# EUROPEAN PATENT SPECIFICATION

(11) **EP 3 048 172 B1**
(45) Date of publication and mention of the grant of the patent: **13.11.2019**
(21) Application number: 14845641.1
(22) Date of filing: 17.09.2014
(51) Int. Cl.: C12P 13/04, C12N 15/09, C12P 13/14, C12N 1/20, C12N 1/21, C12N 9/88, C12P 13/08, C12N 9/12, C07K 14/405, C12R 1/63

(54) **METHOD FOR PRODUCING L-AMINO ACID FROM SEAWEED-DERIVED BIOMASS**
VERFAHREN ZUR HERSTELLUNG VON L-AMINOSÄURE AUS BIOMASSE AUS ALGEN
PROCÉDÉ DE PRODUCTION D'UN ACIDE L-AMINÉ À PARTIR D'UNE BIOMASSE D'ORIGINE ALGALE

(30) Priority: 17.09.2013 JP 2013192420
(43) Date of publication of application: 27.07.2016
(73) Proprietor: Ajinomoto Co., Inc., Tokyo 104-8315 (JP)
(72) Inventor: DOI, Hidetaka, Kawasaki-shi Kanagawa 210-8681 (JP)
(74) Representative: Strehl Schübel-Hopf & Partner
(86) International application number: PCT/JP2014/074604
(87) International publication number: WO 2015/041265

(56) References cited:
- EP-A1- 2 248 906
- EP-A1- 2 460 883
- EP-B1- 1 352 966
- WO-A1-2015/041264
- WO-A2-2009/046375
- WO-A2-2011/044279
- FR-A1- 2 847 265
- US-A1- 2012 220 004
- US-A1- 2012 329 115
- ISHIKAWA K. ET AL: "Improvement of L-Lysine Production by Methylophilus methylotrophus from Methanol via the Entner-Doudoroff Pathway, Originating in Escherichia coli", BIOSCIENCE BIOTECHNOLOGY BIOCHEMISTRY., vol. 72, no. 10, 7 October 2008 (2008-10-07), pages 2535-2542, XP055362851, TOKYO, JAPAN ISSN: 0916-8451, DOI: 10.1271/bbb.80183
- THOMAS F. ET AL: "Characterization of the first alginolytic operons in a marine bacterium: from their emergence in marine Flavobacteriia to their independent transfers to marine Proteobacteria and human gut Bacteroides", ENVIRONMENTAL MICROBIOLOGY, vol. 14, no. 9, 19 April 2012 (2012-04-19) , pages 2379-2394, XP055362849, GB ISSN: 1462-2912, DOI: 10.1111/j.1462-2920.2012.02751.x
- DOI H. ET AL: "Identification of enzymes responsible for extracellular alginate depolymerization and alginate metabolism inVibrio algivorus", APPLIED MICROBIOLOGY AND BIOTECHNOLOGY, vol. 101, no. 4, 3 December 2016 (2016-12-03), pages 1581-1592, XP036137850, ISSN: 0175-7598, DOI: 10.1007/S00253-016-8021-7 [retrieved on 2016-12-03]
- DOI H. ET AL: "Vibrio algivorus sp. nov., an alginate- and agarose-assimilating bacterium isolated from the gut flora of a turban shell marine snail", INTERNATIONAL JOURNAL OF SYSTEMATIC AND EVOLUTIONARY MICROBIOLOGY, vol. 66, no. 8, 19 May 2016 (2016-05-19), pages 3164-3169, XP055362560, GB ISSN: 1466-5026, DOI: 10.1099/ijsem.0.001165
- WARGACKI A.J. ET AL.: 'An engineered microbial platform for direct biofuel production from brown macroalgae' SCIENCE vol. 335, no. 6066, 2012, pages 303 - 308, XP055332297
- YUMOTO I. ET AL.: 'Purification and characterization of a catalase from the facultatively psychrophilic bacterium Vibrio rumoiensis S-1(T) exhibiting high catalase activity' J.BACTERIOL. vol. 182, no. 7, 2000, pages 1903 - 1909, XP055332298
- NAM Y.D. ET AL.: 'Vibrio litoralis sp. nov., isolated from a Yellow Sea tidal flat in Korea' INT.J.SYST.EVOL.MICROBIOL. vol. 57, no. PT3, 2007, pages 562 - 5, XP055332299

## Description

### TECHNICAL FIELD

The present invention relates to a method of producing an L-amino acid by fermentation using bacteria.

### BACKGROUND ART

L-amino acids are industrially useful as animal feed additives, components in health food, or an amino acid infusion or the like.

In the industrial production of L-amino acids by a fermentation method, sugars produced from food crops such as sugar cane and maize, that is, glucose, fructose, sucrose, molasses, starch hydrolysate and the like are used as a carbon source.

Moreover, because of the rise of grain prices due to the increased food demand caused by the recently increased world population, sugars, fats and oils obtainable from non-food terrestrial biomass such as stalks and leaves of sugar cane and maize, and jatropha oil are likewise useful in industrial settings as main sources of raw materials for fermentation using microorganisms.

However, some problems have been pointed out with regard to the production of non-food terrestrial biomass, including not direct but indirect competition with the production of food grains in terms of usage of agricultural land, agricultural water, and fertilizer, and the like; and, additionally, land exploitation all over the world and the like (Non-Patent Document 1).

Thus, a focus has been placed on marine biomass including seaweed as a main raw material for fermentation using microorganisms. Marine biomass can be produced without usage of agricultural land, agricultural water, and fertilizer and, furthermore, it is known that brown algae, among seaweed, have an ability to produce main raw materials for fermentation per unit area twice more and five times more than sugar cane and maize including non-food parts, respectively (Non-Patent Document 2). Brown algae store a significant amount of polysaccharides consisting of D-mannuronic acid and L-glucuronic acid, that is, sugars such as alginic acid, glucose, and mannitol.

However, with regard to the usage of sugars stored in brown algae as main raw materials for fermentation, the difficulty in microorganism-mediated assimilation of alginic acid, which is contained as a primary component among sugars of brawn algae, is pointed out as a problem in the industrial usage of the sugars (Non-Patent Document 3, Non-Patent Document 4, Patent Document 1, Patent Document 2).

To solve such a problem, methods for the production of ethanol from alginic acid by fermentation have been proposed, the method in which heterogeneous genes required for ethanol fermentation are introduced and expressed by recombination in a microorganism having an ability to assimilate alginic acid (Patent Document 2); and the method in which the ability to assimilate alginic acid is imparted to a microorganism by heterogeneous recombination and the resultant microorganism capable of performing ethanol fermentation is used for the ethanol production (Patent Document 2).

However, no report has been provided so far with regard to a method for the production of an L-amino acid from a seaweed-derived carbon source including alginic acid by fermentation using microorganisms.

EP1352966 B1 discloses production of L-glutamic acid by genetically modified *Escherichia coli* overexpressing the endogenous *eda* gene encoding 2-keto-3-deoxy-6-phosphogluconate aldolase.

FR2847265 A1 and Ishikawa, et al. "Improvement of L-Lysine Production by Methylophilus methylotrophus from Methanol via the Entner-Doudoroff Pathway, Originating in Escherichia coli", (2008) Biosci. Biotech Biochem. 72, p2535-2542, disclose production of L-lysine by genetically modified *Methylophilus methylotrophus* expressing the heterologous *E. coli eda* gene encoding 2-keto-3-deoxy-6-phosphogluconate aldolase.

EP2248906 A1 and EP2460883 A1 disclose production of L-lysine and L-glutamic acid resp. of L-lysine by bacteria, e.g. modified bacteria, e.g. *E. coli,* grown in a culture medium containing a processed product of a microalga which promotes production and accumulation of the L-amino acid by the bacterium in the culture medium, the processed product e.g. containing fatty acids as carbon source.

### RELATED ART

### Patent Documents

Patent Document 1: International Patent Application WO2009/046375
Patent Document 2: International Patent Application WO2011/024858

### Non-Patent Documents

Non-Patent Document 1: Bringezu, et al. Towards sustainable production and use of resources; Assesing Biofuels (2009) United Nations Environmental Programme.
Non-Patent Document 2: Somerville, et al. "Feedstocks for Lignocellulosic Biofuels" (2010) Science. 329, p790-792.
Non-Patent Document 3: Takeda, et al. Bioethanolproduction from marine biomass alginic acid by metabolically engineered bacteria (2011) Energy Environ. Sci. 4 p2575-2581.
Non-Patent Document 4: Wargacki, et al. "An Engineered Microbial Platform for Direct Biofuel Production from Brown Macroalgae" (2012) Science. 335. p308-313.

### SUMMARY OF THE INVENTION

### PROBLEMS TO BE SOLVED BY THE INVENTION

An object of the present invention is to provide a method of producing an L-amino acid from a seaweed-derived carbon source including alginic acid by fermentation using microorganisms.

### MEANS FOR SOLVING THE PROBLEM

The inventors of the present invention isolated a *Vibrio* bacterium capable of assimilating alginic acid from the digestive tract of turban shell (scientific name: *Turbo cornutus*), which is believed to ingest brown algae on daily basis. Furthermore, the inventors succeeded in direct fermentation of an L-amino acid from alginic acid through the introduction of a set of genes involved in alginate assimilation into an L-amino acid-producing bacterium.
[1] A method of producing an L-amino acid by a fermentation method, the method comprising culturing a bacterium modified to have an ability to produce the L-amino acid and an ability to assimilate alginic acid in a culture medium containing alginic acid as a carbon source obtained from seaweed-derived biomass, or containing alginic acid as a carbon source obtained from seaweed-derived biomass which alginic acid is subjected to an enzymatic degradation reaction by an alginate lyase or an acid hydrolysis reaction, to produce and accumulate the L-amino acid in the culture medium; and collecting the L-amino acid from the culture medium.
[2] The method according to the method described above, wherein the seaweed is a large type of seaweed included in brown algae, red algae, and green algae.
[3] The method according to the method described above, wherein the carbon source obtained from seaweed-derived biomass additionally contains one or more selected from cellulose, mannitol, pectin, galacturonic acid, carrageenan, agar, fucoidan, laminarin, and xylan.
[5] The method according to the method described above, wherein the bacterium is a bacterium having an enhanced alginate lyase.
[6] The method according to the method described above, wherein the alginate lyase is AlyB protein or AlyD protein.
[7] The method according to the method described above, wherein the bacterium is a bacterium having an increased alginate importer activity.
[8] The method according to the method described above, wherein the alginate importer is Kdg protein or Toa protein.
[9] The method according to the method described above, wherein the bacterium is a bacterium having an increased keto acid reductase activity.
[10] The method according to the method described above, wherein the keto acid reductase is an enzyme selected from NADPH/NADH dehydrogenase, D-mannonate dehydratase, 2-keto-3-deoxygluconokinase, and 2-keto-3-deoxy-6-phosphogluconate aldolase.
[11] The method according to the method described above, wherein the alginate lyase, alginate importer and keto acid reductase are enzymes derived from a *Vibrio* bacterium having an ability to assimilate alginic acid.
[12] The method according to the method described above, wherein the *Vibrio* bacterium having an ability to assimilate alginic acid belongs to *Vibrio alginovora*.
[13] The method according to the method described above, wherein the *Vibrio* bacterium having an ability to assimilate alginic acid is derived from the *Vibrio* sp. strain NITE BP-01635.
[14] The method according to the method described above, wherein the bacterium is *Pantoea ananatis* or *Escherichia coli*.
[17] An isolated DNA selected from (a) and (b) below:
   (a) a DNA encoding the amino acid sequence of SEQ ID NO: 3,
   (b) a DNA encoding a protein having the amino acid sequence of SEQ ID NO: 3 in which 1 to 10 amino acids are substituted, deleted, inserted or added, the DNA encoding an alginate importer.
[18] An isolated DNA selected from (c) and (d) below:
   (c) a DNA encoding the amino acid sequence of SEQ ID NO: 5,
   (d) a DNA encoding a protein having the amino acid sequence of SEQ ID NO: 5 in which 1 to 10 amino acids are substituted, deleted, inserted or added, the DNA encoding a protein having the alginate lyase activity.
[19] An isolated DNA selected from (e) and (f) below:
   (e) a DNA encoding the amino acid sequence of SEQ ID NO: 7,
   (f) a DNA encoding a protein having the amino acid sequence of SEQ ID NO: 7 in which 1 to 10 amino acids are substituted, deleted, inserted or added, the DNA encoding a protein having the 2-keto-3-deoxy-6-phosphogluconate aldolase activity.
[20] An isolated DNA selected from (g) and (h) below:
   (g) a DNA encoding the amino acid sequence of SEQ ID NO: 9,
   (h) a DNA encoding a protein having the amino acid sequence of SEQ ID NO: 9 in which 1 to 10 amino acids are substituted, deleted, inserted or added, the DNA encoding a protein having the 2-keto-3-deoxygluconokinase activity.
[21] An isolated DNA selected from (i) and (j) below:
   (i) a DNA encoding the amino acid sequence of SEQ ID NO: 11,
   (j) a DNA encoding a protein having the amino acid sequence of SEQ ID NO: 11 in which 1 to 10 amino acids are substituted, deleted, inserted or added, the DNA encoding a protein having the alginate lyase activity.
[22] An isolated DNA selected from (k) and (1) below:
   (k) a DNA encoding the amino acid sequence of SEQ ID NO: 13,
   (1) a DNA encoding a protein having the amino acid sequence of SEQ ID NO: 13 in which 1 to 10 amino acids are substituted, deleted, inserted or added, the DNA encoding a protein having the alginate lyase activity.
[23] An isolated DNA selected from (m) and (n) below:
   (m) a DNA encoding the amino acid sequence of SEQ ID NO: 15,
   (n) a DNA encoding a protein having the amino acid sequence of SEQ ID NO: 15 in which 1 to 10 amino acids are substituted, deleted, inserted or added, the DNA encoding an alginate importer protein.
[24] An isolated DNA selected from (o) and (p) below:
   (o) a DNA encoding the amino acid sequence of SEQ ID NO: 17,
   (p) a DNA encoding a protein having the amino acid sequence of SEQ ID NO: 17 in which 1 to 10 amino acids are substituted, deleted, inserted or added, the DNA encoding an alginate importer protein.
[25] An isolated DNA selected from (q) and (r) below:
   (q) a DNA encoding the amino acid sequence of SEQ ID NO: 19,
   (r) a DNA encoding a protein having the amino acid sequence of SEQ ID NO: 19 in which 1 to 10 amino acids are substituted, deleted, inserted or added, the DNA encoding an alginate importer protein.
[26] An isolated DNA selected from (s) and (t) below:
   (s) a DNA encoding the amino acid sequence of SEQ ID NO: 21,
   (t) a DNA encoding a protein having the amino acid sequence of SEQ ID NO: 21 in which 1 to 10 amino acids are substituted, deleted, inserted or added, the DNA encoding a protein having the alginate lyase activity.
[27] An isolated DNA selected from (u) and (w) below:
   (u) a DNA encoding the amino acid sequence of SEQ ID NO: 23,
   (w) a DNA encoding a protein having the amino acid sequence of SEQ ID NO: 23 in which 1 to 10 amino acids are substituted, deleted, inserted or added, the DNA encoding a protein having the NADP/NADPH dehydrogenase activity.

### BRIEF DESCRIPTION OF THE DRAWINGS

Figure 1 shows the comparison between the sequences of the 16S rRNA genes of the *Vibrio* sp. strain SA2 and the type strain of *Vibrio rumoiensis* (upper sequence, the *Vibrio* sp. strain SA2 (SEQ ID NO: 1); lower sequence, *Vibrio rumoiensis* type strain S-1 (SEQ ID NO: 98); each box frame with dotted line represents a difference due to a gap, while each box frame with solid line represents a difference due to a substitution).
Figure 2 shows a molecular phylogenetic tree based on the full-length sequence of the 16S rRNA gene in the *Vibrio* sp. strain SA2 (a number beside each branch represents a bootstrap value).
Figure 3 shows the result of the test-tube culture of the *Vibrio* sp. strain SA2 in a minimal medium containing sodium alginate (1: the turbidity at a wavelength of 600 nm in the *Vibrio* sp. strain SA2, 2: the turbidity at a wavelength of 600 nm in the strain MG1655).
Figure 4 shows the maximum specific growth rate in a liquid minimal medium containing only glucose or sodium alginate as a carbon source.
Figure 5 shows a model diagram of the assimilatory metabolic pathway for alginic acid in *Escherichia coli.*
Figure 6 shows a model diagram of the alginate lyase reaction.
Figure 7 shows a model diagram of the scheme to obtain fractionated samples (bacterial cell lysate sample, supernatant sample, and pellet sample) of the *Vibrio* sp. strain SA2.
Figure 8 shows a model diagram representing the expression of a long-chain alginic acid-degrading enzyme. A: excretion model for a long-chain alginic acid-degrading enzyme, B: cell surface functional expression model for a long-chain alginic acid-degrading enzyme.
Figure 9 shows an explanatory drawing that illustrates the screening of a clone expressing the function of an alginate lyase on the cell surface. A: a model diagram of a system to screen a clone expressing the function of an alginate lyase on the cell surface, B: a depression on the alginic acid-containing top agar medium caused by a colony of the *Vibrio* sp. strain SA2 and a model diagram (photograph), C: a result of the screening of a clone expressing the function of an alginate lyase on the cell surface by using the *E*. *coli* strain EPI300 (photograph).
Figure 10 shows an explanatory drawing that illustrates a detection method for the alginate lyase activity. A: a model diagram of a simple detection method for the alginate lyase activity, the *in vitro* viscosity test system, B: the *in vitro* viscosity test; a solidified liquid culture medium with an inclined plane due to a decreased viscosity caused by the addition of sodium alginate (photograph), C: the *in vitro* viscosity test; a reduction of viscosity in liquid culture medium using a fully grown cells of the *Vibrio* sp. strain SA2 (photograph).
Figure 11 shows the results of performed *in vitro* viscosity tests (photographs). A-1: the EPI300/pCCFOS1 control vector strain, 2: the prospective EPI300-based alginate lyase-expressing strain, 3: a comparison control consisting of liquid culture medium alone and no added bacterial strain; B-1: the JM109/pMW119-attR-cat-attL-P₁₄ strain, 2: the JM109/pMW119-attR-cat-attL-P₁₄-alyB strain, 3: the JM109/pMW119-attR-cat-attL-P₁₄-alyD strain, 4: a comparison control consisting of liquid culture medium alone and no added bacterial strain.
Figure 12 shows electron micrographs of the *Vibrio* sp. strain SA2.
Figure 13 shows a Gram stain of the *Vibrio* sp. strain SA2 (photograph).
Figure 14 shows a molecular phylogenetic tree based on a nucleotide sequence of linked housekeeping genes of the *Vibrio* sp. strain SA2 (a line on the lower left corner represents a scale bar and a number beside each branch represents a bootstrap value).

### DETAILED DESCRIPTION

### <1> Bacteria used in the present invention

The bacterium used in the present invention refers to a bacterium used in the manufacturing method of the present invention, the bacterium modified to have an ability to produce an L-amino acid and the ability to assimilate alginic acid.

### <1-1> Bacteria having an ability to produce an L-amino acid

In the present invention, L-amino acid refers to one or more amino acids selected from L-lysine, L-ornithine, L-arginine, L-histidine, L-citrulline, L-isoleucine, L-alanine, L-valine, L-leucine, glycine, L-threonine, L-serine, L-proline, L-phenylalanine, L-tyrosine, L-tryptophan, L-cysteine, L-cystine, L-methionine, L-glutamic acid, L-aspartic acid, L-glutamine, L-citrulline, L-ornithine and L-asparagine.

"The ability to produce an L-amino acid" refers to the ability to produce and accumulate the substance of interest up to an amount in a cell or in a culture medium when a bacterium used in the present invention is cultured in the culture medium, which amount allows the substance of interest to be collected from the cell or the culture medium. The bacterium having an ability to produce an L-amino acid may be a bacterium which can accumulate the substance of interest more abundantly in a culture medium than unmodified strains. Examples of the unmodified strains include a wild-type strain and a parent strain. Moreover, the bacterium having an ability to produce a substance of interest may also be a bacterium which can accumulate the substance of interest preferably in an amount of not less than 0.5 g/L, more preferably in an amount of not less than 1.0 g/L, in a culture medium. The L-amino acid produced by the bacterium used in the present invention may be one kind of substance or not less than two kinds of substances.

Examples of the bacterium include, for example, bacteria belonging to the family Enterobacteriaceae and coryneform bacteria.

Examples of a bacterium belonging to the family Enterobacteriaceae include bacteria belonging to the genera *Escherichia*, *Enterobacter*, *Pantoea*, *Klebsiella*, *Serratia*, *Erwinia*, *Photorhabdus*, *Providencia*, *Salmonella*, *Morganella* and the like. Specifically, bacteria classified into Enterobacteriaceae by a classification method employed for the database (http://www.ncbi.nlm.nih.gov/Taxonomy/Browser/wwwtax.cgi?id=91347) in NCBI (National Center for Biotechnology Information) can be used.

Examples of a bacterium in the genus *Escherichia* include, but not particularly limited to, bacteria classified into the genus *Escherichia* by the classification known to microbiology experts. Examples of a bacterium in the genus *Escherichia* include, for example, bacteria described in the literature by Neidhardt *et al.*, (Backmann, B. J. 1996. Derivations and Genotypes of some mutant derivatives of Escherichia coli K-12, p. 2460-2488. Table 1. In F. D. Neidhardt (ed.), Escherichia coli and Salmonella Cellular and Molecular Biology/Second Edition, American Society for Microbiology Press, Washington, D.C.). Examples of a bacterium in the genus *Escherichia* include, for example, *Escherichia coli.* Specifically, examples of *Escherichia coli* include, for example, *Escherichia coli* W3110 (ATCC 27325) and *Escherichia coli* MG1655 (ATCC 47076) derived from the prototype wild-type strain K12.

Examples of a bacterium in the genus *Enterobacter* include, but not particularly limited to, bacteria classified into the genus *Enterobacter* by the classification known to microbiology experts. Examples of a bacterium in the genus *Enterobacter* include, for example, *Enterobacter agglomerans* and *Enterobacter aerogenes.* Specifically, examples of *Enterobacter agglomerans* include, for example, the *Enterobacter agglomerans* strain ATCC12287. Specifically, examples of *Enterobacter aerogenes* include, for example, the *Enterobacter aerogenes* strains ATCC13048, NBRC12010 (Biotechonol Bioeng. 2007 Mar 27; 98(2) 340-348), and AJ110637 (FERM BP-10955). Moreover, examples of a bacterium in the genus *Enterobacter* include, for example, bacteria described in EP0952221. Incidentally, some breeds of *Enterobacter agglomerans* have been classified into *Pantoea agglomerans*.

Examples of a bacterium in the genus *Pantoea* include, but not particularly limited to, bacteria classified into the genus *Pantoea* by the classification known to microbiology experts. Examples of a bacterium in the genus *Pantoea* include, for example, *Pantoea ananatis*, *Pantoea stewartii*, *Pantoea agglomerans*, and *Pantoea citrea*. Specifically, examples of *Pantoea ananatis* include, for example, the *Pantoea ananatis* strain LMG20103, the *Pantoea ananatis* strains AJ13355 (FERM BP-6614), AJ13356 (FERM BP-6615), AJ13601 (FERM BP-7207), SC17 (FERM BP-11091), and SC17(0) (VKPM B-9246). Incidentally, some breeds of *Enterobacter agglomerans* have recently been re-classified into *Pantoea agglomerans*, *Pantoea ananatis*, *Pantoea stewartii* and the like based on the nucleotide sequence analysis of the 16S rRNA and the like (Int. J. Syst. Bacteriol., 43, 162-173 (1993)). The bacteria in the genus *Pantoea* also include bacteria re-classified into the genus *Pantoea* as described above.

Examples of a bacterium in the genus *Erwinia* include *Erwinia amylovora* and *Erwinia carotovora*. Examples of a bacterium in the genus *Klebsiella* include *Klebsiella planticola.*

Examples of a coryneform bacterium include bacteria belonging to the genera *Corynebacterium*, *Brevibacterium*, and *Microbacterium* and the like.

Specifically, examples of a coryneform include species as indicated below:
*Corynebacterium acetoacidophilum*,
*Corynebacterium acetoglutamicum*,
*Corynebacterium alkanolyticum*,
*Corynebacterium callunae*,
*Corynebacterium glutamicum*,
*Corynebacterium lilium*,
*Corynebacterium melassecola*,
*Corynebacterium thermoaminogenes* (*Corynebacterium efficiens*),
*Corynebacterium herculis*,
*Brevibacterium divaricatum*,
*Brevibacterium flavum*,
*Brevibacterium immariophilum*,
*Brevibacterium lactofermentum* (*Corynebacterium glutamicum*),
*Brevibacterium roseum*,
*Brevibacterium saccharolyticum*,
*Brevibacterium thiogenitalis*,
*Corynebacterium ammoniagenes* (*Corynebacterium stationis*),
*Brevibacterium album*,
*Brevibacterium cerinum*, and
*Microbacterium ammoniaphilum*.

Specifically, examples of a coryneform bacterium include the bacterial strains as indicated below:
*Corynebacterium acetoacidophilum* ATCC 13870,
*Corynebacterium acetoglutamicum* ATCC 15806,
*Corynebacterium alkanolyticum* ATCC 21511,
*Corynebacterium callunae* ATCC 15991,
*Corynebacterium glutamicum* ATCC 13020, ATCC 13032, ATCC 13060, ATCC 13869, and FERM BP-734,
*Corynebacterium lilium* ATCC 15990,
*Corynebacterium melassecola* ATCC 17965,
*Corynebacterium thermoaminogenes* AJ12340 (FERM BP-1539),
*Corynebacterium herculis* ATCC 13868,
*Brevibacterium divaricatum* ATCC 14020,
*Brevibacterium flavum* ATCC 13826, ATCC 14067, and AJ12418 (FERM BP-2205),
*Brevibacterium immariophilum* ATCC 14068,
*Brevibacterium lactofermentum* ATCC 13869,
*Brevibacterium roseum* ATCC 13825,
*Brevibacterium saccharolyticum* ATCC 14066,
*Brevibacterium thiogenitalis* ATCC 19240,
*Corynebacterium ammoniagenes* (*Corynebacterium stationis*) ATCC 6871, and ATCC 6872,
*Brevibacterium album* ATCC 15111,
*Brevibacterium cerinum* ATCC 15112, and
*Microbacterium ammoniaphilum* ATCC 15354.

Additionally, the bacteria in the genus *Corynebacterium* also include bacteria that had been conventionally classified into the genus *Brevibacterium* but have currently been integrated into the genus *Corynebacterium* (Int. J. Syst. Bacteriol., 41, 255 (1991)). Moreover, *Corynebacterium stationis* also include a bacterium that had been conventionally classified into *Corynebacterium ammoniagenes* but have been re-classified into *Corynebacterium stationis* based on the nucleotide sequence analysis of the 16S rRNA and the like (Int. J. Syst. Evol. Microbiol., 60, 874-879 (2010)).

These bacterial strains can be provided from, for example, the American Type Culture Collection (address: 12301 Parklawn Drive, Rockville, Maryland 20852 P.O. Box 1549, Manassas, VA 20108, United States of America). That is, each bacterial strain is given a registration number and can be provided by using this registration number (see http://www.atcc.org/). The registration number corresponding to each bacterial strain is listed in the catalog of the American Type Culture Collection.

The bacterium used in the present invention may be a bacterium intrinsically having an ability to produce a substance of interest or a bacterium modified to have an ability to produce a substance of interest. A bacterium having an ability to produce a substance of interest can be obtained, for example, by imparting the ability to produce the substance of interest to a bacterium as described above or by increasing the ability to produce the substance of interest in a bacterium as described above.

The ability to produce a substance of interest can be imparted or increased by a method conventionally employed in the breeding of amino acid-producing bacteria in a coryneform bacterium or a bacterium in the genus *Escherichia* and the like (see Amino Acid Fermentation, the first edition, Gakkai Shuppan Center, Ltd., May 30, 1986, pp77-100). Such a method includes, for example, acquisition of an auxotrophic mutant strain, acquisition of a strain resistant to analogs of a substance of interest, acquisition of a metabolic control mutant strain, and production of a recombinant strain having an increased activity of a biosynthesis enzyme for a substance of interest. In the breeding of bacteria that produce a substance of interest, only one of, or two, three, or more of such characteristics as auxotrophy, analog resistance and altered metabolic control may be imparted. Moreover, in the breeding of bacteria that produce a substance of interest, the activity may be increased in only one of, or two, three, or more of biosynthesis enzymes for the substance of interest. Furthermore, the increase of the activity of a biosynthesis enzyme may be combined with the impartment of such characteristics as auxotrophy, analog resistance and altered metabolic control.

An auxotrophic mutant strain, an analog resistant strain or a metabolic control mutant strain having an ability to produce an L-amino acid can be obtained by subjecting a parent strain or a wild-type strain to a conventional mutation treatment and selecting from the obtained mutant strains a strain exhibiting auxotrophy, analog resistance, or altered metabolic control and having an ability to produce a substance of interest. Examples of the conventional mutation treatment include irradiation of X-rays or ultraviolet rays, as well as a treatment with a mutation agent such as N-methyl-N'-nitro-N-nitrosoguanidine (MNNG), ethyl methanesulfonate (EMS), methyl methanesulfonate (MMS) and the like.

Moreover, the ability to produce an L-amino acid can also be imparted or increased by increasing the activity of an enzyme involved in the biosynthesis of a substance of interest. The activity of the enzyme can be increased, for example, by modifying a microorganism to have an increased expression of a gene encoding the same enzyme. Methods to increase the expression of a gene are described in WO00/18935, European Patent Application Publication No. 1010755, and the like. The procedures to increase the enzymatic activity will be described in detail below.

Moreover, the ability to produce an L-amino acid can also be imparted or increased by reducing the activity of an enzyme that catalyzes a reaction to produce a compound other than a substance of interest in a pathway branching from the biosynthesis pathway of the substance of interest. Incidentally, examples of "an enzyme that catalyzes a reaction to produce a compound other than a substance of interest in a pathway branching from the biosynthesis pathway of the substance of interest" as used herein also include an enzyme involved in the degradation of the substance of interest. The procedures to reduce the enzymatic activity will be described below.

Now, L-amino acid-producing bacteria and the method to impart or increase the ability to produce an L-amino acid will be specifically illustrated below.

### <L-glutamic acid-producing bacteria>

Examples of a method to impart or increase the ability to produce L-glutamic acid include, for example, a method in which a microorganism is modified to have increased activities of one or more enzymes selected from the L-glutamic acid biosynthesis enzymes. Such enzymes include, but not particularly limited to, glutamate dehydrogenase (*gdhA*), glutamine synthetase (*glnA*), glutamate synthase (*gltAB*), isocitrate dehydrogenase (*icdA*), aconitate hydratase (*acnA*, *acnB*), citrate synthase (*gltA*), methylcitrate synthase (*prpC*), phosphoenolpyruvate carboxylase (*ppc*), pyruvate carboxylase (*pyc*), pyruvate kinase (*pykA*, *pykF*), pyruvate dehydrogenase (*aceEF*, *lpdA*), phosphoenolpyruvate synthase (*ppsA*), enolase (*eno*), phosphoglyceromutase (*pgmA*, *pgmI*), phosphoglycerate kinase (*pgk*), glyceraldehyde-3-phosphate dehydrogenase (*gapA*), triosephosphate isomerase (*tpiA*), fructose-bisphosphate aldolase (*fbp*), phosphofructokinase (*pfkA*, *pfkB*), glucose phosphate isomerase (*pgi*), 6-phosphogluconate dehydratase (*edd*), 2-keto-3-deoxy-6-phosphogluconate aldolase (*eda*), transhydrogenase and the like. Incidentally, the name of a gene is indicated within the parentheses following each enzyme name (the same is true in the following description). The activity is preferably increased in one or more enzymes selected from, for example, among these enzymes, glutamate dehydrogenase, citrate synthase, phosphoenolpyruvate carboxylase, and methylcitrate synthase. Examples of strains modified to have an increased expression of the citrate synthase gene, the phosphoenolpyruvate carboxylase gene, and/or the glutamate dehydrogenase gene include strains disclosed in EP1078989A, EP955368A and EP952221A. Moreover, examples of a microorganism modified to have an increased expression of an L-glutamic acid biosynthesis enzyme gene also include microorganisms disclosed in WO99/07853 and EP1352966B.

Examples of a method to impart or increase the ability to produce L-glutamic acid also include, for example, a method in which a microorganism is modified to have reduced activities of one or more enzymes selected from enzymes that catalyze reactions to produce compounds other than L-glutamic acid in pathways branching from the biosynthesis pathway of L-glutamic acid. Examples of such enzymes include, but not particularly limited to, α-ketoglutarate dehydrogenase (*sucA*), succinate dehydrogenase (*sdhABCD*), acetate kinase (*ack*), acetohydroxy acid synthase (*ilvG*), acetolactate synthase (*ilvI* and the like), formate acetyltransferase (*pfl*), lactate dehydrogenase (*ldh*), alcohol dehydrogenase (*adh*), glutamate decarboxylase (*gadAB*), 1-pyrroline-5-carboxylate dehydrogenase (*putA*) and the like. The activity is preferably reduced or compromised especially in α-ketoglutarate dehydrogenase among these enzymes.

Methods to compromise or reduce the activity of α-ketoglutarate dehydrogenase in a bacterium in the genus *Escherichia* is described in Japanese Unexamined Patent Application Publication Nos. Hei 5-244970 and Hei 7-203980, and the like. Moreover, a method to compromise or reduce the activity of α-ketoglutarate dehydrogenase in a coryneform bacterium is described in WO95/34672. Furthermore, a method to compromise or reduce the activity of α-ketoglutarate dehydrogenase in a bacterium in the genus *Enterobacter* is disclosed in Japanese Unexamined Patent Application Publication No. 2001-333769. Furthermore, methods to compromise or reduce the activity of α-ketoglutarate dehydrogenase in enteric bacteria such as a bacterium in the genus *Pantoea,* a bacterium in the genus *Enterobacter*, a bacterium in the genus *Klebsiella,* a bacterium in the genus *Erwinia,* and the like are disclosed in U.S. Patent Nos. 6197559, 6682912, 6331419, and 8129151. Furthermore, a method to reduce the activity of α-ketoglutarate dehydrogenase and succinate dehydrogenase in a coryneform bacterium and a bacterium in the genus *Pantoea* is disclosed in WO2008/075483.

For example, to reduce the activity of α-ketoglutarate dehydrogenase, modification of the *sucA* (*odhA*) gene that encodes E1o subunit in the enzyme is sufficient. Examples of a strain having a reduced α-ketoglutarate dehydrogenase activity include, for example, the strains below:
*Brevibacterium lactofermentum* ΔS strain (WO95/34672),
*Brevibacterium lactofermentum* AJ12821 (FERM BP-4172, see French Patent No. 9401748),
*Brevibacterium flavum* AJ12822 (FERM BP-4173; see French Patent No. 9401748),
*Corynebacterium glutamicum* AJ12823 (FERM BP-4174; see French Patent No. 9401748),
*Corynebacterium glutamicum* ATCC13869 OAGN, OA2-2, OAGN2-2 (see WO2006/028298),
*Escherichia coli* W3110 *sucA*:: Kmr,
*Escherichia coli* AJ12624 (FERM BP-3853),
*Escherichia coli* AJ12628 (FERM BP-3854),
*Escherichia coli* AJ12949 (FERM BP-4881),
*Brevibacterium lactofermentum* ΔS strain (see WO95/34672),
*Pantoea ananatis* AJ13601 (FERM BP-7207, European Patent Application Publication No. 1078989),
*Pantoea ananatis* AJ13356 (FERM BP-6615, U.S. Patent No. 6,331,419),
*Pantoea ananatis* SC17sucA (FERM BP-8646, WO2005/085419), and
*Klebsiella planticola* AJ13410 (FERM BP-6617, U.S. Patent No. 6,197,559).

Moreover, examples of an L-glutamic acid-producing bacterium or a parent strain to derive that bacterium include the *Pantoea ananatis* strains AJ13355 (FERM BP-6614) and SC17 (FERM BP-11091). The AJ13355 strain was isolated from the soil in Iwata city, Shizuoka prefecture, Japan as a strain which can grow on a culture medium containing L-glutamic acid and a carbon source at a low pH. The SC17 strain was selected as a low mucus-producing mutant strain from the AJ13355 strain (U.S. Patent No. 6,596,517). The SC17 strain has been deposited in the International Patent Organism Depositary, National Institute of Advanced Industrial Science and Technology (International Patent Organism Depositary, current National Institute of Technology and Evaluation; address: #120, 2-5-8, Kazusakamatari, Kisarazu-shi, Chiba 292-0818 Japan) on February 4, 2009 and given the accession number FERM BP-11091. *Pantoea ananatis* AJ13355 had been deposited in the International Patent Organism Depositary, National Institute of Advanced Industrial Science and Technology (International Patent Organism Depositary, current National Institute of Technology and Evaluation; address: #120, 2-5-8, Kazusakamatari, Kisarazu-shi, Chiba 292-0818 Japan) on February 19, 1998 under the accession number FERM P-16644 and has been put into the state of international deposit under the provision of the Budapest Treaty on January 11, 1999 and given the accession number FERM BP-6614.

Moreover, examples of an L-glutamic acid-producing bacterium or a parent strain to derive that bacterium include bacteria belonging to the genus *Pantoea* and having a compromised or reduced activity of α-ketoglutarate dehydrogenase (αKGDH). Examples of such a strain include AJ13356 (U.S. Patent No. 6,331,419), which is a strain with the deletion of the αKGDH-E1 subunit gene (*sucA*) in the background of the AJ13355 strain, and SC17sucA (U.S. Patent No. 6,596,517), which is a strain with the deletion of the *sucA* gene in the background of the SC17 strain. AJ13356 had been deposited in the National Institute of Bioscience and Human-Technology, Agency of Industrial Science and Technology (International Patent Organism Depositary, current National Institute of Technology and Evaluation; address: #120, 2-5-8, Kazusakamatari, Kisarazu-shi, Chiba 292-0818 Japan) on February 19, 1998 under the accession number FERM P-16645 and has been put into the state of international deposit under the provision of the Budapest Treaty on January 11, 1999 and given the accession number FERM BP-6616. Moreover, the SC17sucA strain has given the private strain number AJ417 and deposited in the International Patent Organism Depositary, National Institute of Advanced Industrial Science and Technology (International Patent Organism Depositary, current National Institute of Technology and Evaluation; address: #120, 2-5-8, Kazusakamatari, Kisarazu-shi, Chiba 292-0818 Japan) on February 26, 2004 under the accession number FERM BP-08646.

Moreover, examples of an L-glutamic acid-producing bacterium or a parent strain to derive that bacterium include the SC17sucA/RSFCPG+pSTVCB strain, the AJ13601 strain, the NP106 strain, and the NA1 strain. The SC17sucA/RSFCPG+pSTVCB strain is a strain obtained by introducing into the SC17sucA strain the plasmid RSFCPG containing the citrate synthase gene (*gltA*), the phosphoenolpyruvate carboxylase gene (*ppsA*), and the glutamate dehydrogenase gene (*gdhA*), all of which genes are derived from *Escherichia coli,* and the plasmid pSTVCB containing the citrate synthase gene (*gltA*) derived from *Brevibacterium lactofermentum.* The AJ13601 strain is a strain selected from this SC17sucA/RSFCPG+pSTVCB strain as exhibiting the resistance to a high concentration of L-glutamic acid at a low pH. Moreover, the NP106 strain is a strain obtained by removing the plasmids RSFCPG and pSTVCB from the AJ13601 strain. The AJ13601 strain had been deposited in the International Patent Organism Depositary, National Institute of Advanced Industrial Science and Technology (International Patent Organism Depositary, current National Institute of Technology and Evaluation; address: #120, 2-5-8, Kazusakamatari, Kisarazu-shi, Chiba 292-0818 Japan) on August 18, 1999 under the accession number FERM P-17516 and has been put into the state of international deposit under the provision of the Budapest Treaty on July 6, 2000 and given the accession number FERM BP-7207.

Incidentally, AJ13355 had been identified as *Enterobacter agglomerans* at the time of isolation but has recently been re-classified into *Pantoea ananatis* based on the nucleotide sequence analysis of the 16S rRNA and the like. Accordingly, AJ13355, AJ13356 and AJ13601 are described herein as *Pantoea ananatis* although they have been deposited as *Enterobacter agglomerans* in the above-described depositary.

Furthermore, as a method to impart the ability to produce L-glutamic acid to a coryneform bacterium, a method to amplify the *yggB* gene (NCgl 1221; NP_600492 [gi:19552490]) and a method to introduce a mutant *yggB* gene having a mutation in the coding region may also be employed (WO2006/070944).

The ability to produce L-glutamic acid may also be imparted by amplifying L-glutamic acid exporter genes, the y*hfK* gene (WO2005/085419) and/or the *ybjL* gene (WO2008/133161).

Examples of another method to impart or increase the ability to produce L-glutamic acid also include a method to confer the resistance to an organic acid analog or a respiratory inhibitor and a method to confer the sensitivity to a cell wall synthesis inhibitor. For example, methods to confer the resistance to monofluoroacetic acid (Japanese Unexamined Patent Application Publication No. Sho 50-113209), to confer the resistance to adenine or thymine (Japanese Unexamined Patent Application Publication No. Sho 57-065198), to weaken a urease (Japanese Unexamined Patent Application Publication No. Sho 52-038088), to confer the resistance to malonic acid (Japanese Unexamined Patent Application Publication No. Sho 52-038088), to confer the resistance to benzopyrones or naphthoquinones (Japanese Unexamined Patent Application Publication No. Sho 56-1889), to confer the resistance to HOQNO (Japanese Unexamined Patent Application Publication No. Sho 56-140895), to confer the resistance to α-ketomalonic acid (Japanese Unexamined Patent Application Publication No. Sho 57-2689), to confer the resistance to guanidine (Japanese Unexamined Patent Application Publication No. Sho 56-35981), to confer the sensitivity to penicillin (Japanese Unexamined Patent Application Publication No. Hei 4-88994), and the like are included as examples.

Specific examples of such resistant bacteria include bacterial strains as indicated below:
*Brevibacterium flavum* AJ3949 (FERM BP-2632: see Japanese Unexamined Patent Application Publication No. Sho 50-113209),
*Corynebacterium glutamicum* AJ11628 (FERM P-5736; see Japanese Unexamined Patent Application Publication No. Sho 57-065198),
*Brevibacterium flavum* AJ11355 (FERM P-5007; see Japanese Unexamined Patent Application Publication No. Sho 56-1889),
*Corynebacterium glutamicum* AJ11368 (FERM P-5020; see Japanese Unexamined Patent Application Publication No. Sho 56-1889),
*Brevibacterium flavum* AJ11217 (FERM P-4318; see Japanese Unexamined Patent Application Publication No. Sho 57-2689),
*Corynebacterium glutamicum* AJ11218 (FERM P-4319; see Japanese Unexamined Patent Application Publication No. Sho 57-2689),
*Brevibacterium flavum* AJ11564 (FERM P-5472; see Japanese Unexamined Patent Application Publication No. Sho 56-140895),
*Brevibacterium flavum* AJ11439 (FERM P-5136; see Japanese Unexamined Patent Application Publication No. Sho 56-35981),
*Corynebacterium glutamicum* H7684 (FERM BP-3004; see Japanese Unexamined Patent Application Publication No. Hei 04-88994),
*Brevibacterium lactofermentum* AJ11426 (FERM P-5123; see Japanese Unexamined Patent Application Publication No. Hei 56-048890),
*Corynebacterium glutamicum* AJ11440 (FERM P-5137; see Japanese Unexamined Patent Application Publication No. Hei 56-048890), and
*Brevibacterium lactofermentum* AJ11796 (FERM P-6402; see Japanese Unexamined Patent Application Publication No. Hei 58-158192).

Moreover, for coryneform bacteria, examples of a method to impart or increase the ability to produce L-glutamic acid also include a method to increase the expression of the *yggB* gene and a method to introduce a mutant *yggB* gene having a mutation in the coding region (WO2006/070944). The *yggB* gene is a gene encoding a mechanosensitive channel. The *yggB* gene in *Corynebacterium glutamicum* ATCC13032 corresponds to a sequence complementary to a sequence from position 1,336,091 to 1,337,692 in the genomic sequence registered as GenBank Accession No. NC_003450 in the NCBI database and is also referred to as NCgl1221. The YggB protein encoded by the *yggB* gene in *Corynebacterium glutamicum* ATCC13032 has been registered as GenBank accession No. NP_600492.

### <L-glutamine-producing bacteria>

Examples of an L-glutamine-producing bacterium or a parent strain to derive that bacterium include a bacterium having an increased glutamate dehydrogenase activity, a bacterium having an increased glutamine synthase (*glnA*) activity, and a bacterium with a disrupted glutaminase gene (European Patent Application Publication Nos. 1229121 and 1424398). An increased glutamine synthase activity can also be achieved by disrupting the glutamine adenylyltransferase gene (*glnE*) or the regulatory PII protein gene (*glnB*) (EP1229121). Moreover, a bacterial strain belonging to the genus *Escherichia* and having a mutant glutamine synthase, in which the tyrosine residue at position 397 of the glutamine synthase has been substituted with another amino acid residue, can be illustrated as a preferable L-glutamine-producing bacterium (U.S. Patent Application Publication No. 2003-0148474).

Moreover, examples of a method to impart or increase the ability to produce L-glutamine include methods to confer the resistance to 6-diazo-5-oxo-norleucine (Japanese Unexamined Patent Application Publication No. Hei 3-232497), to confer the resistance to purine analogs and the resistance to methionine sulfoxide (Japanese Unexamined Patent Application Publication No. Sho 61-202694), to confer the resistance to α-ketomaleic acid (Japanese Unexamined Patent Application Publication No. Sho 56-151495), and the like.

Specific examples of a coryneform bacterium having an ability to produce L-glutamine include bacterial strains as indicated below:
*Brevibacterium flavum* AJ11573 (FERM P-5492, Japanese Unexamined Patent Application Publication No. Sho 56-161495),
*Brevibacterium flavum* AJ11576 (FERM BP-10381, Japanese Unexamined Patent Application Publication No. Sho 56-161495), and
*Brevibacterium flavum* AJ12212 (FERM P-8123, Japanese Unexamined Patent Application Publication No. Sho 61-202694).

### <L-proline-producing bacteria>

Examples of an L-proline-producing bacterium or a parent strain to derive that bacterium include a bacterium having a γ-glutamyl kinase desensitized to the feedback inhibition by L-proline and a bacterium having a weakened L-proline degradation system. A method to modify a bacterium by using a DNA that encodes a γ-glutamyl kinase desensitized to the feedback inhibition by L-proline is disclosed in the literature by Dandekar and Uratsu (J. Bacteriol. 170, 12: 5943-5945 (1988)). Moreover, examples of a method to obtain a bacterium having a weakened L-proline degradation system include, for example, a method to introduce into the proline dehydrogenase a mutation that causes reduction of the enzymatic activity. Specific examples of a bacterium having an ability to produce L-proline include the *Escherichia coli* strains NRRL B-12403 and NRRL B-12404 (GB Patent No. 2075056), the *Escherichia coli* strain VKPM B-8012 (U.S. Patent Application Publication No. 2002-0058315), a mutant strain of *Escherichia coli* carrying the plasmid disclosed in German Patent No. 3127361, and a mutant strain of *Escherichia coli* carrying the plasmid disclosed in the literature by Bloom F.R. et al., (The 15th Miami winter symposium, 1983, p.34).

Moreover, specific examples of a bacterium having an ability to produce L-proline also include the *Escherichia coli* strain 702 (VKPMB-8011), which is a strain resistant to 3,4-dehydroxyproline and azetidine-2-carboxylate, the 702ilvA strain obtained by deleting *ilvA* in 702 (VKPMB-8012 strain), *E. coli* having an increased activity of a protein encoded by the b2682, b2683, b1242 or b3434 gene, and the like (Japanese Unexamined Patent Application Publication No. 2002-300874).

### <L-arginine-producing bacteria>

Examples of a method to impart or increase the ability to produce L-arginine include, for example, a method in which a microorganism is modified to have increased activities of one or more enzymes selected from the L-arginine biosynthesis enzymes. Such enzymes include, but not particularly limited to, N-acetylglutamate synthase (*argA*), N-acetylglutamyl-phosphate reductase (*argC*), ornithine acetyltransferase (*argJ*), N-acetylglutamate kinase (*argB*), acetylornithine transaminase (*argD*), acetylornithine deacetylase (*argE*), ornithine carbamoyltransferase (*argF*), argininosuccinate synthase (*argG*), argininosuccinate lyase (*argH*) and carbamoyl-phosphate synthase (*carAB*). As an N-acetylglutamate synthase (*argA*) gene, for example, a mutant gene desensitized to the feedback inhibition by L-arginine is preferably used, in which the amino acid sequence corresponding to the wild-type sequence from position 15 to 19 has been replaced (European Patent Application Publication No. 1170361).

Examples of a microorganism having an ability to produce L-arginine include mutant strains of *Escherichia coli* having the resistance to α-methylmethionine, p-fluorophenylalanine, D-arginine, arginine hydroxamate, S-(2-aminoethyl)-cysteine, α -methylserine, β-2-thienylalanine, or sulfaguanidine (see Japanese Unexamined Patent Application Publication No. Sho 56-106598), and the like. Moreover, examples of a microorganism having an ability to produce L-arginine include the *Escherichia coli* strain 237 (Russian Patent Application No. 2000117677), which is an L-arginine-producing bacterium having a mutation that causes the resistance to the feedback inhibition by L-arginine and having a high activity of N-acetylglutamate synthase. The same strain had been deposited in the Russian National Collection of Industrial Microorganisms (VKPM), GNII Genetika on April 10, 2000 under the accession number VKPM B-7925 and has been put into the state of international deposit under the provision of the Budapest Treaty on May 18, 2001. Moreover, examples of a microorganism having an ability to produce L-arginine also include the *Escherichia coli* strain 382 (Japanese Unexamined Patent Application Publication No. 2002-017342), which is a derivative of the 237 strain and is an L-arginine-producing bacterium having an increased activity to assimilate acetic acid. The *Escherichia coli* strain 382 strain has been deposited in the Russian National Collection of Industrial Microorganisms (VKPM) on April 10, 2000 under the accession number VKPM B-7926.

Examples of a microorganism having an ability to produce L-arginine also include a wild-type strain of a coryneform bacterium; a coryneform bacterium having the resistance to agents such as a sulfa drug, 2-thiazol-alanine or α-amino-β-hydroxyvaleric acid, or the like; a coryneform bacterium having auxotrophy for L-histidine, L-proline, L-threonine, L-isoleucine, L-methionine or L-tryptophan in addition to the resistance to 2-thiazol-alanine (Japanese Unexamined Patent Application Publication No. Sho 54-44096); a coryneform bacterium having the resistance to ketomalonic acid, fluoromalonic acid, or monofluoroacetic acid (Japanese Unexamined Patent Application Publication No. Sho 57-18989); a coryneform bacterium having the resistance to argininol (Japanese Unexamined Patent Application Publication No. Sho 62-24075); or a coryneform bacterium having the resistance to X-guanidine (X represents a fatty acid or a derivative of fatty acid chain) (Japanese Unexamined Patent Application Publication No. Hei 2-186995); and the like. Moreover, examples of a coryneform bacterium having an ability to produce L-arginine also include a mutant strain resistant to 5-azauracil, 6-azauracil, 2-thiouracil, 5-fluorouracil, 5-bromouracil, 5-azacytosine, 6-azacytosine and the like; a mutant strain resistant to arginine hydroxamate and 2-thiouracil, a mutant strain resistant to arginine hydroxamate and 6-azauracil (Japanese Unexamined Patent Application Publication No. Sho 49-126819); a mutant strain resistant to a histidine analog or tryptophan analogs (Japanese Unexamined Patent Application Publication No. Sho 52-114092); a mutant strain having auxotrophy for at least one of methionine, histidine, threonine, proline, isoleucine, lysine, adenine, guanine or uracil (or uracil precursor) (see Japanese Unexamined Patent Application Publication No. Sho 52-99289); a mutant strain resistant to arginine hydroxamate (Japanese Examined Patent Application Publication No. Sho 51-6754); a mutant strain auxotrophic for succinic acid or resistant to a nucleobase analog (Japanese Unexamined Patent Application Publication No. Sho 58-9692); a mutant strain deficient in the ability to degrade arginine, resistant to an antagonist of arginine and canavanine, and auxotrophic for lysine (Japanese Unexamined Patent Application Publication No. Sho 52-8729); a mutant strain resistant to arginine, arginine hydroxamate, homoarginine, D-arginine, and canavanine, or resistant to arginine hydroxamate and 6-azauracil (Japanese Unexamined Patent Application Publication No. Sho 53-143288); and a mutant strain resistant to canavanine (Japanese Unexamined Patent Application Publication No. Sho 53-3586); and the like.

Specific examples of a coryneform bacterium having an ability to produce L-arginine include bacterial strains as indicated below:
*Brevibacterium flavum* AJ11169 (FERM BP-6892),
*Brevibacterium lactofermentum* AJ12092 (FERM BP-6906),
*Brevibacterium flavum* AJ11336 (FERM BP-6893),
*Brevibacterium flavum* AJ11345 (FERM BP-6894), and
*Brevibacterium lactofermentum* AJ12430 (FERM BP-2228).

Moreover, examples of an L-arginine-producing bacterium or a parent strain to derive that bacterium also include a strain with a defect of an arginine repressor, ArgR (U.S. Patent Application Publication No. 2002-0045223) and a strain having an increased cellular glutamine synthetase activity (U.S. Patent Application Publication No. 2005-0014236).

### <L-citrulline-producing bacteria and L-ornithine-producing bacteria>

L-citrulline and L-ornithine share the biosynthesis pathway with L-arginine. Thus, the ability to produce L-citrulline and/or L-ornithine can be imparted or increased by increasing the enzymatic activity of N-acetylglutamate synthase (*argA*), N-acetylglutamyl-phosphate reductase (*argC*), ornithine acetyltransferase (*argJ*), N-acetylglutamate kinase (*argB*), acetylornithine transaminase (*argD*), and/or acetylornithine deacetylase (*argE*) (WO2006-35831).

### <L-phenylalanine-producing bacteria>

Examples of an L-phenylalanine-producing bacterium or a parent strain to derive that bacterium include, but not limited to, strains belonging to the genus *Escherichia,* such as *E. coli* AJ12739 (*tyrA*::Tn10, *tyrR*) with defects of a chorismate mutase-prephenate dehydrogenase and a tyrosine repressor (VKPM B-8197) (WO03/044191), *E*. *coli* HW1089 (ATCC 55371) carrying a mutant *pheA34* gene that encodes a chorismate mutase-prephenate dehydratase desensitized to the feedback inhibition (U.S. Patent No. 5,354,672), *E*. *coli* MWEC101-b (KR8903681), *E*. *coli* NRRL B-12141, NRRL B-12145, NRRL B-12146 and NRRL B-12147 (U.S. Patent No. 4,407,952). Moreover, as a parent strain, *E*. *coli* K-12 [W3110 (*tyrA*)/pPHAB] (FERM BP-3566), *E*. *coli* K-12 [W3110 (*tyrA*)/pPHAD] (FERM BP-12659), *E*. *coli* K-12 [W3110 (*tyrA*)/pPHATerm] (FERM BP-12662) and *E*. *coli* K-12 [W3110 (*tyrA*)/pBR-aroG4, pACMAB] named AJ 12604 (FERM BP-3579) (EP 488424 B1) may also be used, all of which carry a gene encoding a chorismate mutase-prephenate dehydratase desensitized to the feedback inhibition. Furthermore, an L-phenylalanine-producing bacterium having an increased activity of a protein encoded by the *yedA* gene or the *yddG* gene and belonging to the genus *Escherichia* may also be used (U.S. Patent Application Publication No. 2003/0148473 A1 and 2003/0157667 A1, WO03/044192).

### <L-tryptophan-producing bacteria>

Examples of an L-tryptophan-producing bacterium or a parent strain to derive that bacterium include, but not limited to, strains belonging to the genus *Escherichia,* such as *E*. *coli* JP4735/pMU3028 (DSM10122) and JP6015/pMU91 (DSM10123), which have a defective tryptophanyl-tRNA synthetase encoded by a mutant *trpS* gene (U.S. Patent No. 5,756,345); *E*. *coli* SV164 (pGH5) that has a *serA* allele encoding a phosphoglycerate dehydrogenase desensitized to the feedback inhibition by serine and a *trpE* allele encoding a anthranilate synthase desensitized to the feedback inhibition by tryptophan (U.S. Patent No. 6,180,373); *E*. *coli* AGX17 (pGX44) (NRRL B-12263) and AGX6 (pGX50) *aroP* (NRRL B-12264), which have a defect of a tryptophanase (U.S. Patent No. 4,371,614); *E*. *coli* AGX17/pGX50 or pACKG4-pps having an increased ability to produce phosphoenolpyruvic acid (WO9708333, U.S. Patent No. 6,319,696). An L-tryptophan-producing bacterium having an increased activity of a protein encoded by the *yedA* gene or the *yddG* gene and belonging to the genus *Escherichia* may also be used (U.S. Patent Application Publication No. 2003/0148473 A1 and 2003/0157667 A1).

Examples of an L-tryptophan-producing bacterium or a parent strain to derive that bacterium also include strains having an increased activity of one enzyme or two or more enzymes selected from anthranilate synthase (*trpE*), phosphoglycerate dehydrogenase (*serA*), 3-deoxy-D-arabino-heptulosonate-7-phosphate synthase (*aroG*), 3-dehydroquinate synthase (*aroB*), shikimate dehydrogenase (*aroE*), shikimate kinase (*aroL*), 5-enol-pyruvilshikimate-3-phosphate synthase (*aroA*), chorismate synthase (*aroC*), prephenate dehydratase, chorismate mutase, and tryptophan synthase (*trpAB*). The prephenate dehydratase and the chorismate mutase are encoded as a bifunctional enzyme (CM-PD) by the *pheA* gene. Among those enzymes, phosphoglycerate dehydrogenase, 3-deoxy-D-arabino-heptulosonate-7-phosphate synthase, 3-dehydroquinate synthase, shikimate dehydratase, shikimate kinase, 5-enol-pyruvilshikimate-3-phosphate synthase, chorismate synthase, prephenate dehydratase, and chorismate mutase-prephenate dehydrogenase are particularly preferable. Because both of the anthranilate synthase and the phosphoglycerate dehydrogenase are susceptible to the feedback inhibition by L-tryptophan and L-serine, mutations that cause desensitization to the feedback inhibition may be introduced into these enzymes. Specific examples of a strain having such a mutation include *E*. *coli* SV164 having a desensitized anthranilate synthase and a transformed strain obtained by introducing into *E*. *coli* SV164 the plasmid pGH5 containing a mutant *serA* gene that encodes a phosphoglycerate dehydrogenase desensitized to the feedback inhibition (WO94/08031).

Examples of an L-tryptophan-producing bacterium or a parent strain to derive that bacterium also include strains to which a tryptophan operon including a gene that encodes an anthranilate synthase desensitized to the inhibition has been introduced (Japanese Unexamined Patent Application Publication No. Sho 57-71397, Japanese Unexamined Patent Application Publication No. Sho 62-244382, U.S. Patent No. 4,371,614). Furthermore, the ability to produce L-tryptophan may be imparted by increasing the expression of genes encoding a tryptophan synthase in the tryptophan operon (*trpBA*). The tryptophan synthase is composed of the α- and β-subunits encoded by the *trpA* and *trpB* genes, respectively. Furthermore, the ability to produce L-tryptophan may also be improved by increasing the expression of the isocitrate lyase-malate synthase operon (WO2005/103275).

### <L-histidine-producing bacteria>

Examples of an L-histidine-producing bacterium or a parent strain to derive that bacterium include, but not limited to, strains belonging to the genus *Escherichia,* such as the *E*. *coli* strain 24 (VKPM B-5945, RU2003677), the *E*. *coli* strain 80 (VKPM B-7270, RU2119536), *E*. *coli* NRRL B-12116 - B12121 (U.S. Patent No. 4,388,405), *E*. *coli* H-9342 (FERM BP-6675) and H-9343 (FERM BP-6676) (U.S. Patent No. 6,344,347), *E*. *coli* H-9341 (FERM BP-6674) (EP1085087), and *E*. *coli* AI80/pFM201 (U.S. Patent No. 6,258,554).

Examples of an L-histidine-producing bacterium or a parent strain to derive that bacterium also include strains in which the expression is increased in one or more of genes encoding L-histidine biosynthesis enzymes. Examples of such a gene include the ATP phosphoribosyl transferase gene (*hisG*), the phosphoribosyl AMP cyclohydrolase gene (*hisI*), the phosphoribosyl-ATP pyrophosphohydrolase gene (*hisI*), the phosphoribosylformimino-5-aminoimidazole carboxamide ribotide isomerase gene (*hisA*), the amidotransferase gene (*hisH*), the histidinol-phosphate aminotransferase gene (*hisC*), the histidinol phosphatase gene (*hisB*), the histidinol dehydrogenase gene (*hisD*), and the like.

The L-histidine biosynthesis enzymes encoded by *hisG* and *hisBHAFI* are known to be inhibited by L-histidine and, thus, the ability to produce L-histidine can be efficiently increased by introducing into the ATP phosphoribosyl transferase gene (*hisG*) a mutation that confers the resistance to the feedback inhibition (Russian Patent Nos. 2003677 and 2119536).

Specific examples of a strain having an ability to produce L-histidine include *E*. *coli* FERM-P 5038 and 5048 to which a vector carrying a DNA that encodes an L-histidine biosynthesis enzyme has been introduced (Japanese Unexamined Patent Application Publication No. Sho 56-005099), an *E*. *coli* strain to which an amino acid transporter gene has been introduced (EP1016710A), the *E*. *coli* strain 80 to which the resistance to sulfaguanidine, DL-1,2,4-triazole-3-alanine and streptomycin has been conferred (VKPM B-7270, Russian Patent No. 2119536), and the like.

### <L-threonine-producing bacteria>

Preferred examples of a microorganism having an ability to produce L-threonine include bacteria having an increased activity of one enzyme or two or more enzymes of the L-threonine biosynthesis enzymes. Examples of L-threonine biosynthesis enzymes include aspartokinase III (*lysC*), aspartate-semialdehyde dehydrogenase (*asd*), aspartokinase I encoded by the *thr* operon (*thrA*), homoserine kinase (*thrB*), threonine synthase (*thrC*), and aspartate aminotransferase (aspartate transaminase) (*aspC*). The abbreviation of each gene is indicated within the parentheses (the same is true in the following description). Among those enzymes, aspartokinase III, aspartate-semialdehyde dehydrogenase, aspartokinase I, homoserine kinase, aspartate aminotransferase, and threonine synthase are particularly preferable. An L-threonine biosynthesis gene may be introduced into a bacterium in the genus *Escherichia* exhibiting decreased degradation of threonine. Examples of a bacterium in the genus *Escherichia* exhibiting decreased degradation of threonine include, for example, the TDH6 strain having the defect in the threonine dehydrogenase activity (Japanese Unexamined Patent Application Publication No. 2001-346578), and the like.

The enzymatic activity of the L-threonine biosynthesis enzymes is suppressed by the final product L-threonine. Accordingly, to establish an L-threonine-producing bacterium, an L-threonine biosynthesis gene is desirably modified not to be susceptible to the feedback inhibition by L-threonine. Moreover, the above-described *thrA*, *thrB*, and *thrC* genes constitute the threonine operon and the threonine operon forms an attenuator structure, and the expression of the threonine operon is inhibited by isoleucine and threonine in a culture medium and suppressed by attenuation. This modification can be achieved by removing the leader sequence of the attenuation region or the attenuator (see Lynn, S. P., Burton, W. S., Donohue, T. J., Gould, R. M., Gumport, R. I., and Gardner, J. F. J. Mol. Biol. 194:59-69 (1987); WO02/26993; and WO2005/049808).

The native promoter of the threonine operon is present upstream of the threonine operon and may be replaced with a non-native promoter (see WO98/04715) or a threonine operon may be constructed such that the expression of genes involved in the threonine biosynthesis is controlled by a repressor and a promoter of lambda phage (see European Patent No. 0593792). Moreover, to modify the bacterium to be insusceptible to the feedback inhibition by L-threonine, a bacterial strain resistant to α-amino-β-hydroxyvaleric acid (AHV) may be selected.

The threonine operon modified as described above to be insusceptible to the feedback inhibition by L-threonine is preferably increased in copy number in a host or linked to a strong promoter to increase the expression level. The increase of copy number can be achieved by the plasmid-mediated amplification as well as by transposition of the threonine operon into the genome using a transposon, Mu-phage and the like.

In addition to the L-threonine biosynthesis enzymes, genes involved in glycolysis, TCA cycle, and respiratory chain, genes involved in the regulation of gene expression, or genes involved in sugar uptake are also preferably enhanced. Examples of these useful genes in the production of L-threonine include the transhydrogenase genes (*pntAB*) (European Patent No. 733712), the phosphoenolpyruvate carboxylase gene (*pepC*) (WO95/06114), the phosphoenolpyruvate synthase gene (*pps*) (European Patent No. 877090), the pyruvate carboxylase gene in a coryneform bacterium or a *Bacillus* bacterium (WO99/18228, European Patent Application Publication No. 1092776).

Moreover, it is also preferable to increase the expression of genes that confer the resistance to L-threonine or L-homoserine or to confer the resistance to L-threonine or L-homoserine on a host. Examples of a gene that confers the resistance include the *rhtA* gene (Res. Microbiol. 154:123-135 (2003)), the *rhtB* gene (European Patent Application Publication No. 0994190), the *rhtC* gene (European Patent Application Publication No. 1013765), and the *yfiK* and *yeaS* genes (European Patent Application Publication No. 1016710). Moreover, methods described in European Patent Application Publication No. 0994190 and WO90/04636 may be referenced as a method to confer the resistance to L-threonine on a host.

Examples of an L-threonine-producing bacterium or a parent strain to derive that bacterium include, but not limited to, strains belonging to the genus *Escherichia,* such as *E*. *coli* TDH-6/pVIC40 (VKPM B-3996) (U.S. Patent No. 5,175,107; U.S. Patent No. 5,705,371), *E*. *coli* 472T23/pYN7 (ATCC 98081) (U.S. Patent No. 5,631,157), *E*. *coli* NRRL-21593 (U.S. Patent No. 5,939,307), *E*. *coli* FERM BP-3756 (U.S. Patent No. 5,474,918), *E*. *coli* FERM BP-3519 and FERM BP-3520 (U.S. Patent No. 5,376,538), *E*. *coli* MG442 (Gusyatiner et al., Genetika (in Russian), 14, 947-956 (1978)), *E*. *coli* VL643 and VL2055 (EP 1149911 A).

The TDH-6 strain has the deletion of the *thrC* gene and the sucrose assimilating property and, moreover, a leaky mutation in the *ilvA* gene. Moreover, this strain has a mutation in the *rhtA* gene that confers the resistance to threonine or homoserine in high concentration. The B-3996 strain carries the plasmid pVIC40, which is an RSF1010-derived vector inserted with the *thrA***BC* operon including a mutant *thrA* gene. This mutant *thrA* gene encodes an aspartokinase homoserine dehydrogenase I that has been desensitized substantially to the feedback inhibition by threonine. The B-3996 strain has been deposited in the All-Union Scientific Center of Antibiotics (Nagatinskaya Street 3-A, 117105 Moscow, Russia) on November 19, 1987 under the accession number RIA 1867. This strain was also deposited in the Russian National Collection of Industrial Microorganisms (VKPM) (1 Dorozhny proezd., 1 Moscow 117545, Russia) on April 7, 1987 under the accession number B-3996.

*E*. *coli* VKPM B-5318 (EP 0593792B) may also be used as an L-threonine-producing bacterium or a parent strain to derive that bacterium. The B-5318 strain is prototrophic for isoleucine and the regulatory region of the threonine operon in the plasmid pVIC40 in this strain has been replaced with the C1 repressor and the PR promoter of a thermo-sensitive lambda phage. VKPM B-5318 has been deposited as an international deposit in the Russian National Collection of Industrial Microorganisms (VKPM) (1 Dorozhny proezd., 1 Moscow 117545, Russia) on May 3, 1990 under the accession number VKPM B-5318.

The *thrA* gene encoding the aspartokinase homoserine dehydrogenase I *of E. coli* has been identified (from nucleotide position 337 to 2799, GenBank accession NC_000913.2, gi: 49175990). The *thrA* gene is located between the *thrL* and *thrB* genes on the chromosome in *E*. *coli* K-12. The *thrB* gene encoding the homoserine kinase of *Escherichia coli* has been identified (from nucleotide position 2801 to 3733, GenBank accession NC_000913.2, gi: 49175990). The *thrB* gene is located between the *thrA* and *thrC* genes on the chromosome in *E*. *coli* K-12. The *thrC* gene encoding the threonine synthase *of E. coli* has been identified (from nucleotide position 3734 to 5020, GenBank accession NC_000913.2, gi: 49175990). The *thrC* gene is located between the *thrB* gene and the open reading frame of *yaaX* on the chromosome in *E*. *coli* K-12. These three genes all function as a single threonine operon. To increase the expression of the threonine operon, the attenuator region that affects the transcription is preferably removed from the operon (WO2005/049808, WO2003/097839).

A mutant *thrA* gene encoding an aspartokinase homoserine dehydrogenase I resistant to the feedback inhibition by threonine, as well as the *thrB* and the *thrC* genes can be obtained as a single operon from the well-known plasmid pVIC40 present in the threonine-producing strain *E*. *coli* VKPM B-3996. Details of the plasmid pVIC40 are described in U.S. Patent No. 5,705,371.

The *rhtA* gene *of E. coli* is located on *E*. *coli* chromosome at 18 minutes near the *glnHPQ* operon that encodes components of the glutamate transport system. The *rhtA* gene is identical to ORF1 (the *ybiF* gene, from nucleotide position 764 to 1651, GenBank accession number AAA218541, gi:440181) and located between the *pexB* and *ompX* genes. The unit to express a protein encoded by ORF1 is referred to as the *rhtA* gene (rht: resistant to homoserine and threonine). Moreover, the *rhtA23* mutation has been identified to be a G to A substitution at position -1 relative to the ATG initiator codon (ABSTRACTS of the 17th International Congress of Biochemistry and Molecular Biology in conjugation with Annual Meeting of the American Society for Biochemistry and Molecular Biology, San Francisco, California August 24-29, 1997, abstract No. 457, EP 1013765 A).

The *asd* gene *of E. coli* has already been identified (from nucleotide position 3572511 to 3571408, GenBank accession NC _000913.1, gi:16131307) and can be obtained by PCR using primers produced based on the nucleotide sequence of the gene (see White, T.J. et al., Trends Genet., 5, 185 (1989)). The *asd* genes of other microorganisms can also be similarly obtained.

Moreover, the *aspC* gene *of E. coli* has likewise already been identified (from nucleotide position 983742 to 984932, GenBank accession NC_000913.1, gi:16128895) and can be obtained by PCR. The *aspC* genes of other microorganisms can also be similarly obtained.

### <L-lysine-producing bacteria>

Now, examples of an L-lysine-producing bacterium or/and a method to establish that strain will be indicated below.

For example, examples of a strain having an ability to produce L-lysine include L-lysine analog-resistant strains or metabolic control mutant strains. Examples of an L-lysine analog include, but not limited to, oxalysine, lysine hydroxamate, S-(2-aminoethyl)-L-cysteine (AEC), γ-methyllysine, α-chlorocaprolactam and the like. Mutant strains having the resistance to these lysine analogs can be obtained by subjecting a bacterium belonging to the family Enterobacteriaceae to a conventional artificial mutation treatment. Specifically, examples of an L-lysine-producing bacterium include the *Escherichia coli* strain AJ11442 (FERM BP-1543, NRRL B-12185; see Japanese Unexamined Patent Application Publication No. Sho 56-18596 and U.S. Patent No. 4346170), the *Escherichia coli* strain VL611 (Japanese Unexamined Patent Application Publication No. 2000-189180), and the like. Moreover, the WC196 strain (see WO96/17930) may also be used as an L-lysine-producing bacterium of *Escherichia coli.*

Moreover, L-lysine-producing bacteria can also be established by increasing the activity of the L-lysine biosynthesis enzymes. The activity can be increased in these enzymes by increasing the copy number of an enzyme-coding gene in a cell or modifying the expression regulatory sequence of the gene. An increase of the copy number of a gene encoding an L-lysine biosynthesis enzyme and the modification of the expression regulatory sequence can be achieved by the same method as in the case of the *gltP* and *gltS* genes described below.

Examples of a gene encoding an L-lysine biosynthesis enzyme include genes for enzymes in the diaminopimelate pathway, such as the dihydrodipicolinate synthase gene (*dapA*), the aspartokinase gene (*lysC*), the dihydrodipicolinate reductase gene (*dapB*), the diaminopimelate decarboxylase gene (*lysA*), the diaminopimelate dehydrogenase gene (*ddh*) (all in WO96/40934), the phosphoenolpyruvate carboxylase gene (*ppc*) (Japanese Unexamined Patent Application Publication No. Sho 60-87788), the aspartate aminotransferase gene (*aspC*) (Japanese Examined Patent Application Publication No. Hei 6-102028), the diaminopimelate epimerase gene (*dapF*) (Japanese Unexamined Patent Application Publication No. 2003-135066), the aspartate-semialdehyde dehydrogenase gene (*asd*) (WO00/61723), and the like; or genes for enzymes in the aminoadipate pathway and the like, such as the homoaconitate hydratase gene (Japanese Unexamined Patent Application Publication No. 2000-157276), and the like. Moreover, the expression level of a gene involved in the energy efficiency (*cyo*) (EP 1170376 A), a gene encoding a nitocinamide nucleotide transhydrogenase (*pntAB*) (U.S. Patent No. 5,830,716), the *ybjE* gene encoding a protein having the activity to export L-lysine (WO2005/073390), a gene encoding a glutamate dehydrogenase (*gdhA*) (Gene23:199-209(1983)), or any combination of these genes may be increased in the parent strain. The abbreviation of each gene is indicated within the parentheses. Among the above-described genes, the *ybjE* gene is preferable. Examples of the *ybjE* gene include a gene encoding the amino acid sequence of SEQ ID NO: 86, specifically, a gene having the nucleotide sequence of SEQ ID NO: 85.

The wild-type dihydrodipicolinate synthase derived from *Escherichia coli* is known to be susceptible to the feedback inhibition by L-lysine and the wild-type aspartokinase derived from *Escherichia coli* is known to be susceptible to the suppression and the feedback inhibition by L-lysine. Thus, in cases where the *dapA* gene and the *lysC* gene are used, these genes are preferred to be mutant genes insusceptible to the feedback inhibition by L-lysine.

Examples of DNA encoding a mutant dihydrodipicolinate synthase that is insusceptible to the feedback inhibition by L-lysine include DNA encoding a protein having a sequence in which the histidine residue at position 118 has been substituted with a tyrosine residue. Moreover, examples of DNA encoding a mutant aspartokinase that is insusceptible to the feedback inhibition by L-lysine include DNA encoding an AK III having a sequence in which the threonine residue at position 352, the glycine residue at position 323 and the methionine at position 318 are substituted with an isoleucine residue, an aspartic acid residue and isoleucine, respectively (see U.S. Patent Nos. 5661012 and 6040160 for these mutants). The mutant DNA can be obtained by a site-directed mutagenesis method using PCR and the like.

Additionally, as examples of a plasmid containing a mutant *dapA* encoding a mutant dihydrodipicolinate synthase and a mutant *lysC* encoding a mutant aspartokinase, the broad-host-range plasmids RSFD80, pCAB1, and pCABD2 are known (U.S. Patent No. 6040160). An *Escherichia coli* strain JM109 transformed with the same plasmid (U.S. Patent No. 6040160) had been named AJ12396 and the same strain had been deposited in the National Institute of Bioscience and Human-Technology, Agency of Industrial Science and Technology, Ministry of International Trade and Industry (International Patent Organism Depositary, current National Institute of Technology and Evaluation) on October 28, 1993 under the accession number FERM P-13936 and has been put into the state of international deposit under the provision of the Budapest Treaty on November 1, 1994 and has been deposited under the accession number FERM BP-4859. RSFD80 can be obtained from the AJ12396 strain by a known method.

Furthermore, in an L-amino acid-producing bacterium, the activity may be reduced or compromised in enzymes that catalyze reactions to produce other compounds in pathways branching from the biosynthesis pathway of the L-amino acid and in enzymes that function negatively in the synthesis or accumulation of the L-amino acid. In the production of L-lysine, examples of such an enzyme include homoserine dehydrogenase, lysine decarboxylase (*cadA*, *ldcC*), malic enzyme and the like, and examples of strains having reduced or compromised activities of the enzymes are described in WO95/23864, WO96/17930, WO2005/010175 and the like.

To reduce or compromise the lysine decarboxylase activity, the expression is preferably reduced in both the *cadA* gene and the *ldcC* gene, which encode lysine decarboxylases. Reduction of the expression from both genes can be performed according to the method described in WO2006/078039.

In a method to reduce or compromise the activity of these enzymes, a mutation that reduces or compromises the activities of the enzymes may be introduced into the genes for the above-described enzymes on the genome by a conventional mutation treatment or the gene recombination technique. The introduction of such a mutation can be achieved, for example, through the deletion of a gene encoding an enzyme on the genome by gene recombination or through the modification of an expression regulatory sequence such as promoter and Shine-Dalgarno (SD) sequence. Moreover, the introduction of such a mutation can also be achieved by introducing into a coding region for an enzyme on the genome an amino acid substitution (missense mutation), a termination codon (nonsense mutation), or a frame-shift mutation that causes an addition or deletion of one to two bases, or by deleting a portion or the entire region of a gene (J. Biol. Chem. 272:8611-8617 (1997)). Moreover, the activity of an enzyme can also be reduced or compromised by constructing a mutant enzyme-encoding gene with the deletion of the entire part or a part of the coding region and replacing with the same gene the normal gene on the genome by gene recombination and the like, or by introducing a transposon or IS element into the same gene on the genome.

For example, a method as described below is used to introduce by gene recombination a mutation that reduces or compromises the activity of the above-described enzyme. A gene of interest on the genome can be replaced with a mutant gene by modifying a partial sequence in the gene of interest to produce a mutant gene that does not produce a normally functioning enzyme and then transforming a bacterium belonging to the family Enterobacteriaceae with DNA containing the same gene to induce a recombination event between the mutant gene and the gene on the genome. Examples of such gene replacement using homologous recombination include methods using a linear DNA fragment, such as a method called "Red-driven integration" (Datsenko, K. A, and Wanner, B. L. Proc. Natl. Acad. Sci. USA. 97:6640-6645 (2000)), a combination method of the Red-driven integration method and the excision system originated from λ phage (Cho, E. H., Gumport, R. I., Gardner, J. F. J. Bacteriol. 184: 5200-5203 (2002)) (see WO2005/010175), and the like; or a method using a plasmid containing a thermosensitive replication origin; and the like (U.S. Patent No. 6303383; Japanese Unexamined Patent Application Publication No. Hei 05-007491). Moreover, a site-directed mutagenesis through the gene replacement using homologous recombination as mentioned above can also be performed using a plasmid that has no ability to replicate in a host.

Examples of a preferable L-lysine-producing bacterium include *Escherichia coli* WC196 Δ*cadA* Δ*ldcC*/pCABD2 (WO2006/078039). This bacterial strain is a strain established by disrupting the *cadA* and *ldcC* genes encoding lysine decarboxylases in the WC196 strain and introducing the plasmid pCABD2 containing a lysine biosynthesis gene (U.S. Patent No. 6,040,160). The WC196 strain is a strain obtained from the W3110 strain, which is derived from *E. coli* K-12, and was bred by replacing the wild-type *lysC* gene on the chromosome of the W3110 strain with a mutant *lysC* gene encoding an aspartokinase III which has been desensitized to the feedback inhibition by L-lysine through the substitution of threonine 352 with isoleucine (U.S. Patent No. 5,661,012) and then imparting the AEC resistance (U.S. Patent No. 5,827,698). The WC196 strain had been named *Escherichia coli* AJ13069 and deposited in the National Institute of Bioscience and Human-Technology, Agency of Industrial Science and Technology (International Patent Organism Depositary, current National Institute of Technology and Evaluation; address: #120, 2-5-8, Kazusakamatari, Kisarazu-shi, Chiba 292-0818 Japan) on December 6, 1994 under the accession number FERM P-14690 and has been put into the state of international deposit under the provision of the Budapest Treaty on September 29, 1995 and given the accession number FERM BP-5252 (U.S. Patent No. 5,827,698). WC196 Δ*cadA* Δ*ldcC* itself is a preferable L-lysine-producing bacterium. WC196 Δ*cadA* Δ*ldcC* has been named AJ110692 and deposited as an international deposit in the National Institute of Bioscience and Human-Technology, Agency of Industrial Science and Technology (International Patent Organism Depositary, current National Institute of Technology and Evaluation; address: #120, 2-5-8, Kazusakamatari, Kisarazu-shi, Chiba 292-0818 Japan) on October 7, 2008 and given the accession number FERM BP-11027.

Moreover, examples of a preferable L-lysine-producing bacterium also include the *E*. *coli* strain AJIK01 (NITE BP-01520). The AJIK01 strain had been named *E*. *coli* AJ111046 and deposited in the NITE Patent Microorganism Depositary, National Institute of Technology and Evaluation (#120, 2-5-8, Kazusakamatari, Kisarazu-shi, Chiba 292-0818 Japan) on January 29, 2013 and has been put into the state of international deposit under the provision of the Budapest Treaty on May 15, 2014 and given the accession number NITE BP-01520.

The plasmid pCABD2 contains a mutant *dapA* gene encoding a dihydrodipicolinate synthase (DDPS) derived from *Escherichia coli,* which carries a mutation causing the desensitization to the feedback inhibition by L-lysine; a mutant *lysC* gene encoding an aspartokinase III derived from *Escherichia coli,* which carries a mutation causing the desensitization to the feedback inhibition by L-lysine; the *dapB* gene encoding a dihydrodipicolinate reductase derived from *Escherichia coli*; and the *ddh* gene encoding a diaminopimelate dehydrogenase derived from *Brevibacterium lactofermentum.*

The procedures to increase the expression of genes for enzymes involved in the biosynthesis of L-lysine as described above and the methods to reduce the activity of the enzymes can be applied similarly to genes encoding enzymes in the biosynthesis of other L-amino acids.

### <L-valine-producing bacteria>

Examples of an L-valine-producing bacterium or a parent strain to derive that bacterium include, but not limited to, a strain modified to overexpress the *ilvGMEDA* operon (U.S. Patent No. 5,998,178). A region in the *ilvGMEDA* operon required for attenuation is preferably removed to prevent the produced valine from attenuating the expression of the operon. Furthermore, the threonine deaminase activity is preferably reduced by disrupting the *ilvA* gene in the operon.

Examples of an L-valine-producing bacterium or a parent strain to derive that bacterium also include a mutant strain having a mutation in an amino acyl tRNA synthetase (U.S. Patent No. 5,658,766). For example, *E*. *coli* VL1970 having a mutation in the *ileS* gene that encodes the isoleucyl tRNA synthetase can be used. *E*. *coli* VL1970 has been deposited in the Russian National Collection of Industrial Microorganisms (VKPM) (1 Dorozhny proezd., 1 Moscow 117545, Russia) on June 24, 1988 under the accession number VKPM B-4411.

Furthermore, mutant strains which require lipoic acid for their growth and/or lack H⁺-ATPase (WO96/06926) can be used as a parent strain.

### <L-isoleucine-producing bacteria>

Examples of an L-isoleucine-producing bacterium or a parent strain to derive that bacterium include, but not limited to, mutant strains having the resistance to 6-dimethylaminopurine (Japanese Unexamined Patent Application Publication No. Hei 5-304969); to isoleucine analogs such as thiaisoleucine, isoleucine hydroxamate and the like; and, furthermore, to DL-ethionine and/or arginine hydroxamate (Japanese Unexamined Patent Application Publication No. Hei 5-130882). Furthermore, a recombinant strain which has been transformed with a gene encoding a protein involved in the biosynthesis of L-isoleucine, such as threonine deaminase, acetohydroxy acid synthase and the like, can also be used as a parent strain (Japanese Unexamined Patent Application Publication No. Hei 2-458, FR 0356739, and U.S. Patent No. 5,998,178).

### <L-methionine-producing bacteria>

Examples of an L-methionine-producing bacterium or a parent strain to derive that bacterium include, but not limited to, a strain auxotrophic for L-threonine and a mutant strain having the resistance to norleucine (Japanese Unexamined Patent Application Publication No. 2000-139471). Furthermore, strains with the deletion of the methionine repressor, and recombinant strains which have been transformed with a gene encoding a protein involved in the biosynthesis of L-methionine, such as homoserine succinyltransferase, cystathionine γ-synthase and the like, can also be used as a parent strain (Japanese Unexamined Patent Application Publication No. 2000-139471).

### <L-leucine-producing bacteria>

Examples of an L-leucine-producing bacterium or a parent strain to derive that bacterium include, but not limited to, strains belonging to the genus *Escherichia,* such as leucine-resistant *E*. *coli* strains (for example, the 57 strain (VKPM B-7386, U.S. Patent No. 6,124,121)) or *E. coli* strains resistant to leucine analogs including β-2-thienylalanine, 3-hydroxyleucine, 4-azaleucine, 5,5,5-trifluoroleucine and the like (Japanese Examined Patent Application Publication No. Sho 62-34397 and Japanese Unexamined Patent Application Publication No. Hei 8-70879), and an *E*. *coli* strain obtained by a gene engineering method described in WO96/06926, *E*. *coli* H-9068 (Japanese Unexamined Patent Application Publication No. Hei 8-70879).

Bacteria used in the present invention may be improved by increasing the expression of one or more of genes involved in the biosynthesis of L-leucine. Examples of such a gene include genes in the *leuABCD* operon, preferably represented by a mutant *leuA* gene encoding an isopropylmalate synthase that has been desensitized to the feedback inhibition by L-leucine (U.S. Patent No. 6,403,342). Furthermore, bacteria used in the present invention may also be improved by increasing the expression of one or more of genes encoding proteins which function to export L-amino acid from a bacterial cell. Examples of such a gene include the b2682 gene and the b2683 gene (the *ygaZH* genes) (EP 1239041 A2).

### <1-2> Impartment of the ability to assimilate alginic acid

The bacterium used in the present invention is a bacterium modified to have the alginate-assimilating property.

In the present invention, alginic acid means polysaccharides consisting of D-mannuronic acid and L-glucuronic acid.

The term "alginic acid" as used herein refers to polysaccharides composed of two kinds of uronic acids, β-D-mannuronic acid (M), which is also referred to as β-1,4'-mannurono-1,4'-L-gulurono-glycan, and α-L-glucuronic acid (G), whose pyranose rings are connected each other mainly by a β1→4 linkage. In alginic acid, a homopolymer region of β-D-mannuronic acid (hereinafter referred to as "poly-(M)" or "M-block"), a homopolymer region of α-L-glucuronic acid (hereinafter referred to as "poly-(G)" or "G-block"), and a heteropolymer region of β-D-mannuronic acid and α-L-glucuronic acid (hereinafter referred to as "poly-(MG)" or "MG-block") exist. The source of alginic acid is not limited but alginic acid can be obtained from, for example, algae including seaweed and the like; bacteria; and the like.

Either of the ability to produce an L-amino acid and the ability to assimilate alginic acid may be imparted first. The phrase "modified to have the alginate-assimilating property" refers to bacteria capable of growing with a carbon source mainly including alginic acid. Such bacteria are preferably modified to assimilate a more amount of alginic acid than wild-type strains and are preferred to be modified such that the bacteria can assimilate, for example, alginic acid in an amount of 0.5 g/L or more, desirably 1 g/L or more, and further desirably 2.5 g/L or more in a culture medium.

The alginate-assimilating property can be effected by imparting any one or more characteristics or all characteristics of
i) increasing the alginate lyase activity,
ii) increasing the alginate importer activity, and
iii) increasing the keto acid reductase activity.

The term "alginate lyase" refers to a lyase that acts on alginic acid. "Lyase" is the general term for enzymes that catalyze a reaction to remove a certain group from a substrate neither by hydrolysis nor by oxidation and leave a double bond. In detail, an alginate lyase degrades alginic acid through a β-elimination reaction. Specifically, alginate lyase is an enzyme that produces oligo-sugars having a 4-deoxy-α-D-mann-4-enuronosyl group at the non-reducing end through the elimination reaction of alginic acid. The term "alginate lyase activity" as used herein means the activity of the enzyme as described above.

Alginate lyases can be classified based on their substrate specificity into a group of enzymes that specifically act on poly-(M) (EC4.2.2.3), a group of enzymes that specifically act on poly-(G) (EC4.2.2.11), and, furthermore, a group of enzymes that specifically act on poly-(MG), and the like. The alginate lyase as used herein may be an alginate lyase which acts on any of the substrates and, in one embodiment, the alginate lyase is poly-(M)-specific and, in another embodiment, is poly-(G)-specific. The term "poly-(M)-specific" as used herein refers to an alginate lyase which acts preferentially on poly-(M) compared with poly-(G) and the term "poly-(G)-specific" as used herein refers to an alginate lyase which acts preferentially on poly-(G) compared with poly-(M). In any case, the alginate lyase may further have an action on poly-(MG).

An increased alginate lyase activity can be confirmed, for example, by preparing a crude enzyme solution from each of microorganisms before and after modification and comparing the alginate lyase activity in the crude enzyme solutions. The increase of the alginate lyase activity can be determined by measuring the absorbance of an unsaturated reaction product at 235 nm with reference to, for example, Uchimura et al. Marine Biotechnology (2010) Vol.12, p526-533, and *Escherichia coli* W3110 and MG1655 and *Corynebacterium glutamicum* ATCC13869 and ATCC13032 can be used as subjects for comparison.

Alginate lyases derived from bacteria in the genera *Azotobacter*, *Pseudomonas*, *Cobetia*, *Sphingomonas*, *Klebsiella*, *Alteromonas*, *Rhodobacter*, and *Shewanella* may be used and, among those, alginate lyases of bacteria in the genus *Vibrio* can be used. For example, *Vibrio alginolytics*, *Vibrio splendidus*, *Vibrio kanaloaei*, *Vibrio pomeroyi*, *Vibrio chagasii*, *Vibrio lentus*, *Vibrio cyclitrophicus*, *Vibrio crassostreae*, *Vibrio hemicentroti*, *Vibrio* sp. JAM-A9m, *Vibrio rumoiensis* or *Vibrio alginovora* can be used.

Moreover, the alginate lyase genes of the genus *Vibrio* can be obtained by using the GenBank database below:
*Vibrio cholerae* O1 biovar eltor str. N16961 chromosome I, complete sequence; AE003852,
*Vibrio cholerae* O1 biovar eltor str. N16961 chromosome II, complete sequence; AE003853,
*Vibrio parahaemolyticus* RIMD 2210633 chromosome I, complete sequence; BA000031,
*Vibrio parahaemolyticus* RIMD 2210633 chromosome II, complete sequence; BA000032,
*Vibrio fischeri* ES114 chromosome I, complete sequence; CP000020,
*Vibrio fischeri* ES114 chromosome II, complete sequence; CP000021,
*Vibrio vulnificus* CMCP6 chromosome I, complete sequence; AE016795,
*Vibrio vulnificus* CMCP6 chromosome II, complete sequence; AE016796,
*Vibrio vulnificus* YJ016 chromosome I, complete sequence; BA000037,
*Vibrio vulnificus* YJ016 chromosome II, complete sequence; BA000038.

Moreover, genes of the genus *Vibrio* can be obtained with reference to the ID in the GenBank database as listed in the tables below. Specifically, the genes of the genus *Vibrio* can be obtained by entering each Assembly ID in the tables below on a page with URL: http://www.ncbi.nlm.nih.gov/ in NCBI and downloading the data corresponding to each Assembly ID from the WGS (Whole Genome Sequence) Project page.

### [Table 1-1]

**Table 1-1**

| GenBank Assembly ID | |
|---|---|
| (Accession.version) | GenBank release ID |
| GCA_000400425.1 | 687838 |
| GCA_000400405.1 | 687798 |
| GCA_000400385.1 | 687758 |
| GCA_000400365.1 | 687718 |
| GCA_000400345.1 | 687678 |
| GCA_000400325.1 | 687638 |
| GCA_000400305.1 | 687598 |
| GCA_000400285.1 | 687558 |
| GCA_000400265.1 | 687518 |
| GCA_000400245.1 | 687478 |
| GCA_000400225.1 | 687438 |
| GCA_000400205.1 | 687398 |
| GCA_000388025.1 | 672908 |
| GCA_000390165.1 | 670918 |
| GCA_000387725.1 | 651438 |
| GCA_000387705.1 | 651418 |
| GCA_000387685.1 | 651398 |
| GCA_000387665.1 | 651378 |
| GCA_000387645.1 | 651358 |
| GCA_000387625.1 | 651338 |
| GCA_000387605.1 | 651318 |
| GCA_000387585.1 | 651298 |
| GCA_000272105.1 | 390998 |
| GCA_000269765.1 | 388758 |
| GCA_000269745.1 | 388738 |
| GCA_000269725.1 | 388718 |
| GCA_000256485.1 | 404808 |
| GCA_000257185.1 | 367268 |
| GCA_000257165.1 | 367248 |
| GCA_000195475.2 | 345468 |
| GCA_000354175.1 | 573118 |
| GCA_000347555.1 | 559258 |
| GCA_000342305.1 | 552488 |
| GCA_000338215.1 | 534938 |
| GCA_000316925.1 | 502608 |

### [Table 1-2]

**Table 1-2**

| GenBank Assembly ID | |
|---|---|
| (Accession.version) | GenBank release ID |
| GCA_000316985.1 | 502668 |
| GCA_000315135.1 | 500288 |
| GCA_000299635.1 | 427738 |
| GCA_000303175.1 | 438938 |
| GCA_000280885.1 | 406338 |
| GCA_000275705.1 | 400088 |
| GCA_000272445.1 | 391338 |
| GCA_000272425.1 | 391318 |
| GCA_000272365.1 | 391258 |
| GCA_000272385.1 | 391278 |
| GCA_000272405.1 | 391298 |
| GCA_000272305.1 | 391198 |
| GCA_000272285.1 | 391178 |
| GCA_000272345.1 | 391238 |
| GCA_000272325.1 | 391218 |
| GCA_000272265.1 | 391158 |
| GCA_000272245.1 | 391138 |
| GCA_000272125.1 | 391018 |
| GCA_000272225.1 | 391118 |
| GCA_000272085.1 | 390978 |
| GCA_000272205.1 | 391098 |
| GCA_000272065.1 | 390958 |
| GCA_000272045.1 | 390938 |
| GCA_000272185.1 | 391078 |
| GCA_000272165.1 | 391058 |
| GCA_000272145.1 | 391038 |
| GCA_000287135.1 | 411338 |
| GCA_000287115.1 | 411318 |
| GCA_000287095.1 | 411298 |
| GCA_000287155.1 | 411358 |
| GCA_000287075.1 | 411278 |
| GCA_000287055.1 | 411258 |
| GCA_000287035.1 | 411238 |
| GCA_000287015.1 | 411218 |
| GCA_000287195.1 | 411398 |

### [Table 1-3]

**Table 1-3**

| GenBank Assembly ID | |
|---|---|
| (Accession.version) | GenBank release ID |
| GCA_000286995.1 | 411198 |
| GCA_000286975.1 | 411178 |
| GCA_000286955.1 | 411158 |
| GCA_000257415.2 | 572558 |
| GCA_000256465.1 | 369678 |
| GCA_000256445.1 | 369688 |
| GCA_000256425.1 | 369698 |
| GCA_000256405.1 | 369718 |
| GCA_000256385.1 | 369708 |
| GCA_000256365.1 | 369728 |
| GCA_000256345.1 | 369738 |
| GCA_000256325.1 | 369748 |
| GCA_000256305.1 | 369758 |
| GCA_000256285.1 | 369768 |
| GCA_000256245.1 | 369788 |
| GCA_000256605.1 | 369668 |
| GCA_000256205.1 | 369798 |
| GCA_000256185.1 | 369808 |
| GCA_000256165.1 | 369818 |
| GCA_000256135.1 | 369828 |
| GCA_000256115.1 | 369838 |
| GCA_000256095.1 | 370488 |
| GCA_000252345.2 | 432028 |
| GCA_000247005.1 | 354498 |
| GCA_000237785.2 | 340248 |
| GCA_000237745.2 | 340308 |
| GCA_000237725.2 | 340228 |
| GCA_000237705.2 | 340488 |
| GCA_000237685.2 | 340188 |
| GCA_000237665.2 | 341088 |
| GCA_000237645.2 | 339688 |
| GCA_000237625.2 | 344228 |
| GCA_000237605.2 | 340628 |
| GCA_000237585.2 | 340008 |
| GCA_000237565.2 | 340068 |

### [Table 1-4]

**Table 1-4**

| GenBank Assembly ID | |
|---|---|
| (Accession.version) | GenBank release ID |
| GCA_000237545.2 | 339988 |
| GCA_000237525.2 | 341208 |
| GCA_000237505.2 | 343908 |
| GCA_000237485.2 | 341008 |
| GCA_000237465.2 | 340048 |
| GCA_000237445.2 | 339828 |
| GCA_000237425.2 | 339808 |
| GCA_000237405.2 | 339668 |
| GCA_000348505.1 | 570068 |
| GCA_000348485.1 | 570118 |
| GCA_000348465.1 | 570128 |
| GCA_000348445.1 | 571148 |
| GCA_000348425.1 | 571128 |
| GCA_000348405.1 | 571118 |
| GCA_000348385.1 | 571088 |
| GCA_000348365.1 | 571078 |
| GCA_000348345.1 | 571048 |
| GCA_000348325.1 | 571038 |
| GCA_000348305.1 | 571028 |
| GCA_000348285.1 | 570998 |
| GCA_000348265.1 | 570988 |
| GCA_000348245.1 | 570958 |
| GCA_000348225.1 | 570948 |
| GCA_000348205.1 | 570938 |
| GCA_000348185.1 | 570908 |
| GCA_000348165.1 | 570898 |
| GCA_000348145.1 | 570888 |
| GCA_000348125.1 | 570878 |
| GCA_000348105.1 | 570868 |
| GCA_000348085.1 | 570858 |
| GCA_000348065.1 | 570848 |
| GCA_000348045.1 | 570838 |
| GCA_000259935.1 | 373798 |
| GCA_000347495.1 | 558488 |
| GCA_000327105.3 | 555018 |

### [Table 1-5]

**Table 1-5**

| GenBank Assembly ID | |
|---|---|
| (Accession.version) | GenBank release ID |
| GCA_000327125.3 | 555038 |
| GCA_000327145.3 | 555048 |
| GCA_000327165.3 | 555008 |
| GCA_000327185.3 | 554958 |
| GCA_000327205.3 | 554998 |
| GCA_000327225.3 | 554988 |
| GCA_000327245.3 | 554968 |
| GCA_000299535.1 | 427638 |
| GCA_000299515.1 | 427618 |
| GCA_000299495.1 | 427598 |
| GCA_000341445.1 | 541358 |
| GCA_000275645.1 | 395148 |
| GCA_000257415.1 | 367498 |
| GCA_000237765.2 | 339328 |
| GCA_000223095.2 | 339048 |
| GCA_000166495.2 | 328438 |
| GCA_000166475.2 | 340908 |
| GCA_000338875.1 | 538258 |
| GCA_000338075.1 | 534948 |
| GCA_000334195.1 | 529738 |
| GCA_000318505.2 | 528248 |
| GCA_000327245.2 | 528208 |
| GCA_000318485.2 | 528168 |
| GCA_000327225.2 | 528128 |
| GCA_000327205.2 | 528088 |
| GCA_000327185.2 | 528048 |
| GCA_000327165.2 | 528008 |
| GCA_000327145.2 | 527968 |
| GCA_000327125.2 | 527918 |
| GCA_000327105.2 | 527848 |
| GCA_000330905.1 | 521758 |
| GCA_000328405.1 | 516618 |
| GCA_000327245.1 | 515378 |
| GCA_000327225.1 | 515338 |
| GCA_000327205.1 | 515298 |

### [Table 1-6]

**Table 1-6**

| GenBank Assembly ID | |
|---|---|
| (Accession.version) | GenBank release ID |
| GCA_000327185.1 | 515258 |
| GCA_000327165.1 | 515218 |
| GCA_000327145.1 | 515178 |
| GCA_000327125.1 | 515138 |
| GCA_000327105.1 | 515098 |
| GCA_000318075.1 | 505058 |
| GCA_000318505.1 | 505198 |
| GCA_000318485.1 | 505178 |
| GCA_000303085.1 | 442508 |
| GCA_000303125.1 | 442478 |
| GCA_000279375.1 | 403218 |
| GCA_000303005.1 | 442538 |
| GCA_000279205.1 | 403228 |
| GCA_000302895.1 | 442608 |
| GCA_000302875.1 | 442548 |
| GCA_000302855.1 | 442558 |
| GCA_000303105.1 | 442488 |
| GCA_000302835.1 | 442598 |
| GCA_000279955.1 | 403188 |
| GCA_000279185.1 | 403238 |
| GCA_000302755.1 | 442588 |
| GCA_000302775.1 | 442578 |
| GCA_000303065.1 | 442518 |
| GCA_000279785.1 | 403198 |
| GCA_000303045.1 | 442498 |
| GCA_000302985.1 | 442568 |
| GCA_000302965.1 | 442528 |
| GCA_000279555.1 | 403208 |
| GCA_000222145.1 | 297418 |
| GCA_000279285.1 | 400228 |
| GCA_000279265.1 | 400208 |
| GCA_000279455.1 | 400368 |
| GCA_000279435.1 | 400348 |
| GCA_000279415.1 | 400328 |
| GCA_000279395.1 | 400308 |

### [Table 1-7]

**Table 1-7**

| GenBank Assembly ID | |
|---|---|
| (Accession.version) | GenBank release ID |
| GCA_000281655.1 | 402598 |
| GCA_000279345.1 | 400288 |
| GCA_000279245.1 | 400188 |
| GCA_000279325.1 | 400268 |
| GCA_000279305.1 | 400248 |
| GCA_000305755.1 | 445598 |
| GCA_000305735.1 | 445608 |
| GCA_000305715.1 | 445618 |
| GCA_000305695.1 | 445628 |
| GCA_000305625.1 | 449978 |
| GCA_000305545.1 | 450018 |
| GCA_000305645.1 | 449968 |
| GCA_000305605.1 | 443328 |
| GCA_000305565.1 | 450008 |
| GCA_000305585.1 | 449988 |
| GCA_000305675.1 | 449958 |
| GCA_000305525.1 | 450028 |
| GCA_000305135.1 | 450348 |
| GCA_000305115.1 | 450448 |
| GCA_000305095.1 | 450438 |
| GCA_000305075.1 | 450428 |
| GCA_000304795.1 | 441018 |
| GCA_000305055.1 | 450418 |
| GCA_000305195.1 | 450408 |
| GCA_000305015.1 | 450398 |
| GCA_000304995.1 | 450388 |
| GCA_000304955.1 | 450378 |
| GCA_000304775.1 | 440998 |
| GCA_000304935.1 | 450368 |
| GCA_000304915.1 | 450358 |
| GCA_000304755.1 | 440978 |
| GCA_000259295.1 | 372098 |
| GCA_000234455.2 | 334828 |
| GCA_000234865.1 | 315908 |
| GCA_000234435.2 | 334588 |

### [Table 1-8]

**Table 1-8**

| GenBank Assembly ID | |
|---|---|
| (Accession.version) | GenBank release ID |
| GCA_000234885.1 | 315918 |
| GCA_000234905.1 | 315898 |
| GCA_000234415.2 | 343308 |
| GCA_000234395.2 | 334108 |
| GCA_000234925.1 | 315888 |
| GCA_000234945.1 | 315878 |
| GCA_000234965.1 | 315868 |
| GCA_000234375.2 | 342808 |
| GCA_000222645.2 | 341648 |
| GCA_000222625.2 | 340808 |
| GCA_000221485.1 | 297158 |
| GCA_000221465.1 | 297148 |
| GCA_000221445.1 | 297138 |
| GCA_000221425.1 | 297128 |
| GCA_000220785.2 | 334448 |
| GCA_000220765.2 | 334428 |
| GCA_000221405.1 | 297118 |
| GCA_000220745.2 | 334928 |
| GCA_000221385.1 | 297108 |
| GCA_000220725.2 | 334908 |
| GCA_000221365.1 | 297098 |
| GCA_000221345.1 | 297088 |
| GCA_000195415.2 | 335888 |
| GCA_000166455.2 | 315778 |
| GCA_000256265.1 | 369778 |
| GCA_000259875.1 | 373738 |
| GCA_000195065.1 | 255188 |
| GCA_000257205.1 | 367288 |
| GCA_000021625.1 | 22428 |
| GCA_000250855.1 | 359928 |
| GCA_000241385.1 | 325738 |
| GCA_000184325.1 | 237938 |
| GCA_000195225.2 | 306588 |
| GCA_000222685.2 | 300348 |
| GCA_000222665.2 | 300308 |

### [Table 1-9]

**Table 1-9**

| GenBank Assembly ID | |
|---|---|
| (Accession.version) | GenBank release ID |
| GCA_000222605.2 | 300268 |
| GCA_000222585.2 | 300228 |
| GCA_000222565.2 | 300188 |
| GCA_000217675.1 | 288828 |
| GCA_000039765.1 | 273878 |
| GCA_000186585.1 | 241138 |
| GCA_000196095.1 | 256918 |
| GCA_000189275.2 | 252618 |
| GCA_000189255.2 | 252578 |
| GCA_000165125.2 | 235678 |
| GCA_000182685.1 | 231268 |
| GCA_000182465.1 | 230868 |
| GCA_000182385.1 | 230728 |
| GCA_000182365.1 | 230688 |
| GCA_000182345.1 | 230648 |
| GCA_000181535.1 | 229298 |
| GCA_000176715.1 | 221458 |
| GCA_000176455.1 | 220938 |
| GCA_000176435.1 | 220898 |
| GCA_000176415.1 | 220858 |
| GCA_000176395.1 | 220818 |
| GCA_000176375.1 | 220778 |
| GCA_000176235.1 | 220498 |
| GCA_000176215.1 | 220458 |
| GCA_000176175.1 | 220378 |
| GCA_000176155.1 | 220338 |
| GCA_000176135.1 | 220298 |
| GCA_000176055.1 | 220138 |
| GCA_000175995.1 | 220018 |
| GCA_000175975.1 | 219978 |
| GCA_000175695.1 | 219418 |
| GCA_000174335.1 | 216758 |
| GCA_000174315.1 | 216718 |
| GCA_000174295.1 | 216678 |
| GCA_000174275.1 | 216638 |

### [Table 1-10]

**Table 1-10**

| GenBank Assembly ID | |
|---|---|
| (Accession.version) | GenBank release ID |
| GCA_000174255.1 | 216598 |
| GCA_000174235.1 | 216558 |
| GCA_000174115.1 | 216318 |
| GCA_000171815.1 | 211958 |
| GCA_000168935.1 | 206318 |
| GCA_000168915.1 | 206278 |
| GCA_000168895.1 | 206238 |
| GCA_000167935.1 | 204398 |
| GCA_000166455.1 | 201958 |
| GCA_000158115.1 | 187118 |
| GCA_000154045.1 | 179038 |
| GCA_000154025.1 | 178998 |
| GCA_000154005.1 | 178958 |
| GCA_000153985.1 | 178918 |
| GCA_000153965.1 | 178878 |
| GCA_000153945.1 | 178838 |
| GCA_000153865.1 | 178678 |
| GCA_000153785.1 | 178518 |
| GCA_000153505.1 | 177998 |
| GCA_000153005.1 | 176998 |
| GCA_000152765.1 | 176518 |
| GCA_000152485.1 | 175958 |
| GCA_000152465.1 | 175918 |
| GCA_000152445.1 | 175878 |
| GCA_000152425.1 | 175838 |
| GCA_000091465.1 | 112098 |
| GCA_000024825.1 | 25628 |
| GCA_000426765.1 | 777318 |
| GCA_000464435.1 | 774868 |
| GCA_000462975.1 | 773048 |
| GCA_000461895.1 | 771938 |
| GCA_000454475.1 | 764028 |
| GCA_000454455.1 | 764008 |
| GCA_000454265.1 | 763838 |
| GCA_000454245.1 | 763818 |

### [Table 1-11]

**Table 1-11**

| GenBank Assembly ID | |
|---|---|
| (Accession.version) | GenBank release ID |
| GCA_000454225.1 | 763798 |
| GCA_000454205.1 | 763778 |
| GCA_000454185.1 | 763758 |
| GCA_000454165.1 | 763738 |
| GCA_000454145.1 | 763718 |
| GCA_000442925.1 | 750228 |
| GCA_000438805.1 | 745598 |
| GCA_000438785.1 | 745578 |
| GCA_000022585.1 | 23388 |
| GCA_000021605.1 | 22408 |
| GCA_000430425.1 | 737928 |
| GCA_000430405.1 | 737908 |
| GCA_000418995.1 | 726938 |
| GCA_000017705.1 | 18508 |
| GCA_000417905.1 | 719578 |
| GCA_000417625.1 | 719378 |
| GCA_000016245.1 | 17048 |
| GCA_000009745.1 | 10548 |
| GCA_000006745.1 | 7548 |

Alginate lyases can be obtained from microorganisms which are attached to seaweed and organisms that eat seaweed to grow. For example, alginate lyases can be obtained from microorganisms attached to turban shell, abalone, skullcap shell, black scraper, parrot fish, red seabream, wrasse and the like.

In one example, an alginate lyase derived from NITE BP-01635 (hereinafter, also represented by *Vibrio* sp. strain SA2 in this specification) and particularly the polypeptide indicated below can be used. The NITE BP-01635 strain had been deposited as a domestic deposit in the National Institute of Technology and Evaluation (#122, 2-5-8, Kazusakamatari, Kisarazu-shi, Chiba, Japan) under the accession number NITE P-01635 (Date of deposition: June 13, 2013) and has been put into the state of international deposit under the provision of the Budapest Treaty on September 13, 2013 and given the accession number NITE BP-01635.

AlyB is an alginate lyase and the coding *alyB* gene sequence is represented by SEQ ID NO: 20 and the amino acid sequence is represented by SEQ ID NO: 21.

AlyD is an alginate lyase localized in the periplasm and the coding *alyD* gene sequence is represented by SEQ ID NO: 4 and the amino acid sequence is represented by SEQ ID NO: 5.

OalB is an alginate lyase localized in the cytoplasm and the coding *oalB* gene sequence is represented by SEQ ID NO: 10 and the amino acid sequence is represented by SEQ ID NO: 11.

OalC is an alginate lyase localized in the cytoplasm and the coding *oalC* gene sequence is represented by SEQ ID NO: 12 and the amino acid sequence is represented by SEQ ID NO: 13.

The alginate lyase (hereinafter referred to as the enzyme) may be variants of the above-indicated alginate lyase of the present invention as long as the variants retain the original function. Incidentally, such a variant may be referred to as a "conservative variant". Examples of a conservative variant include, for example, homologs or artificial mutants of the above-indicated enzyme of the present invention, such as proteins encoded by the *alyB*, *alyD*, *oalB*, and *oalC* genes.

The phrase "retaining the original function" refers to a variant of a protein having an activity corresponding to that of the original protein. That is, "retaining the original function" in regard to an alginate lyase refers to a protein having the alginate lyase activity and "retaining the original function" in regard to the alginate lyase activity refers to the alginate lyase activity retained in a protein.

Incidentally, in cases where a protein functions in a multisubunit complex, "retaining the original function" in regard to each subunit may refer to each subunit forming a complex with the remaining subunits and the resultant complex having the corresponding activity. That is, for example, "retaining the original function" in regard to each subunit of an alginate lyase may refer to each subunit forming a complex with the remaining subunits and the resultant complex having the alginate lyase activity.

Genes encoding homologs of the above-indicated enzyme can easily be obtained from a public database, for example, by BLAST search or FASTA search using the nucleotide sequence of a gene encoding the above-indicated enzyme as a query sequence. Moreover, genes encoding homologs of the above-indicated enzyme can be obtained by, for example, PCR using yeast chromosomes or a bacterial chromosome as a template and oligonucleotides produced based on those known gene sequences as primers.

Genes encoding conservative variants of the above-indicated enzyme may be, for example, genes as described below. That is, a gene encoding the enzyme may be a gene encoding a protein having the above-described amino acid sequence in which one or several amino acids are substituted, deleted, inserted, or added at one position or several positions in the amino acid sequence, as long as the gene encodes a protein retaining the original function. In this case, the corresponding activity can be retained normally 60% or greater, preferably 70% or greater, more preferably 80% or greater, further preferably 90% or greater in relative to the activity of the protein before one or several amino acids are substituted, deleted, inserted, or added. Incidentally, "1 to 10" in the above means preferably 1 to 5 amino acids.

The above-described substitution, deletion, insertion, or addition of 1 to 10 amino acids is a conservative mutation that retains the normal function of the protein. Conservative substitution is a representative of conservative mutations. A conservative substitution is a mutation in which substitution occurs mutually among Phe, Trp, and Tyr when the substitution site is occupied with an aromatic amino acid, among Leu, Ile, and Val when the substitution site is occupied with a hydrophobic amino acid, between Gln and Asn when the substitution site is occupied with a polar amino acid, among Lys, Arg, and His when the substitution site is occupied with a basic amino acid, between Asp and Glu when the substitution site is occupied with an acidic amino acid, and between Ser and Thr when the substitution site is occupied with an amino acid having a hydroxyl group. Specifically, examples of a substitution considered to be a conservative substitution include a substitution from Ala to Ser or Thr; a substitution from Arg to Gln, His or Lys; a substitution from Asn to Glu, Gln, Lys, His or Asp; a substitution from Asp to Asn, Glu or Gln; a substitution from Cys to Ser or Ala; a substitution from Gln to Asn, Glu, Lys, His, Asp or Arg; a substitution from Glu to Gly, Asn, Gln, Lys or Asp; a substitution from Gly to Pro; a substitution from His to Asn, Lys, Gln, Arg or Tyr; a substitution from Ile to Leu, Met, Val or Phe; a substitution from Leu to Ile, Met, Val or Phe; a substitution from Lys to Asn, Glu, Gln, His or Arg; a substitution from Met to He, Leu, Val or Phe; a substitution from Phe to Trp, Tyr, Met, Ile or Leu; a substitution from Ser to Thr or Ala; a substitution from Thr to Ser or Ala; a substitution from Trp to Phe or Tyr; a substitution from Tyr to His, Phe or Trp; and a substitution from Val to Met, Ile or Leu. Moreover, examples of the amino acid substitution, deletion, insertion, addition, inversion or the like as described above also include naturally occurring mutations based on the individual difference in the organism from which the gene is derived or on the differences of species (mutant or variant).

Furthermore, a gene having a conservative mutation as described above may be a gene encoding a protein that has a homology of 80% or greater, preferably 90% or greater, more preferably 95% or greater, further preferably 97% or greater, and especially preferably 99% or greater to the entirety of the above-described amino acid sequence and retains the original function. Incidentally, "homology" herein may refer to "identity".

Moreover, a gene encoding the enzyme may be a DNA which hybridizes under stringent conditions to a probe that can be prepared from a known gene sequence, for example, a sequence complementary to the entirety or a portion of the above-described nucleotide sequence and encodes a protein retaining the original function. The term "stringent conditions" refers to conditions under which a so-called specific hybrid is formed and no non-specific hybrid is formed. Examples of the stringent conditions can include, for one example, conditions under which DNAs having a high homology, for example, DNAs having a homology of 80% or greater, preferably 90% or greater, more preferably 95% or greater, still more preferably 97% or greater, and especially preferably 99% or greater hybridize to each other and DNAs having a lesser homology do not hybridize to each other, or conventional conditions for washing in Southern hybridization, that is, conditions consisting of washing once, preferably twice to three times with a salt concentration and at a temperature corresponding to washing at 60°C with 1 x SSC, 0.1% SDS, preferably washing at 60°C with 0.1 x SSC, 0.1% SDS, and more preferably washing at 68°C with 0.1 x SSC, 0.1% SDS.

As mentioned above, a probe used in the above-described hybridization may be a portion of a complementary sequence of the gene. Such a probe can be produced by PCR using oligonucleotides produced based on the known gene sequence as primers and a DNA fragment containing the nucleotide sequences of these primers as a template. For example, in cases where a DNA fragment with a size of around 300 bp is used as a probe, examples of the hybridization washing conditions include washing at 50°C with 2 x SSC, 0.1% SDS.

Moreover, a gene encoding the enzyme may be a gene in which any codon has been replaced with a synonymous codon, as long as it encodes a protein retaining the original function. For example, a gene encoding the enzyme of the present invention may be a gene modified to have the optimal codons according to the codon usage of a host to be used.

Incidentally, the description related to the conservative variants of the above-described gene and protein can also be applied in terms of any proteins described below and genes encoding them.

Alginate importer means a protein having a function in the uptake of alginic acid into cytoplasm from the outside of a cell.

Alginate importers derived from bacteria in the genera *Azotobacter*, *Pseudomonas*, *Cobetia*, *Sphingomonas*, *Klebsiella*, *Alteromonas*, *Rhodobacter*, and *Shewanella* are preferable and, among those, alginate importers of bacteria in the genus *Vibrio* can be used. For example, *Vibrio alginolytics*, *Vibrio splendidus*, *Vibrio kanaloaei*, *Vibrio pomeroyi*, *Vibrio chagasii*, *Vibrio lentus*, *Vibrio cyclitrophicus*, *Vibrio crassostreae*, *Vibrio hemicentroti*, *Vibrio* sp. JAM-A9m, and *Vibrio alginovora* can be used.

Alginate importers exist in both the outer and inner membranes and examples of the protein existing in the outer membrane include, for example, the KdgN protein, which is a component of porin.

As one example, the sequence of the *kdgN* gene encoding KdgN of the strain NITE BP-01635 in the genus *Vibrio* is represented by SEQ ID NO: 14 and the KdgN protein is represented by SEQ ID NO: 15.

Examples of the protein existing in the inner membrane include ToaA, ToaB, and ToaC. For example, the *toaA* gene encoding ToaA of the strain NITE BP-01635 in the genus *Vibrio* is represented by SEQ ID NO: 2 and the ToaA protein is represented by SEQ ID NO: 3. The *toaB* gene encoding ToaB of the strain NITE BP-01635 is represented by SEQ ID NO: 16 and the ToaB protein is represented by SEQ ID NO: 17. The *toaC* gene encoding ToaC of the strain NITE BP-01635 is represented by SEQ ID NO: 18 and the ToaC protein is represented by SEQ ID NO: 19.

An increased alginate uptake activity can be confirmed, for example, by culturing each of microorganisms before and after modification in a culture medium containing alginic acid and measuring the intracellular and extracellular concentrations of alginic acid with the carbazole sulfate method (Methods of Biochemical Experiments, Volume 1. Methods for Quantification of Reducing Sugars). *Escherichia coli* W3110 and MG1655 and *Corynebacterium glutamicum* ATCC13869 and ATCC13032 can be used as subjects for comparison.

Keto acid reductase means an enzyme involved in a pathway in which uronic acid polysaccharides produced by an alginate lyase is degraded to pyruvic acid and glyceraldehyde-3-phosphate, and specifically means an enzyme selected from NADPH/NADH dehydrogenase, D-mannonate dehydratase, 2-keto-3-deoxygluconokinase, and 2-keto-3-deoxy-6-phosphogluconate aldolase.

NADPH/NADH dehydrogenase means an enzyme which reduces 4-deoxy-L-erythro-5-hexoseulose uronic acid (abbreviation of the compound: DEH or DEHU) to 2-keto-3-deoxy-D-gluconate (abbreviation of the compound: 2-keto-3-deoxygluconate or KDG) by using the reducing power of NADPH or NADH.

Enhancement of NADPH/NADH dehydrogenase can be confirmed, for example, by preparing a crude enzyme solution from each of microorganisms before and after modification and comparing the NADPH/NADH dehydrogenase activity in the crude enzyme solutions. The increase of the NADPH/NADH dehydrogenase activity can be determined by measuring the activity according to the method of Wargacki et al., (Science 335, 308 2012). *Escherichia coli* W3110 and MG1655 and *Corynebacterium glutamicum* ATCC13869 and ATCC13032 can be used as subjects for comparison.

NADPH/NADH dehydrogenase is encoded by the *dehR* gene and the *dehR* gene is preferably obtained from a microorganism having an ability to assimilate alginic acid and is preferably derived from bacteria in the genera *Azotobacter*, *Pseudomonas*, *Cobetia*, *Sphingomonas*, *Klebsiella*, *Alteromonas*, *Rhodobacter*, and *Shewanella*, and, among those, NADPH/NADH dehydrogenases of bacteria in the genus *Vibrio* can be used. For example, *Vibrio alginolytics*, *Vibrio splendidus*, *Vibrio kanaloaei*, *Vibrio pomeroyi*, *Vibrio chagasii*, *Vibrio lentus, Vibrio cyclitrophicus*, *Vibrio crassostreae*, *Vibrio hemicentroti*, *Vibrio* sp. JAM-A9m, and *Vibrio alginovora* can be used.

Particularly, the *dehR* genes derived from bacteria in the genus *Vibrio* are preferably used. As one example, the *dehR* gene of the strain NITE BP-01635 in the genus *Vibrio* is represented by SEQ ID NO: 22 and the DehR protein is represented by SEQ ID NO: 23.

D-mannonate dehydratase means an enzyme represented by the EC Number: 4.2.1.8, which dehydrates D-mannuronic acid and produces 2-dehydro-3-deoxy-D-gluconate. D-Mannonate dehydrogenase is also referred to as mannuronate dehydrogenase, or D-mannonate hydrolyase.

An increased D-mannonate dehydratase activity can be confirmed, for example, by preparing a crude enzyme solution from each of microorganisms before and after modification and comparing the D-mannonate dehydratase activity in the crude enzyme solutions. The increase of the D-mannonate dehydratase activity can be determined by measuring the activity through the quantification of 2-dehydro-3-deoxy-D-gluconate (Methods Enzymol. 1982 (90) p288-94). *Escherichia coli* W3110 and MG1655 and *Corynebacterium glutamicum* ATCC13869 and ATCC13032 can be used as subjects for comparison.

D-mannonate dehydratase is found widely in microorganisms belonging to the family Enterobacteriaceae and microorganisms having an ability to assimilate alginic acid and the genes derived from the genera *Escherichia*, *Enterobacter*, *Pantoea*, and *Vibrio* can be used. Particularly D-mannonate dehydratases derived from bacteria in the genus *Vibrio* and *Escherichia coli* are preferably used.

A gene encoding a D-mannonate dehydratase derived from *Escherichia coli, uxuA*, is represented by SEQ ID NO: 54 and the UxuA protein is represented by SEQ ID NO: 55.

2-keto-3-deoxygluconokinase refers to an enzyme represented by the EC Number: 2.7.1.178/2.7.1.45, which reversibly catalyzes a reaction to produce 2-keto-3-deoxy-6-phosphogluconate from 2-dehydro-3-deoxy-D-gluconate. The 2-keto-3-deoxygluconokinase activity is also referred to as ketodeoxygluconokinase or KDG kinase.

An increased 2-keto-3-deoxygluconokinase activity can be confirmed, for example, by preparing a crude enzyme solution from each of microorganisms before and after modification and comparing the 2-keto-3-deoxygluconokinase activity in the crude enzyme solutions.

An increased 2-keto-3-deoxygluconokinase activity can be confirmed by a method to enzymatically measure the increase of ADP, which method is described in the article of Mandran-Berthlot M-A et al., 1984, Journal of Bacteriology Vol.160: p600-606. *Escherichia coli* W3110 and MG1655 and *Corynebacterium glutamicum* ATCC13869 and ATCC13032 can be used as subjects for comparison.

2-keto-3-deoxygluconokinase is found widely in microorganisms belonging to the family Enterobacteriaceae and microorganisms having an ability to assimilate alginic acid and the genes derived from the genera *Escherichia*, *Enterobacter*, *Pantoea*, and *Vibrio* can be used. In the present invention, particularly 2-keto-3-deoxygluconokinases derived from bacteria in the genus *Vibrio* and *Escherichia coli* are preferably used.

The *kdgK* gene sequence encoding a 2-keto-3-deoxygluconokinase derived from the strain NITE BP-01635 in the genus *Vibrio* is represented by SEQ ID NO: 8 and the KdgK protein is represented by SEQ ID NO: 9.

The *kdgK* gene sequence encoding a 2-keto-3-deoxygluconokinase of *Escherichia coli* is represented by SEQ ID NO: 56 and the KdgK protein is represented by SEQ ID NO: 57.

2-keto-3-deoxy-6-phosphogluconate aldolase means an enzyme referred to as EDA and represented by the EC Number: 4.1.2.14, which degrades 2-keto-3-deoxy-6-phosphogluconate (2-keto-3-deoxygluconate 6-phosphate) into pyruvic acid and D-glyceraldehyde-3-phosphate (also referred to as glyceraldehyde 3-phosphate).

An increased 2-keto-3-deoxy-6-phosphogluconate aldolase (hereinafter referred to as EDA) activity can be confirmed, for example, by preparing a crude enzyme solution from each of microorganisms before and after modification and comparing the EDA activity in the crude enzyme solutions.

The EDA activity can be determined by using the absorbance at 340 nm of the NADH in a reaction of the produced pyruvate and a commercially available LDH enzyme. *Escherichia coli* W3110 and MG1655 and *Corynebacterium glutamicum* ATCC13869 and ATCC13032 can be used as subjects for comparison.

Genes for 2-keto-3-deoxy-6-phosphogluconate aldolase derived from Gram-negative bacteria having the Entner-Doudoroff pathway can be used. Examples of such bacteria include *Escherichia coli*, bacteria in the genus *Vibrio,* and bacteria in the genus *Pantoea. Corynebacterium glutamicum* also have an EDA.

Examples of the *EDA* gene in *Escherichia coli* include the *eda* gene represented by SEQ ID NO: 58 and the EDA protein represented by SEQ ID NO: 59.

As the *eda* gene derived from the strain NITE BP-01635 in the genus *Vibrio*, the gene represented by SEQ ID NO: 6 and the protein represented by SEQ ID NO: 7 can be used.

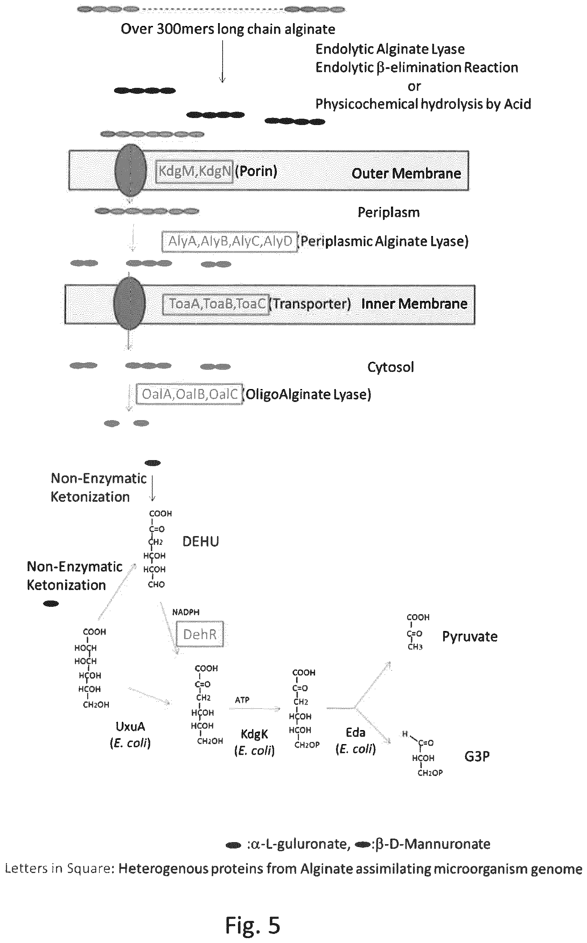

In the present invention, any one or more characteristics of
i) increasing the alginate lyase activity,
ii) increasing the alginate importer activity,
iii) increasing the keto acid reductase activity
are desirably imparted and, more preferably, all of those characteristics are preferably imparted. A model of the alginate assimilatory metabolic pathway in a microorganism to which all the characteristics have been imparted is shown in Figure 5, in which *Escherichia coli* is used as an example.

### <1-3> Procedures to increase the activity of a protein

Now, procedures to increase the activity of a protein will be described below.

The phrase "the activity of a protein increases" means that the activity of the same protein is increased compared with those in unmodified strains such as a wild-type strain, a parent strain and the like. Incidentally, "the activity of a protein increases" is also expressed as "the activity of a protein is increased". The phrase "the activity of a protein increases" refers to, specifically, an increase of the same protein in molecular number per cell and/or an increase of the function of the same protein per molecule as compared with unmodified strains. That is, "the activity" in the phrase "the activity of a protein increases" is not limited to the catalytic activity of the protein but may mean the transcription level (the amount of mRNA) of a gene encoding the protein or the amount of the protein. Moreover, the phrase "the activity of a protein increases" includes not only increasing the activity of the same protein in bacterial strains originally having the activity of the target protein but also providing the activity of the same protein to bacterial strains originally having no activity of the target protein. Moreover, a preferable activity of a target protein may be provided to a microorganism after reducing or attenuating the activity of the target protein intrinsically possessed by the microorganism, as long as the activity of the protein is eventually increased. Incidentally, "protein" is synonymous with enzyme.

The activity of a protein is not particularly limited as long as the activity is increased as compared with unmodified strains, but the activity may be increased, for example, 1.5 times or more, 2 times or more, or 3 times or more as compared with unmodified strains. Moreover, when an unmodified strain does not have the activity of a target protein, the same protein should be produced by introducing a gene encoding the same protein and, for example, the same protein may be produced to such a degree that the enzymatic activity can be determined.

Such a modification as to increase the activity of a protein can be achieved, for example, by increasing the expression of a gene encoding the same protein. Incidentally, "the expression of a gene is increased" is also expressed as "the expression of a gene is enhanced". The expression of a gene may be increased, for example, 1.5 times or more, 2 times or more, or 3 times or more as compared with unmodified strains. Moreover, the phrase "the expression of a gene is increased" includes not only increasing the expression level of the same gene in bacterial strains originally having the expression of the target gene but also expressing the same gene in bacterial strains originally having no expression of the target gene. That is, the phrase "the expression of a gene is increased" includes, for example, introducing a target gene into bacterial strains not carrying the same gene to express the same gene.

An increased expression of a gene can be achieved, for example, by increasing the copy number of the gene.

An increase of the copy number of a gene can be achieved by introducing the same gene into the chromosome of a host microorganism. The introduction of a gene into a chromosome can be performed, for example, by using homologous recombination (MillerI, J. H. Experiments in Molecular Genetics, 1972, Cold Spring Harbor Laboratory). Only one copy of a gene or two or more copies of a gene may be introduced. For example, multiple copies of a gene can be introduced into a chromosome by homologous recombination, which targets multiple copies of a sequence on the chromosome. Examples of a sequence which exists on the chromosome in multiple copies include repetitive DNA sequences (repetitive DNA) and inverted repeats that are present at both ends of transposons. Moreover, homologous recombination may be performed by targeting a suitable sequence of a gene and the like on the chromosome which is not required for the production of a substance of interest. Homologous recombination can be performed by, for example, methods using a linear DNA fragment, such as the Red-driven integration method (Datsenko, K. A, and Wanner, B. L. Proc. Natl. Acad. Sci. USA. 97:6640-6645 (2000)) and the like; a method using a plasmid containing a thermosensitive replication origin, a method using a plasmid capable of being transferred by conjugation, a method using a suicide vector without a replication origin functional in a host, or a transduction method using a phage. Moreover, a gene may be introduced randomly into a chromosome by using a transposon or Mini-Mu (Japanese Unexamined Patent Application Publication No. Hei 2-109985; US 5,882,888; EP 805867B1).

Introduction of a target gene into the chromosome can be confirmed by Southern hybridization using a probe having a complementary sequence of the entirety or a portion of the same gene or by PCR using primers produced based on the sequence of the same gene, and the like.

Moreover, an increase of the copy number of a gene can also be achieved by introducing a vector containing the target gene into a host microorganism. For example, a DNA fragment containing the target gene is linked to a vector functional in a host microorganism to construct a vector for the expression of the same gene and the host microorganism is transformed with the same expression vector and thereby the copy number of the same gene can be increased. The DNA fragment containing the target gene can be obtained, for example, by PCR using as a template the genomic DNA of a microorganism having the target gene. As the vector, a vector which is able to autonomously replicate in cells of a host microorganism can be used. The vector is preferably a multi-copy vector. Moreover, the vector preferably carries a marker such as an antibiotic resistance gene to select transformants. The vector may be, for example, a vector originated from a bacterium plasmid, a vector originated from a yeast plasmid, a vector derived from a bacteriophage, cosmid, or phagemid, or the like. Specifically, examples of a vector capable of autonomously replicating in bacteria in the family Enterobacteriaceae such as *Escherichia coli* include, for example, pUC19, pUC18, pHSG299, pHSG399, pHSG398, pACYC184, pBR322, pSTV29 (all of which are available from Takara Bio Inc.), pMW219 (Nippon Gene Co., Ltd.), pTrc99A (Pharmacia Biotech Inc.), pPROK-series vectors (Clontech Laboratories Inc.), pKK233-2 (manufactured by Clontech Laboratories Inc.), pET-series vectors (Novagen), pQE-series vectors (QIAGEN Inc.), and the broad-host-range vector RSF1010. Specifically, examples of a vector capable of autonomously replicating in a coryneform bacterium include, for example, pHM1519 (Agric, Biol. Chem., 48, 2901-2903(1984)); pAM330 (Agric. Biol. Chem., 48, 2901-2903(1984)); plasmids improved from those plasmids and carrying a drug resistance gene; the plasmid pCRY30, which is described in Japanese Unexamined Patent Application Publication No. Hei 3-210184; the plasmids pCRY21, pCRY2KE, pCRY2KX, pCRY31, pCRY3KE and pCRY3KX, which are described in Japanese Unexamined Patent Application Publication No. Hei 2-72876 and U.S. Patent No. 5,185,262; the plasmids pCRY2 and pCRY3, which are described in Japanese Unexamined Patent Application Publication No. Hei 1-191686; pAJ655, pAJ611 and pAJ1844, which are described in Japanese Unexamined Patent Application Publication No. Sho 58-192900; pCG1, which is described in Japanese Unexamined Patent Application Publication No. Sho 57-134500; pCG2, which is described in Japanese Unexamined Patent Application Publication No. Sho 58-35197; and pCG4 and pCG11, which are described in Japanese Unexamined Patent Application Publication No. Sho 57-183799.

In cases where a gene is introduced, the gene may be maintained in such a state that the gene can be expressed in the bacterium used in the present invention. Specifically, a gene may be introduced such that the gene is expressed under the control of a promoter sequence functional in the bacterium used in the present invention. The promoter may be a host-derived promoter or a heterogeneous promoter. The promoter may be a promoter intrinsic to a gene to be introduced or a promoter of a different gene. As a promoter, for example, a stronger promoter as described below may also be used.

Moreover, in cases where two or more genes are introduced, the genes may be maintained in such a state that each gene can be expressed in the bacterium used in the present invention. For example, each of all genes may be maintained on a single expression vector or on the chromosome. Moreover, each of all genes may be maintained separately on plural expression vectors or on a single or plural expression vectors and the chromosome. Moreover, an operon composed of two or more genes may be introduced. The phrase "cases where two or more genes are introduced" includes, for example, a case where genes encoding two or more enzymes, respectively, are introduced, a case where genes encoding two or more subunits that compose a single enzyme, respectively, are introduced, and a combination thereof.

A gene to be introduced is not particularly limited as long as it encodes a protein functional in a host. The gene to be introduced may be a host-derived gene or a heterogeneous gene. The gene to be introduced can be obtained by PCR, for example, using primers designed based on the nucleotide sequence of the same gene and the genomic DNA of an organism having the same gene or a plasmid carrying the same gene and the like as a template. Moreover, the entirety of the gene to be introduced may be synthesized, for example, based on the nucleotide sequence of the same gene.

Incidentally, in cases where a protein functions in a multisubunit complex, all or only a part of those plural subunits may be modified as long as the activity of the protein is eventually increased. That is, for example, in cases where the activity of the protein is increased by increasing the gene expression, the gene expression may be increased in all of plural genes that encode those subunits or in only a part of the genes. Typically, the gene expression is preferably increased in all of the plural genes that encode those subunits. Moreover, each of subunits that compose a complex may be derived from one kind of organism or from two or more different kinds of organisms as long as the complex has a protein function of interest. That is, for example, genes which encode plural subunits and are derived from the same organism may be introduced into a host or genes each derived from a different organism may be introduced into a host.

Moreover, an increased expression of a gene can be achieved by increasing the transcription efficiency of the gene. An increased transcription efficiency of a gene can be achieved, for example, by replacing the promoter of the gene on the chromosome with a stronger promoter. The term "stronger promoter" means a promoter which enhances the transcription of a gene than the originally existing wild-type promoter. Examples of a stronger promoter include, for example, T7 promoter, *trp* promoter, *lac* promoter, *thr* promoter, *tac* promoter, *trc* promoter, and PL promoter, all of which are known high expression promoters. Moreover, when a coryneform bacterium is used, examples of a promoter that can be used include the artificially designed and modified P54-6 promoter (Appl. Microbiol. Biotechnolo., 53, 674-679 (2000)); the *pta*, *aceA*, *aceB*, *adh*, and *amyE* promoters, which can be induced by acetic acid, ethanol, pyruvic acid and the like in coryneform bacteria; the *cspB*, *SOD*, and *tuf* promoters, which are strong promoters of coryneform bacteria exhibiting high expression levels (Journal of Biotechnology 104 (2003) 311-323, Appl Environ Microbiol. 2005 Dec; 71(12):8587-96.); and the like. Moreover, as a stronger promoter, a highly active form of a conventional promoter may be obtained by using various reporter genes. For example, the activity of a promoter can be increased by modifying the -35 region and the -10 region within a promoter region to have sequences close to the consensus sequences (WO00/18935). Examples of a highly active promoter include various *tac*-like promoters (Katashkina JI *et al.* Russian Federation Patent application 2006134574) and *pnlp8* promoter (WO2010/027045). Examples of methods to evaluate the promoter strength and of strong promoters are described in the article of Goldstein et al., (Prokaryotic promoters in biotechnology. Biotechnol. Annu. Rev., 1, 105-128 (1995)), and the like.

Moreover, an increased expression of a gene can be achieved by increasing the translation efficiency of the gene. An increased translation efficiency of a gene can be achieved, for example, by replacing the Shine-Dalgarno (SD) sequence (also referred to as ribosome binding site (RBS)) of the gene on the chromosome with a stronger SD sequence. The term "stronger SD sequence" means a SD sequence which enhances the translation of mRNA than the originally existing wild-type SD sequence. Examples of a stronger SD sequence include, for example, the RBS of gene 10 from the phage T7 (Olins P. O. et al, Gene, 1988, 73, 227-235). Furthermore, it has been known that a substitution, insertion, or deletion of several nucleotides in the spacer region between the RBS and the initiator codon, particularly in the sequence immediately upstream of the initiator codon (5'-UTR) make a severe influence on the mRNA stability and the translation efficiency and the translation efficiency of the gene may also be increased by modifying those regions.

Elements which affect the gene expression, such as promoter, SD sequence, and the spacer region between the RBS and the initiator codon, are also referred to collectively as "expression regulatory region". Expression regulatory regions can be identified by using a promoter search vector or gene analysis software programs such as GENETYX and the like. Modification of these expression regulatory regions can be performed, for example, by a method using a thermosensitive vector or the Red-driven integration method (WO2005/010175).

An increased translation efficiency of a gene can also be achieved, for example, by codon modification. A clear bias in codon usage is present among 61 amino acid codons observed in a population of mRNA molecules in *Escherichia coli* and the like and the amount of a certain tRNA seems to be directly proportional to the corresponding codon usage frequency (Kane, J.F., Curr. Opin. Biotechnol., 6(5), 494-500 (1995)). That is, translational problems can be created when mRNA containing an excess of rare codons is abundant. Recent studies have suggested that especially clusters of AGG/AGA, CUA, AUA, CGA, or CCC codons can reduce both the quantity and quality of a synthesized protein. Such a problem can arise particularly during the expression of a heterogeneous gene. Thus, when a heterogeneous gene and the like are expressed, the translation efficiency of the gene can be increased by replacing rare codons present in the gene with synonymous codons which are more frequently used. The replacement of codons can be performed, for example, by a site-directed mutagenesis method, in which a mutation of interest is introduced into a DNA site of interest. Examples of a site-directed mutagenesis method include a method using PCR (Higuchi, R., 61, in PCR technology, Erlich, H. A. Eds., Stockton press (1989); Carter, P., Meth. in Enzymol., 154, 382 (1987)) and a method using a phage (Kramer, W. and Frits, H. J., Meth. in Enzymol., 154, 350 (1987); Kunkel, T. A. et al., Meth. in Enzymol., 154, 367 (1987)). Moreover, the entirety of a gene fragment carrying replaced codons may be synthesized. Codon usage frequencies in various organisms are disclosed in "the Codon Usage Database" (http://www.kazusa.or.jp/codon; Nakamura, Y. et al, Nucl. Acids Res., 28, 292 (2000)).

Moreover, an increased expression of a gene can also be achieved by amplifying such a regulator as to increase the expression of the gene or by deleting or weakening such a regulator as to reduce the expression of the gene.

The procedures as described above to increase the gene expression may be used individually or in any combination thereof.

Moreover, such a modification as to increase the activity of a protein can also be achieved, for example, by increasing the specific activity of the protein. Reduction of the feedback inhibition and desensitization to the feedback inhibition are also included in the increase of specific activity. A protein having an increased specific activity can be obtained, for example, by examining various organisms. Moreover, a conventional protein may be mutagenized to obtain a highly active variant of the protein. Examples of a mutation to be introduced may include, for example, a substitution, deletion, insertion, or addition of 1 to 10 amino acids at 1 to 10 positions of a protein. The introduction of a mutation can be performed, for example, by the site-directed mutagenesis method as mentioned above. Moreover, the introduction of a mutation may be performed, for example, by a mutation treatment. Examples of the mutation treatment include irradiation of X-rays, irradiation of ultraviolet rays, as well as a treatment with a mutation agent such as N-methyl-N'-nitro-N-nitrosoguanidine (MNNG), ethyl methanesulfonate (EMS), methyl methanesulfonate (MMS) and the like. Moreover, random mutations may be induced by direct treatment of DNA with hydroxylamine *in vitro.* The increase of specific activity may be employed alone or in any combination with the procedures as described above to increase the gene expression.

A method used for transformation is not particularly limited but a conventionally known method may be used. For example, a method as reported for *Escherichia coli* K-12, in which recipient bacterial cells are treated with calcium chloride to increase the penetrating property of DNA (Mandel, M. and Higa, A., J. Mol. Biol. 1970, 53, 159-162), or a method as reported for *Bacillus subtilis*, in which competent cells are prepared from cells in the propagation stage and DNA is introduced thereto (Duncan, C. H., Wilson, G. A. and Young, F. E.., 1997. Gene 1: 153-167), can be used. Alternatively, such a method as to be known for *Bacillus subtilis*, actinomycetes and yeasts can also be applied, in which cells of a DNA-receiving germ are converted into protoplasts or spheroplasts, into which recombinant DNA is easily taken, followed by introduction of recombinant DNA into the DNA-receiving germ (Chang, S. and Choen, S.N., 1979.Mol. Gen. Genet. 168: 111-115; Bibb, M. J., Ward, J. M. and Hopwood, O. A. 1978.Nature 274: 398-400; Hinnen, A., Hicks, J. B. and Fink, G. R. 1978. Proc. Natl. Acad. Sci. USA 75: 1929-1933).

An increased activity of a protein can be confirmed by measuring the activity of the same protein.

An increased activity of a protein can also be confirmed by identifying an increased expression of a gene encoding the same protein. An increased expression of a gene can be confirmed by identifying an increased transcription level of the same gene or by identifying an increased amount of a protein expressed by the same gene.

An increased transcription level of a gene can be identified by comparing the amount of mRNA transcribed from the same gene with that of an unmodified strain, such as a wild-type strain or parent strain. Examples of a method to evaluate the amount of mRNA include Northern hybridization, RT-PCR, and the like (Sambrook, J., et al., Molecular Cloning A Laboratory Manual/Third Edition, Cold spring Harbor Laboratory Press, Cold spring Harbor (USA), 2001). The amount of the mRNA may be increased, for example, 1.5 times or more, 2 times or more, or 3 times or more as compared with unmodified strains.

An increased amount of a protein can be identified by Western blotting using an antibody (Molecular cloning (Cold spring Harbor Laboratory Press, Cold spring Harbor (USA), 2001)). The amount of the protein may be increased, for example, 1.5 times or more, 2 times or more, or 3 times or more as compared with unmodified strains.

### <2> Manufacturing method of the present invention

The manufacturing method of the present invention is a method of producing an L-amino acid by a fermentation method comprising: culturing a bacterium modified from one of the above-described bacteria to have an ability to produce the L-amino acid and an ability to assimilate alginic acid in a culture medium containing alginic acid as a carbon source obtained from seaweed-derived biomass, or containing alginic acid as a carbon source obtained from seaweed-derived biomass which alginic acid is subjected to an enzymatic degradation reaction by an alginate lyase or an acid hydrolysis reaction, to produce and accumulate the L-amino acid in the culture medium; and collecting the L-amino acid from the culture medium.

In the present invention, the term "seaweed" means large algae called macroalgae and refers to green algae such as sea lettuce, green laver; red algae such as *Pyropia* sp., *Gracilaria vermiculophylla*; brown algae such as kombu, *Eisenia bicyclis*, *Ecklonia cava*, *Undaria pinnatifida*, *Sargassum fulvellum*; and the like, and combinations of plural kinds of seaweed selected from those. Brown algae are particularly preferable. Brown algae are preferable because of abundant storage of mannitol, a sugar alcohol, among the primary assimilation products produced by photosynthesis. Examples of a brown alga that can be used in the present invention include brown algae belonging to the orders Scytosiphonales, Ectocarpales, Dictyosiphonales, Chordariales, Ralfsiales, Sporochnales, Desmarestiales, Laminariales, Cutleriales, Syringodermatales, Sphacelariales, Dictyotales, Tilopteridales, Ascoseirales, Fucales, and Durvilaeales in the class Phaeophyceae, and *Laminaria japonica* is preferable. Because *Laminaria japonica* as a marine biomass material is produced quite abundantly and is large in size, *Laminaria japonica* can be supplied steadily and abundantly as a raw material for the production of a substance of interest.

In the present invention, although either raw seaweed or dried seaweed may be used, raw seaweed is preferably used. Using raw seaweed is advantageous because it allows the exclusion of a step to dry seaweed, which requires quite high energy consumption, and, moreover, almost completely eliminates the necessity to add water in the step to obtain a saccharified liquid of seaweed. Inorganic salts in seaweed can be extracted by immersing seaweed in water and allowing the seaweed to swell. In the present invention, seaweed may be swollen as necessary.

In the present invention, seaweed may be cleaved into pieces as necessary. Seaweed can be cleaved into pieces by a known method and may be cleaved into pieces with a desired size by using, for example, scissors, a mixer, sonication, a French press, a stone mill, a mortar, a homogenizer, glass beads, a milling machine, and the like.

Carbon sources such as alginic acid, cellulose, mannitol, pectin, galacturonic acid, carrageenan, agar, and the like included in seaweed can be obtained from seaweed by a conventional method. For example, alginic acid can be obtained by the method below.

### (1) Extraction step

Seaweed raw materials are washed well, followed by the addition of an alkaline material and heating to solubilize alginic acid in the algal body. Alginic acid included in seaweed forms salts with minerals included in seawater and the salts in the form of insoluble gel are filled in the space between cell walls. The addition of a sodium salt to seaweed exchanges the insoluble salts of alginic acid with watersoluble sodium alginate, which is released into the outside of the algal body.

### (2) Filtration step

After a sufficient amount of the alginic acid is released into a solution, the solution is filtrated to remove insoluble components and thereby a solution of sodium alginate is obtained. The extracted liquid is highly viscous because of the viscosity of the solubilized alginic acid. To this liquid, a large amount of water is added to reduce the viscosity and to filtrate the liquid.

### (3) Deposition

An addition of an acid to the solution of sodium alginate decreases the pH of the solution and results in the deposition of insoluble alginic acid again. Using a calcium salt instead of an acid can also result in the deposition of calcium alginate, which is similarly insoluble.

### (4) Drying

After the deposited alginic acid was dehydrated, it was washed well and dried to obtain alginic acid. Neutralization of this alginic acid with an alkaline material produces an alginic acid salt. Sodium alginate and potassium alginate are produced by using sodium and potassium as an alkaline material for the neutralization, respectively.

Alginic acid obtained by a process as described above is preferably used as a carbon source in a culture medium after the alginic acid is subjected to an enzymatic degradation reaction with an alginate lyase or an acid hydrolysis reaction.

For example, to obtain from seaweed a carbon source that is a main raw material for fermentation, seaweed is subjected to an acid treatment by adding an acid, including an inorganic acid such as hydrochloric acid or phosphoric acid; a sulfonic acid such as sulfuric acid; or a carboxylic acid such as acetic acid or formic acid, to the seaweed and thereby a saccharified liquid of seaweed can be obtained, in which the degradation of polysaccharides in the seaweed, that is, saccharification has been induced.

Moreover, to obtain from seaweed a carbon source that is a main raw material for fermentation, seaweed is preferably subjected to an enzyme treatment with an alginate lyase (EC.4.2.2.3) or lambda-carrageenase (EC 3.2.1.162) or pectinase, or an enzyme having the enzymatic activity of cellulase. The enzyme treatment enables a saccharified liquid of seaweed to be obtained, in which the degradation of polysaccharides in the seaweed, that is, saccharification has been induced.

The cleavage of seaweed into pieces, swelling, acid treatment, enzyme treatment, which are steps to obtain the saccharified liquid of seaweed mentioned above, may be performed individually in a sequential manner or any of the steps may be performed simultaneously. Moreover, these steps may be performed in different orders.

Among alginate lyases, any of the alginate lyase mentioned above can be used and an alginate lyase expressed directly in a microorganism may be used or an alginate lyase isolated independently may be used.

Among pectinases, a polygalacturonase, pectin lyase, pectin esterase, pectin methyl esterase and the like can be used.

Cellulose included in seaweed can be degraded by adding a cellulase after the extraction and separation of polysaccharides including alginic acid. In the present invention, cellulase is also referred to as endo-1,4-β-glucanase and means an enzyme which hydrolyzes β1→4 glucoside linkages in cellulose and mainly produces cellobiose. Cellulases are present in higher plants, bacteria, filamentous fungi, wood rotting fungus, molluscs and the like. In the present invention, the source of cellulase is not limited but cellulases extracted and purified from microorganisms or cellulases produced by molecular biological techniques may be similarly used.

The bacterium used in the present invention can assimilate alginic acid salts purified by the extraction step as illustrated above. In the present invention, the alginic acid salts mean sodium alginate and potassium alginate and the bacterium used in the present invention is preferably a bacterium capable of growing with alginic acid and alginic acid salt as a main carbon source.

As a culture medium to be used, a culture medium conventionally used in the production of a substance of interest by fermentation using microorganisms can be used. That is, a conventional culture medium containing a carbon source, a nitrogen source, inorganic ions and, as necessary, other organic components can be used. Here, a carbon source obtained from seaweed can be used as a carbon source.

Examples of a carbon source obtained from seaweed include alginic acid, cellulose, glucose, mannitol, pectin, galacturonic acid, carrageenan, and agar. For example, typical brown algae contain alginic acid, mannitol and glucose in a ratio of 5:8:1 (Wargacki, et al. (2012) Science. 335. p308-313).

The culture medium may contain other sugar sources, such as glucose, sucrose, fructose, glycerol, or ethanol, as long as the culture medium contains a carbon source obtained from seaweed.

Inorganic ammonium salts such as ammonium sulfate, ammonium chloride, ammonium phosphate; organic nitrogen such as soybean hydrolysates; ammonia gas, ammonia water, and the like can be used as a nitrogen source. Suitable amounts of essential materials such as vitamin B₁, L-homoserine and the like, or yeast extract or the like are desirably contained in the culture medium as organic trace nutrients. In addition to these, small amounts of potassium phosphate, magnesium sulfate, ferric ions, manganese ions and the like are added as necessary. Moreover, when the bacterium used in the present invention is cultured, a certain concentration of salt is still further preferably contained in the culture medium. The salt may be a salt formed by binding of a substance of interest to a counterion or may be table salt (NaCl). Incidentally, the culture medium used in the present invention may be either a natural medium or a synthetic medium as long as it is a medium containing a carbon source, a nitrogen source, inorganic ions and, as necessary, other organic trace components.

Moreover, such L-amino acids as to improve the growth and productivity may be added. For example, L-threonine, L-homoserine, and L-isoleucine are preferably added in case of the L-lysine fermentation; and L-isoleucine, L-lysine, L-glutamic acid, and L-homoserine are preferably added in case of the L-threonine fermentation; and L-phenylalanine, L-tyrosine and the like are preferably added in case of the L-tryptophan fermentation. The concentrations of the added amino acids are around 0.01 to 10 g/L.

The culture is desirably performed under aerobic conditions for one to seven days and preferably at an incubation temperature in the range of 25°C to 40°C, more preferably 30°C to 39°C, further preferably 34°C to 38°C. The pH during the culture period is preferably in the range of 5 to 9. Additionally, an inorganic or organic acidic or basic material, furthermore, ammonia gas and the like can be used for the pH adjustment. Collection of the L-amino acid from the fermentation liquid can typically be performed by combining an ion exchange resin method, a precipitation method and other known methods. Incidentally, in cases where the L-amino acid is stored in bacterial cells, for example, the bacterial cells are disrupted with ultrasonic treatment and the like and removed by centrifugation to obtain supernatant and thereby the L-amino acid can be collected from the supernatant by the ion exchange resin method and the like. The collected L-amino acid may be a free form of the L-amino acid or may be a salt of the L-amino acid including a sulfate, hydrochloride, carbonate, ammonium salt, sodium salt, and potassium salt of the L-amino acid. Moreover, in addition to the L-amino acid of interest, the collected L-amino acid may contain cell debris of the microorganism, components of the culture medium, water, and metabolic by-products of the microorganism. The purity of the collected L-amino acid is 50% or greater, preferably 85% or greater, and especially preferably 95% or greater.

### EXAMPLES

Now, the present invention will be described more specifically with reference to the examples.

### <Example 1>

### Identification of alginate assimilation-functioning genes in the genome of the Vibrio sp. strain SA2 (the strain NITE BP-01635)

The whole genomic DNA of the *Vibrio* sp. strain SA2 was analyzed on the next-generation sequencer Miseq (manufactured by Illumina Inc.) and, by BLAST analysis (Altshul, et al. (1997) Nucleic Acids Research. 25. p3389-3402), the nucleotide sequence information of novel genes in the genome of the *Vibrio* sp. strain SA2, which have homologies to the sequences of already reported alginate assimilation-functioning genes of *Vibrio splendidus* (Wargacki, et al. (2012) Science. 335, p308-313; NCBI: Number AAMR0000 V12B), and the amino acid sequence information of novel proteins encoded by those novel genes were obtained (Table 2). This analysis suggested the possibility that alginate assimilation-functioning genes might be present in the genome of the *Vibrio* sp. strain SA2 (the strain NITE BP-01635). The construction of plasmids to heterogeneously express in *Escherichia coli* these novel genes in the genome of the *Vibrio* sp. strain SA2 was performed as described in the following Examples.

### [Table 2]

**Table 2. The list of alginate assimilation-functioning genes in the genome of Vibrio splendidus and genes homologous thereto in the genome of the Vibrio sp. strain SA2**

| NCBI: Number AAMR0000 V12B number | Gene name | The length of the ORF in *V. splendidus* (bp) | The length of the ORF of a homolog in *Vibrio* sp. SA2 (bp) | DNA identity of the ORF (%) | Amino acid identity of the ORF (%) | SEQ ID of the DNA | SEQ ID of the amino acid |
|---|---|---|---|---|---|---|---|
| 24194 | *toaA* | 1767 | 1749 | 75 | 84 | 2 | 3 |
| 24274 | *alyD* | 1041 | 1050 | 65 | 66 | 4 | 5 |
| 24199 | *eda* | 627 | 624 | 73 | 74 | 6 | 7 |
| 24204 | *kdgK* | 933 | 933 | 66 | 66 | 8 | 9 |
| 24214 | *oalB* | 2208 | 2181 | 69 | 75 | 10 | 11 |
| 24219 | *oalC* | 2154 | 2145 | 74 | 84 | 12 | 13 |
| 24309 | *kdgN* | 726 | 720 | 53 | 40 | 14 | 15 |
| 24234 | *toaB* | 1779 | 1062 | 45 | 14 | 16 | 17 |
| 24324 | *toaC* | 1659 | 483 | 46 | 19 | 18 | 19 |
| 24259 | *alyB* | 1569 | 1581 | 69 | 72 | 20 | 21 |
| 24244 | *dehR* | 882 | 885 | 81 | 87 | 22 | 23 |

### <Example 2>

### Outline of the plasmid construction for the expression of the alginate assimilation-functioning genes in the genome of the Vibrio sp. strain SA2

To heterogeneously express in a strain of *Escherichia coli* the above-described eleven genes present in the genome of the *Vibrio* sp. strain SA2 (SEQ ID NO: 2 (*toaA*), SEQ ID NO: 4 (*alyD*), SEQ ID NO: 6 (*eda*), SEQ ID NO: 8 (*kdgK*), SEQ ID NO: 10 (*oalB*), SEQ ID NO: 12 (*oalC*), SEQ ID NO: 14 (*kdgN*), SEQ ID NO: 16 (*toaB*), SEQ ID NO: 18 (*toaC*), SEQ ID NO: 20 (*alyB*), and SEQ ID NO: 22 (*dehR*)), each gene sequence was amplified and a plasmid carrying the amplified sequences was constructed. In the procedure, the plasmids pSTV29-kdgN-alyB-toaA, pMW219-dehR-oalB-toaB-toaC, pMW219-oalC and pMW219-alyD-eda-kdgN were constructed by amplification from the genomic DNA of the *Vibrio* sp. strain SA2 as a template and the In Fusion HD cloning kit (manufactured by Takara Bio Inc.), which can connect plural gene fragments and incorporate them into a plasmid with one operation based on the commercially available plasmids pSTV29 (manufactured by Takara Bio Inc.) and pMW219 (manufactured by Nippon Gene Co., Ltd.). To unify these plasmids, PCR amplification was performed again by using those plasmids as templates with the In Fusion HD cloning kit to construct the plasmids pSTV29-kdgN-alyB-toaA-oalC (named pSA2S2; SEQ ID NO: 24) and pMW219-dehR-oalB-toaB-toaC-alyD-eda-kdgK (named pSA2M4; SEQ ID NO: 25). The primer sequences used for the construction and the procedure of the construction will be shown below.

### (2-1) Construction of the plasmid pSAS2 for the expression of the alginate assimilation-functioning genes in the genome of the Vibrio sp. strain SA2

The *kdgN* gene was amplified by using primers represented by SEQ ID NOs: 26 and 27 and the genomic DNA of the *Vibrio* sp. strain SA2 as a template. The *alyB* gene was amplified by using primers represented by SEQ ID NOs: 28 and 29 and the genomic DNA of the *Vibrio* sp. strain SA2 as a template. The *toaA* gene was amplified by using primers represented by SEQ ID NOs: 30 and 31 and the genomic DNA of the *Vibrio* sp. strain SA2 as a template. The obtained three fragments were connected to pSTV29, which had been treated with *Hin*dIII and *Sal*I and then purified, by using the In Fusion HD cloning kit to construct pSTV29-kdgN-alyB-toaA.

Similarly, the *oalC* gene was amplified by using primers represented by SEQ ID NOs: 32 and 33 and the genomic DNA of the *Vibrio* sp. strain SA2 as a template and connected to pMW219, which had been treated with *Hin*dIII and *Sal*I and then purified, by using the In Fusion HD cloning kit to construct pMW219-oalC.

Furthermore, the entirety of pSTV29-kdgN-alyB-toaA and a fragment containing the *oalC* gene were amplified by PCR using primers represented by SEQ ID NOs: 34 and 35 and primers represented by SEQ ID NOs: 36 and 37, respectively, and both fragments were connected by using the In Fusion HD cloning kit to construct pSA2S2 (SEQ ID NO: 24).

### (2-2) Construction of the plasmid pSAM4 for the expression of the alginate assimilation-functioning genes in the genome of the Vibrio sp. strain SA2

The *dehR* gene was amplified by using primers represented by SEQ ID NOs: 38 and 39 and the genomic DNA of the *Vibrio* sp. strain SA2 as a template. The *oalB* gene was amplified by using primers represented by SEQ ID NOs: 40 and 41 and the genomic DNA of the *Vibrio* sp. strain SA2 as a template. The *toaB* and *toaC* genes were amplified together by using primers represented by SEQ ID NOs: 42 and 43 and the genomic DNA of the *Vibrio* sp. strain SA2 as a template (the *toaB* and *toaC* genes exist side by side in the genome). The obtained three fragments were connected to pMW219, which had been treated with *Hin*dIII and *Sal*I and then purified, by using the In Fusion HD cloning kit to construct pMW219-dehR-oalB-toaB-toaC.

Similarly, the *alyD* gene was amplified by using primers represented by SEQ ID NOs: 44 and 45 and the genomic DNA of the *Vibrio* sp. strain SA2 as a template. The *eda* gene was amplified by using primers represented by SEQ ID NOs: 46 and 47 and the genomic DNA of the *Vibrio* sp. strain SA2 as a template. The *kdgK* gene was amplified by using primers represented by SEQ ID NOs: 48 and 49 and the genomic DNA of the *Vibrio* sp. strain SA2 as a template. The obtained three fragments were connected to pMW219, which had been treated with *Hin*dIII and *Sal*I and then purified, by using the In Fusion HD cloning kit to construct pMW219-alyD-eda-kdgK.

Furthermore, the entirety of pMW219-dehR-oalB-toaB-toaC and a fragment containing the *alyD*, *eda*, and *kdgK* genes were amplified by PCR using primers represented by SEQ ID NOs: 50 and 51 and primers represented by SEQ ID NOs: 52 and 53, respectively, and both fragments were connected by using the In Fusion HD cloning kit to construct pSA2M4 (SEQ ID NO: 25).

### (2-3) Introduction of pSA2S2 and pSA2M4 into the Escherichia coli L-glutamine-producing MG1655 ΔsucA strain

Electrocompetent cells of the *Escherichia coli* strain MG1655 Δ*sucA* (see US7090988) were produced according to a conventional method and subjected to the electroporation with the added ethanol-precipitated pSA2S2 plasmid DNA (not less than 1 µg) at 1.8 kV and 25 µF to establish the MG1655 Δ*sucA*/pSA2S2 strain. Electrocompetent cells of the MG1655 Δ*sucA*/pSA2S2 strain were produced according to a conventional method and subjected to the electroporation with the added ethanol-precipitated pSA2M4 plasmid DNA (not less than 1 µg) at 1.8 kV and 25 µF to establish the MG1655 Δ*sucA*/pSA2S2,pSA2M4 strain.

### <Example 3>

The L-glutamic acid production using *Escherichia coli* L-glutamic acid-producing strains in a culture medium containing alginic acid as a sole carbon source (3-1) The production of a culture medium using alginic acid hydrolysates as a carbon source

Sodium alginate was added to give a final concentration of 75 g/L in 3 N sulfuric acid and subjected to a heat treatment at 65°C for three hours in a water bath to perform acid hydrolysis of alginic acid. The obtained sodium alginate solution was adjusted with KOH to pH7.0 and a sodium alginate solution was produced. The obtained solution of sodium alginate hydrolysate and various salts were mixed to prepare a liquid minimal medium containing sodium alginate hydrolysate as indicated below.

**The composition of the liquid minimal medium containing sodium alginate hydrolysate**

| | |
|---|---|
| Sodium alginate hydrolysate | 2.5 g/L |
| Na₂HPO₄ | 6 g/L |
| KH₂PO₄ | 3 g/L |
| NaCl | 0.5 g/L |
| NH₄Cl | 1 g/L |
| (NH₄)₂SO₄ | 1.25 g/L |
| MgSO₄·7H₂O | 0.246 g/L |
| Thiamine·HCl | 10 mg/L |
| Kanamycin | 40 mg/L |
| Chloramphenicol | 40 mg/L |

### (3-2) The L-glutamic acid production from alginic acid hydrolysate by the MG1655 ΔsucA/pSA2S2,pSA2M4 strain

The glycerol stock of the MG1655 Δ*sucA*/pSA2S2,pSA2M4 strain was thawed and 100 µL each of the glycerol stock was applied uniformly onto LB-agar medium containing 40 mg/L of kanamycin and 40 mg/L of chloramphenicol and incubated statically at 37°C for 16 hours. About one fourth of the bacterial cells in each obtained plate were suspended in 0.5 mL of saline and the turbidity at a wavelength of 600 nm was measured with a U-2000 spectrophotometer (manufactured by Hitachi, Ltd.). Five mL of the above-described liquid minimal medium containing sodium alginate hydrolysate was poured into an L-shaped tube and inoculated with the each obtained suspension containing bacteria in such a liquid volume as to produce a turbidity of 0.15 at a wavelength of 600 nm and incubated at 39°C for 72 hours in a TN-1506 rocking incubator (manufactured by Advantec Toyo Kaisha, Ltd.) at a revolution speed of 70 rpm. After completion of the culture, the amount of L-glutamic acid accumulated in the culture medium was determined by using the Biotec Analyzer AS-310 (Sakura SI Co. Ltd.).

The result is shown in Table 3. As indicated in Table 3, the MG1655 Δ*sucA*/pSA2S2,pSA2M4 strain, which expressed eleven genes homologous to the alginate assimilation-functioning genes, accumulated a significant amount of L-glutamic acid in the culture medium containing alginic acid hydrolysates as a sole carbon source as compared with the vector control strain as a comparison control.

### [Table 3]

**Table 3. The results in the production of L-glutamic acid by using the MG1655ΔsucA/SA2S2,SA2M4 strain**

| Bacterial strain | Accumulation of L-glutamic acid (mg/L) |
|---|---|
| MG1655ΔsucA/pSTV29, pMW219 | 6.7 |
| MG1655ΔsucA/pSA2S2, pSA2M4 | 20.0 |

### <Example 4> Measurement of the alginate lyase activity in the whole bacterial cell lysate from the Vibrio sp. strain SA2

Fifty µL of each glycerol stock of the *Vibrio* sp. strain SA2 (the strain NITE BP-01635) and *Vibrio splendidus* (type strain ATCC33125) described in Patent Document 1 (WO 2009/046375) was individually plated on LB-agar medium supplemented with Daigo's artificial seawater SP (Wako Pure Chemical Industries, Ltd.; catalog number: 395-01343) and incubated statically at 30°C for 40 hours as a seed culture.

About 200 µL of the obtained wet bacterial cells were suspended in 2.5 mL of the BugBuster Master Mix manufactured by Merck Millipore Co. with a blue inoculation loop and subsequently shaken at 30°C for 15 minutes by reciprocal shaking at 120 rpm to disrupt bacterial cells and to obtain whole cell lysates of the *Vibrio* sp. strain SA2 and *Vibrio splendidus.* The protein concentrations of the cell lysates were measured using BSA manufactured by Bio-Rad Laboratories, Inc. as a standard according to a conventional method, that is, by using the CBB staining method.

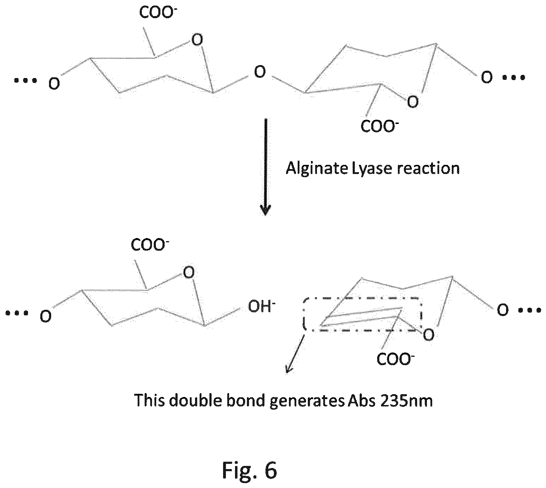

Each of these whole bacterial cell lysate samples was diluted with the BugBuster Master Mix to a protein concentration of 0.48 g/L in terms of BSA. Ninety µL of the each diluted whole bacterial cell lysate sample was mixed with the 2X alginate lyase reaction buffer having the composition indicated below in a 96-well plate and allowed to react for 18 minutes at 30°C, 34°C, 37°C, 40°C, 42°C, and 44°C to determine the alginate lyase activity by measuring the absorbance at 235 nm by using a Spectramax 190 plate reader manufactured by Molecular Devices Corporation Japan. As for the unit of the alginate lyase enzyme activity, one unit was defined according to Uchimura et al., Marine Biotechnology (2010) Vol. 12, p526-533 as an enzymatic activity causing a change of 0.1 absorbance unit at 235 nm (Figure 6). Consequently, as indicated in Table 4, it was found that a significantly higher alginate lyase activity was shown in the whole bacterial cell lysate from the *Vibrio* sp. strain SA2 (the strain NITE BP-01635) as compared with the whole bacterial cell lysate from *Vibrio splendidus* (type strain ATCC33125) at all the temperatures in the reactions carried out in the present invention.

**The 2X alginate lyase reaction buffer**

| | |
|---|---|
| Sodium alginate | 4 g/L |
| Na₂HPO₄ | 6 g/L |
| KH₂PO₄ | 3 g/L |
| NaCl | 0.5 g/L |
| NH₄Cl | 1 g/L |

### [Table 4]

**Table 4. The results of the measurement of the alginate lyase activity using whole bacterial cell lysate samples (unit: U/mg protein/min)**

| Reaction temperature (°C) | Whole bacterial cell lysate from *Vibrio splendidus* | Whole bacterial cell lysate from the *Vibrio* sp. SA2 |
|---|---|---|
| 30 | 0.65 | 0.79 |
| 34 | 1.04 | 1.38 |
| 37 | 1.27 | 1.92 |
| 40 | 1.39 | 1.89 |
| 44 | 0.74 | 1.85 |

### <Example 5> Estimation of the alginate lyase localization by the measurement of the alginate lyase activity in the Vibrio sp. strain SA2

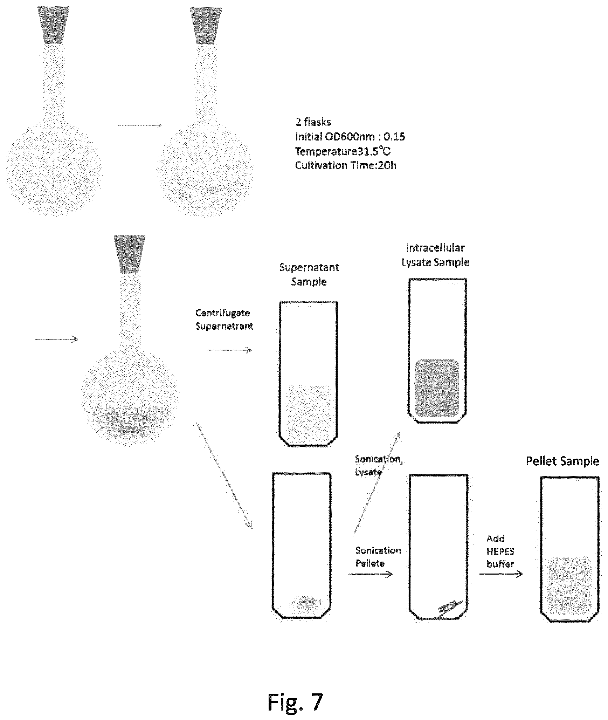

To examine the localization of the alginate lyase observed in the whole bacterial cell lysate from the *Vibrio* sp. strain SA2, the alginate lyase activity measurement was performed by separating three kinds of samples as shown in Figure 7, that is, culture supernatant, bacterial cell lysate, and bacterial cell pellet solution.

Fifty µL each of the glycerol stock of the *Vibrio* sp. strain SA2 was plated on LB-agar medium supplemented with Daigo's artificial seawater SP and incubated statically at an incubation temperature of 30°C for 16 hours as a seed culture. After the static culture, about 200 µL of the obtained wet bacterial cells were collected and suspended in 0.7 mL of saline sterilized by autoclaving with a blue inoculation loop and the absorbance at a wavelength of 600 nm was measured with a U-2900 spectrophotometer manufactured by Hitachi, Ltd. Then, 100 mL of a liquid minimal medium for the measurement of the enzymatic activity having the composition described below was poured into each of two Sakaguchi flasks sterilized by autoclaving and the suspension of the *Vibrio* sp. strain SA2 in saline was added to give an absorbance of 0.15 at a wavelength of 600 nm and incubated with shaking reciprocally for 20 hours under the conditions of 120 rpm of reciprocal shaking speed and 31.5°C of incubation temperature.

**The liquid minimal medium for the measurement of the enzymatic activity**

| | |
|---|---|
| Sodium alginate | 4 g/L |
| Na₂HPO₄ | 12 g/L |
| KH₂PO₄ | 36 g/L |
| NaCl | 16 g/L |
| NH₄Cl | 2 g/L |
| MgSO₄·7H₂O | 0.246 g/L |
| VB1 HCl | 10 mg/L |

Four aliquots of 40 mL each of the obtained culture were isolated and centrifuged under the conditions of 4°C, 6000 rpm, and 5 minutes with a CR20GIII centrifugal machine manufactured by Hitachi, Ltd. to collect bacterial cells and obtain a supernatant sample and a bacterial cell sample. Then, 50 mM HEPES buffer at pH 7.0 cooled to 4°C in a full volume of 5 mL was added to the bacterial cell sample to suspend bacterial cells. The suspension was disrupted for 10 minutes using a Bioruptor sonicator manufactured by Cosmo Bio Co., Ltd. at the range M under such a condition that a cycle of 10 seconds of disruption and 1 second of rest was repeated. The obtained bacterial cell lysate was centrifuged under the conditions of 4°C, 6000 rpm, and 5 minutes with a CR20GIII centrifugal machine manufactured by Hitachi, Ltd. to obtain supernatant as a bacterial cell lysate sample. To the remaining residue, 50 mM HEPES buffer at pH 7.0 cooled to 4°C in a full volume of 5 mL was added and suspended well by pipetting to obtain a pellet sample (Figure 7).

The protein concentrations of the supernatant, bacterial cell lysate and pellet samples of the *Vibrio* sp. strain SA2 obtained by the above-described method were measured using BSA manufactured by Bio-Rad Laboratories, Inc. as a standard by using the CBB staining method.

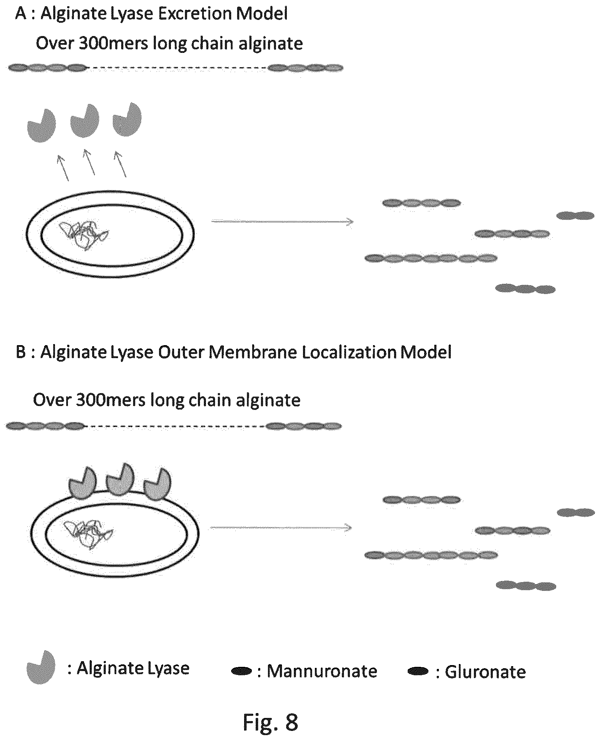

Each of the obtained bacterial cell lysate, supernatant, and pellet samples was diluted with 50 mM HEPES buffer at pH 7.0 cooled to 4°C to have a protein concentration of 0.136 g/L in terms of BSA and, then, an equal volume of the above-described 2X alginate lyase reaction buffer was added and mixed in a 96-well plate and allowed to react at 30°C for 18 minutes to determine the alginate lyase activity by measuring the absorbance at 235 nm by using a Spectramax 190 plate reader manufactured by Molecular Devices Corporation Japan. Consequently, no alginate lyase activity was detected from the bacterial cell lysate and supernatant samples, while a significant alginate lyase activity was detected from the pellet sample (Table 5). It is considered that, in the present example, proteins exported to the outside of cells of the *Vibrio* sp. strain SA2 are accumulated in the supernatant sample and proteins localized in the cytoplasm of a bacterial cell are accumulated in the bacterial cell lysate sample. Moreover, proteins localized in the cell surface such as cell membrane are considered to be accumulated in the pellet sample. Thus, the possibility was suggested that an alginate lyase expressed in the *Vibrio* sp. strain SA2, which degrades a highly polymerized alginic acid such as a reagent grade sodium alginate, is not exported outside the cell (Figure 8, A) but is expressed on the cell membrane (Figure 8, B).

### [Table 5]

**Table 5. The results of the measurement of the alginate lyase activity using fractionated samples (bacterial cell lysate sample, supernatant sample, and pellet sample) of the Vibrio sp. strain SA2 (unit: U/mg protein/min)**

| Sample | Activity (U/mg protein/min) |
|---|---|
| Supernatant | N.D |
| Bacterial Cell Lysate | N.D |
| Pellet | 0.19 |

### <Example 6> Identification of the gene for an alginate lyase functionally expressed on the cell surface in the Vibrio sp. strain SA2

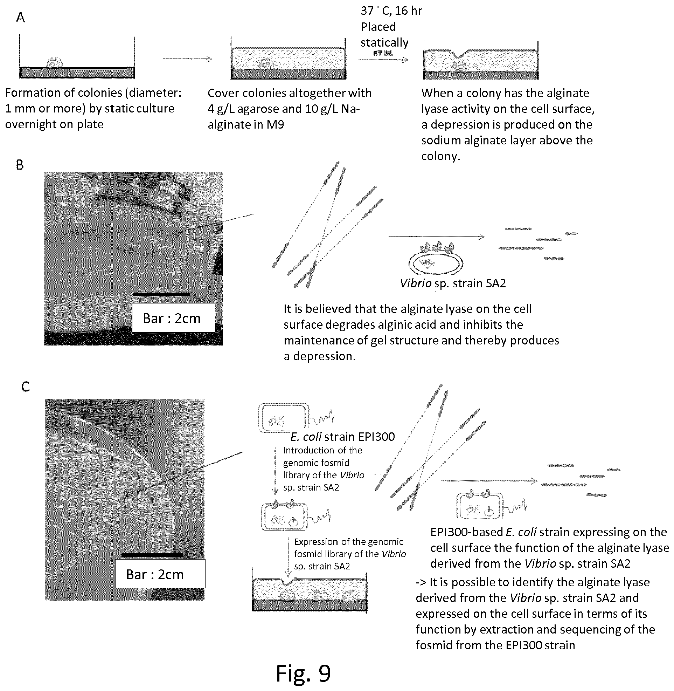

For the purpose of identifying an alginate lyase functionally expressed on the cell surface and of heterogeneously expressing in *Escherichia* bacteria the alginate lyase functionally expressed on the cell surface, a system to screen a clone expressing the function of an alginate lyase on the cell surface was established and a fosmid library carrying the genome of the *Vibrio* sp. strain SA2 was constructed (Figure 9, A).

### Establishment of a system to screen a clone expressing the function of an alginate lyase on the cell surface

About 50 µL of the glycerol stock of the *Vibrio* sp. strain SA2 was applied onto LB-agar medium supplemented with 15 g/L of NaCl and incubated at 37°C for 16 hours. After the culture, about 5 µL of the obtained wet bacterial cells were collected and applied onto a new plate of LB-agar medium supplemented with 15 g/L of NaCl to draw a circular shape with a diameter of about 5 mm and incubated at 37°C for 16 hours. After confirming the bacterial cells of the *Vibrio* sp. strain SA2 rising on the agar, an alginic acid-containing top agar medium (the composition is described below) sterilized by autoclaving at 120°C for 20 minutes was layered and solidified at room temperature. After the obtained plate carrying bacterial cells sandwiched between two layers of the agar media was placed statically at 37°C for 16 hours, visible depressions were found to be produced at the places where the *Vibrio* sp. strain SA2 had grown (Figure 9, B).

**The alginic acid-containing top agar medium**

| | |
|---|---|
| Sodium alginate | 10 g/L |
| Na₂HPO₄ | 6 g/L |
| KH₂PO₄ | 3 g/L |
| NH₄Cl | 1 g/L |
| NaCl | 0.5 g/L |
| Agarose | 4 g/L |

### Construction of a genomic fosmid library of the Vibrio sp. strain SA2 Preparation of insert DNA

About 50 µL of the glycerol stock of the *Vibrio* sp. strain SA2 was applied onto LB-agar medium supplemented with 15 g/L of NaCl and incubated at 37°C for 16 hours. After the culture, the obtained wet bacterial cells were collected and the genomic DNA extraction was performed using the PurElute Bacterial Genomic Kit manufactured by Edge BioSystems, Inc. The obtained the genomic DNA was fragmented using a DNA fragmentation machine HyeroShear manufactured by Gene Machines, Inc. and subjected to pulsed-field gel electrophoresis to fractionate DNA fragments in the range of 33-40 kb. The fractionated DNA fragments were purified using β-agarase manufactured by New England Bio Labs, Inc. and then blunt-ended using the Mighty Cloning Reagent Set (Blunt End) manufactured by Takara Bio Inc. to use these fragments as insert DNA fragments.

### Ligation to the fosmid vector pCC1FOS

The above-described insert DNA fragments were ligated to pCC1FOS manufactured by Epicenter Biotechnologies by a ligation reaction at 4°C overnight according to a conventional method by using T4 DNA ligase manufactured by Takara Bio Inc. Subsequently, *in vitro* packaging was performed according to a conventional method by using the Lambda packaging Extract manufactured by Epicenter Biotechnologies and thereby a genomic fosmid library of the *Vibrio* sp. strain SA2 was obtained.

### Transformation of the E. coli strain EPI300

The glycerol stock of the *E. coli* strain EPI300 manufactured by Epicenter Biotechnologies was transformed according to a conventional method with the obtained genomic fosmid library of the *Vibrio* sp. strain SA2 and incubated statically overnight on LB-agar medium containing 12.5 mg/L of chloramphenicol. After confirming the formation of 200 or more colonies per plate of the agar medium, culture was performed similarly on 20 plates of LB-agar medium supplemented with 12.5 mg/L of chloramphenicol to obtain the plates of the agar medium having a total of 4000 or more visible colonies formed with a diameter of 1 mm or more.

The 20 agar medium plates containing the formed colonies of the EPI300 strain expressing the genomic fosmid library of the *Vibrio* sp. strain SA2 were layered with the alginic acid-containing top agar medium sterilized by autoclaving at 120°C for 20 minutes. After the obtained plates carrying many colonies sandwiched between two layers of the agar media were placed statically at 37°C for 16 hours, a visible depression was found to be produced above the place where one colony had grown (Figure 9, C). The inventors collected with a sterilized toothpick the colony that formed a depression and applied the colony on LB-agar medium containing 12.5 mg/L of chloramphenicol to produce single colonies and, then, named them prospective EPI300-based alginate lyase-expressing strains and produced glycerol stocks.

### Establishment of the in vitro viscosity test system as a simple detection method for the alginate lyase activity

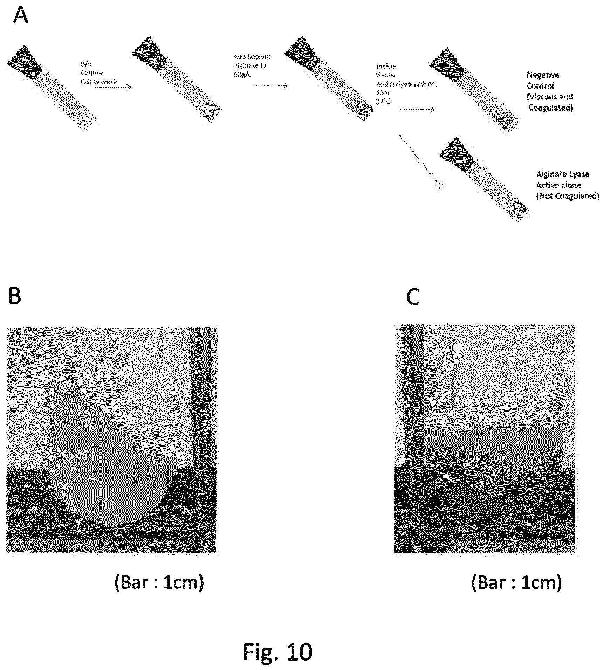

For the purpose of conveniently finding microorganisms that can degrade alginic acid outside the cell, an *in vitro* viscosity test system was tried to be established (Figure 10, A). Specifically, about 50 µL of the glycerol stock of the *Vibrio* sp. strain SA2 was incubated overnight at a temperature of 37°C with reciprocal shaking at 120 rpm in liquid LB medium supplemented with Daigo's artificial seawater SP to have a fully grown culture and then supplemented with sodium alginate to a final concentration of 50 g/L. When agitated with an inclination angle of about 45° by reciprocal shaking at 120 rpm for 16 hours at a temperature of 37°C, the liquid culture medium became viscous and the liquid surface was fixed with a plane inclined at an angle of about 45° because of the sodium alginate in a test tube into which the *Vibrio* sp. strain SA2 had not been inoculated (Figure 10, B). On the other hand, the culture medium did not become viscous but was maintained in liquid form in a test tube into which the *Vibrio* sp. strain SA2 had been inoculated, though sodium alginate had been added to a concentration of 50 g/L (Figure 10, C). This is thought to be a confirmable indication of degradation of a high concentration of sodium alginate by the alginate lyase activity of the *Vibrio* sp. strain SA2. Therefore, we decided to use this *in vitro* viscosity test system as a simple method to examine the presence or absence of the alginate degradation activity.

### Simple detection of the alginate lyase activity using the in vitro viscosity test system

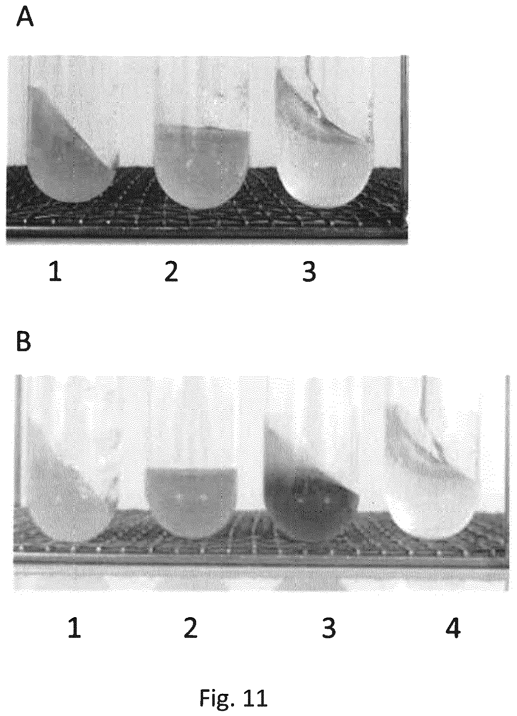

About 50 µL of each glycerol stock of the EPI300/pCCFOS1 control vector strain and the prospective EPI300-based alginate lyase-expressing strain was individually incubated overnight at a temperature of 37°C with reciprocal shaking at 120 rpm in 5 mL of liquid LB medium supplemented with 12.5 mg/L of chloramphenicol in a test tube to have a fully grown culture. Then, sodium alginate was added to the each test tube to give a final concentration of 50 g/L. When agitated with an inclination angle of about 45° by reciprocal shaking at 120 rpm for 16 hours at a temperature of 37°C, an increased viscosity of the culture medium due to the sodium alginate was observed in the test tube into which the EPI300/pCCFOS1 control vector strain had been inoculated, while a significant increase of the viscosity was not observed and the liquid culture medium was maintained in liquid form in the test tube into which the prospective EPI300-based alginate lyase-expressing strain had been inoculated (Figure 11, A). Thus, the prospective EPI300-based alginate lyase-expressing strain was suggested to have an activity to degrade a long-chain alginic acid outside the cell.

To extract a fosmid carried by the prospective EPI300-based alginate lyase-expressing strain, 20 mL of liquid LB medium poured into a Sakaguchi flask was inoculated with about 50 µL of the glycerol stock of the prospective EPI300-based alginate lyase-expressing strain and incubated overnight at 37°C with reciprocal shaking at 120 rpm to produce a fully grown culture. The absorbance at a wavelength of 600 nm was measured with a U-2900 spectrophotometer manufactured by Hitachi, Ltd. and the culture was diluted with liquid LB medium to give an absorbance of 0.8 at a wavelength of 600 nm. Subsequently, the Copy Control Induction Solution manufactured by Epicenter Biotechnologies (catalog number: CCIS125) was added to the culture to give a final concentration of 0.1% (v/v) to carry out an induction while shaken reciprocally at 120 rpm for 6 hours at 37°C. The fosmid DNA carried by the prospective EPI300-based alginate lyase-expressing strain was extracted and purified from the culture after the induction by using the QIAGEN plasmid Midi kit manufactured by QIAGEN Inc.

DNA sequencing analysis was performed on the extracted fosmid DNA by using primers represented by SEQ ID NOs: 60 and 61, the Big Dye Terminator v1.1 Cycle Sequencing Kit manufactured by Life Technologies, Inc. and the 3130 Genetic Analyzer manufactured by Life Technologies, Inc. to obtain the information about the terminal sequences of the insert sequence derived from the genome of the *Vibrio* sp. strain SA2 in the fosmid carried by the prospective EPI300-based alginate lyase-expressing strain.

BLAST analysis (Altshul, et al. "Gapped BLAST and PSI-BLAST: new generation of protein database search programs" (1997) Nucleic Acids Research. 25. p3389-3402) was performed on the information about the whole genomic DNA of the *Vibrio* sp. strain SA2 obtained by the above-described next-generation sequencer Miseq (manufactured by Illumina Inc.) to acquire the information about the insert sequence with a size of 27 kbp derived from the genome of the *Vibrio* sp. strain SA2. The inventors found that this 27 kb-insert sequence included the sequence of the *alyB* gene sequence (SEQ ID NO: 20), which encodes an alginate lyase.

The *alyB* gene derived from the genome of the *Vibrio* sp. strain SA2 (SEQ ID NO: 20) was cloned into a plasmid carrying the promoter P₁₄, pMW119-attR-cat-attL-P₁₄, to construct a plasmid to express the *alyB* gene. Specifically, a PCR was performed by using synthetic oligonucleotides having the nucleotide sequences represented by SEQ ID NOs: 62 and 63 as primers and the genomic DNA of the *Vibrio* sp. strain SA2 as a template to amplify a sequence including the *alyB* gene. A PCR was performed by using synthetic oligonucleotides having the nucleotide sequences represented by SEQ ID NOs: 64 and 65 as primers and the plasmid pMW119-attR-cat-attL-P₁₄ as a template to amplify pMW119-attR-cat-attL-P₁₄ as a linear DNA sequence. The sequence including the *alyB* gene and the linear pMW119-attR-cat-attL-P₁₄ sequence were connected by using the In-Fusion HD Cloning kit (manufactured by Takara Bio Inc.) to construct the plasmid pMW119-attR-cat-attL-P₁₄-alyB, which heterogeneously expresses the *alyB* gene derived from the genome of the *Vibrio* sp. strain SA2 under the control of the promoter P₁₄. The obtained plasmid pMW119-attR-cat-attL-P₁₄-alyB was introduced into the *E. coli* strain JM109 according to a conventional method to establish the JM109/pMW119-attR-cat-attL-P₁₄-alyB strain, which heterogeneously expresses the *alyB* gene derived from the genome of the *Vibrio* sp. strain SA2.

The plasmid pMW119-attR-cat-attL-P₁₄, which carries the promoter P₁₄, was constructed as described below. A PCR was performed by using synthetic oligonucleotides represented by SEQ ID NOs: 79 and 80 as primers and the chromosomal DNA of the *Escherichia coli* strain MG1655 as a template to amplify the sequence of the *gdhA* gene including the promoter P₁₄ represented by SEQ ID NO: 78. The PCR product was purified and treated with the Takara BKL Kit (manufactured by Takara Bio Inc.) and connected to pMW219 (manufactured by Nippon Gene Co., Ltd.), which had been digested with *Sma*I and treated with the Takara BKL Kit, to obtain the plasmid pMW219-P₁₄-gdhA. A PCR was performed by using synthetic oligonucleotides represented by SEQ ID NOs: 81 and 82 as primers and pMW219-P₁₄-gdhA as a template to amplify a part of the sequence corresponding to the promoter P₁₄ (the P₁₄ sequence). A PCR was performed by using synthetic oligonucleotides represented by SEQ ID NOs: 83 and 84 as primers and the plasmid pMW118-attL-Cm-attR (WO2005/010175) as a template to amplify the attR-cat-attL sequence, which contains the chloramphenicol resistance gene between the sequences of the lambda phage attachment sites attR and attL. The attR-cat-attL sequence and the P₁₄ sequence were connected to pMW119 (manufactured by Nippon Gene Co., Ltd.), which had been digested with *Hind*III and *Sal*I*,* by using the In-Fusion HD Cloning kit (manufactured by Takara Bio Inc.) to construct the plasmid pMW119-attR-cat-attL-P₁₄, which carries the promoter P₁₄.

The *alyD* gene derived from the genome of the *Vibrio* sp. strain SA2 (SEQ ID NO: 4) was cloned into the plasmid pMW119-attR-cat-attL-P₁₄, which carries the promoter P₁₄, to construct a plasmid to express the *alyD* gene. Specifically, a PCR was performed by using synthetic oligonucleotides having the nucleotide sequences represented by SEQ ID NOs: 66 and 67 as primers and the genomic DNA of the *Vibrio* sp. strain SA2 as a template to amplify a sequence including the *alyB* gene. A PCR was performed by using synthetic oligonucleotides having the nucleotide sequences represented by SEQ ID NOs: 64 and 65 as primers and the plasmid pMW119-attR-cat-attL-P₁₄ as a template to amplify pMW119-attR-cat-attL-P₁₄ as a linear DNA sequence. The sequence including the *alyD* gene and the linear pMW119-attR-cat-attL-P₁₄ sequence were connected by using the In-Fusion HD Cloning kit (manufactured by Takara Bio Inc.) to construct the plasmid pMW119-attR-cat-attL-P₁₄-alyD, which heterogeneously expresses the *alyD* gene derived from the genome of the *Vibrio* sp. strain SA2 under the control of the promoter P₁₄. The obtained plasmid pMW119-attR-cat-attL-P₁₄-alyD was introduced into the *E. coli* strain JM109 according to a conventional method to establish the JM109/pMW119-attR-cat-attL-P₁₄-alyD strain, which heterogeneously expresses the *alyD* gene derived from the genome of the *Vibrio* sp. strain SA2.

Furthermore, the plasmid pMW119-attR-cat-attL-P₁₄ was introduced into the *E. coli* strain JM109 according to a conventional method to establish the JM109/pMW119-attR-cat-attL-P₁₄ strain.

About 50 µL of each glycerol stock of the JM109/pMW119-attR-cat-attL-P₁₄ strain, the JM109/pMW119-attR-cat-attL-P₁₄-alyB strain and the JM109/pMW119-attR-cat-attL-P₁₄-alyD strain was individually incubated overnight at a temperature of 37°C with reciprocal shaking at 120 rpm in 5 mL of liquid LB medium supplemented with 12.5 mg/L of chloramphenicol in a test tube to have a fully grown culture. Then, sodium alginate was added to the each test tube to give a final concentration of 50 g/L. When agitated with an inclination angle of about 45° by reciprocal shaking at 120 rpm for 16 hours at a temperature of 37°C, an increased viscosity of the culture medium due to the sodium alginate was observed in the test tubes into which the JM109/pMW119-attR-cat-attL-P₁₄ strain and the JM109/pMW119-attR-cat-attL-P₁₄-alyD strain had been inoculated, respectively, while a significant increase of the viscosity was not observed and the liquid culture medium was maintained in liquid form in the test tube into which the JM109/pMW119-attR-cat-attL-P₁₄-alyB strain had been inoculated (Figure 11, B). Thus, the enzyme AlyB (SEQ ID NO: 5), which is encoded by the *alyB* gene derived from the genome of the *Vibrio* sp. strain SA2 and expressed in the prospective EPI300-based alginate lyase-expressing strain, was suggested to have activities to be expressed heterogeneously in *E. coli* and to degrade a long-chain alginic acid outside the cell.

The protein localization of AlyB represented by SEQ ID NO: 21 and derived from the genome of the *Vibrio* sp. strain SA2 and of AlyD represented by SEQ ID NO: 5 and derived from the genome of the *Vibrio* sp. strain SA2 were predicted by using the open software program SOSUI-GramN, which can predict subcellular localization of proteins expressed in Gram-negative bacteria (Imai, et al. "SOSUI-GramN: high performance prediction for sub-cellular localization of proteins in gram-negative bacteria." (2008) Bioinformation. Vol. 2, p417-421). Consequently, the AlyB and AlyD proteins derived from the genome of the *Vibrio* sp. strain SA2 were predicted to be localized in the outer and inner membranes of a cell, respectively. Thus, along with the result of the above-described *in vitro* viscosity test system (Figure 11, B), AlyB was suggested to be the alginate lyase expressed on the cell surface in the *Vibrio* sp. strain SA2 and *E. coli* and degrading alginic acid outside the cell to smaller molecules. Only one case example has been reported so far as a report about the expression of an alginate lyase in *E. coli* to degrade alginic acid outside the cell to smaller molecules, in which a fusion protein of an artificially modified partial fragment of Antigen 43 autotransporter (Ag43) and a heterogeneous alginate lyase was used to forcibly export heterogeneous alginate lyase proteins outside the cell, thereby degrading alginic acid outside the cell to smaller molecules (Non-Patent Document 4 (Wargacki, et al. "An Engineered Microbial Platform for Direct Biofuel Production from Brown Macroalgae" (2012) Science. Vol.335, p308-313); Patent Document 1 (WO 2009/046375)). In contrast, the AlyB derived from the genome of the *Vibrio* sp. strain SA2 and found in the present invention is believed to be an alginate lyase that is automatically localized on the cell surface upon heterogeneous expression in *E. coli* and that can degrade alginic acid outside the cell to smaller molecules and, thus, can be considered to be a novel alginate lyase useful for the conversion of alginic acid into useful molecules in microbial engineering.

### <Example 7> Measurement of the alginate lyase activity of AlyB derived from the genome of the Vibrio sp. strain SA2

### Cloning of an already reported alyB gene as a comparison control

A sequence including the *alyB* gene derived from the genome of the *Vibrio splendidus* strain ATCC33125 was amplified by employing the genomic DNA from *Vibrio splendidus* (type strain ATCC33125) as a template, which had been extracted by using the PurElute Bacterial Genomic Kit manufactured by Edge BioSystems, Inc., and synthetic oligonucleotides having the nucleotide sequences represented by SEQ ID NOs: 68 and 69 as primers. A PCR was performed by using synthetic oligonucleotides having the nucleotide sequences represented by SEQ ID NOs: 64 and 65 as primers and the plasmid pMW119-attR-cat-attL-P₁₄ as a template to amplify pMW119-attR-cat-attL-P₁₄ as a linear DNA sequence. The sequence including the *alyB* gene derived from the genome of the *Vibrio splendidus* strain ATCC33125 and the linear pMW119-attR-cat-attL-P₁₄ sequence were connected by using the In-Fusion HD Cloning kit (manufactured by Takara Bio Inc.) to construct the plasmid pMW119-attR-cat-attL-P₁₄-alyB (*V. splendidus*), which heterogeneously expresses the *alyB* gene derived from the genome of the *Vibrio splendidus* strain ATCC33125 under the control of the promoter P₁₄. The obtained plasmid pMW119-attR-cat-attL-P₁₄-alyB (*V. splendidus*) was introduced into the *E. coli* strain JM109 according to a conventional method to establish the JM109/pMW119-attR-cat-attL-P₁₄-alyB *(V. splendidus*) strain, which heterogeneously expresses the *alyB* gene derived from the genome of the *Vibrio splendidus* strain ATCC33125.

Fifty µL of each glycerol stock of the JM109/pMW119-attR-cat-attL-P₁₄ strain, the JM109/pMW119-attR-cat-attL-P₁₄-alyB strain, and the JM109/pMW119-attR-cat-attL-P₁₄-alyB (*V. splendidus*) strain was individually plated on LB-agar medium supplemented with 12.5 mg/L of chloramphenicol and incubated statically at an incubation temperature of 30°C for 16 hours as a seed culture.

About 200 µL of the obtained wet bacterial cells were suspended in 2.5 mL of the BugBuster Master Mix manufactured by Merck Millipore Co. with a blue inoculation loop and subsequently shaken at 30°C for 15 minutes by reciprocal shaking at 120 rpm to disrupt bacterial cells and to obtain whole cell lysates of the JM109/pMW119-attR-cat-attL-P₁₄ strain, the JM109/pMW119-attR-cat-attL-P₁₄-alyB strain, and the JM109/pMW119-attR-cat-attL-P₁₄-alyB (*V. splendidus*) strain. The protein concentrations of these whole cell lysates were measured using BSA manufactured by Bio-Rad Laboratories, Inc. as a standard according to a conventional method, that is, by using the CBB staining method.

Each of these whole bacterial cell lysate samples was diluted with the BugBuster Master Mix to a protein concentration of 0.48 g/L in terms of BSA. Ninety µL of the each diluted whole bacterial cell lysate sample was mixed with the 2X alginate lyase reaction buffer having the composition indicated above in a 96-well plate and allowed to react for 18 minutes at 30°C, 34°C, 37°C, 40°C, 42°C, and 44°C to determine the alginate lyase activity by measuring the absorbance at 235 nm by using a Spectramax 190 plate reader manufactured by Molecular Devices Corporation Japan. As for the unit of the alginate lyase enzyme activity, one unit was defined according to Uchimura et al., Marine Biotechnology (2010) Vol. 12, p526-533 as an enzymatic activity causing a change of 0.1 absorbance unit at 235 nm. The result is shown in Table 6. The whole bacterial cell lysate from the JM109/pMW119-attR-cat-attL-P₁₄ strain did not exhibit a significant activity at all the temperatures in the reactions carried out in the present invention. Furthermore, it was found that a significantly higher alginate lyase activity was shown in the whole bacterial cell lysate from the JM109/pMW119-attR-cat-attL-P₁₄-alyB strain (described as "Expressing AlyB from *Vibrio* sp. SA2" in Table 6) as compared with the whole bacterial cell lysate from the JM109/pMW119-attR-cat-attL-P₁₄-alyB (*V*. *splendidus*) strain (described as "Expressing AlyB from *Vibrio splendidus*" in Table 6) at all the temperatures in the reactions carried out in the present invention. This result indicated that the alginate lyase activity of AlyB derived from the genome of the *Vibrio* sp. strain SA2 was significantly higher than the alginate lyase activity of AlyB derived from the genome of the *Vibrio splendidus* strain ATCC33125.

### [Table 6]

**Table 6. The results of the measurement of the alginate lyase activity using whole bacterial cell lysate samples of strains expressing AlyB, in which each AlyB is expressed by the same gene expression system in the E.coli strain JM109 (unit: U/mg protein/min)**

| Reaction temperature (°C) | Expressing AlyB from *Vibrio splendidus* | Expressing AlyB from *Vibrio* sp. SA2 |
|---|---|---|
| 30 | 0.58 | 1.86 |
| 34 | 1.07 | 2.48 |
| 37 | 1.56 | 2.55 |
| 40 | 1.15 | 3.00 |
| 44 | 0.81 | 3.22 |

### <Example 8> Establishment of an E. coli L-lysine-producing bacterium that heterogeneously expresses the dehR and alyD and alyB genes derived from the genome of the Vibrio sp. strain SA2

The *dehR* gene derived from the genome of the *Vibrio* sp. strain SA2 (SEQ ID NO: 22) was cloned into the plasmid pMW119-attR-cat-attL-P₁₄, which carries the promoter P₁₄, to construct a plasmid to express the *dehR* gene. Specifically, a PCR was performed by using synthetic oligonucleotides having the nucleotide sequences represented by SEQ ID NOs: 70 and 71 as primers and the genomic DNA of the *Vibrio* sp. strain SA2 as a template to amplify a sequence including the *dehR* gene. A PCR was performed by using synthetic oligonucleotides having the nucleotide sequences represented by SEQ ID NOs: 64 and 65 as primers and the plasmid pMW119-attR-cat-attL-P₁₄ as a template to amplify pMW119-attR-cat-attL-P₁₄ as a linear DNA sequence. The sequence including the *dehR* gene and the linear pMW119-attR-cat-attL-P₁₄ sequence were connected by using the In-Fusion HD Cloning kit (manufactured by Takara Bio Inc.) to construct the plasmid pMW119-attR-cat-attL-P₁₄-dehR, which heterogeneously expresses the *dehR* gene derived from the genome of the *Vibrio* sp. strain SA2 under the control of the promoter P₁₄.

Next, by using the L-lysine-producing *E. coli* strain AJIK01 (the strain NITE BP-01520) as a parent strain, the *dehR* gene derived from the genome of the *Vibrio* sp. strain SA2 was inserted into the locus of the *narI* gene by following the procedures described below to establish the L-lysine-producing bacterium strain AJIK01 *ΔnarI*::P₁₄-*dehR,* which performs the heterogeneous expression.

A gene fragment including attR-cat-attL-P₁₄-dehR was produced by a PCR method using the above-described plasmid pMW119-attR-cat-attL-P₁₄-dehR as a template and synthetic oligonucleotides having the nucleotide sequences represented by SEQ ID NOs: 72 and 73 of the Additional Example as primers. The AJIK01 Δ*narI*::attR-cat-attL-P₁₄-*dehR* strain, in which the *narI* gene of the AJIK01 strain is deleted, was established using the obtained gene fragment by the λ-red method (U.S. Patent Application Publication No. 2006/0160191; Datsenko, K. A, and Wanner, B. L. Proc. Natl. Acad. Sci. USA. 97:6640-6645 (2000)).

Next, the helper plasmid pMW-intxis-ts (U.S. Patent Application Publication No. 20060141586) was used to remove the attR-cat-attL gene introduced upstream of the P₁₄ promoter. The plasmid pMW-intxis-ts is a plasmid carrying genes that encode an integrase (Int) and an excisionase (Xis) of lambda phage and having an ability to replicate the plasmid itself in a thermosensitive manner.

Competent cells of the AJIK01 Δ*narI*::attR-cat-attL-P₁₄-*dehR* strain were produced according to a conventional method, transformed with the helper plasmid pMW-intxis-ts, and cultured at 30°C on a plate of LB-agar medium containing 50 mg/L ampicillin to select an ampicillin resistant strain. The transformant strain was incubated overnight at 42°C on LB-agar medium to remove the plasmid pMW-intxis-ts and the obtained colonies were tested for the resistance to ampicillin and tetracycline to obtain an ampicillin sensitive strain. Thus, a P₁₄-*dehR*-introduced strain lacking the attR-cat-attL gene and pMW-intxis-ts was obtained. This strain was named AJIK01 *ΔnarI*::P₁₄*-dehR.*

Furthermore, by using the AJIK01 *ΔnarI*::P₁₄*-dehR* strain as a parent strain, the AJIK01 *ΔnarI*::P₁₄*-dehR ΔycgG*::P₁₄*-alyD* strain was established by following the procedures described below, which strain heterogeneously expresses the *dehR* and *alyD* genes derived from the genome of the *Vibrio* sp. strain SA2.

A gene fragment including attR-cat-attL-P₁₄-alyD was produced by a PCR method using the above-described plasmid pMW119-attR-cat-attL-P₁₄-alyD as a template and synthetic oligonucleotides having the nucleotide sequences represented by SEQ ID NOs: 74 and 75 as primers. The AJIK01 *ΔnarI*::P₁₄*-dehR ΔycgG*::attR-cat-attL-P₁₄-alyD strain, in which the *ycgG* gene of the AJIK01 *ΔnarI*::P₁₄*-dehR* strain is deleted, was established using the obtained gene fragment by the λ-red method.

Competent cells of the AJIK01 *ΔnarI*::P₁₄*-dehR* Δ*ycgG*::attR-cat-attL-P₁₄-*alyD* strain were produced according to a conventional method, transformed with the helper plasmid pMW-intxis-ts, and cultured at 30°C on a plate of LB-agar medium containing 50 mg/L ampicillin to select an ampicillin resistant strain. The transformant strain was incubated overnight at 42°C on LB-agar medium to remove the plasmid pMW-intxis-ts and the obtained colonies were tested for the resistance to ampicillin and tetracycline to obtain an ampicillin sensitive strain. Thus, a P₁₄-*alyD*-introduced strain lacking the attR-cat-attL gene and pMW-intxis-ts was obtained. This strain was named AJIK01 *ΔnarI*::P₁₄*-dehR ΔycgG*::P₁₄*-alyD.*

Furthermore, by using the AJIK01 *ΔnarI*::P₁₄*-dehR ΔycgG*::P₁₄*-alyD* strain as a parent strain, the AJIK01 *ΔnarI*::P₁₄*-dehR ΔycgG*::P₁₄*-alyD ΔycgV*::P₁₄-*alyB* strain was established by following the procedures described below, which strain heterogeneously expresses the *dehR* and *alyD* and *alyB* genes derived from the genome of the *Vibrio* sp. strain SA2.

A gene fragment including attR-cat-attL-P₁₄-alyB was produced by a PCR method using the above-described plasmid pMW119-attR-cat-attL-P₁₄-alyB as a template and synthetic oligonucleotides having the nucleotide sequences represented by SEQ ID NOs: 76 and 77 as primers. The AJIK01 *ΔnarI*::P₁₄*-dehR ΔycgG*::P₁₄-*alyD* Δ*ycgV*::attR-cat-attL-P₁₄-*alyB* strain, in which the *ycgV* gene of the AJIK01 *ΔnarI*::P₁₄*-dehR ΔycgG*::P₁₄*-alyD* strain is deleted, was established using the obtained gene fragment by the λ-red method.
Competent cells of the AJIK01 *ΔnarI*::P₁₄*-dehR ΔycgG*::P₁₄*-alyD* Δ*ycgV*::attR-cat-attL-P₁₄*-alyB* strain were produced according to a conventional method, transformed with the helper plasmid pMW-intxis-ts, and cultured at 30°C on a plate of LB-agar medium containing 50 mg/L ampicillin to select an ampicillin resistant strain. The transformant strain was incubated overnight at 42°C on LB-agar medium to remove the plasmid pMW-intxis-ts and the obtained colonies were tested for the resistance to ampicillin and tetracycline to obtain an ampicillin sensitive strain. Thus, a P₁₄-*alyB*-introduced strain lacking the attR-cat-attL gene and pMW-intxis-ts was obtained. This bacterial strain was named AJIK01 *ΔnarI*::P₁₄*-dehR ΔycgG*::P₁₄-*alyD ΔycgV*::P₁₄*-alyB.*

### <Example 9> Production of lysine by fermentation of alginic acid as a carbon source

About 50 µL of each glycerol stock of the AJIK01 strain (the strain NITE BP-01520) and the AJIK01 *ΔnarI*::P₁₄*-dehR ΔycgG*::P₁₄*-alyD ΔycgV*::P₁₄*-alyB* strain was individually applied onto LB-agar medium and incubated statically at 37°C for 16 hours. About 200 µL of the obtained wet bacterial cells of the each strain were collected and suspended in 0.7 mL of saline sterilized by autoclaving and the absorbance at a wavelength of 600 nm was measured with a U-2900 spectrophotometer manufactured by Hitachi, Ltd. Then, 25 mL of a liquid medium for the lysine fermentation with the composition described below, which had been adjusted to pH 7.0 with aqueous KOH solution and subsequently sterilized by autoclaving, was poured into each of two Sakaguchi flasks sterilized by autoclaving. To the Sakaguchi flasks into each of which 25 mL of the liquid medium for the lysine fermentation had been poured, saline suspensions of the AJIK01 strain and the AJIK01 *ΔnarI*::P₁₄*-dehR ΔycgG*::P₁₄*-alyD ΔycgV*::P₁₄*-alyB* strain were individually added to give an absorbance of 0.5 at a wavelength of 600 nm and subsequently 0.5 g of sodium alginate sterilized by autoclaving was added as a carbon source and incubated with shaking reciprocally for 88 hours under the conditions of 120 rpm of reciprocal shaking speed and 37°C of incubation temperature. After completion of the culture, the amount of L-lysine accumulated in the culture medium was determined by using the Biotec Analyzer AS-310 (Sakura SI Co. Ltd.). Moreover, the amount of the bacterial cells at the end of the culturing period was determined by diluting the culture with distilled water as required immediately after the end of the culturing period and measuring the absorbance at a wavelength of 600 nm (OD600) with a U-2900 spectrophotometer (manufactured by Hitachi, Ltd.).

The result is shown in Table 7. In Table 7, "Strain" indicates the names of bacterial strains. Moreover, in Table 7, "Lys (mg/L)" indicates the amount of L-lysine accumulated in the culture medium in the flasks. The strain that heterogeneously expresses the *dehR* and *alyD* and *alyB* genes derived from the genome of the *Vibrio* sp. strain SA2 (the AJIK01 *ΔnarI*::P₁₄*-dehR ΔycgG*::P₁₄*-alyD ΔycgV*::P₁₄*-alyB* strain) demonstrated a significantly higher amount of accumulated bacterial cells as compared with the control strain (the AJIK01 strain). Moreover, the strain that heterogeneously expresses the *dehR* and *alyD* and *alyB* genes derived from the genome of the *Vibrio* sp. strain SA2 (the AJIK01 *ΔnarI*::P₁₄*-dehR ΔycgG*::P₁₄*-alyD ΔycgV*::P₁₄*-alyB* strain) demonstrated a significantly higher ability to produce L-lysine as compared with the control strain (the AJIK01 strain). The results shown in Table 7 indicated that the simultaneous heterogeneous expression of DehR, AlyD, and AlyB derived from the genome of the *Vibrio* sp. strain SA2 in the *E. coli* L-lysine-producing AJIK01 strain allowed the acquirement of the ability to assimilate alginic acid and to increase the ability to produce L-lysine.

**The composition of the liquid culture medium for the lysine fermentation**

| | |
|---|---|
| (NH₄)₂SO₄ | 24 g/L |
| FeSO₄·7H₂O | 10 mg/L |
| MnSO₄·4H₂O | 8.2 mg/L |
| Yeast Extract (Difco) | 2 g/L |
| Na₂HPO₄ | 12 g/L |
| KH₂PO₄ | 37 g/L |
| NaCl | 16 g/L |
| NH₄Cl | 2 g/L |
| MgSO₄·7H₂O | 1 g/L |

### [Table 7]

**Table 7. The results of the lysine fermentation using alginic acid as a main carbon source**

| Strain | CT: 88hr OD₆₀₀ | CT: 88hr Lys (mg/L) |
|---|---|---|
| AJIK01 | 1.1 | 79 |
| AJIK01Δ*narI*::P₁₄*-dehR*, Δ*ycgG*::P₁₄-*alyD*, Δ*ycgV*::P₁₄-*alyB* | 2.1 | 120 |
| Blank (no addition of a strain) | 0.7 | 60 |

### <Reference Example 1> Obtainment of the Vibrio sp. strain SA2

Turban shells collected in Nadaura area, Nanao city, Ishikawa prefecture, Japan were used for the screening of new microorganisms capable of assimilating alginic acid (sampling place: the marine area near the point at latitude 37.0981 degree north and longitude 137.0499 degree east).

The shell of the above-described turban shell was smashed with a hammer and one inoculation loop of brownish-green digestive tract content was scooped with a blue inoculation loop and suspended in Daigo's artificial seawater SP solution (manufactured by Wako Pure Chemical Industries, Ltd.; see the composition below), which had been sterilized by autoclaving at 120°C and 0.15 MPa for 20 minutes. The suspension of the digestive tract of turban shell was appropriately diluted with the Daigo's artificial seawater and plated on the sodium alginate-agar selection medium containing a sodium alginate having a polymerization degree of not less than 2000 (a first grade reagent manufactured by Wako Pure Chemical Industries, Ltd.; 300-400 cP; catalog number: 192-09995) as a sole carbon source (see the composition below). After static culturing at an incubation temperature of 25°C for an incubation period of 75 hours, many colonies were confirmed to be formed.

Colonies were collected and suspended in saline with an inoculation loop and then washed with saline three times. Subsequently, the suspensions were individually plated on a newly prepared sodium alginate-agar selection medium and incubated statically at an incubation temperature of 37°C for an incubation period of 50 hours and strains confirmed for their colony formation were further subjected to the single-colony isolation (SI) with the sodium alginate-agar selection medium and then suspended in 4 mL of a culture medium composed of liquid LB medium additionally supplemented with 15 g/L of NaCl to perform test-tube culture. The culture was performed by shaking test tubes under the conditions of 37°C of incubation temperature and 120 rpm of reciprocal shaking speed. Once the OD determined by the absorbance at 600 nm reached about 0.3 in each culture, a glycerol stock of the culture was produced by adding an equal volume of 20% sterilized glycerol and freezing at -80°C and stored in an ultra-deep freezer. A bacterial strain that formed single colonies at the earliest time point was named SA2.

**The composition of the Daigo's artificial seawater SP**

| | |
|---|---|
| MgCl₂·6H₂O | 9.474 g/L |
| CaCl₂·2H₂O | 1.328 g/L |
| Na₂SO₄ | 3.505 g/L |
| KCl | 0.597 g/L |
| NaHCO₃ | 0.171 g/L |
| KBr | 0.085 g/L |
| Na₂B₄O₇·10H₂O | 0.034 g/L |
| SrCl₂ | 0.012 g/L |
| NaF | 3 mg/L |
| LiCl | 1 mg/L |
| KI | 0.07 mg/L |
| CoCl₂·6H₂O | 0.0002 mg/L |
| AlCl₃·6H₂O | 0.008 mg/L |
| FeCl₃·6H₂O | 0.006 mg/L |
| Na₂WO₄·2H₂O | 0.0002 mg/L |
| (NH₄)₆MO₇O₂₄·4H₂O | 0.0008 mg/L |
| NaCl | 20.747 g/L |

**The composition of the sodium alginate-agar selection medium**

| | |
|---|---|
| Sodium alginate | 5 g/L |
| Na₂HPO₄ | 6 g/L |
| KH₂PO₄ | 3 g/L |
| NaCl | 0.5 g/L |
| NH₄Cl | 1 g/L |
| NaCl | 21.247 g/L |
| MgSO₄·7H₂O | 0.246 g/L |
| Thiamine·HCl | 10 mg/L |
| MgCl₂·6H₂O | 9.474 g/L |
| CaCl₂·2H₂O | 1.328 g/L |
| Na₂SO₄ | 3.505 g/L |
| KCl | 0.597 g/L |
| NaHCO₃ | 0.171 g/L |
| KBr | 0.085 g/L |
| Na₂B₄O₇·10H₂O | 0.034 g/L |
| SrCl₂ | 0.012 g/L |
| NaF | 3 mg/L |
| LiCl | 1 mg/L |
| KI | 0.07 mg/L |
| CoCl₂·6H₂O | 0.0002 mg/L |
| AlCl₃·6H₂O | 0.008 mg/L |
| FeCl₃·6H₂O | 0.006 mg/L |
| Na₂WO₄·2H₂O | 0.0002 mg/L |
| (NH₄)₆MO₇O₂₄·4H₂O | 0.0008 mg/L |
| Bacto Agar | 15 g/L |

### <Reference Example 2>

### Molecular phylogenetic tree-based analysis of the Vibrio sp. strain SA2

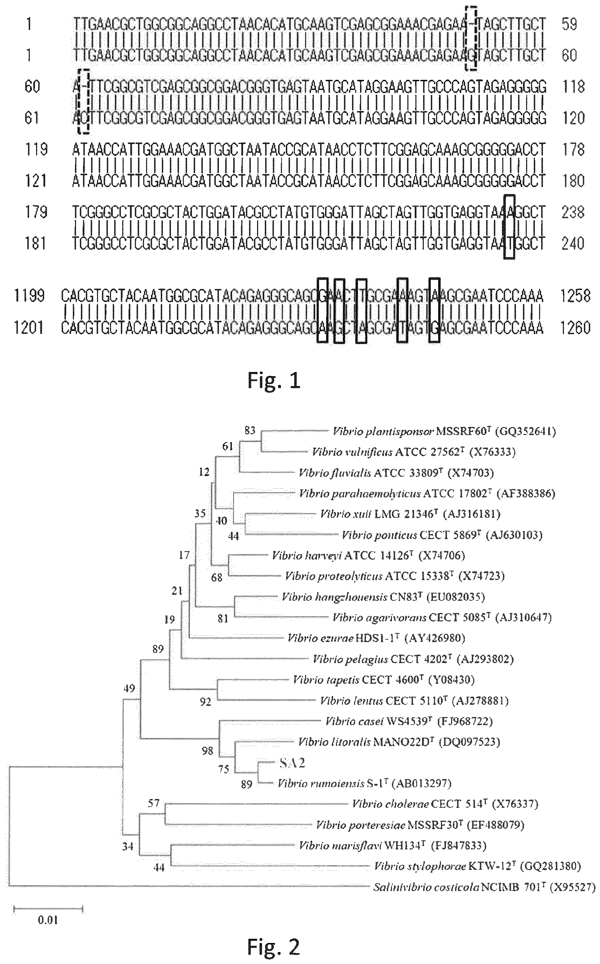

Fifty µL of the glycerol stock of the SA2 strain was incubated statically at 37°C for 16 hours on an agar medium composed of liquid LB medium supplemented with 15 g/L of NaCl and the whole genomic DNA was extracted from the obtained bacterial cells by using the PurElute Bacterial Genomid Kit (manufactured by Edge BioSystems, Inc.). The obtained whole genomic DNA was analyzed on the next-generation sequencer Miseq (manufactured by Illumina Inc.) and, by BLAST analysis (Altshul, et al. "Gapped BLAST and PSI-BLAST: new generation of protein database search programs" (1997) Nucleic Acids Research. 25, p3389-3402), the full-length sequence of the 16S rRNA gene (SEQ ID NO: 1) was obtained. Consequently, the SA2 strain showed 99.5% homology with the *Vibrio rumoiensis* type strain S-1 (the DSM19141 strain) in the comparison of the full-length sequence of the 16S rRNA gene (including the difference due to a gap of two bases and a substitution of six bases; Figure 1).

A molecular phylogenetic tree was generated based on the obtained full-length sequence of the 16S rRNA gene of the SA2 strain by the neighbor-joining method (Saitou, et al. "The neighbor-joining method: a new method for reconstructing phylogenic trees" (1987) Mol. Biol. Evol. 4, p406-425) using the MEGA molecular phylogenetic tree prediction software ver.5.0 (Tamura, et al. "MEGA5: Molecular Evolutionary Genetics Analysis using Maximum Likelihood, Evolutionary Distance, and Maximum Parsimony Methods" (2011) Mol. Biol. Evol. 28, p2731-2739) (Figure 2). The Kimura 2-parameter model was used as a model of base substitution used in the neighbor-joining method (see Kimura. "A simple method for estimating evolutionary rates of base substitutions through comparative studies of nucleotide sequences" (1980) J. Mol. Evol. 16, p111-120). Moreover, to assess the confidence of the topology of the generated molecular phylogenetic tree, the assessment was performed with 1000 bootstrap replicates (see Felsenstein. "Confidence limits on phylogenics: an approach using the bootstrap" (1985) Evolution. 39, p783-791).

The result of the molecular phylogenetic analysis indicated that the SA2 strain belonged to a cluster formed by species of the genus *Vibrio.* It was supported by a bootstrap value of 98% that the SA2 strain belonged to a cluster formed by *Vibrio rumoiensis*, *Vibrio litoralis*, and *Vibrio casei.* Moreover, the SA2 strain formed a cluster together with *Vibrio rumoiensis* and the cluster was supported by a bootstrap value of 89%. The above result indicated that the SA2 strain was closely related to the genus *Vibrio.* Thus, the SA2 strain was named *Vibrio* sp. strain SA2 (the strain NITE BP-01635).

### <Reference Example 3>

### Identification of the species of the Vibrio sp. strain SA2 by the DNA-DNA hybridization method

BLAST analysis indicated that the full-length sequence of the 16S rRNA gene of the *Vibrio* sp. strain SA2 (the strain NITE BP-01635) showed 99.5% homology with that of *Vibrio rumoiensis*, 97.9% homology with that of *Vibrio litoralis*, and 96.3% homology with that of *Vibrio casei.* Because it had been reported that microorganism species mutually exhibiting a homology of less than 98.7% in the full-length sequence of the 16S rRNA gene were considered to belong to different species, it was likely that the *Vibrio* sp. strain SA2 was the same species of microorganism as *Vibrio rumoiensis* alone (Stackebrandt, et al. "Taxonomic parameters revisited: tarnished gold standards." (2006) Microbiol. Today. 33. p152-155.).

Thus, the identification of the species of the *Vibrio* sp. strain SA2 by the DNA-DNA hybrid formation test was performed three times each on the *Vibrio* sp. strain SA2 and the *Vibrio rumoiensis* type strain by switching the immobilized DNA and the probe DNA between the strains (see Yoshiaki Kawamura "Phylogenetic Systematics and Identification Method of Bacteria" (2000) Japanese journal of bacteriology. 55. p545-584; Ken-ichiro Suzuki et al., Eds. "Experimental Methods of Classification of Identification of Microorganisms, focused on molecular genetics and molecular biological procedures" (2000) p34-47).

Consequently, the genomic DNA of the *Vibrio* sp. strain SA2 and the *Vibrio rumoiensis* type strain showed a value in the range of 36-39% and a homology value of 37.5% on average (Table 8 below). Currently, a species of microorganisms is defined as follows: bacterial strains exhibiting a homologous value of 70% or greater as a result of the comparison of homologous value by the DNA-DNA hybrid formation test are included in the same species (see Wayne, et al. "Report of the ad hoc committee on reconciliation of approaches to bacterial systematics." (1987) Int. J. Syst. Bacteriol., 37, p463-464.). Thus, the *Vibrio* sp. strain SA2 was found to be a novel species of microorganism belonging to a different species from the *Vibrio rumoiensis* type strain.

### [Table 8]

**Table 8. The results of the DNA-DNA hybridization test (homologous value (%))**

| | DNA used as labelled DNA (probe) | |
|---|---|---|
| Immobilized DNA | *Vibrio* sp. SA2 | *Vibrio rumoiensis* type strain |
| *Vibrio* sp. SA2 | 100 | 37 |
| *Vibrio rumoiensis* type strain | 38 | 100 |

### <Reference Example 4>

### Electron micrography of the Vibrio sp. strain SA2

### (Fixation of bacterial cells)

Fifty µL each of the glycerol stock of the *Vibrio* sp. strain SA2 (the strain NITE BP-01635) was plated on LB-agar medium supplemented with 15 g/L of NaCl and incubated statically at an incubation temperature of 25°C for 16 hours as a seed culture. The obtained bacterial cells were immersed overnight in 2% paraformaldehyde, 2% glutaraldehyde, 0.1 M cacodylate buffer (pH7.4) under the temperature condition of 4°C (about 16 hours) to fix the bacterial cells. The fixed bacterial cells were washed four times with 0.1 M cacodylate buffer (pH7.4) under the temperature condition of 4°C. Furthermore, the washed fixed bacterial cells were placed statically for 2 hours in 1% tannic acid, 0.1 M cacodylate buffer (pH7.4) under the temperature condition of 4°C to perform the second fixation. After this second fixation, the fixed bacterial cells were washed four times with 0.1 M cacodylate buffer (pH7.4) under the temperature condition of 4°C, and then placed statically for three hours in 2% osmium tetroxide, 0.1 M cacodylate buffer (pH7.4) under the temperature condition of 4°C.

### (Dehydration treatment)

The fixed bacterial cells which had undergone the above-described treatment were dehydrated by replacement with 50% ethanol for 30 minutes under the temperature condition of 4°C. Next, the dehydration treatment was performed by replacement with 70% ethanol for 30 minutes under the temperature condition of 4°C. Next, the dehydration treatment was performed by replacement with 90% ethanol for 30 minutes under the temperature condition of 25°C. Furthermore, the replacement with 100% ethanol was carried out three times at room temperature for 30 minutes to perform the dehydration treatment and the replacement with 100% etnhanol was further carried out at room temperature overnight to obtain the dehydrated fixed bacterial cell sample.

### (Freeze dry)

The above-described dehydrated fixed bacterial cell sample of the *Vibrio* sp. strain SA2 was subjected to the replacement with a 50%/50% mixture of tert-butyl alcohol/ethanol at room temperature for one hour. Furthermore, after the replacement with tert-butyl alcohol at room temperature for one hour, the replacement with tert-butyl alcohol for one hour under the temperature condition of 4°C was repeated three times. The sample which had undergone the replacement was freeze-dried.

### (Coating)

The freeze-dried sample was coated with osmium film (60 nm) by using an osmium plasma coater manufactured by Nippon Laser and Electronics Laboratory Co., Ltd. (catalog number: NL-OPC80NS).

### (Observation and photography)

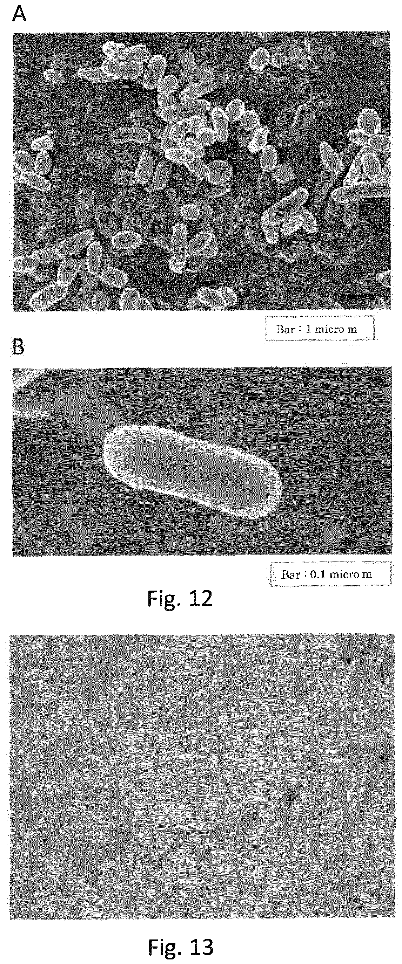

The coated sample was observed and photographed with an electron microscope manufactured by JEOL Ltd. (catalog number: JSM-6340F) under an acceleration voltage condition of 5 kV and thereby electron micrographs of the *Vibrio* sp. strain SA2 shown in Figure 12 were obtained. This result indicated that the *Vibrio* sp. strain SA2 grown on LB-agar medium supplemented with 15 g/L of NaCl was an aflagellate rod-shaped bacterial cell.

### <Reference Example 5> The result from the Gram-staining test of the Vibrio sp. strain SA2

Fifty µL each of the glycerol stock of the *Vibrio* sp. strain SA2 (the strain NITE BP-01635) was plated on agar medium supplemented with MB2216 (manufactured by Becton Dickinson and Co.) and incubated statically at an incubation temperature of 30°C for 24 hours as a seed culture. The obtained bacterial cells was stained according to a conventional method by using the Favor G "Nissui" gram-identification stain (manufactured by Nihon Pharmaceutical Co., Ltd.) and observed and photographed with a BX50F4 optical microscope manufactured by Olympus Corporation and thereby an optical micrograph of the *Vibrio* sp. strain SA2 shown in Figure 13 was obtained. That result indicated that the *Vibrio* sp. strain SA2 belonged to Gram-negative bacteria.

### <Reference Example 6> Multi-locus gene analysis of the Vibrio sp. strain SA2

The multi-locus gene analysis (a molecular phylogenetic tree-based analysis comparing sequences each including plural interconnected genes) is generally performed using housekeeping genes, which are conserved in the genome of the genus *Vibrio,* to identify the species of a microorganism in the genus *Vibrio.* Therefore, the multi-locus gene analysis was performed using the sequences of six genes (*atpA*, *pyrH*, *recA*, *rpoA*, *rpoD* and the 16S rRNA gene) among housekeeping genes conserved in the genome of bacteria in the genus *Vibrio* with reference to Kim, et al. Int. J. Syst. Evol. Microbiol., 2013, vol 63, p3697-3703.

By a method similar to the above-described obtainment of the full-length sequence of the 16S rRNA gene represented by SEQ ID NO: 1, the sequences of the *atpA*, *pyrH*, *recA*, *rpoA*, and *rpoD* genes in the *Vibrio* sp. strain SA2 were obtained.
The *atpA* gene of the *Vibrio* sp. strain SA2 is represented by SEQ ID NO: 89.
The *pyrH* gene of the *Vibrio* sp. strain SA2 is represented by SEQ ID NO: 90.
The *recA* gene of the *Vibrio* sp. strain SA2 is represented by SEQ ID NO: 91.
The *rpoA* gene of the *Vibrio* sp. strain SA2 is represented by SEQ ID NO: 92.
The *rpoD* gene of the *Vibrio* sp. strain SA2 is represented by SEQ ID NO: 93.

Next, the sequences of 150 genes used for the multi-locus gene analysis indicated in Table 9 were obtained by using sequences available from database (GenBank/DDBJ/EMBL), the *rpoD* gene sequences of *Vibrio casei*, *Vibrio litoralis*, and *Vibrio rumoiensis* obtained according to the method of Yamamoto et al., Int. J. Syst. Evol. Microbiol., 1998, vol48, p813-819, and, furthermore, the *pyrH* gene sequence of *Vibrio rumoiensis* obtained from the web site "The Taxonomy of *Vibrios*" (http://www.taxvibrio.lncc.br/).
The *rpoD* gene sequence of *Vibrio casei* is represented by SEQ ID NO: 94.
The *rpoD* gene sequence of *Vibrio litoralis* is represented by SEQ ID NO: 95.
The *rpoD* gene sequence of *Vibrio rumoiensis* is represented by SEQ ID NO: 96.
The *pyrH* gene sequence of *Vibrio rumoiensis* is represented by SEQ ID NO: 97.

For the multi-locus gene analysis, the MEGA ver 5.0 described in Tamura et al. Mol. Biol. Evol., 2011, vol28, p2731-2739 was used. Moreover, the neighbor-joining method described in Saitou et al. Mol. Biol. Evol., 1987, vol4, p406-425 was used as a method to predict a phylogenetic tree. The Jukes and Cantor's method described in Jukes et al. New York: Academic Press.1969 was used as a model of base substitution. The bootstrap method with 1000 replicates described in Felsenstein et al. Evolution, 1985, vol39, p783-791 was used to assess the confidence of the topology.

### [Table 9]

**Table 9. List of the sequences used in the multi-locus gene analysis**

| (The gene sequences obtained in the Reference Example are represented by SEQ ID NO: 1 and SEQ ID NOs: 89-97) | | | | | | |
|---|---|---|---|---|---|---|
| taxon | Accession No.(GenBank/DDBJ/EMBL) | | | | | |
| | *atpA* | *pyrH* | *recA* | *rpoA* | *rpoD* | 16S rRNA |
| *Vibrio* sp.SA2 | SEQ ID NO: 89 | SEQ ID NO: 90 | SEQ ID NO: 91 | SEQ ID NO: 92 | SEQ ID NO: 93 | SEQ ID NO: 1 |
| *Vibrio alginolyticus* | EF601231 | GU266285 | AJ842373 | AJ842558 | FM202535 | X56576 |
| *Vibrio brasiliensis* | EF601320 | HM771374 | AJ842376 | HM771384 | AEVS01000072 | HM771338 |
| *Vibrio campbellii* | EF601232 | EF596641 | AJ842377 | AJ842564 | FM202508 | X56575 |
| *Vibrio casei* | FJ968711 | FJ968720 | FJ968714 | FJ968717 | SEQ ID NO: 94 | FJ968722 |
| *Vibrio chagasii* | EF601280 | EU118252 | AJ842385 | AJ842572 | AY75I356 | AJ316199 |
| *Vibrio cholerae* | EF601300 | EF990257 | AJ842386 | AJ842573 | AM942060 | X76337 |
| *Vibrio coralliilyticus* | JN039158 | GU266292 | AJ842402 | AJ842587 | ACZN01000 013 | AJ440005 |
| *Vibrio gigantis* | EU541556 | EU871951 | EU541593 | EU541573 | AY751347 | EF094888 |
| *Vibrio halioticoli* | EF601260 | EU871952 | EU871966 | AJ842617 | BAUJ01000027 | AB000390 |
| *Vibrio harveyi* | BAOD0100 0015 | EU118238 | AJ842440 | AJ842627 | FM202498 | X74706 |
| *Vibrio ichthyoenteri* | EF601262 | HM771375 | AJ842446 | HM771385 | HF679140 | AJ437192 |
| *Vibrio lentus* | EU541558 | JX401842 | AJ842452 | AJ842639 | AY751358 | AJ278881 |
| *Vibrio litoralis* | FJ968710 | AUFZ01000 011 | FJ968713 | FJ968716 | SEQ ID NO: 95 | DQ097523 |
| *Vibrio mediterranei* | EF601242 | GU266288 | AJ842459 | AJ842644 | HF542043 | X74710 |
| *Vibrio orientalis* | EF601341 | EU118243 | AJ842485 | AJ842672 | HF542105 | X74719 |
| *Vibrio parahaemolyticus* | EF601274 | EU118240 | AJ842490 | AJ842677 | FM202531 | AF388386 |
| *Vibrio proteolyticus* | EF601259 | BATJ01000010 | AJ842499 | AJ842686 | HE805638 | X74723 |
| *Vibrio rotiferianus* | EF601340 | EF596722 | AJ842501 | AJ842688 | FM202525 | AJ316187 |
| *Vibrio rumoiensis* | EF601329 | SEQ ID NO: 97 | AJ842503 | AJ842690 | SEQ ID NO: 96 | AB013297 |
| *Vibrio scophthalmi* | EF601261 | HM771376 | AJ842505 | HM771386 | HF679141 | U46579 |
| *Vibrio tapetis* | HE795159 | HE795189 | HE795219 | AJ842730 | HF542104 | Y08430 |
| *Vibrio vulnificus* | AMQV0100 0037 | GQ382226 | AJ842523 | AJ842737 | HE805634 | X76333 |
| *Enterovibrio norvegicus* | EF601330 | JF739391 | AJ842348 | AJ842531 | AM942062 | AJ316208 |
| *Grimontia hollisae* | EF601247 | JF739393 | AJ842351 | AJ842535 | AM942061 | AJ514909 |

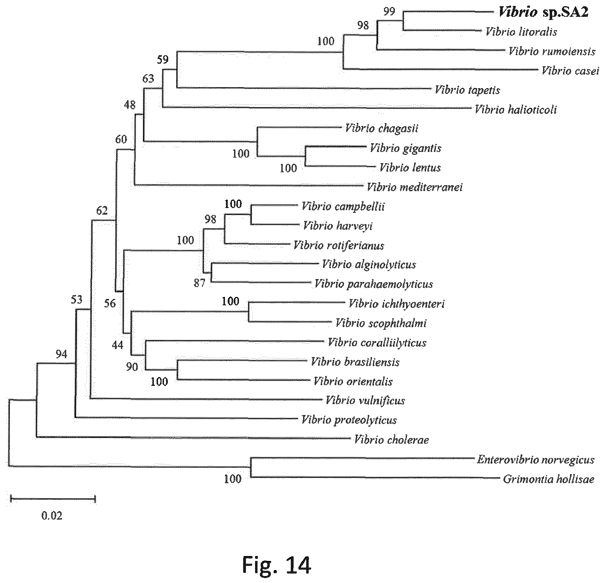

The above multi-locus gene analysis using the sequences of the housekeeping genes characteristic in bacteria of the genus *Vibrio* (*atpA, pyrH, recA, rpoA, rpoD* and the 16S rRNA gene) found the *Vibrio* sp. strain SA2 to be included in a cluster formed by three species of bacteria in the genus *Vibrio: Vibrio casei, Vibrio litoralis,* and *Vibrio rumoiensis* (Figure 14). This cluster was supported by a bootstrap value as high as 100%. Furthermore, also in this cluster, the *Vibrio* sp. strain SA2 formed the cluster together with *Vibrio litoralis.* Thus, this multi-locus gene analysis indicated that the *Vibrio* sp. strain SA2 was most closely related to *Vibrio litoralis.* This cluster was supported by a bootstrap value of 99% but a genetic distance was still observed between both the species, indicating that the *Vibrio* sp. strain SA2 belonged to a different species from *Vibrio litoralis.* Furthermore, as described above, the *Vibrio* sp. strain SA2 exhibited a homology of 97.9% in the full-length sequence of the 16S rRNA gene as compared with *Vibrio litoralis.* It has been reported that microorganism species exhibiting a homology of less than 98.7% in the full-length sequence of the 16S rRNA gene to each other are considered to belong to different species (Stackebrandt, et al. "Taxonomic parameters revisited: tarnished gold standards." (2006) Microbiol. Today. 33, p152-155.). Thus, the *Vibrio* sp. strain SA2 was indicated to belong to a different species from *Vibrio litoralis,* which had been indicated by the multi-locus gene analysis to be most closely related to the *Vibrio* sp. strain SA2, and was indicated to be a microorganism belonging to a new species of bacteria in the genus *Vibrio.*

### <Reference Example 7> Composition analysis of fatty acids in bacterial cells of the Vibrio sp. strain SA2 and closely related species

Since the *Vibrio* sp. strain SA2 was indicated to belong to a new species of bacteria in the genus *Vibrio,* the analysis of fatty acid composition in bacterial cells, which is a commonly described chemical property useful for the classification of microorganism species, was performed on the *Vibrio* sp. strain SA2 and closely related species thereof.

Fifty µL of each glycerol stock of the *Vibrio* sp. strain SA2 (the strain NITE BP-01635) and comparison control strains *Vibrio rumoiensis* (type strain DSM19141), *Vibrio litoralis* (type strain DSM17657), and *Vibrio casei* (type strain DSM 22364) was individually plated on agar medium supplemented with MB2216 (manufactured by Becton Dickinson and Co.) and incubated statically at an incubation temperature of 30°C for 24 hours as a seed culture. The major fatty acid composition listed in Table 10 was obtained from the obtained bacterial cells by following the operation manual (Version 6) for the composition analysis of fatty acids in bacterial cells in the Sherlock Microbial Identification System (Version 6.0) and using the TSBA6 Calculation Method and the TSBA6 Library.

### [Table 10]

**Table 10. The results of the composition analysis of fatty acids in bacterial cells of the Vibrio sp. strain SA2 and the closely related species**

| Fatty acids (%) | *Vibrio* sp.SA2 | *Vibrio rumoiensis* | *Vibrio litoralis* | *Vibrio casei* |
|---|---|---|---|---|
| C_{12:0} | 3.9 | 3.9 | 3.9 | 4.3 |
| C_{14:0} | 1.7 | 4.7 | 1.8 | 2.1. |
| C_{16:0} | 28.0 | 26.0 | 23.2 | 22.6 |
| C_{18:0} | 2.0 | 1.6 | 3.2 | 2.2 |
| C_{17:1}w8c | ND | 0.3 | 0.5 | 0.7 |
| C_{20:1}w7c | 0.2 | ND | 0.4 | 0.4 |

The above result indicated that the *Vibrio* sp. strain SA2 had a bacterial cell-fatty acid composition different from those of the closely related species which formed a cluster together with the *Vibrio* sp. strain SA2 in the multi-locus gene analysis and the analysis of the 16S rRNA gene.

### <Reference Example 8> The results of analyses of biochemical and physiological properties in the Vibrio sp. strain SA2

The catalase reaction, oxidase reaction, acid production from glucose, gas production from glucose, glucose oxidation ability/fermentation ability (O/F test), and sensitivity to 0129 of the *Vibrio* sp. strain SA2 were analyzed based on the methods described in Barrow and Feltham, Cowan and Steel's Manual for the Identification of the Medical Bacteria. 3rd Edition. 1993, Cambridge University Press. Consequently, the *Vibrio* sp. strain SA2 exhibited the physiological properties as shown in Table 11.

### [Table 11]

**Table 11. Physiological properties of the Vibrio sp. strain SA2**

| Test item | Result |
|---|---|
| catalase reaction | + |
| oxidase reaction | + |
| acid production from glucose | + |
| gas production from glucose | + |
| O/F test (oxidation/fermentation) | +/+ |
| O129 sensitivity (150 µg) | + |

| | |
|---|---|
| (+: positive, -: negative) | |

Furthermore, the *Vibrio* sp. strain SA2 was tested by using a kit manufactured by bioMerieux SA to analyze physiological properties in Gram-negative bacteria, the API20NE kit. The results are shown in Table 12.

### [Table 12]

**Table 12. The test results of the Vibrio sp. strain SA2 with the API20NE kit**

| Test item | Result |
|---|---|
| Nitrate reduction biochemical test | + |
| Indole production biochemical test | - |
| Glucose acidification biochemical test | + |
| Arginine dihydrolase biochemical test | - |
| Urease biochemical test | - |
| Aesculin hydrolysis biochemical test | - |
| Gelatin hydrolysis biochemical test | - |
| β-Galactosidase biochemical test | + |
| Glucose assimilation test | - |
| L-Arabinose assimilation test | - |
| D-Mannose assimilation test | - |
| D-Mannitol assimilation test | - |
| N-Acetyl-D-glucosamine assimilation test | - |
| Maltose assimilation test | - |
| Potassium gluconate assimilation test | + |
| n-Capric acid assimilation test | - |
| Adipic acid assimilation test | - |
| dl-Malic acid assimilation test | + |
| Sodium citrate assimilation test | - |
| Phenyl acetate assimilation test | - |
| Cytochrome oxidase biochemical test | + |

| | |
|---|---|
| (+: positive, -: negative) | |

Furthermore, various enzymatic activities carried by the *Vibrio* sp. strain SA2 were tested by using a kit manufactured by bioMerieux SA to analyze biochemical properties (enzymatic activities) in microorganisms, the API ZYM kit. The results are shown in Table 13.

### [Table 13]

**Table 13. The results of biochemical tests with the API ZYM kit in the Vibrio sp. strain SA2**

| Test item | Result |
|---|---|
| Alkaline phosphatase activity | + |
| Esterase (C4) activity | + |
| Esterase/lipase (C8) activity | - |
| Lipase (C14) activity | - |
| Leucine aryl-amidase activity | + |
| Valine aryl-amidase activity | + |
| Cystine aryl-amidase activity | - |
| Trypsin activity | - |
| Chymotrypsin activity | - |
| Acid phosphatase activity | + |
| Naphthol-AS-BI-phosphohydrolase activity | + |
| α-Galactosidase activity | - |
| β-Galactosidase activity | + |
| β-Glucuronidase activity | - |
| α-Glucosidase activity | - |
| β-Glucosidase activity | - |
| N-Acetyl-β-glucosaminidase activity | + |
| α-Mannosidase activity | - |
| α-Fucosidase activity | - |

| | |
|---|---|
| (+: positive, -: negative) | |

From the above results and the investigation on the optimal culturing conditions, the *Vibrio* sp. strain SA2 (the strain NITE BP-01635) may be considered to be a microorganism having the characteristics described below.

The *Vibrio* sp. strain SA2 (the strain NITE BP-01635) is a Gram-negative bacterium. When cultured on agar or liquid medium supplemented with MB2216, the strain appears to be a rod-shaped bacterium with a diameter in the range of 0.7-0.8 µm and a width in the range of 1.5-2.5 µm without flagella and thus to be immotile. When cultured on agar medium supplemented MB2216 at 30°C for an incubation period of 24 hours, pale-yellow smooth-looking colonies were formed in a circular shape with a diameter in the range of 2.0-3.0 mm. When cultured on agar medium supplemented with MB2216 and in liquid culture medium supplemented with MB2216, the strain can be grown at a temperature in the range of 4-40°C and at pH in the range of 5-10. When the liquid medium supplemented with MB2216 is used, the conditions of temperature in the range of 25-30°C, pH in the range of 7-8 and the concentration of added NaCl in the range of 1-14% are the optimal conditions for the growth. When the concentration of added NaCl is above 15%, the strain cannot grow. Moreover, major constituent bacterial cell fatty acids were 16:1 w7c/16:1 w6c (38.18 %), 16:0 (27.97 %), 18:1 w7c (14.28 %), 14:0 3OH/16:1 iso I (8.65 %), 12:0 (3.86%), 18:0 (2.03%) and 14:0 (1.67%).

The above result and the results of the comparative analysis of the full-length 16S rRNA sequence, the multi-locus gene analysis and the DNA-DNA hybridization test in the present invention indicated that the *Vibrio* sp. strain SA2 (the strain NITE BP-01635) belonged to a new species of bacteria in the genus *Vibrio* but did not correspond to any already reported microorganism species. Thus, the inventors found that the *Vibrio* sp. strain SA2 (the strain NITE BP-01635) belonged to a new species of bacteria in the genus *Vibrio,* naming the new species, to which the *Vibrio* sp. strain SA2 belongs, *Vibrio alginovora* according to the International Code of Nomenclature of Bacteria.

### <Reference Example 9> Test-tube culture of the Vibrio sp. strain SA2 in minimal medium

As characteristics of conventional microorganism strains belonging to *Vibrio rumoiensis*, it had been reported that a temperature of 40°C was too high for the strains to grow and that the strains did not have the alginate lyase activity and, thus, the ability to assimilate alginic acid (Garrity, et al. (2005) Bergey's manual of systematic bacteriology, 2nd Edition Part B p526-527). Thus, the inventors plated 50 µL of each glycerol stock of the *Vibrio* sp. strain SA2 (the strain NITE BP-01635) and the *Escherichia coli* strain MG1655 (ATCC 47076 strain) as a comparison control strain individually on LB-agar medium supplemented with 15 g/L of NaCl and incubated statically at an incubation temperature of 37°C for 16 hours as a seed culture. After seed culturing, bacterial cells grown on agar medium were collected and suspended in 1 mL of sterilized saline with an inoculation loop and the turbidity determined by the absorbance at 600 nm was measured with a U-2000 spectrophotometer (manufactured by Hitachi, Ltd.). Then, the seed culture was inoculated as a main culture into 5 mL of the liquid minimal medium indicated below supplemented with sodium alginate as a sole carbon source to give a turbidity of 0.05, which is determined by the absorbance at 600 nm. By using a TVS062CA shaking incubator (manufactured by Advantec), test-tube culture was continuously performed for 9 hours under the conditions of 37°C and 70 rpm. Consequently, significant growth of the *Vibrio* sp. strain SA2 (the strain NITE BP-01635) caused by the alginate assimilation was observed. Thus, it was indicated that the *Vibrio* sp. strain SA2 was a new microorganism strain that had an ability to assimilate alginic acid and was different from conventional microorganism strains belonging to *Vibrio rumoiensis.*

**The composition of the liquid minimal medium with sodium alginate**

| | |
|---|---|
| Sodium alginate | 2.5 g/L |
| Na₂HPO₄ | 6 g/L |
| KH₂PO₄ | 3 g/L |
| NH₄Cl | 1 g/L |
| NaCl | 15.5 g/L |
| MgSO₄·7H₂O | 0.246 g/L |
| Thiamine·HCl | 10 mg/L |

### <Reference Example 10> Determination of the maximum specific growth rate of the Vibrio sp. strain SA2 in minimal medium at 37°C

Fifty µL of each glycerol stock of the *Vibrio* sp. strain SA2 (the strain NITE BP-01635) and the *Escherichia coli* strain MG1655 (ATCC 47076 strain) as a comparison control strain was individually plated on LB-agar medium supplemented with 15 g/L of NaCl and incubated statically at an incubation temperature of 37°C for 16 hours as a seed culture. After seed culturing, bacterial cells grown on agar medium were collected and suspended in 1 mL of sterilized saline with an inoculation loop and the turbidity determined by the absorbance at 600 nm was measured with a U-2000 spectrophotometer (manufactured by Hitachi, Ltd.). Then, the seed culture was inoculated into 5 mL of the liquid minimal medium indicated above supplemented with sodium alginate as a sole carbon source to give a turbidity of 0.05, which is determined by the absorbance at 600 nm. By using a TVS062CA shaking incubator (manufactured by Advantec), test-tube culture was continuously performed for 24 hours under the conditions of 37°C and 70 rpm.

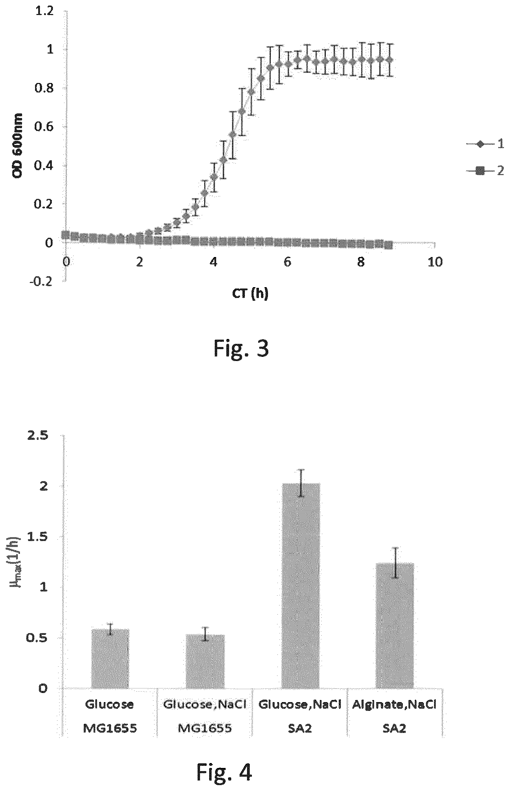

Similar test-tube culture was continuously performed for 24 hours under the conditions of 37°C and 70 rpm by exchanging the culture medium used in the main culture with each of the below-indicated two types of M9 minimal medium supplemented with glucose as a sole carbon source. The result is shown in Table 14 and Figure 3. The maximum specific growth rate in each condition was determined from the obtained data on the growth (Figure 4). Consequently, the maximum specific growth rate of the *Vibrio* sp. strain SA2 in the liquid minimal medium with sodium alginate was shown to be about twice as high as the specific growth rate of the *Escherichia coli* strain MG1655 in liquid M9 medium with glucose.

**The composition of liquid M9 medium with glucose**

| | |
|---|---|
| Glucose | 2.5 g/L |
| Na₂HPO₄ | 6 g/L |
| KH₂PO₄ | 3 g/L |
| NH₄Cl | 1 g/L |
| NaCl | 0.5 g/L |
| MgSO₄·7H₂O | 0.246 g/L |
| Thiamine·HCl | 10 mg/L |

**The composition of liquid M9 medium with glucose and NaCl**

| | |
|---|---|
| Glucose | 2.5 g/L |
| Na₂HPO₄ | 6 g/L |
| KH₂PO₄ | 3 g/L |
| NH₄Cl | 1 g/L |
| NaCl | 15.5 g/L |
| MgSO₄·7H₂O | 0.246 g/L |
| Thiamine·HCl | 10 mg/L |

### [Table 14]

**Table 14. Growth in minimal medium containing glucose or sodium alginate as a sole carbon source ○: good growth, ×: no growth observed**

| Culture medium used in a main culture | MG1655 | SA2 |
|---|---|---|
| Liquid M9 medium with glucose | ○ | × |
| Liquid M9 medium with glucose and NaCl | ○ | ○ |
| Liquid minimal medium with sodium alginate | × | ○ |

### <Reference Example 11> Determination of the growth-permissive temperature of the Vibrio sp. strain SA2 in minimal medium containing sodium alginate

Fifty µL of each glycerol stock of the *Vibrio* sp. strain SA2 (the strain NITE BP-01635) and the *Escherichia coli* strain MG1655 (ATCC 47076 strain) as a comparison control strain was individually plated on LB-agar medium supplemented with 15 g/L of NaCl and incubated statically at an incubation temperature of 37°C for 16 hours as a seed culture. Moreover, 50 µL each of a glycerol stock of the *Vibrio rumoiensis* strain S-1 (DSM 19141 strain) was plated on LB-agar medium supplemented with 15 g/L of NaCl and incubated statically at an incubation temperature of 31.5°C for 16 hours as a seed culture. After seed culturing, bacterial cells grown on agar medium were collected and suspended in 1 mL of sterilized saline with an inoculation loop and the turbidity determined by the absorbance at 600 nm was measured with a U-2000 spectrophotometer (manufactured by Hitachi, Ltd.). Then, the seed culture was inoculated into 5 mL of the liquid minimal medium indicated above supplemented with sodium alginate as a sole carbon source to give a turbidity of 0.05, which is determined by the absorbance at 600 nm. By using a TVS062CA shaking incubator (manufactured by Advantec), test-tube culture was continuously performed for 24 hours under the condition of 70 rpm while the temperature was changed in one degree increments from 37°C to 43°C. Consequently, as shown in Table 15, the *Vibrio* sp. strain SA2 (the strain NITE BP-01635) grew well in liquid minimal medium with sodium alginate at an incubation temperature of 40°C, indicating that the *Vibrio* sp. strain SA2 (the strain NITE BP-01635) was a microorganism capable of assimilating sodium alginate at 40°C. The above experimental result indicated that the *Vibrio* sp. strain SA2 was a new microorganism strain capable of growing at 40°C that had an ability to assimilate alginic acid and was different from conventional microorganism strains belonging to *Vibrio rumoiensis.*

### [Table 15]

**Table 15. The results of the determination of the growth-permissive temperature in minimal medium containing sodium alginate as a sole carbon source (○: good growth, ×: no growth observed)**

| Incubation temperature | MG1655 | *Vibrio rumoiensis* | SA2 |
|---|---|---|---|
| 37°C | × | × | ○ |
| 38°C | × | × | ○ |
| 39°C | × | × | ○ |
| 40°C | × | × | ○ |
| 41°C | × | × | × |
| 42°C | × | × | × |
| 43°C | × | × | × |

### <Reference Example 12> Comparison of the growth-permissive temperatures of the Vibrio sp. strain SA2 and other bacteria in the genus Vibrio on agar medium

Fifty µL of each glycerol stock of the *Vibrio* sp. strain SA2 (the strain NITE BP-01635) and *Vibrio rumoiensis* (type strain DSM19141 strain) and *Vibrio splendidus* (type strain ATCC33125) as comparison control strains was individually plated on LB-agar medium supplemented with Daigo's artificial seawater SP (Wako Pure Chemical Industries, Ltd.; catalog number: 395-01343) and incubated statically at an incubation temperature of 25°C for 16 hours as a seed culture. The obtained seed culture bacterial cells in a volume of about 10 µL were collected with a white inoculation loop and streaked on three types of culture media, that is, LB-agar medium supplemented with Daigo's artificial seawater SP, the sodium alginate-agar selection medium, and M9-agar medium supplemented with Daigo's artificial seawater SP and glucose and incubated statically at a temperature in the range from 25 to 39°C for 48 hours. Consequently, as shown in Tables 16 to 18, only the *Vibrio* sp. strain SA2 (the strain NITE BP-01635) was grown well at an incubation temperature of 34°C in the sodium alginate-agar selection medium, indicating that only the *Vibrio* sp. strain SA2 (the strain NITE BP-01635) was a microorganism capable of assimilating sodium alginate at a temperature of 34°C or higher.

**The composition of M9-agar medium supplemented with Daigo's artificial seawater SP and glucose**

| | |
|---|---|
| Glucose | 5 g/L |
| Na₂HPO₄ | 6 g/L |
| KH₂PO₄ | 3 g/L |
| NH₄Cl | 1 g/L |
| NaCl | 21.247 g/L |
| MgSO₄·7H₂O | 0.246 g/L |
| Thiamine·HCl | 10 mg/L |
| MgCl₂·6H₂O | 9.474 g/L |
| CaCl₂·2H₂O | 1.328 g/L |
| Na₂SO₄ | 3.505 g/L |
| KCl | 0.597 g/L |
| NaHCO₃ | 0.171 g/L |
| KBr | 0.085 g/L |
| Na₂B₄O₇·10H₂O | 0.034 g/L |
| SrCl₂ | 0.012 g/L |
| NaF | 3 mg/L |
| LiCl | 1 mg/L |
| KI | 0.07 mg/L |
| CoCl₂-6H₂O | 0.0002 mg/L |
| AlCl₃-6H₂O | 0.008 mg/L |
| FeCl₃·6H₂O | 0.006 mg/L |
| Na₂WO₄-2H₂O | 0.0002 mg/L |
| (NH₄)₆MO₇O₂₄·4H₂O | 0.0008 mg/L |
| Bacto Agar | 15 g/L |

### [Table 16]

**Table 16. The results of static culture in LB-agar medium supplemented with Daigo's artificial seawater SP**

| Incubation temperature | *Vibrio splendidus* | *Vibrio rumoiensis* | SA2 |
|---|---|---|---|
| 25°C | ○ | ○ | ○ |
| 30°C | Δ | ○ | ○ |
| 34°C | × | ○ | ○ |
| 37°C | × | ○ | ○ |
| 39°C | × | Δ | ○ |

| | | | |
|---|---|---|---|
| (○: good growth, Δ: faint growth, ×: no growth observed) | | | |

### [Table 17]

**Table 17. The results of static culture in the sodium alginate-agar selection medium**

| Incubation temperature | *Vibrio splendidus* | *Vibrio rumoiensis* | SA2 |
|---|---|---|---|
| 25°C | Δ | × | ○ |
| 30°C | Δ | × | ○ |
| 34°C | × | × | ○ |
| 37°C | × | × | ○ |
| 39°C | × | × | ○ |

| | | | |
|---|---|---|---|
| (○: good growth, Δ: faint growth, ×: no growth observed) | | | |

### [Table 18]

**Table 18. The results of static culture in M9-agar medium supplemented with Daigo's artificial seawater SP and glucose**

| Incubation temperature | *Vibrio splendidus* | *Vibrio rumoiensis* | SA2 |
|---|---|---|---|
| 25°C | × | × | ○ |
| 30°C | × | × | ○ |
| 34°C | × | × | ○ |
| 37°C | × | × | ○ |
| 39°C | × | × | ○ |

| | | | |
|---|---|---|---|
| (○: good growth, Δ: faint growth, ×: no growth observed) | | | |

### <Reference Example 13> Impartment of the ability to produce L-glutamic acid to the Vibrio sp. strain SA2

The genomic DNA of the *Escherichia coli* strain MG1655 was extracted by using the PurElute Bacterial Genomic kit (manufactured by Edge BioSystems, Inc.). To heterogeneously express in the *Vibrio* sp. strain SA2 the YbjL protein represented by SEQ ID NO: 86, a PCR was performed by using the obtained genomic DNA as a template and primers represented by SEQ ID NOs: 87 and 88 and the amplified DNA was purified according to a conventional method and connected to the vector pMW219 (manufactured by Nippon Gene Co., Ltd.), which had been digested with the restriction enzymes *Hin*dIII and *Sal*I*,* by using the In-Fusion HD Cloning Kit (manufactured by Takara Bio Inc.) to construct the plasmid pMW219-ybjL for the expression of the amplified *ybjL.*

The *Vibrio* sp. strain SA2 was cultured with agitation at an incubation temperature of 37°C and a revolution speed of 120 rpm in two Sakaguchi flasks each containing 40 mL of liquid LB medium containing 15 g/L of NaCl until the OD at 600 nm reached around 0.5. Eighty mL of the obtained culture was centrifuged under the conditions of a revolution speed of 7000 rpm, an operation time of 7 minutes, and a temperature of 4°C to collect bacterial cells. The collected cells were washed twice with 15 mL of an ice-cold sterilized solution of 2 mM HEPES, 100 mM sucrose, 5 mM CaCl₂ (after suspended, centrifuged under the conditions of a revolution speed of 7000 rpm, an operation time of 7 minutes, and a temperature of 4°C to collect bacterial cells) and then suspended in 1 mL of 10% sterilized glycerol solution to obtain electrocompetent cells of the *Vibrio* sp. strain SA2. Ethanol-precipitated pMW219-ybjL plasmid DNA (not less than 1 µg) was added to 100 µL of the *Vibrio* sp. strain SA2 and the pMW219-ybjL plasmid wad introduced into the *Vibrio* sp. strain SA2 by electroporation at 1.8 kV and 25 µF. To the electroporated electrocompetent cells, 0.5 mL of liquid LB medium containing 15 g/L of NaCl was added and incubated statically at 37°C for 2.5 hours to make a recovery culture and then incubated statically for 24 hours on LB-agar medium containing 15 g/L of NaCl and 40 mg/L of kanamycin to observe the colony formation. The obtained colonies were further subjected to SI with LB-agar medium containing 15 g/L of NaCl and 40 mg/L of kanamycin and then suspended in 4 mL of liquid LB medium containing 15 g/L of NaCl and 40 mg/L of kanamycin to perform test-tube culture. The culture was performed by shaking a test tube under the conditions of an incubation temperature of 37°C and a reciprocal shaking speed of 120 rpm. Once the OD determined by the absorbance at 600 nm reached about 0.3, a glycerol stock was produced by adding an equal volume of 20% sterilized glycerol and freezing at -80°C, and the glycerol stock of the obtained strain was named the glycerol stock of the *Vibrio* sp. SA2/pMW219-ybjL strain.

Sodium alginate in an amount of 5 g was added to 100 mL of 3 N sulfuric acid and subjected to a heat treatment at 65°C for three hours. The obtained sodium alginate hydrolysate solution was adjusted to pH 7.0 by adding KOH to obtain a sugar solution of sodium alginate hydrolysate. The obtained sugar solution of sodium alginate hydrolysate was used to prepare the below-indicated liquid minimal medium containing sodium alginate hydrolysate.

**The composition of the liquid minimal medium containing sodium alginate hydrolysate**

| | |
|---|---|
| Sodium alginate hydrolysate | 2.5 g/L |
| Na₂HPO₄ | 6 g/L |
| KH₂PO₄ | 3 g/L |
| NH₄Cl | 1 g/L |
| NaCl | 15.5 g/L |
| MgSO₄·7H₂O | 0.246 g/L |
| Thiamine·HCl | 10 mg/L |
| Kanamycin | 40 mg/L |

The glycerol stock of the *Vibrio* sp. SA2/pMW219-ybjL strain was thawed and 100 µL each of the glycerol stock was applied uniformly onto LB-agar medium containing 15 g/L of NaCl and 40 mg/L of kanamycin and incubated statically at 30°C for 48 hours. About one fourth of the bacterial cells in the obtained plate were suspended in 0.5 mL of saline and the turbidity at a wavelength of 600 nm was measured with a U-2000 spectrophotometer (manufactured by Hitachi, Ltd.). Five mL of the above-described liquid minimal medium containing sodium alginate hydrolysate was poured into an L-shaped tube and inoculated with the obtained suspension containing bacteria in such a liquid volume as to produce a turbidity of 0.05 at a wavelength of 600 nm and incubated at 34°C for 22 hours in a TN-1506 rocking incubator (manufactured by Advantec Toyo Kaisha, Ltd.) at a revolution speed of 70 rpm. After completion of the culture, the amount of L-glutamic acid accumulated in the culture medium was determined by using the Biotec Analyzer AS-310 (Sakura SI Co. Ltd.).

The result is shown in Table 19. As indicated in Table 19, the bacterial strain obtained through the impartment of the ability to produce L-glutamic acid by introducing pMW219-ybjL into the *Vibrio* sp. strain SA2, which had been originally isolated as a microorganism capable of assimilating alginic acid as a sole carbon source, accumulated a significant amount of L-glutamic acid in the culture medium containing alginic acid as a sole carbon source.

### [Table 19]

**Table 19. The result of the production of L-glutamic acid from alginic acid using the Vibrio sp.SA2/pMW219-ybjL strain**

| Bacterial strain | Accumulated L-glutamic acid (mg/L) |
|---|---|
| *Vibrio* sp. strain SA2/pMW219 | 1.7 |
| *Vibrio* sp. SA2/pMW219-ybjL | 21.7 |

### Description of sequences

SEQ ID NO: 1: the nucleotide sequence of the 16S ribosomal RNA gene in the *Vibrio* sp. strain SA2,
SEQ ID NO: 2: the nucleotide sequence of the *toaA* gene in the *Vibrio* sp. strain SA2,
SEQ ID NO: 3: the amino acid sequence of the ToaA protein in the *Vibrio* sp. strain SA2,
SEQ ID NO: 4: the nucleotide sequence of the *alyD* gene in the *Vibrio* sp. strain SA2,
SEQ ID NO: 5: the amino acid sequence of the AlyD protein in the *Vibrio* sp. strain SA2,
SEQ ID NO: 6: the nucleotide sequence of the *eda* gene in the *Vibrio* sp. strain SA2,
SEQ ID NO: 7: the amino acid sequence of the EDA protein in the *Vibrio* sp. strain SA2,
SEQ ID NO: 8: the nucleotide sequence of the *kdgK* gene in the *Vibrio* sp. strain SA2,
SEQ ID NO: 9: the amino acid sequence of the KdgK protein in the *Vibrio* sp. strain SA2,
SEQ ID NO: 10: the nucleotide sequence of the *oalB* gene in the *Vibrio* sp. strain SA2,
SEQ ID NO: 11: the amino acid sequence of the OalB protein in the *Vibrio* sp. strain SA2,
SEQ ID NO: 12: the nucleotide sequence of the *oalC* gene in the *Vibrio* sp. strain SA2,
SEQ ID NO: 13: the amino acid sequence of the OalC protein in the *Vibrio* sp. strain SA2,
SEQ ID NO: 14: the nucleotide sequence of the *kdgN* gene in the *Vibrio* sp. strain SA2,
SEQ ID NO: 15: the amino acid sequence of the KdgN protein in the *Vibrio* sp. strain SA2,
SEQ ID NO: 16: the nucleotide sequence of the *toaB* gene in the *Vibrio* sp. strain SA2,
SEQ ID NO: 17: the amino acid sequence of the ToaB protein in the *Vibrio* sp. strain SA2,
SEQ ID NO: 18: the nucleotide sequence of the *toaC* gene in the *Vibrio* sp. strain SA2,
SEQ ID NO: 19: the amino acid sequence of the ToaC protein in the *Vibrio* sp. strain SA2,
SEQ ID NO: 20: the nucleotide sequence of the *alyB* gene in the *Vibrio* sp. strain SA2,
SEQ ID NO: 21: the amino acid sequence of the AlyB protein in the *Vibrio* sp. strain SA2,
SEQ ID NO: 22: the nucleotide sequence of the *dehR* gene in the *Vibrio* sp. strain SA2,
SEQ ID NO: 23: the amino acid sequence of the DehR protein in the *Vibrio* sp. strain SA2,
SEQ ID NO: 24: pSTV29-kdgN-alyB-toaA-oalC (pSA2S2),
SEQ ID NO: 25: pMW219-dehR-oalB-toaB-toaC-alyD-eda-kdgK (pSA2M4),
SEQ ID NO: 26: a primer for amplifying *kdgN* derived from the *Vibrio* sp. strain SA2,
SEQ ID NO: 27: a primer for amplifying *kdgN* derived from the *Vibrio* sp. strain SA2,
SEQ ID NO: 28: a primer for amplifying *alyB* derived from the *Vibrio* sp. strain SA2,
SEQ ID NO: 29: a primer for amplifying *alyB* derived from the *Vibrio* sp. strain SA2,
SEQ ID NO: 30: a primer for amplifying *toaA* derived from the *Vibrio* sp. strain SA2,
SEQ ID NO: 31: a primer for amplifying *toaA* derived from the *Vibrio* sp. strain SA2,
SEQ ID NO: 32: a primer for amplifying *oalC* derived from the *Vibrio* sp. strain SA2,
SEQ ID NO: 33: a primer for amplifying *oalC* derived from the *Vibrio* sp. strain SA2,
SEQ ID NO: 34: a primer for amplifying the entirety of pSTV29-kdgN-alyB-toaA,
SEQ ID NO: 35: a primer for amplifying the entirety of pSTV29-kdgN-alyB-toaA,
SEQ ID NO: 36: a primer for amplifying the *oalC* gene,
SEQ ID NO: 37: a primer for amplifying the *oalC* gene,
SEQ ID NO: 38: a primer for amplifying *dehR* derived from the *Vibrio* sp. strain SA2,
SEQ ID NO: 39: a primer for amplifying *dehR* derived from the *Vibrio* sp. strain SA2,
SEQ ID NO: 40: a primer for amplifying *oalB* derived from the *Vibrio* sp. strain SA2,
SEQ ID NO: 41: a primer for amplifying *oalB* derived from the *Vibrio* sp. strain SA2,
SEQ ID NO: 42: a primer for amplifying *toaB* and *toaC* derived from the *Vibrio* sp. strain SA2,
SEQ ID NO: 43: a primer for amplifying *toaB* and *toaC* derived from the *Vibrio* sp. strain SA2,
SEQ ID NO: 44: a primer for amplifying *alyD* derived from the *Vibrio* sp. strain SA2,
SEQ ID NO: 45: a primer for amplifying *alyD* derived from the *Vibrio* sp. strain SA2,
SEQ ID NO: 46: a primer for amplifying *eda* derived from the *Vibrio* sp. strain SA2,
SEQ ID NO: 47: a primer for amplifying *eda* derived from the *Vibrio* sp. strain SA2,
SEQ ID NO: 48: a primer for amplifying *kdgK* derived from the *Vibrio* sp. strain SA2,
SEQ ID NO: 49: a primer for amplifying *kdgK* derived from the *Vibrio* sp. strain SA2,
SEQ ID NO: 50: a primer for amplifying the entirety of pMW219-dehR-oalB-toaB-toaC,
SEQ ID NO: 51: a primer for amplifying the entirety of pMW219-dehR-oalB-toaB-toaC,
SEQ ID NO: 52: a primer for amplifying the *alyD*, *eda* and *kdgK* genes,
SEQ ID NO: 53: a primer for amplifying the *alyD*, *eda* and *kdgK* genes,
SEQ ID NO: 54: the nucleotide sequence of the *uxuA* gene in *Escherichia coli,*
SEQ ID NO: 55: the amino acid sequence of the UxuA protein in *Escherichia coli,*
SEQ ID NO: 56: the nucleotide sequence of the *kdgK* gene in *Escherichia coli,*
SEQ ID NO: 57: the amino acid sequence of the KdgK protein in *Escherichia coli,*
SEQ ID NO: 58: the nucleotide sequence of the *eda* gene in *Escherichia coli,*
SEQ ID NO: 59: the amino acid sequence of the EDA protein in *Escherichia coli,*
SEQ ID NO: 60: a primer for amplifying EPI,
SEQ ID NO: 61: a primer for amplifying EPI,
SEQ ID NO: 62: a forward primer for constructing pMW119-attR-cat-attL-P₁₄-alyB,
SEQ ID NO: 63: a reverse primer for constructing pMW119-attR-cat-attL-P₁₄-alyB,
SEQ ID NO: 64: a forward primer for amplifying a linear DNA of pMW119-attR-cat-attL-P₁₄,
SEQ ID NO: 65: a reverse primer for amplifying a linear DNA of pMW119-attR-cat-attL-P₁₄,
SEQ ID NO: 66: a forward primer for constructing pMW119-attR-cat-attL-P₁₄-alyD,
SEQ ID NO: 67: a reverse primer for constructing pMW119-attR-cat-attL-P₁₄-alyD,
SEQ ID NO: 68: a forward primer for cloning *alyB* derived from *Vibrio splendidus* (type strain ATCC33125),
SEQ ID NO: 69: a reverse primer for cloning *alyB* derived from *Vibrio splendidus* (type strain ATCC33125),
SEQ ID NO: 70: a forward primer for constructing pMW119-attR-cat-attL-P₁₄-dehR,
SEQ ID NO: 71: a reverse primer for constructing pMW119-attR-cat-attL-P₁₄-dehR,
SEQ ID NO: 72: a forward primer for enhancing *dehR* on the chromosome,
SEQ ID NO: 73: a reverse primer for enhancing *dehR* on the chromosome,
SEQ ID NO: 74: a forward primer for enhancing *alyD* on the chromosome,
SEQ ID NO: 75: a reverse primer for enhancing *alyD* on the chromosome,
SEQ ID NO: 76: a forward primer for enhancing *alyB* on the chromosome,
SEQ ID NO: 77: a reverse primer for enhancing *alyB* on the chromosome,
SEQ ID NO: 78: the nucleotide sequence of the promoter P₁₄
SEQ ID NO: 79: a primer,
SEQ ID NO: 80: a primer,
SEQ ID NO: 81: a primer,
SEQ ID NO: 82: a primer,
SEQ ID NO: 83: a primer,
SEQ ID NO: 84: a primer,
SEQ ID NO: 85: the nucleotide sequence of the *ybjL* gene derived from *Escherichia coli,*
SEQ ID NO: 86: the amino acid sequence of the YbjL protein derived from *Escherichia coli,*
SEQ ID NO: 87: a primer,
SEQ ID NO: 88: a primer,
SEQ ID NO: 89: the nucleotide sequence of the *atpA* gene in the *Vibrio* sp. strain SA2,
SEQ ID NO: 90: the nucleotide sequence of the *pyrH* gene in the *Vibrio* sp. strain SA2,
SEQ ID NO: 91: the nucleotide sequence of the *recA* gene in the *Vibrio* sp. strain SA2,
SEQ ID NO: 92: the nucleotide sequence of the *rpoA* gene in the *Vibrio* sp. strain SA2,
SEQ ID NO: 93: the nucleotide sequence of the *rpoD* gene in the *Vibrio* sp. strain SA2,
SEQ ID NO: 94: the nucleotide sequence of the *rpoD* gene in *Vibrio casei,*
SEQ ID NO: 95: the nucleotide sequence of the *rpoD* gene in *Vibrio litoralis,*
SEQ ID NO: 96: the nucleotide sequence of the *rpoD* gene in *Vibrio rumoiensis,*
SEQ ID NO: 97: the nucleotide sequence of the *pyrH* gene in *Vibrio rumoiensis,*
SEQ ID NO: 98: the nucleotide sequence of the 16S ribosomal RNA gene in *Vibrio rumoiensis.*

### INDUSTRIAL APPLICABILITY

The present invention enables L-amino acids to be produced by fermentation from a seaweed-derived carbon source including alginic acid.

## Claims

1. A method of producing an L-amino acid by a fermentation method, the method comprising: culturing a bacterium modified to have an ability to produce the L-amino acid and an ability to assimilate alginic acid in a culture medium containing alginic acid as a carbon source obtained from seaweed-derived biomass, or containing alginic acid as a carbon source obtained from seaweed-derived biomass which alginic acid is subjected to an enzymatic degradation reaction by an alginate lyase or an acid hydrolysis reaction, to produce and accumulate the L-amino acid in the culture medium in an amount of not less than 1.0 g/l; and collecting the L-amino acid from the culture medium.

2. The method according to claim 1, wherein the seaweed is a large type of seaweed included in brown algae, red algae, and green algae.

3. The method according to claim 1 or 2, wherein the carbon source obtained from seaweed-derived biomass additionally contains one or more selected from cellulose, mannitol, pectin, galacturonic acid, carrageenan, agar, fucoidan, laminarin, and xylan.

4. The method according to any one of claims 1 to 3, wherein the bacterium is a bacterium having an enhanced alginate lyase.

5. The method according to claim 4, wherein the alginate lyase is AlyB protein or AlyD protein.

6. The method according to any one of claims 1 to 5, wherein the bacterium is a bacterium having an increased alginate importer activity.

7. The method according to claim 6, wherein the alginate importer is Kdg protein or Toa protein.

8. The method according to any one of claims 1 to 7, wherein the bacterium is a bacterium having an increased keto acid reductase activity.

9. The method according to claim 8, wherein the keto acid reductase is an enzyme selected from NADPH/NADH dehydrogenase, D-mannonate dehydratase, 2-keto-3-deoxygluconokinase, and 2-keto-3-deoxy-6-phosphogluconate aldolase.

10. The method according to any one of claims 4 to 9, wherein the alginate lyase, alginate importer and keto acid reductase are enzymes derived from a *Vibrio* bacterium having an ability to assimilate alginic acid.

11. The method according to claim 10, wherein the *Vibrio* bacterium having an ability to assimilate alginic acid belongs to *Vibrio alginovora.*

12. The method according to claim 10, wherein the *Vibrio* bacterium having an ability to assimilate alginic acid is derived from the *Vibrio* sp. strain NITE BP-01635.

13. The method according to any one of claims 1 to 12, wherein the bacterium is *Pantoea ananatis* or *Escherichia coli.*

14. An isolated DNA selected from (a) to (w) below:
(a) a DNA encoding the amino acid sequence of SEQ ID NO: 3,
(b) a DNA encoding a protein having the amino acid sequence of SEQ ID NO: 3 in which 1 to 10 amino acids are substituted, deleted, inserted or added, the DNA encoding an alginate importer,
(c) a DNA encoding the amino acid sequence of SEQ ID NO: 5,
(d) a DNA encoding a protein having the amino acid sequence of SEQ ID NO: 5 in which 1 to 10 amino acids are substituted, deleted, inserted or added, the DNA encoding a protein having the alginate lyase activity,
(e) a DNA encoding the amino acid sequence of SEQ ID NO: 7,
(f) a DNA encoding a protein having the amino acid sequence of SEQ ID NO: 7 in which 1 to 10 amino acids are substituted, deleted, inserted or added, the DNA encoding a protein having the 2-keto-3-deoxy-6-phosphogluconate aldolase activity,
(g) a DNA encoding the amino acid sequence of SEQ ID NO: 9,
(h) a DNA encoding a protein having the amino acid sequence of SEQ ID NO: 9 in which 1 to 10 amino acids are substituted, deleted, inserted or added, the DNA encoding a protein having the 2-keto-3-deoxygluconokinase activity,
(i) a DNA encoding the amino acid sequence of SEQ ID NO: 11,
(j) a DNA encoding a protein having the amino acid sequence of SEQ ID NO: 11 in which 1 to 10 amino acids are substituted, deleted, inserted or added, the DNA encoding a protein having the alginate lyase activity,
(k) a DNA encoding the amino acid sequence of SEQ ID NO: 13,
(1) a DNA encoding a protein having the amino acid sequence of SEQ ID NO: 13 in which 1 to 10 amino acids are substituted, deleted, inserted or added, the DNA encoding a protein having the alginate lyase activity,
(m) a DNA encoding the amino acid sequence of SEQ ID NO: 15,
(n) a DNA encoding a protein having the amino acid sequence of SEQ ID NO: 15 in which 1 to 10 amino acids are substituted, deleted, inserted or added, the DNA encoding an alginate importer protein,
(o) a DNA encoding the amino acid sequence of SEQ ID NO: 17,
(p) a DNA encoding a protein having the amino acid sequence of SEQ ID NO: 17 in which 1 to 10 amino acids are substituted, deleted, inserted or added, the DNA encoding an alginate importer protein,
(q) a DNA encoding the amino acid sequence of SEQ ID NO: 19,
(r) a DNA encoding a protein having the amino acid sequence of SEQ ID NO: 19 in which 1 to 10 amino acids are substituted, deleted, inserted or added, the DNA encoding an alginate importer protein,
(s) a DNA encoding the amino acid sequence of SEQ ID NO: 21,
(t) a DNA encoding a protein having the amino acid sequence of SEQ ID NO: 21 in which 1 to 10 amino acids are substituted, deleted, inserted or added, the DNA encoding a protein having the alginate lyase activity,
(u) a DNA encoding the amino acid sequence of SEQ ID NO: 23,
(w) a DNA encoding a protein having the amino acid sequence of SEQ ID NO: 23 in which 1 to 10 amino acids are substituted, deleted, inserted or added, the DNA encoding a protein having the NADP/NADPH dehydrogenase activity.

## Patentansprüche

1. Verfahren zur Herstellung einer L-Aminosäure durch ein Fermentationsverfahren, wobei das Verfahren umfasst: das Kultivieren eines Bakteriums, das so modifiziert ist, dass es die Fähigkeit zur Herstellung der L-Aminosäure und die Fähigkeit zur Assimilation von Alginsäure in einem Kulturmedium aufweist, das Alginsäure als Kohlenstoffquelle enthält, die aus Seetang-abgeleiteter Biomasse erhalten wird, oder das Alginsäure als Kohlenstoffquelle enthält, die aus Seetang-abgeleiteter Biomasse erhalten wird, wobei die Alginsäure einer enzymatischen Abbaureaktion durch eine Alginatlyase oder eine Säurehydrolysereaktion unterworfen wird, zur Herstellung und Anreicherung der L-Aminosäure im Kulturmedium in einer Menge von nicht weniger als 1,0 g/l; und das Gewinnen der L-Aminosäure aus dem Kulturmedium.

2. Verfahren nach Anspruch 1, worin der Seetang eine große Art von Seetang ist, die in Braun-, Rottalgen und Grünalgen enthalten ist.

3. Verfahren nach Anspruch 1 oder 2, worin die aus Seetang-abgeleiteter Biomasse erhaltene Kohlenstoffquelle zusätzlich eine oder mehrere enthält, die aus Cellulose, Mannitol, Pektin, Galacturonsäure, Carrageenan, Agar, Fucoidan, Laminarin und Xylan ausgewählt ist.

4. Verfahren nach einem der Ansprüche 1 bis 3, worin das Bakterium ein Bakterium mit einer verstärkten Alginatlyase ist.

5. Verfahren nach Anspruch 4, worin die Alginatlyase AlyB-Protein oder AlyD-Protein ist.

6. Verfahren nach einem der Ansprüche 1 bis 5, worin das Bakterium ein Bakterium mit einer erhöhten Alginat-Importeraktivität ist.

7. Verfahren nach Anspruch 6, worin der Alginatimporter Kdg-Protein oder Toa-Protein ist.

8. Verfahren nach einem der Ansprüche 1 bis 7, worin das Bakterium ein Bakterium mit einer erhöhten Ketosäurereduktaseaktivität ist.

9. Verfahren nach Anspruch 8, worin die Ketosäure-Reduktase ein aus NADPH/NADH-Dehydrogenase, D-Mannonatdehydratase, 2-Keto-3-Desoxygluconokinase und 2-Keto-3-Desoxy-6-phosphogluconataldolase ausgewähltes Enzym ist.

10. Verfahren nach einem der Ansprüche 4 bis 9, worin die Alginatlyase, der Alginatimporter und die Ketosäurereduktase Enzyme sind, die von einem Vibriobakterium mit der Fähigkeit zur Aufnahme von Alginsäure abgeleitet sind.

11. Verfahren nach Anspruch 10, worin das *Vibriobakterium* mit der Fähigkeit zur Aufnahme von Alginsäure zu *Vibrio alginovora* gehört.

12. Verfahren nach Anspruch 10, worin das *Vibriobakterium* mit der Fähigkeit zur Aufnahme von Alginsäure von dem *Vibrio* sp. Stamm NITE BP-01635 abgeleitet ist.

13. Verfahren nach einem der Ansprüche 1 bis 12, worin das zur Familie der Enterobacteriaceae gehörende Bakterium *Pantoea ananatis* oder *Escherichia coli* ist.

14. Isolierte DNA, ausgewählt aus den nachstehenden (a) bis (w):
(a) eine DNA, die für die Aminosäuresequenz von SEQ ID NO: 3 kodiert,
(b) eine DNA, die für ein Protein mit der Aminosäuresequenz von SEQ ID NO: 3 kodiert, in der 1 bis 10 Aminosäuren substituiert, deletiert, inseriert oder hinzugefügt sind, wobei die DNA für einen Alginat-Importer kodiert,
(c) eine DNA, die für die Aminosäuresequenz von SEQ ID NO: 5 kodiert,
(d) eine DNA, die für ein Protein mit der Aminosäuresequenz von SEQ ID NO: 5 kodiert, in der 1 bis 10 Aminosäuren substituiert, deletiert, inseriert oder hinzugefügt sind, wobei die DNA für ein Protein mit der Alginatlyaseaktivität kodiert,
(e) eine DNA, die für die Aminosäuresequenz von SEQ ID NO: 7 kodiert,
(f) eine DNA, die für ein Protein mit der Aminosäuresequenz von SEQ ID NO: 7 kodiert, in der 1 bis 10 Aminosäuren substituiert, deletiert, inseriert oder hinzugefügt sind, wobei die DNA für ein Protein mit der Aktivität der 2-Keto-3-deoxy-6-phosphogluconat-Aldolase kodiert,
(g) eine DNA, die für die Aminosäuresequenz von SEQ ID NO: 9 kodiert,
(h) eine DNA, die für ein Protein mit der Aminosäuresequenz von SEQ ID NO: 9 kodiert, in der 1 bis 10 Aminosäuren substituiert, deletiert, inseriert oder hinzugefügt sind, wobei die DNA für ein Protein mit der Aktivität der 2-Keto-3-deoxygluconokinase kodiert,
(i) eine DNA, die für die Aminosäuresequenz von SEQ ID NO: 11 kodiert,
(j) eine DNA, die für ein Protein mit der Aminosäuresequenz von SEQ ID NO: 11 kodiert, in der 1 bis 10 Aminosäuren substituiert, deletiert, inseriert oder hinzugefügt sind, wobei die DNA für ein Protein mit der Alginatlyaseaktivität kodiert,
(k) eine DNA, die für die Aminosäuresequenz von SEQ ID NO: 13 kodiert,
(1) eine DNA, die für ein Protein mit der Aminosäuresequenz von SEQ ID NO: 13 kodiert, in der 1 bis 10 Aminosäuren substituiert, deletiert, inseriert oder hinzugefügt sind, wobei die DNA für ein Protein mit der Alginatlyaseaktivität kodiert,
(m) eine DNA, die für die Aminosäuresequenz von SEQ ID NO: 15 kodiert,
(n) eine DNA, die für ein Protein mit der Aminosäuresequenz von SEQ ID NO: 15 kodiert, in der 1 bis 10 Aminosäuren substituiert, deletiert, inseriert oder hinzugefügt sind, wobei die DNA für ein Alginat-Importerprotein kodiert,
(o) eine DNA, die für die Aminosäuresequenz von SEQ ID NO: 17 kodiert,
(p) eine DNA, die für ein Protein mit der Aminosäuresequenz von SEQ ID NO: 17 kodiert, in der 1 bis 10 Aminosäuren substituiert, deletiert, inseriert oder hinzugefügt sind, wobei die DNA für ein Alginat-Importerprotein kodiert,
(q) eine DNA, die für die Aminosäuresequenz von SEQ ID NO: 19 kodiert,
(r) eine DNA, die für ein Protein mit der Aminosäuresequenz von SEQ ID NO: 19 kodiert, in der 1 bis 10 Aminosäuren substituiert, deletiert, inseriert oder hinzugefügt sind, wobei die DNA für ein Alginat-Importerprotein kodiert,
(s) eine DNA, die für die Aminosäuresequenz von SEQ ID NO: 21 kodiert,
(t) eine DNA, die für ein Protein mit der Aminosäuresequenz von SEQ ID NO: 21 kodiert, in der 1 bis 10 Aminosäuren substituiert, deletiert, inseriert oder hinzugefügt sind, wobei die DNA für ein Protein mit der Alginatlyaseaktivität kodiert,
(u) eine DNA, die für die Aminosäuresequenz von SEQ ID NO: 23 kodiert,
(w) eine DNA, die für ein Protein mit der Aminosäuresequenz von SEQ ID NO: 23 kodiert, in der 1 bis 10 Aminosäuren substituiert, deletiert, inseriert oder hinzugefügt sind, wobei die DNA für ein Protein mit der NADP/NADPH-Dehydrogenaseaktivität kodiert.

## Revendications

1. Procédé de production d'un acide L-aminé par un procédé de fermentation, le procédé comprenant : la culture d'une bactérie modifiée pour présenter une aptitude à la production de l'acide L-aminé et une aptitude à l'assimilation d'acide alginique dans un milieu de culture contenant de l'acide alginique en tant que source de carbone obtenue à partir d'une biomasse d'origine algale, ou contenant de l'acide alginique en tant que source de carbone obtenue à partir d'une biomasse d'origine algale, lequel acide alginique est soumis à une réaction de dégradation enzymatique par une alginate lyase ou une réaction d'hydrolyse acide, pour produire et accumuler l'acide L-aminé dans le milieu de culture en une quantité non inférieure à 1,0 g/l ; et la récupération de l'acide L-aminé à partir du milieu de culture.

2. Procédé selon la revendication 1, dans lequel l'algue est un type d'algue de grande taille incluse parmi les algues brunes, les algues rouges et les algues vertes.

3. Procédé selon la revendication 1 ou 2, dans lequel la source de carbone obtenue à partir d'une biomasse d'origine algale contient en outre un ou plusieurs choisis parmi la cellulose, le mannitol, la pectine, l'acide galacturonique, la carraghénine, l'agar-agar, le fucoïdane, la laminarine et le xylane.

4. Procédé selon l'une quelconque des revendications 1 à 3, dans lequel la bactérie est une bactérie présentant une teneur accrue en alginate lyase.

5. Procédé selon la revendication 4, dans lequel l'alginate lyase est une protéine AlyB ou une protéine AlyD.

6. Procédé selon l'une quelconque des revendications 1 à 5, dans lequel la bactérie est une bactérie présentant une activité importateur d'alginate accrue.

7. Procédé selon la revendication 6, dans lequel l'importateur d'alginate est une protéine Kdg ou une protéine Toa.

8. Procédé selon l'une quelconque des revendications 1 à 7, dans lequel la bactérie est une bactérie présentant une activité cétoacide réductase accrue.

9. Procédé selon la revendication 8, dans lequel la cétoacide réductase est une enzyme choisie parmi la NADPH/NADH déshydrogénase, la D-mannonate déshydratase, la 2-céto-3-désoxygluconokinase et la 2-céto-3-désoxy-6-phosphogluconate aldolase.

10. Procédé selon l'une quelconque des revendications 4 à 9, dans lequel l'alginate lyase, l'importateur d'alginate et la cétoacide réductase sont des enzymes dérivées d'une bactérie *Vibrio* présentant une aptitude à l'assimilation d'acide alginique.

11. Procédé selon la revendication 10, dans lequel la bactérie *Vibrio* présentant une aptitude à l'assimilation d'acide alginique appartient à *Vibrio alginovora.*

12. Procédé selon la revendication 10, dans lequel la bactérie *Vibrio* présentant une aptitude à l'assimilation d'acide alginique est dérivée de la souche NITE BP-01635 de l'espèce *Vibrio.*

13. Procédé selon l'une quelconque des revendications 1 à 12, dans lequel la bactérie est *Pantoea ananatis* ou *Escherichia coli.*

14. ADN isolé choisi parmi (a) à (w) ci-dessous :
(a) un ADN codant pour la séquence d'acides aminés de SEQ ID N° : 3,
(b) un ADN codant pour une protéine présentant la séquence d'acides aminés de SEQ ID N° : 3 dans laquelle 1 à 10 acides aminés sont substitués, supprimés, insérés ou ajoutés, l'ADN codant pour un importateur d'alginate,
(c) un ADN codant pour la séquence d'acides aminés de SEQ ID N° : 5,
(d) un ADN codant pour une protéine présentant la séquence d'acides aminés de SEQ ID N° : 5 dans laquelle 1 à 10 acides aminés sont substitués, supprimés, insérés ou ajoutés, l'ADN codant pour une protéine présentant l'activité alginate lyase,
(e) un ADN codant pour la séquence d'acides aminés de SEQ ID N° : 7,
(f) un ADN codant pour une protéine présentant la séquence d'acides aminés de SEQ ID N° : 7 dans laquelle 1 à 10 acides aminés sont substitués, supprimés, insérés ou ajoutés, l'ADN codant pour une protéine présentant l'activité 2-céto-3-désoxy-6-phosphogluconate aldolase,
(g) un ADN codant pour la séquence d'acides aminés de SEQ ID N° : 9,
(h) un ADN codant pour une protéine présentant la séquence d'acides aminés de SEQ ID N° : 9 dans laquelle 1 à 10 acides aminés sont substitués, supprimés, insérés ou ajoutés, l'ADN codant pour une protéine présentant l'activité 2-céto-3-désoxygluconokinase,
(i) un ADN codant pour la séquence d'acides aminés de SEQ ID N° : 11,
(j) un ADN codant pour une protéine présentant la séquence d'acides aminés de SEQ ID N° : 11 dans laquelle 1 à 10 acides aminés sont substitués, supprimés, insérés ou ajoutés, l'ADN codant pour une protéine présentant l'activité alginate lyase,
(k) un ADN codant pour la séquence d'acides aminés de SEQ ID N° : 13,
(1) un ADN codant pour une protéine présentant la séquence d'acides aminés de SEQ ID N° : 13 dans laquelle 1 à 10 acides aminés sont substitués, supprimés, insérés ou ajoutés, l'ADN codant pour une protéine présentant l'activité alginate lyase,
(m) un ADN codant pour la séquence d'acides aminés de SEQ ID N° : 15,
(n) un ADN codant pour une protéine présentant la séquence d'acides aminés de SEQ ID N° : 15 dans laquelle 1 à 10 acides aminés sont substitués, supprimés, insérés ou ajoutés, l'ADN codant pour une protéine importatrice d'alginate,
(o) un ADN codant pour la séquence d'acides aminés de SEQ ID N° : 17,
(p) un ADN codant pour une protéine présentant la séquence d'acides aminés de SEQ ID N° : 17 dans laquelle 1 à 10 acides aminés sont substitués, supprimés, insérés ou ajoutés, l'ADN codant pour une protéine importatrice d'alginate,
(q) un ADN codant pour la séquence d'acides aminés de SEQ ID N° : 19,
(r) un ADN codant pour une protéine présentant la séquence d'acides aminés de SEQ ID N° : 19 dans laquelle 1 à 10 acides aminés sont substitués, supprimés, insérés ou ajoutés, l'ADN codant pour une protéine importatrice d'alginate,
(s) un ADN codant pour la séquence d'acides aminés de SEQ ID N° : 21,
(t) un ADN codant pour une protéine présentant la séquence d'acides aminés de SEQ ID N° : 21 dans laquelle 1 à 10 acides aminés sont substitués, supprimés, insérés ou ajoutés, l'ADN codant pour une protéine présentant l'activité alginate lyase,
(u) un ADN codant pour la séquence d'acides aminés de SEQ ID N° : 23,
(w) un ADN codant pour une protéine présentant la séquence d'acides aminés de SEQ ID N° : 23 dans laquelle 1 à 10 acides aminés sont substitués, supprimés, insérés ou ajoutés, l'ADN codant pour une protéine présentant l'activité NADP/NADPH déshydrogénase.


### SEQUENCE LISTING

<110> Ajinomoto Co., Inc.
<120> Method for producing L-amino acid from seaweed biomass
<130> D618-14244
<150> JP 2013-192420
   <151> 2013-09-17
<160> 98
<170> PatentIn version 3.5
<210> 1
   <211> 1492
   <212> DNA
   <213> Vibrio sp.
<400> 1
   ttgaacgctg gcggcaggcc taacacatgc aagtcgagcg gaaacgagaa tagcttgcta      60
   ttcggcgtcg agcggcggac gggtgagtaa tgcataggaa gttgcccagt agagggggat     120
   aaccattgga aacgatggct aataccgcat aacctcttcg gagcaaagcg ggggaccttc      180
   gggcctcgcg ctactggata cgcctatgtg ggattagcta gttggtgagg taaaggctca     240
   ccaaggcgac gatccctagc tggtctgaga ggatgatcag ccacactggg actgagacac     300
   ggcccagact cctacgggag gcagcagtgg ggaatattgc acaatgggcg aaagcctgat     360
   gcagccatgc cgcgtgtatg aagaaggcct tcgggttgta aagtactttc agttgtgagg     420
   aaggtttcgt agttaataac tgcgttgctt gacgttagca acagaagaag caccggctaa     480
   ctccgtgcca gcagccgcgg taatacggag ggtgcgagcg ttaatcggaa ttactgggcg     540
   taaagcgcat gcaggtggtt tgttaagtca gatgtgaaag cccggggctc aacctcggaa     600
   ggtcatttga aactggcaaa ctagagtact gtagaggggg gtagaatttc aggtgtagcg     660
   gtgaaatgcg tagagatctg aaggaatacc agtggcgaag gcggccccct ggacagatac     720
   tgacactcag atgcgaaagc gtggggagca aacaggatta gataccctgg tagtccacgc     780
   cgtaaacgat gtctacttgg aggttgtggc cttgagccgt ggctttcgga gctaacgcgt     840
   taagtagacc gcctggggag tacggtcgca agattaaaac tcaaatgaat tgacgggggc     900
   ccgcacaagc ggtggagcat gtggtttaat tcgatgcaac gcgaagaacc ttacctactc     960
   ttgacatcca gagaactttc cagagatgga ttggtgcctt cgggaactct gagacaggtg    1020
   ctgcatggct gtcgtcagct cgtgttgtga aatgttgggt taagtcccgc aacgagcgca    1080
   acccttatcc ttgtttgcca gcgagtaatg tcgggaactc cagggagact gccggtgata    1140
   aaccggagga aggtggggac gacgtcaagt catcatggcc cttacgagta gggctacaca    1200
   cgtgctacaa tggcgcatac agagggcagc gaacttgcga aagtaagcga atcccaaaaa    1260
   gtgcgtcgta gtccggattg gagtctgcaa ctcgactcca tgaagtcgga atcgctagta    1320
   atcgtggatc agaatgccac ggtgaatacg ttcccgggcc ttgtacacac cgcccgtcac    1380
   accatgggag tgggctgcaa aagaagtagg tagtttaacc ttcgggagga cgcttaccac    1440
   tttgtggttc atgactgggg tgaagtcgta acaaggtagc cctaggggaa cc            1492
<210> 2
   <211> 1749
   <212> DNA
   <213> Vibrio sp.
<220>
   <221> CDS
   <222> (1)..(1749)
<400> 2
   atg act gtc gat act att gtt gtt gct gcc tat ttt ctc ttt cta att        48
   Met Thr Val Asp Thr Ile Val Val Ala Ala Tyr Phe Leu Phe Leu Ile
   1               5                   10                  15
   gcg att ggt tgg atg ttc cgt aca ttt act acc tct act agt gat tac        96
   Ala Ile Gly Trp Met Phe Arg Thr Phe Thr Thr Ser Thr Ser Asp Tyr
               20                  25                  30
   ttt agg ggg gga ggt aag atg tta tgg tgg atg gta ggt gct act gcc       144
   Phe Arg Gly Gly Gly Lys Met Leu Trp Trp Met Val Gly Ala Thr Ala
           35                  40                  45
   ttc atg act caa ttc tca gcc tgg acc ttc act gga gca gca ggg cgc       192
   Phe Met Thr Gln Phe Ser Ala Trp Thr Phe Thr Gly Ala Ala Gly Arg
       50                  55                  60
   gct tat agc gat ggc ttt gtt gtc gta atc tta ttc tta gca aac gca       240
   Ala Tyr Ser Asp Gly Phe Val Val Val Ile Leu Phe Leu Ala Asn Ala
   65                  70                  75                  80
   ttt ggt tac ttt gtt aac tat atg tac ttc gcg cct aaa ttc cgt cag       288
   Phe Gly Tyr Phe Val Asn Tyr Met Tyr Phe Ala Pro Lys Phe Arg Gln
                   85                  90                  95
   ctt cgt gta gtg act gca att gaa gct att cgt caa cgt ttt ggt aaa       336
   Leu Arg Val Val Thr Ala Ile Glu Ala Ile Arg Gln Arg Phe Gly Lys
               100                 105                 110
   act tca gaa cag ttc ttt acc tgg ggc ggt atg cca gat agc tta att       384
   Thr Ser Glu Gln Phe Phe Thr Trp Gly Gly Met Pro Asp Ser Leu Ile
           115                 120                 125
   tct gcg ggt att tgg cta aat ggc ctg gct atc ttt gtg gcg gca gtt       432
   Ser Ala Gly Ile Trp Leu Asn Gly Leu Ala Ile Phe Val Ala Ala Val
       130                 135                 140
   ttt gat atc cca atg gaa gca acg att atc gtc aca ggc tta gtt ctt       480
   Phe Asp Ile Pro Met Glu Ala Thr Ile Ile Val Thr Gly Leu Val Leu
   145                 150                 155                 160
   atg ctt atg tct gta acc ggt ggc tct tgg gca gtt gtt gct tct gac       528
   Met Leu Met Ser Val Thr Gly Gly Ser Trp Ala Val Val Ala Ser Asp
                   165                 170                 175
   ttt atg caa atg ctg gtg atc atg gcg gta acc att act tgt gcc gtt        576
   Phe Met Gln Met Leu Val Ile Met Ala Val Thr Ile Thr Cys Ala Val
               180                 185                 190
   gct gct tac ttc caa ggt ggt ggt att act aac att gta aat aac ttc        624
   Ala Ala Tyr Phe Gln Gly Gly Gly Ile Thr Asn Ile Val Asn Asn Phe
           195                 200                 205
   caa ggc gat ttc ttt gtt ggt aac aac ttg aat tac atg agc atc ttt        672
   Gln Gly Asp Phe Phe Val Gly Asn Asn Leu Asn Tyr Met Ser Ile Phe
       210                 215                 220
   gtt ctt tgg gta gtg ttc atc ttc atc aag cag ttt ggt gta atg aat        720
   Val Leu Trp Val Val Phe Ile Phe Ile Lys Gln Phe Gly Val Met Asn
   225                 230                 235                 240
   aat tcg att aac gct tat cgt tat tta tgt gca aaa gac agt gaa aat        768
   Asn Ser Ile Asn Ala Tyr Arg Tyr Leu Cys Ala Lys Asp Ser Glu Asn
                   245                 250                 255
   gct cgt aaa gcg gct ggc ctt gct tgt gta ttg atg att att ggc cct        816
   Ala Arg Lys Ala Ala Gly Leu Ala Cys Val Leu Met Ile Ile Gly Pro
               260                 265                 270
   gta att tgg ttc tta cct gct tgg tat gtt ggt gca ttt atg cct aac        864
   Val Ile Trp Phe Leu Pro Ala Trp Tyr Val Gly Ala Phe Met Pro Asn
           275                 280                 285
   ttt gcc act gag tat ggt gct gtg ggc aat gat gca gct tac ctt gct        912
   Phe Ala Thr Glu Tyr Gly Ala Val Gly Asn Asp Ala Ala Tyr Leu Ala
       290                 295                 300
   ttc gta caa cac atg atg cca gcg ggt atg gtt ggc ttg ctt atg tca        960
   Phe Val Gln His Met Met Pro Ala Gly Met Val Gly Leu Leu Met Ser
   305                 310                 315                 320
   gca atg ttt gcc gcg aca atg tct tca atg gat tct ggc tta aac cgt       1008
   Ala Met Phe Ala Ala Thr Met Ser Ser Met Asp Ser Gly Leu Asn Arg
                   325                 330                 335
   aat gca ggc atc ttt gtg atg aac ttc tat agc ccg att ctt cgt cca       1056
   Asn Ala Gly Ile Phe Val Met Asn Phe Tyr Ser Pro Ile Leu Arg Pro
               340                 345                 350
   aat gct tca caa aaa gaa ttg gtg gta gtg agt aaa acg acc act atc       1104
   Asn Ala Ser Gln Lys Glu Leu Val Val Val Ser Lys Thr Thr Thr Ile
           355                 360                 365
   gtg atg ggc ttt att atc att gcg att ggc ttg ttt att aac tca tta       1152
   Val Met Gly Phe Ile Ile Ile Ala Ile Gly Leu Phe Ile Asn Ser Leu
       370                 375                 380
   cgt cac tta agc tta ttt gat atc gtg tta aat gtg ggt gcg ttg att       1200
   Arg His Leu Ser Leu Phe Asp Ile Val Leu Asn Val Gly Ala Leu Ile
   385                 390                 395                 400
   ggc ttc cca atg ttg att cca gtg tta tta ggc atg tgg att cgt aaa       1248
   Gly Phe Pro Met Leu Ile Pro Val Leu Leu Gly Met Trp Ile Arg Lys
                   405                 410                 415
   aca cca gat tgg tca gct tgg aca act ttg att gtc ggc ggt atc gtt       1296
   Thr Pro Asp Trp Ser Ala Trp Thr Thr Leu Ile Val Gly Gly Ile Val
               420                 425                 430
   tct tgg gtt ttc ggt att tca tta caa gca cac gat gta gaa cac ttc      1344
   Ser Trp Val Phe Gly Ile Ser Leu Gln Ala His Asp Val Glu His Phe
           435                 440                 445
   ttc ggt tta gaa caa gca ctg acc gga cgt gaa tgg tct gac ttg aaa      1392
   Phe Gly Leu Glu Gln Ala Leu Thr Gly Arg Glu Trp Ser Asp Leu Lys
       450                 455                 460
   gta gca tta agc ctt gca gca cac gtt gta ttt act ggc ggt ttc ttc      1440
   Val Ala Leu Ser Leu Ala Ala His Val Val Phe Thr Gly Gly Phe Phe
   465                 470                 475                 480
   ttg cta acg atg ctt ttc tac aga gga cgc act cca gag cgt gaa gct      1488
   Leu Leu Thr Met Leu Phe Tyr Arg Gly Arg Thr Pro Glu Arg Glu Ala
                   485                 490                 495
   gaa gta gaa aga cta ttc gaa aac tgg aac act ccg gtg gtt tca agt      1536
   Glu Val Glu Arg Leu Phe Glu Asn Trp Asn Thr Pro Val Val Ser Ser
               500                 505                 510
   ggt gaa gcg cag cat gat tta gat act aaa caa cgc tct atg cta ggt      1584
   Gly Glu Ala Gln His Asp Leu Asp Thr Lys Gln Arg Ser Met Leu Gly
           515                 520                 525
   cga tta atc tgt act gct gga ttt ggt gtt tta gca atg gca cta cta      1632
   Arg Leu Ile Cys Thr Ala Gly Phe Gly Val Leu Ala Met Ala Leu Leu
       530                 535                 540
   cca aat gaa atg aca gga cgt atg ttg ttc cta tta tgt ggt tca att      1680
   Pro Asn Glu Met Thr Gly Arg Met Leu Phe Leu Leu Cys Gly Ser Ile
   545                 550                 555                 560
   gtt ttg gtt gtt ggt gtg ttg ttg gtg aat gcc tca aaa gta caa cat      1728
   Val Leu Val Val Gly Val Leu Leu Val Asn Ala Ser Lys Val Gln His
                   565                 570                 575
   caa acg gtt tca gaa tcg taa                                          1749
   Gln Thr Val Ser Glu Ser
               580
<210> 3
   <211> 582
   <212> PRT
   <213> Vibrio sp.
<400> 3
   Met Thr Val Asp Thr Ile Val Val Ala Ala Tyr Phe Leu Phe Leu Ile
   1               5                   10                  15
   Ala Ile Gly Trp Met Phe Arg Thr Phe Thr Thr Ser Thr Ser Asp Tyr
               20                  25                  30
   Phe Arg Gly Gly Gly Lys Met Leu Trp Trp Met Val Gly Ala Thr Ala
           35                  40                  45
   Phe Met Thr Gln Phe Ser Ala Trp Thr Phe Thr Gly Ala Ala Gly Arg
       50                  55                  60
   Ala Tyr Ser Asp Gly Phe Val Val Val Ile Leu Phe Leu Ala Asn Ala
   65                  70                  75                  80
   Phe Gly Tyr Phe Val Asn Tyr Met Tyr Phe Ala Pro Lys Phe Arg Gln
                   85                  90                  95
   Leu Arg Val Val Thr Ala Ile Glu Ala Ile Arg Gln Arg Phe Gly Lys
               100                 105                 110
   Thr Ser Glu Gln Phe Phe Thr Trp Gly Gly Met Pro Asp Ser Leu Ile
           115                 120                 125
   Ser Ala Gly Ile Trp Leu Asn Gly Leu Ala Ile Phe Val Ala Ala Val
       130                 135                 140
   Phe Asp Ile Pro Met Glu Ala Thr Ile Ile Val Thr Gly Leu Val Leu
   145                 150                 155                 160
   Met Leu Met Ser Val Thr Gly Gly Ser Trp Ala Val Val Ala Ser Asp
                   165                 170                 175
   Phe Met Gln Met Leu Val Ile Met Ala Val Thr Ile Thr Cys Ala Val
               180                 185                 190
   Ala Ala Tyr Phe Gln Gly Gly Gly Ile Thr Asn Ile Val Asn Asn Phe
           195                 200                 205
   Gln Gly Asp Phe Phe Val Gly Asn Asn Leu Asn Tyr Met Ser Ile Phe
       210                 215                 220
   Val Leu Trp Val Val Phe Ile Phe Ile Lys Gln Phe Gly Val Met Asn
   225                 230                 235                 240
   Asn Ser Ile Asn Ala Tyr Arg Tyr Leu Cys Ala Lys Asp Ser Glu Asn
                   245                 250                 255
   Ala Arg Lys Ala Ala Gly Leu Ala Cys Val Leu Met Ile Ile Gly Pro
               260                 265                 270
   Val Ile Trp Phe Leu Pro Ala Trp Tyr Val Gly Ala Phe Met Pro Asn
           275                 280                 285
   Phe Ala Thr Glu Tyr Gly Ala Val Gly Asn Asp Ala Ala Tyr Leu Ala
       290                 295                 300
   Phe Val Gln His Met Met Pro Ala Gly Met Val Gly Leu Leu Met Ser
   305                 310                 315                 320
   Ala Met Phe Ala Ala Thr Met Ser Ser Met Asp Ser Gly Leu Asn Arg
                   325                 330                 335
   Asn Ala Gly Ile Phe Val Met Asn Phe Tyr Ser Pro Ile Leu Arg Pro
               340                 345                 350
   Asn Ala Ser Gln Lys Glu Leu Val Val Val Ser Lys Thr Thr Thr Ile
           355                 360                 365
   Val Met Gly Phe Ile Ile Ile Ala Ile Gly Leu Phe Ile Asn Ser Leu
       370                 375                 380
   Arg His Leu Ser Leu Phe Asp Ile Val Leu Asn Val Gly Ala Leu Ile
   385                 390                 395                 400
   Gly Phe Pro Met Leu Ile Pro Val Leu Leu Gly Met Trp Ile Arg Lys
                   405                 410                 415
   Thr Pro Asp Trp Ser Ala Trp Thr Thr Leu Ile Val Gly Gly Ile Val
               420                 425                 430
   Ser Trp Val Phe Gly Ile Ser Leu Gln Ala His Asp Val Glu His Phe
           435                 440                 445
   Phe Gly Leu Glu Gln Ala Leu Thr Gly Arg Glu Trp Ser Asp Leu Lys
       450                 455                 460
   Val Ala Leu Ser Leu Ala Ala His Val Val Phe Thr Gly Gly Phe Phe
   465                 470                 475                 480
   Leu Leu Thr Met Leu Phe Tyr Arg Gly Arg Thr Pro Glu Arg Glu Ala
                   485                 490                 495
   Glu Val Glu Arg Leu Phe Glu Asn Trp Asn Thr Pro Val Val Ser Ser
               500                 505                 510
   Gly Glu Ala Gln His Asp Leu Asp Thr Lys Gln Arg Ser Met Leu Gly
           515                 520                 525
   Arg Leu Ile Cys Thr Ala Gly Phe Gly Val Leu Ala Met Ala Leu Leu
       530                 535                 540
   Pro Asn Glu Met Thr Gly Arg Met Leu Phe Leu Leu Cys Gly Ser Ile
   545                 550                 555                 560
   Val Leu Val Val Gly Val Leu Leu Val Asn Ala Ser Lys Val Gln His
                   565                 570                 575
   Gln Thr Val Ser Glu Ser
               580
<210> 4
   <211> 1050
   <212> DNA
   <213> Vibrio sp.
<220>
   <221> CDS
   <222> (1)..(1050)
<400> 4
   atg tta aaa aaa ttc ctc tgc atg tcg gtt atc cta ggc tgc tca gca          48
   Met Leu Lys Lys Phe Leu Cys Met Ser Val Ile Leu Gly Cys Ser Ala
   1               5                   10                  15
   aac gca atg gcg caa gcc gct cca gta cca gct gac aag ttt gac atg          96
   Asn Ala Met Ala Gln Ala Ala Pro Val Pro Ala Asp Lys Phe Asp Met
               20                  25                  30
   atg aac tgg aaa att acc gtt cca gaa gat aga gat aaa aac ggt aag         144
   Met Asn Trp Lys Ile Thr Val Pro Glu Asp Arg Asp Lys Asn Gly Lys
           35                  40                  45
   att gat gaa att gaa ggt gta gcg atg ttc agc tac tca aac ccg gac         192
   Ile Asp Glu Ile Glu Gly Val Ala Met Phe Ser Tyr Ser Asn Pro Asp
       50                  55                  60
   tat ttc ttt tta gat gac gat ggc aac atg gtt ttc caa gtt caa aat         240
   Tyr Phe Phe Leu Asp Asp Asp Gly Asn Met Val Phe Gln Val Gln Asn
   65                  70                  75                  80
   aaa gct atc aca aca tct ggc tca tca aac gct cgt tca gaa cta cgc         288
   Lys Ala Ile Thr Thr Ser Gly Ser Ser Asn Ala Arg Ser Glu Leu Arg
                   85                  90                  95
   caa atg cta cgt ggc acc gat ctt act atc gat gtc aaa gct cca gca         336
   Gln Met Leu Arg Gly Thr Asp Leu Thr Ile Asp Val Lys Ala Pro Ala
               100                 105                 110
   aat aac ttc gct tta gca tct cat ccc aaa gca aaa gaa tat ggt gct         384
   Asn Asn Phe Ala Leu Ala Ser His Pro Lys Ala Lys Glu Tyr Gly Ala
           115                 120                 125
   gtt ggt ggt aaa tta gaa gca acc tta aaa gta aac cat gtt gct act         432
   Val Gly Gly Lys Leu Glu Ala Thr Leu Lys Val Asn His Val Ala Thr
       130                 135                 140
   cat gct aat caa cca aat aaa ctt cct gcc tat tca gtg gtt gtt gga         480
   His Ala Asn Gln Pro Asn Lys Leu Pro Ala Tyr Ser Val Val Val Gly
   145                 150                 155                 160
   caa att cat gcc aac aaa gat cct aaa cta atg gaa gca aac act ggc         528
   Gln Ile His Ala Asn Lys Asp Pro Lys Leu Met Glu Ala Asn Thr Gly
                   165                 170                 175
   ttt ggc tat ggt aat gaa cct tta aaa atc ttc tac aaa aaa tgg cca        576
   Phe Gly Tyr Gly Asn Glu Pro Leu Lys Ile Phe Tyr Lys Lys Trp Pro
               180                 185                 190
   gat cat gat aaa ggt tct gta ttc ttt aac tac gag cgc aac tta gca        624
   Asp His Asp Lys Gly Ser Val Phe Phe Asn Tyr Glu Arg Asn Leu Ala
           195                 200                 205
   aaa gat gat cca aac cgt atc gat att gcc tac cca gtt tgg ggc aat        672
   Lys Asp Asp Pro Asn Arg Ile Asp Ile Ala Tyr Pro Val Trp Gly Asn
       210                 215                 220
   act tgg gaa aac aaa gat gat cct aaa gat gct ggt gta gca ctt ggg        720
   Thr Trp Glu Asn Lys Asp Asp Pro Lys Asp Ala Gly Val Ala Leu Gly
   225                 230                 235                 240
   gaa gag ttt agt tat gta gtg gat gtt gaa ggt aac atc atg cac tta        768
   Glu Glu Phe Ser Tyr Val Val Asp Val Glu Gly Asn Ile Met His Leu
                   245                 250                 255
   act ttc act tcg aaa aac cac aag acc gtt gaa tat aaa atc gac cta        816
   Thr Phe Thr Ser Lys Asn His Lys Thr Val Glu Tyr Lys Ile Asp Leu
               260                 265                 270
   tct aaa aac ctg aag cct gac ggc aca gtt gat gag aaa gat aat cca        864
   Ser Lys Asn Leu Lys Pro Asp Gly Thr Val Asp Glu Lys Asp Asn Pro
           275                 280                 285
   cgt ggt tac gca gaa gat tct ttc tac ttt aaa gcc ggt gct tat ggc        912
   Arg Gly Tyr Ala Glu Asp Ser Phe Tyr Phe Lys Ala Gly Ala Tyr Gly
       290                 295                 300
   caa tgt agt gtg aaa gat gat gaa ggt ttc tgg tct cca ggt tgt gca        960
   Gln Cys Ser Val Lys Asp Asp Glu Gly Phe Trp Ser Pro Gly Cys Ala
   305                 310                 315                 320
   ggt aca ggt gac ttc gca act gac gtg aag aat ggc gac tac aac agt       1008
   Gly Thr Gly Asp Phe Ala Thr Asp Val Lys Asn Gly Asp Tyr Asn Ser
                   325                 330                 335
   gtg aca ttc tct aag tta gaa tta aca tcg cat aaa aaa tag               1050
   Val Thr Phe Ser Lys Leu Glu Leu Thr Ser His Lys Lys
               340                 345
<210> 5
   <211> 349
   <212> PRT
   <213> Vibrio sp.
<400> 5
   Met Leu Lys Lys Phe Leu Cys Met Ser Val Ile Leu Gly Cys Ser Ala
   1               5                   10                  15
   Asn Ala Met Ala Gln Ala Ala Pro Val Pro Ala Asp Lys Phe Asp Met
               20                  25                  30
   Met Asn Trp Lys Ile Thr Val Pro Glu Asp Arg Asp Lys Asn Gly Lys
           35                  40                  45
   Ile Asp Glu Ile Glu Gly Val Ala Met Phe Ser Tyr Ser Asn Pro Asp
       50                  55                  60
   Tyr Phe Phe Leu Asp Asp Asp Gly Asn Met Val Phe Gln Val Gln Asn
   65                  70                  75                  80
   Lys Ala Ile Thr Thr Ser Gly Ser Ser Asn Ala Arg Ser Glu Leu Arg
                   85                  90                  95
   Gln Met Leu Arg Gly Thr Asp Leu Thr Ile Asp Val Lys Ala Pro Ala
               100                 105                 110
   Asn Asn Phe Ala Leu Ala Ser His Pro Lys Ala Lys Glu Tyr Gly Ala
           115                 120                 125
   Val Gly Gly Lys Leu Glu Ala Thr Leu Lys Val Asn His Val Ala Thr
       130                 135                 140
   His Ala Asn Gln Pro Asn Lys Leu Pro Ala Tyr Ser Val Val Val Gly
   145                 150                 155                 160
   Gln Ile His Ala Asn Lys Asp Pro Lys Leu Met Glu Ala Asn Thr Gly
                   165                 170                 175
   Phe Gly Tyr Gly Asn Glu Pro Leu Lys Ile Phe Tyr Lys Lys Trp Pro
               180                 185                 190
   Asp His Asp Lys Gly Ser Val Phe Phe Asn Tyr Glu Arg Asn Leu Ala
           195                 200                 205
   Lys Asp Asp Pro Asn Arg Ile Asp Ile Ala Tyr Pro Val Trp Gly Asn
       210                 215                 220
   Thr Trp Glu Asn Lys Asp Asp Pro Lys Asp Ala Gly Val Ala Leu Gly
   225                 230                 235                 240
   Glu Glu Phe Ser Tyr Val Val Asp Val Glu Gly Asn Ile Met His Leu
                   245                 250                 255
   Thr Phe Thr Ser Lys Asn His Lys Thr Val Glu Tyr Lys Ile Asp Leu
               260                 265                 270
   Ser Lys Asn Leu Lys Pro Asp Gly Thr Val Asp Glu Lys Asp Asn Pro
           275                 280                 285
   Arg Gly Tyr Ala Glu Asp Ser Phe Tyr Phe Lys Ala Gly Ala Tyr Gly
       290                 295                 300
   Gln Cys Ser Val Lys Asp Asp Glu Gly Phe Trp Ser Pro Gly Cys Ala
   305                 310                 315                 320
   Gly Thr Gly Asp Phe Ala Thr Asp Val Lys Asn Gly Asp Tyr Asn Ser
                   325                 330                 335
   Val Thr Phe Ser Lys Leu Glu Leu Thr Ser His Lys Lys
               340                 345
<210> 6
   <211> 624
   <212> DNA
   <213> Vibrio sp.
<220>
   <221> CDS
   <222> (1)..(624)
<400> 6
   atg tct aaa tta gat caa caa ctt gct gac tta aaa gtc att cca gtg        48
   Met Ser Lys Leu Asp Gln Gln Leu Ala Asp Leu Lys Val Ile Pro Val
   1               5                   10                  15
   att gca ttg aac aaa gtt gaa cat gcc ctt cct ctg gcg aaa gca tta        96
   Ile Ala Leu Asn Lys Val Glu His Ala Leu Pro Leu Ala Lys Ala Leu
               20                  25                  30
   atg gaa aac ggc atg cct tgc gca gaa att act ttt cgt act gaa gcc       144
   Met Glu Asn Gly Met Pro Cys Ala Glu Ile Thr Phe Arg Thr Glu Ala
           35                  40                  45
   gca gtc ggc gca att aaa gcc atg cgt gaa gcc tac cct gaa atg cta       192
   Ala Val Gly Ala Ile Lys Ala Met Arg Glu Ala Tyr Pro Glu Met Leu
       50                  55                  60
   att ggt gca ggt acg att tta aca cct gaa caa gtt gac caa gca ata       240
   Ile Gly Ala Gly Thr Ile Leu Thr Pro Glu Gln Val Asp Gln Ala Ile
   65                  70                  75                  80
   gat gct ggt gtt gat ttc att gta agc cca ggc tta aac cca aca acg       288
   Asp Ala Gly Val Asp Phe Ile Val Ser Pro Gly Leu Asn Pro Thr Thr
                   85                  90                  95
   gtt aaa tac tgt caa caa aag aac atg cca atc gtt ccg ggt gtt aac       336
   Val Lys Tyr Cys Gln Gln Lys Asn Met Pro Ile Val Pro Gly Val Asn
               100                 105                 110
   aac cca agc cta gtt gag caa gca atg gaa ctg ggt ctt aaa acc gtt       384
   Asn Pro Ser Leu Val Glu Gln Ala Met Glu Leu Gly Leu Lys Thr Val
           115                 120                 125
   aag ttc ttc cca gct gag cca tct ggt ggc gtt ggt atg ttg aaa gca       432
   Lys Phe Phe Pro Ala Glu Pro Ser Gly Gly Val Gly Met Leu Lys Ala
       130                 135                 140
   tta act gcg gtt tac cca gtg aaa ttt atg cca act ggc ggt gtt agt       480
   Leu Thr Ala Val Tyr Pro Val Lys Phe Met Pro Thr Gly Gly Val Ser
   145                 150                 155                 160
   tta tca aac att gat gac tac cta tct att cca gct gta ctg gca tgt       528
   Leu Ser Asn Ile Asp Asp Tyr Leu Ser Ile Pro Ala Val Leu Ala Cys
                   165                 170                 175
   ggc ggt act tgg atg gtt cct aca tct ctt att gat aac gag caa tgg       576
   Gly Gly Thr Trp Met Val Pro Thr Ser Leu Ile Asp Asn Glu Gln Trp
               180                 185                 190
   gat gaa cta ggt gca tta gtt aaa gat gct gta gcc aaa gtt aac tag       624
   Asp Glu Leu Gly Ala Leu Val Lys Asp Ala Val Ala Lys Val Asn
           195                 200                 205
<210> 7
   <211> 207
   <212> PRT
   <213> Vibrio sp.
<400> 7
   Met Ser Lys Leu Asp Gln Gln Leu Ala Asp Leu Lys Val Ile Pro Val
   1               5                   10                  15
   Ile Ala Leu Asn Lys Val Glu His Ala Leu Pro Leu Ala Lys Ala Leu
               20                  25                  30
   Met Glu Asn Gly Met Pro Cys Ala Glu Ile Thr Phe Arg Thr Glu Ala
           35                  40                  45
   Ala Val Gly Ala Ile Lys Ala Met Arg Glu Ala Tyr Pro Glu Met Leu
       50                  55                  60
   Ile Gly Ala Gly Thr Ile Leu Thr Pro Glu Gln Val Asp Gln Ala Ile
   65                  70                  75                  80
   Asp Ala Gly Val Asp Phe Ile Val Ser Pro Gly Leu Asn Pro Thr Thr
                   85                  90                  95
   Val Lys Tyr Cys Gln Gln Lys Asn Met Pro Ile Val Pro Gly Val Asn
               100                 105                 110
   Asn Pro Ser Leu Val Glu Gln Ala Met Glu Leu Gly Leu Lys Thr Val
           115                 120                 125
   Lys Phe Phe Pro Ala Glu Pro Ser Gly Gly Val Gly Met Leu Lys Ala
       130                 135                 140
   Leu Thr Ala Val Tyr Pro Val Lys Phe Met Pro Thr Gly Gly Val Ser
   145                 150                 155                 160
   Leu Ser Asn Ile Asp Asp Tyr Leu Ser Ile Pro Ala Val Leu Ala Cys
                   165                 170                 175
   Gly Gly Thr Trp Met Val Pro Thr Ser Leu Ile Asp Asn Glu Gln Trp
               180                 185                 190
   Asp Glu Leu Gly Ala Leu Val Lys Asp Ala Val Ala Lys Val Asn
           195                 200                 205
<210> 8
   <211> 933
   <212> DNA
   <213> Vibrio sp.
<220>
   <221> CDS
   <222> (1)..(933)
<400> 8
   atg aca aca ttc aat att gca gtc att ggc gaa tgt atg att gaa ctg         48
   Met Thr Thr Phe Asn Ile Ala Val Ile Gly Glu Cys Met Ile Glu Leu
   1               5                   10                  15
   caa aaa tca ggc gaa ttc cta acg caa aaa ttt ggt ggt gat acg ctt         96
   Gln Lys Ser Gly Glu Phe Leu Thr Gln Lys Phe Gly Gly Asp Thr Leu
               20                  25                  30
   aat act gcg ttg tat ctt gct cgc cta acc aaa cac act gcg att aaa        144
   Asn Thr Ala Leu Tyr Leu Ala Arg Leu Thr Lys His Thr Ala Ile Lys
           35                  40                  45
   aca agc tac att acc gcg ctt ggt cgt gaa ggc ttt agc caa aac atg        192
   Thr Ser Tyr Ile Thr Ala Leu Gly Arg Glu Gly Phe Ser Gln Asn Met
       50                  55                  60
   att gat agc tgg tca gaa gaa gcg att aat acg caa cac att ctt cgt        240
   Ile Asp Ser Trp Ser Glu Glu Ala Ile Asn Thr Gln His Ile Leu Arg
   65                  70                  75                  80
   ttt gaa aac aag caa ccc ggt atg tac tac atc gaa acc gat gac act        288
   Phe Glu Asn Lys Gln Pro Gly Met Tyr Tyr Ile Glu Thr Asp Asp Thr
                   85                  90                  95
   ggt gag cgt agt ttc tat tac tgg cgt aat gat gcc gcg gca aaa tac        336
   Gly Glu Arg Ser Phe Tyr Tyr Trp Arg Asn Asp Ala Ala Ala Lys Tyr
               100                 105                 110
   atg ttt gac caa gcg gag agc gct caa ctg atc gaa aca tta tca acc        384
   Met Phe Asp Gln Ala Glu Ser Ala Gln Leu Ile Glu Thr Leu Ser Thr
           115                 120                 125
   ttc gat gca gtc tat tta agt ggg atc aca ctc gct att ttg act gag        432
   Phe Asp Ala Val Tyr Leu Ser Gly Ile Thr Leu Ala Ile Leu Thr Glu
       130                 135                 140
   caa ggt cgt gag cag tta ttc aca tta ctt agc aaa ttg aaa gct aat        480
   Gln Gly Arg Glu Gln Leu Phe Thr Leu Leu Ser Lys Leu Lys Ala Asn
   145                 150                 155                 160
   ggc agc aaa gtt att ttt gat aat aac ttc cgt cca aaa ttg tgg gca      528
   Gly Ser Lys Val Ile Phe Asp Asn Asn Phe Arg Pro Lys Leu Trp Ala
                   165                 170                 175
   tcg gtt gaa gaa gct caa caa gcg tat ctt aaa aca cta tcg ttc tgt      576
   Ser Val Glu Glu Ala Gln Gln Ala Tyr Leu Lys Thr Leu Ser Phe Cys
               180                 185                 190
   gat att gcc tta tta aca ttc gat gat gaa caa gat ctc ttt ggt gac      624
   Asp Ile Ala Leu Leu Thr Phe Asp Asp Glu Gln Asp Leu Phe Gly Asp
           195                 200                 205
   gac aat atc gaa caa tgt att gct cgt act caa ggt caa ggc gtt aaa      672
   Asp Asn Ile Glu Gln Cys Ile Ala Arg Thr Gln Gly Gln Gly Val Lys
       210                 215                 220
   gag att gcg att aaa cgt ggc agc aaa gat tgt gtt gtt gta gaa gaa      720
   Glu Ile Ala Ile Lys Arg Gly Ser Lys Asp Cys Val Val Val Glu Glu
   225                 230                 235                 240
   gat agc att caa tac gtt gcg aca aaa ccg gtt gat aac gtg gtg gac      768
   Asp Ser Ile Gln Tyr Val Ala Thr Lys Pro Val Asp Asn Val Val Asp
                   245                 250                 255
   acg act gcc gca ggt gac tca ttc agt gct ggc tat tta gct aag cgt      816
   Thr Thr Ala Ala Gly Asp Ser Phe Ser Ala Gly Tyr Leu Ala Lys Arg
               260                 265                 270
   tta aat ggt tac agc gca aaa gac tct gcc cta tca ggt cat aaa gta      864
   Leu Asn Gly Tyr Ser Ala Lys Asp Ser Ala Leu Ser Gly His Lys Val
           275                 280                 285
   gca ggc acc gtt atc caa cat caa ggc gcg att att cca agt gag gca      912
   Ala Gly Thr Val Ile Gln His Gln Gly Ala Ile Ile Pro Ser Glu Ala
       290                 295                 300
   atg cct gat atc ggc tta taa                                          933
   Met Pro Asp Ile Gly Leu
   305                 310
<210> 9
   <211> 310
   <212> PRT
   <213> Vibrio sp.
<400> 9
   Met Thr Thr Phe Asn Ile Ala Val Ile Gly Glu Cys Met Ile Glu Leu
   1               5                   10                  15
   Gln Lys Ser Gly Glu Phe Leu Thr Gln Lys Phe Gly Gly Asp Thr Leu
               20                  25                  30
   Asn Thr Ala Leu Tyr Leu Ala Arg Leu Thr Lys His Thr Ala Ile Lys
           35                  40                  45
   Thr Ser Tyr Ile Thr Ala Leu Gly Arg Glu Gly Phe Ser Gln Asn Met
       50                  55                  60
   Ile Asp Ser Trp Ser Glu Glu Ala Ile Asn Thr Gln His Ile Leu Arg
   65                  70                  75                  80
   Phe Glu Asn Lys Gln Pro Gly Met Tyr Tyr Ile Glu Thr Asp Asp Thr
                   85                  90                  95
   Gly Glu Arg Ser Phe Tyr Tyr Trp Arg Asn Asp Ala Ala Ala Lys Tyr
               100                 105                 110
   Met Phe Asp Gln Ala Glu Ser Ala Gln Leu Ile Glu Thr Leu Ser Thr
           115                 120                 125
   Phe Asp Ala Val Tyr Leu Ser Gly Ile Thr Leu Ala Ile Leu Thr Glu
       130                 135                 140
   Gln Gly Arg Glu Gln Leu Phe Thr Leu Leu Ser Lys Leu Lys Ala Asn
   145                 150                 155                 160
   Gly Ser Lys Val Ile Phe Asp Asn Asn Phe Arg Pro Lys Leu Trp Ala
                   165                 170                 175
   Ser Val Glu Glu Ala Gln Gln Ala Tyr Leu Lys Thr Leu Ser Phe Cys
               180                 185                 190
   Asp Ile Ala Leu Leu Thr Phe Asp Asp Glu Gln Asp Leu Phe Gly Asp
           195                 200                 205
   Asp Asn Ile Glu Gln Cys Ile Ala Arg Thr Gln Gly Gln Gly Val Lys
       210                 215                 220
   Glu Ile Ala Ile Lys Arg Gly Ser Lys Asp Cys Val Val Val Glu Glu
   225                 230                 235                 240
   Asp Ser Ile Gln Tyr Val Ala Thr Lys Pro Val Asp Asn Val Val Asp
                   245                 250                 255
   Thr Thr Ala Ala Gly Asp Ser Phe Ser Ala Gly Tyr Leu Ala Lys Arg
               260                 265                 270
   Leu Asn Gly Tyr Ser Ala Lys Asp Ser Ala Leu Ser Gly His Lys Val
           275                 280                 285
   Ala Gly Thr Val Ile Gln His Gln Gly Ala Ile Ile Pro Ser Glu Ala
       290                 295                 300
   Met Pro Asp Ile Gly Leu
   305                 310
<210> 10
   <211> 2181
   <212> DNA
   <213> Vibrio sp.
<220>
   <221> CDS
   <222> (1)..(2181)
<400> 10
   atg aaa aat gaa gta tca gct gta ttg ctt aac act gca gaa gtg aaa        48
   Met Lys Asn Glu Val Ser Ala Val Leu Leu Asn Thr Ala Glu Val Lys
   1               5                   10                  15
   gcg tta cag aaa gaa gtc ggc cgt gac agt ttg atg ggt aac act att        96
   Ala Leu Gln Lys Glu Val Gly Arg Asp Ser Leu Met Gly Asn Thr Ile
               20                  25                  30
   gcg acc att aaa aaa gat gtt gat gcc ttt atg aca ctg ggc tac gac       144
   Ala Thr Ile Lys Lys Asp Val Asp Ala Phe Met Thr Leu Gly Tyr Asp
           35                  40                  45
   gta cct ggg cat ggt gaa gcg ggt ggt tat gaa cac aat cgc cat aag       192
   Val Pro Gly His Gly Glu Ala Gly Gly Tyr Glu His Asn Arg His Lys
       50                  55                  60
   caa aac tat aac ttt atg aac ctt gct ggt cgt ctt tat cta gta act       240
   Gln Asn Tyr Asn Phe Met Asn Leu Ala Gly Arg Leu Tyr Leu Val Thr
   65                  70                  75                  80
   aaa gaa gaa aaa tac gct cgt ttt gta gct gaa cta tta act atc tat       288
   Lys Glu Glu Lys Tyr Ala Arg Phe Val Ala Glu Leu Leu Thr Ile Tyr
                   85                  90                  95
   gct gac aaa tac ttg caa ttc gat ttc cat att caa aaa aac acc aac       336
   Ala Asp Lys Tyr Leu Gln Phe Asp Phe His Ile Gln Lys Asn Thr Asn
               100                 105                 110
   cca acg ggt cgt tta ttc cac cag atc tta aat gaa cac atg tgg cta       384
   Pro Thr Gly Arg Leu Phe His Gln Ile Leu Asn Glu His Met Trp Leu
           115                 120                 125
   atg tat tcg agc ttg gcg tat tct tgt gtg aaa gga ttt atg tcg ctt       432
   Met Tyr Ser Ser Leu Ala Tyr Ser Cys Val Lys Gly Phe Met Ser Leu
       130                 135                 140
   act gag cgt aat cat gta gaa agc aat tta ttc cta cct atg ctt gat       480
   Thr Glu Arg Asn His Val Glu Ser Asn Leu Phe Leu Pro Met Leu Asp
   145                 150                 155                 160
   atg ttt acc gtg aaa tat ggc cat gat ttt gac cgt att cat aac cac       528
   Met Phe Thr Val Lys Tyr Gly His Asp Phe Asp Arg Ile His Asn His
                   165                 170                 175
   ggt atg tgg gcc gtt gcc gcg gta ggt att tgt ggt tta gct att gat       576
   Gly Met Trp Ala Val Ala Ala Val Gly Ile Cys Gly Leu Ala Ile Asp
               180                 185                 190
   aaa cgt gag tat cta gaa atg gcc gta ttt ggt cta gat aac ttt aac       624
   Lys Arg Glu Tyr Leu Glu Met Ala Val Phe Gly Leu Asp Asn Phe Asn
           195                 200                 205
   gct ggc aaa aaa gaa atg ggt gag tgt ggt ttc tta gcg caa atc tct       672
   Ala Gly Lys Lys Glu Met Gly Glu Cys Gly Phe Leu Ala Gln Ile Ser
       210                 215                 220
   aaa tta ttt gcc cct tct ggt tac tac atg gaa ggt cct tac tac cac       720
   Lys Leu Phe Ala Pro Ser Gly Tyr Tyr Met Glu Gly Pro Tyr Tyr His
   225                 230                 235                 240
   cgc ttt gcg att cgt cct cta tgt gtg ttt gca gaa gtt tta cac cgt       768
   Arg Phe Ala Ile Arg Pro Leu Cys Val Phe Ala Glu Val Leu His Arg
                   245                 250                 255
   cac atg cct gag cta gat att tat aac ttt aaa gac aaa gtg att ggt       816
   His Met Pro Glu Leu Asp Ile Tyr Asn Phe Lys Asp Lys Val Ile Gly
               260                 265                 270
   aat acg gtt caa aca ctt tta gct acc gct tat cca aat ggt gag ttt       864
   Asn Thr Val Gln Thr Leu Leu Ala Thr Ala Tyr Pro Asn Gly Glu Phe
           275                 280                 285
   cca gcg tta aat gat tca tct cat aca atg ggc att acc gat atg ggc       912
   Pro Ala Leu Asn Asp Ser Ser His Thr Met Gly Ile Thr Asp Met Gly
       290                 295                 300
   gta caa gtc gcg gtg agc tta tac agc aag cac tac ggt tta gat gat       960
   Val Gln Val Ala Val Ser Leu Tyr Ser Lys His Tyr Gly Leu Asp Asp
   305                 310                 315                 320
   aac ttg ctt ggt atg gcg aaa att caa aac caa gta tgg atg cat gcg      1008
   Asn Leu Leu Gly Met Ala Lys Ile Gln Asn Gln Val Trp Met His Ala
                   325                 330                 335
   tgt ggt cta gag tta tct cgt gcg ttt gaa aaa gca caa aat gag cgt      1056
   Cys Gly Leu Glu Leu Ser Arg Ala Phe Glu Lys Ala Gln Asn Glu Arg
               340                 345                 350
   cct gat gat att ggt tta cca ttt ttc cca agt gtg gag tta aat gaa      1104
   Pro Asp Asp Ile Gly Leu Pro Phe Phe Pro Ser Val Glu Leu Asn Glu
           355                 360                 365
   ggc cca gcg ggt cat aac ggt gca caa ggt ttt atc cgt atg caa gat      1152
   Gly Pro Ala Gly His Asn Gly Ala Gln Gly Phe Ile Arg Met Gln Asp
       370                 375                 380
   gaa acc aaa gat gtc ttc caa tta gtg atg aac tat ggc caa cat ggt      1200
   Glu Thr Lys Asp Val Phe Gln Leu Val Met Asn Tyr Gly Gln His Gly
   385                 390                 395                 400
   atg gat cat ggt cac ttt gat act tta ggt att agt tat ttt aac cgt      1248
   Met Asp His Gly His Phe Asp Thr Leu Gly Ile Ser Tyr Phe Asn Arg
                   405                 410                 415
   ggt caa gaa gtg tta cgt gaa tat ggt ttc tgc cgt tgg gtt aat gtt      1296
   Gly Gln Glu Val Leu Arg Glu Tyr Gly Phe Cys Arg Trp Val Asn Val
               420                 425                 430
   gaa cct aag ttc ggt ggc cgc tac ctt gat gag aat aaa tct tat gcc      1344
   Glu Pro Lys Phe Gly Gly Arg Tyr Leu Asp Glu Asn Lys Ser Tyr Ala
           435                 440                 445
   cgt caa acc att gca cat aac gct gtg acg att gat gag aaa tgc caa      1392
   Arg Gln Thr Ile Ala His Asn Ala Val Thr Ile Asp Glu Lys Cys Gln
       450                 455                 460
   aat aac ttc tca att gat gaa gca gat acg gtt cat ggc ttg ccg cat      1440
   Asn Asn Phe Ser Ile Asp Glu Ala Asp Thr Val His Gly Leu Pro His
   465                 470                 475                 480
   ttc ttt aaa gta gaa gat gca aaa gtg aat ggt atg agc gct ttt gcc      1488
   Phe Phe Lys Val Glu Asp Ala Lys Val Asn Gly Met Ser Ala Phe Ala
                   485                 490                 495
   aat gat cat tac caa gat ttc gac atg cag cgt agt gtg ttc atg cta      1536
   Asn Asp His Tyr Gln Asp Phe Asp Met Gln Arg Ser Val Phe Met Leu
               500                 505                 510
   aaa ctg gat gaa cta gaa gca cca ttg ctg ctc gat tta tac cgc tta      1584
   Lys Leu Asp Glu Leu Glu Ala Pro Leu Leu Leu Asp Leu Tyr Arg Leu
           515                 520                 525
   acg gca gaa gat aac aaa aca cat cag ttt gac tac tca cac caa tat      1632
   Thr Ala Glu Asp Asn Lys Thr His Gln Phe Asp Tyr Ser His Gln Tyr
       530                 535                 540
   caa ggt caa att gtt cgt act aat ttt gat tat caa gcc cac caa gag      1680
   Gln Gly Gln Ile Val Arg Thr Asn Phe Asp Tyr Gln Ala His Gln Glu
   545                 550                 555                 560
   ctt gac aca cta ggt gac aac ttc ggt tat caa cat cta tgg aat gtg      1728
   Leu Asp Thr Leu Gly Asp Asn Phe Gly Tyr Gln His Leu Trp Asn Val
                   565                 570                 575
   gca agt gga gaa gtt aaa ggc agt gct tta gtc agt tgg cta caa aat      1776
   Ala Ser Gly Glu Val Lys Gly Ser Ala Leu Val Ser Trp Leu Gln Asn
               580                 585                 590
   aat act tac tac act tgg tta ggt acg agt tcg aac aat gat ggt gaa      1824
   Asn Thr Tyr Tyr Thr Trp Leu Gly Thr Ser Ser Asn Asn Asp Gly Glu
           595                 600                 605
   gtc att ttc aca cgt act ggc gct aat gat cca agt ttt aac tta cgt      1872
   Val Ile Phe Thr Arg Thr Gly Ala Asn Asp Pro Ser Phe Asn Leu Arg
       610                 615                 620
   agt gag cct gcc ttt atc tta cgt gct aaa ggc caa tca agc tta ttt      1920
   Ser Glu Pro Ala Phe Ile Leu Arg Ala Lys Gly Gln Ser Ser Leu Phe
   625                 630                 635                 640
   gca tca gtc tta gaa acg cat ggt tac ttt aac gaa gag ttt gaa caa      1968
   Ala Ser Val Leu Glu Thr His Gly Tyr Phe Asn Glu Glu Phe Glu Gln
                   645                 650                 655
   tca gtg aat gct cgt ggt caa gtt caa gac gta cgt gtg att gcc cat      2016
   Ser Val Asn Ala Arg Gly Gln Val Gln Asp Val Arg Val Ile Ala His
               660                 665                 670
   aat gag gtt gga tct gtg att caa att gaa acg caa caa tcg gtg gtt      2064
   Asn Glu Val Gly Ser Val Ile Gln Ile Glu Thr Gln Gln Ser Val Val
           675                 680                 685
   act ttt atg gta agc aat caa tta gcc gcg aca gat acc act gtg cat      2112
   Thr Phe Met Val Ser Asn Gln Leu Ala Ala Thr Asp Thr Thr Val His
       690                 695                 700
   caa att tca gtc gat gac aaa act tat caa tgg aat ggc ttc tat tca      2160
   Gln Ile Ser Val Asp Asp Lys Thr Tyr Gln Trp Asn Gly Phe Tyr Ser
   705                 710                 715                 720
   tta gaa gta caa gtg aaa taa                                          2181
   Leu Glu Val Gln Val Lys
                   725
<210> 11
   <211> 726
   <212> PRT
   <213> Vibrio sp.
<400> 11
   Met Lys Asn Glu Val Ser Ala Val Leu Leu Asn Thr Ala Glu Val Lys
   1               5                   10                  15
   Ala Leu Gln Lys Glu Val Gly Arg Asp Ser Leu Met Gly Asn Thr Ile
               20                  25                  30
   Ala Thr Ile Lys Lys Asp Val Asp Ala Phe Met Thr Leu Gly Tyr Asp
           35                  40                  45
   Val Pro Gly His Gly Glu Ala Gly Gly Tyr Glu His Asn Arg His Lys
       50                  55                  60
   Gln Asn Tyr Asn Phe Met Asn Leu Ala Gly Arg Leu Tyr Leu Val Thr
   65                  70                  75                  80
   Lys Glu Glu Lys Tyr Ala Arg Phe Val Ala Glu Leu Leu Thr Ile Tyr
                   85                  90                  95
   Ala Asp Lys Tyr Leu Gln Phe Asp Phe His Ile Gln Lys Asn Thr Asn
               100                 105                 110
   Pro Thr Gly Arg Leu Phe His Gln Ile Leu Asn Glu His Met Trp Leu
           115                 120                 125
   Met Tyr Ser Ser Leu Ala Tyr Ser Cys Val Lys Gly Phe Met Ser Leu
       130                 135                 140
   Thr Glu Arg Asn His Val Glu Ser Asn Leu Phe Leu Pro Met Leu Asp
   145                 150                 155                 160
   Met Phe Thr Val Lys Tyr Gly His Asp Phe Asp Arg Ile His Asn His
                   165                 170                 175
   Gly Met Trp Ala Val Ala Ala Val Gly Ile Cys Gly Leu Ala Ile Asp
               180                 185                 190
   Lys Arg Glu Tyr Leu Glu Met Ala Val Phe Gly Leu Asp Asn Phe Asn
   195 200 205
   Ala Gly Lys Lys Glu Met Gly Glu Cys Gly Phe Leu Ala Gln Ile Ser
   210 215 220
   Lys Leu Phe Ala Pro Ser Gly Tyr Tyr Met Glu Gly Pro Tyr Tyr His
   225 230 235 240
   Arg Phe Ala Ile Arg Pro Leu Cys Val Phe Ala Glu Val Leu His Arg
   245 250 255
   His Met Pro Glu Leu Asp Ile Tyr Asn Phe Lys Asp Lys Val Ile Gly
   260 265 270
   Asn Thr Val Gln Thr Leu Leu Ala Thr Ala Tyr Pro Asn Gly Glu Phe
   275 280 285
   Pro Ala Leu Asn Asp Ser Ser His Thr Met Gly Ile Thr Asp Met Gly
   290 295 300
   Val Gln Val Ala Val Ser Leu Tyr Ser Lys His Tyr Gly Leu Asp Asp
   305 310 315 320
   Asn Leu Leu Gly Met Ala Lys Ile Gln Asn Gln Val Trp Met His Ala
   325 330 335
   Cys Gly Leu Glu Leu Ser Arg Ala Phe Glu Lys Ala Gln Asn Glu Arg
   340 345 350
   Pro Asp Asp Ile Gly Leu Pro Phe Phe Pro Ser Val Glu Leu Asn Glu
   355 360 365
   Gly Pro Ala Gly His Asn Gly Ala Gln Gly Phe Ile Arg Met Gln Asp
   370 375 380
   Glu Thr Lys Asp Val Phe Gln Leu Val Met Asn Tyr Gly Gln His Gly
   385 390 395 400
   Met Asp His Gly His Phe Asp Thr Leu Gly Ile Ser Tyr Phe Asn Arg
   405 410 415
   Gly Gln Glu Val Leu Arg Glu Tyr Gly Phe Cys Arg Trp Val Asn Val
   420 425 430
   Glu Pro Lys Phe Gly Gly Arg Tyr Leu Asp Glu Asn Lys Ser Tyr Ala
   435 440 445
   Arg Gln Thr Ile Ala His Asn Ala Val Thr Ile Asp Glu Lys Cys Gln
   450 455 460
   Asn Asn Phe Ser Ile Asp Glu Ala Asp Thr Val His Gly Leu Pro His
   465 470 475 480
   Phe Phe Lys Val Glu Asp Ala Lys Val Asn Gly Met Ser Ala Phe Ala
   485 490 495
   Asn Asp His Tyr Gln Asp Phe Asp Met Gln Arg Ser Val Phe Met Leu
   500 505 510
   Lys Leu Asp Glu Leu Glu Ala Pro Leu Leu Leu Asp Leu Tyr Arg Leu
   515 520 525
   Thr Ala Glu Asp Asn Lys Thr His Gln Phe Asp Tyr Ser His Gln Tyr
   530 535 540
   Gln Gly Gln Ile Val Arg Thr Asn Phe Asp Tyr Gln Ala His Gln Glu
   545 550 555 560
   Leu Asp Thr Leu Gly Asp Asn Phe Gly Tyr Gln His Leu Trp Asn Val
   565 570 575
   Ala Ser Gly Glu Val Lys Gly Ser Ala Leu Val Ser Trp Leu Gln Asn
   580 585 590
   Asn Thr Tyr Tyr Thr Trp Leu Gly Thr Ser Ser Asn Asn Asp Gly Glu
   595 600 605
   Val Ile Phe Thr Arg Thr Gly Ala Asn Asp Pro Ser Phe Asn Leu Arg
   610 615 620
   Ser Glu Pro Ala Phe Ile Leu Arg Ala Lys Gly Gln Ser Ser Leu Phe
   625 630 635 640
   Ala Ser Val Leu Glu Thr His Gly Tyr Phe Asn Glu Glu Phe Glu Gln
   645 650 655
   Ser Val Asn Ala Arg Gly Gln Val Gln Asp Val Arg Val Ile Ala His
   660 665 670
   Asn Glu Val Gly Ser Val Ile Gln Ile Glu Thr Gln Gln Ser Val Val
   675 680 685
   Thr Phe Met Val Ser Asn Gln Leu Ala Ala Thr Asp Thr Thr Val His
   690 695 700
   Gln Ile Ser Val Asp Asp Lys Thr Tyr Gln Trp Asn Gly Phe Tyr Ser
   705 710 715 720
   Leu Glu Val Gln Val Lys
   725
<210> 12
   <211> 2145
   <212> DNA
   <213> Vibrio sp.
<220>
   <221> CDS
   <222> (1)..(2145)
<400> 12
   atg aat tac caa cca tta tta atg aat ttt gaa gaa gcg gct gaa ctg        48
   Met Asn Tyr Gln Pro Leu Leu Met Asn Phe Glu Glu Ala Ala Glu Leu
   1               5                   10                  15
   gca aaa gag tta ggt aaa gat act ctg cta ggt aat gca ctg gct cgc        96
   Ala Lys Glu Leu Gly Lys Asp Thr Leu Leu Gly Asn Ala Leu Ala Arg
               20                  25                  30
   gat att gct caa aca gat gca tat atg gca aat gtg ggt att gaa gtg       144
   Asp Ile Ala Gln Thr Asp Ala Tyr Met Ala Asn Val Gly Ile Glu Val
           35                  40                  45
   cca gga cat ggt gaa ggt ggt ggt tat gag cat aat cgt cac aag caa       192
   Pro Gly His Gly Glu Gly Gly Gly Tyr Glu His Asn Arg His Lys Gln
       50                  55                  60
   aac tat att cat atg gac tta gca ggc cgc tta ttt ctg att act ggt       240
   Asn Tyr Ile His Met Asp Leu Ala Gly Arg Leu Phe Leu Ile Thr Gly
   65                  70                  75                  80
   gag caa aaa tac cgt gac tac att gtt gaa cta cta aca gct tac gct       288
   Glu Gln Lys Tyr Arg Asp Tyr Ile Val Glu Leu Leu Thr Ala Tyr Ala
                   85                  90                  95
   act gtg tat cca aca ctt gaa agc aat gta agc cgc gac agt aac cct       336
   Thr Val Tyr Pro Thr Leu Glu Ser Asn Val Ser Arg Asp Ser Asn Pro
               100                 105                 110
   cca ggt aaa ctg ttc cac caa aca ttg aat gaa aat atg tgg atg ctg       384
   Pro Gly Lys Leu Phe His Gln Thr Leu Asn Glu Asn Met Trp Met Leu
           115                 120                 125
   tac tca tca tgt gca tat agc tgt gtt tac cac acg ctt gaa gag tcg       432
   Tyr Ser Ser Cys Ala Tyr Ser Cys Val Tyr His Thr Leu Glu Glu Ser
       130                 135                 140
   caa aaa cgt cag att gaa gat gac ctg ttc aaa cac atg atc cac atg       480
   Gln Lys Arg Gln Ile Glu Asp Asp Leu Phe Lys His Met Ile His Met
   145                 150                 155                 160
   ttt gta gtg act tac ggt cat gac ttt gac att gtg cac aac cac ggt       528
   Phe Val Val Thr Tyr Gly His Asp Phe Asp Ile Val His Asn His Gly
                   165                 170                 175
   cta tgg gcc gtt gcg gca gta ggt att tgt ggt tac gcc atc aat gac        576
   Leu Trp Ala Val Ala Ala Val Gly Ile Cys Gly Tyr Ala Ile Asn Asp
               180                 185                 190
   caa gaa tct gtt gat aaa gca cta tac ggt ttg aag ctt gat aaa gtg        624
   Gln Glu Ser Val Asp Lys Ala Leu Tyr Gly Leu Lys Leu Asp Lys Val
           195                 200                 205
   agc ggt ggc ttc ttg gct caa ctg gat cag ttg ttc tcg cca gat ggc        672
   Ser Gly Gly Phe Leu Ala Gln Leu Asp Gln Leu Phe Ser Pro Asp Gly
       210                 215                 220
   tac tat atg gaa ggt ccg tac tat cac cgc ttc tca ttg cgt cca att        720
   Tyr Tyr Met Glu Gly Pro Tyr Tyr His Arg Phe Ser Leu Arg Pro Ile
   225                 230                 235                 240
   tac ttg ttt gcc gaa gca att gag cgt cgt cag cca gag ttg ggc att        768
   Tyr Leu Phe Ala Glu Ala Ile Glu Arg Arg Gln Pro Glu Leu Gly Ile
                   245                 250                 255
   tat gaa ttt aac gat tca gtg atc aaa acg acg tca tac gca gta ttt        816
   Tyr Glu Phe Asn Asp Ser Val Ile Lys Thr Thr Ser Tyr Ala Val Phe
               260                 265                 270
   aaa acc gct ttt cca gat ggc act cta cct gcg ctt aat gat tca tca        864
   Lys Thr Ala Phe Pro Asp Gly Thr Leu Pro Ala Leu Asn Asp Ser Ser
           275                 280                 285
   aaa acc atc agt att aat gat gaa ggt gtg atc atg gca acg tct gtg        912
   Lys Thr Ile Ser Ile Asn Asp Glu Gly Val Ile Met Ala Thr Ser Val
       290                 295                 300
   tgt tac cac cgc tat gag caa act gaa gta ttg ttg ggt atg gcc agt        960
   Cys Tyr His Arg Tyr Glu Gln Thr Glu Val Leu Leu Gly Met Ala Ser
   305                 310                 315                 320
   cac caa caa aat gtc tgg gta cat aaa gca ggt ctt act cta tct caa       1008
   His Gln Gln Asn Val Trp Val His Lys Ala Gly Leu Thr Leu Ser Gln
                   325                 330                 335
   gcc gtt gaa gca gca gat gac atc aaa cct ttc aac tgg ggc agc ttg       1056
   Ala Val Glu Ala Ala Asp Asp Ile Lys Pro Phe Asn Trp Gly Ser Leu
               340                 345                 350
   ttt atg aca gat ggc cca caa ggt gag aaa ggc ggt gtg agt att ctt       1104
   Phe Met Thr Asp Gly Pro Gln Gly Glu Lys Gly Gly Val Ser Ile Leu
           355                 360                 365
   cgt cat cgt gat gtt aaa gat gat gac acc atg gca cta att tgg ttt       1152
   Arg His Arg Asp Val Lys Asp Asp Asp Thr Met Ala Leu Ile Trp Phe
       370                 375                 380
   ggt caa cat ggt tcc gat cac caa tat cat tca gca tta gat cat ggt       1200
   Gly Gln His Gly Ser Asp His Gln Tyr His Ser Ala Leu Asp His Gly
   385                 390                 395                 400
   cat tac gat ggc tta cat cta agt gtg ttt aac cgt ggt aat gaa gtg       1248
   His Tyr Asp Gly Leu His Leu Ser Val Phe Asn Arg Gly Asn Glu Val
                   405                 410                 415
   ctg cat gat tat ggg ttt ggt cgt tgg gta aac gtt gag cca aaa ttt      1296
   Leu His Asp Tyr Gly Phe Gly Arg Trp Val Asn Val Glu Pro Lys Phe
               420                 425                 430
   ggt ggt cgt tat att cca gaa aat aag tct tac tgt aag caa act gtg      1344
   Gly Gly Arg Tyr Ile Pro Glu Asn Lys Ser Tyr Cys Lys Gln Thr Val
           435                 440                 445
   gca cat aac aca gtt gtc gtt gac caa aaa acg caa aat aac ttc aat      1392
   Ala His Asn Thr Val Val Val Asp Gln Lys Thr Gln Asn Asn Phe Asn
       450                 455                 460
   acg gct tta gcg gaa tct aaa ttc ggt cag aag cac ttt ttc tta acc      1440
   Thr Ala Leu Ala Glu Ser Lys Phe Gly Gln Lys His Phe Phe Leu Thr
   465                 470                 475                 480
   gaa gat caa aaa ttc caa gcg atg agc ggt act att tct gag tac tac      1488
   Glu Asp Gln Lys Phe Gln Ala Met Ser Gly Thr Ile Ser Glu Tyr Tyr
                   485                 490                 495
   gaa ggt gtt gat atg caa cgt agc gta tta ctg cct gag cta gaa gaa      1536
   Glu Gly Val Asp Met Gln Arg Ser Val Leu Leu Pro Glu Leu Glu Glu
               500                 505                 510
   ttt gaa aaa cca ctt gtg att gac gta tac cgt att act gca gag acg      1584
   Phe Glu Lys Pro Leu Val Ile Asp Val Tyr Arg Ile Thr Ala Glu Thr
           515                 520                 525
   gaa cat gaa tac gat ctg cct tac cac cat aaa gga caa atc att cga      1632
   Glu His Glu Tyr Asp Leu Pro Tyr His His Lys Gly Gln Ile Ile Arg
       530                 535                 540
   acc gat ttt gac tac caa acg gaa agc aag ttg cgt cca tta ggt gat      1680
   Thr Asp Phe Asp Tyr Gln Thr Glu Ser Lys Leu Arg Pro Leu Gly Asp
   545                 550                 555                 560
   tca gat ggt tat cag cac ctg tgg aac gtg gct tca ggt aat gtg gaa      1728
   Ser Asp Gly Tyr Gln His Leu Trp Asn Val Ala Ser Gly Asn Val Glu
                   565                 570                 575
   ggt agc tca tta gtt agt tgg tta cat gat tct agc tac tac agc cta      1776
   Gly Ser Ser Leu Val Ser Trp Leu His Asp Ser Ser Tyr Tyr Ser Leu
               580                 585                 590
   gtc tct gca gca cca aca ggc agt caa gtg atc ttt gca cgc act ggt      1824
   Val Ser Ala Ala Pro Thr Gly Ser Gln Val Ile Phe Ala Arg Thr Gly
           595                 600                 605
   gct aat gac cct gat ttc aac tta cgc tca gag cct act ttg atc ttg      1872
   Ala Asn Asp Pro Asp Phe Asn Leu Arg Ser Glu Pro Thr Leu Ile Leu
       610                 615                 620
   cgt caa aaa gcc aaa gat cat gtg ttt gct tca gta ctc gaa acg cat      1920
   Arg Gln Lys Ala Lys Asp His Val Phe Ala Ser Val Leu Glu Thr His
   625                 630                 635                 640
   ggt tac ttt aat gaa tcc atc gaa gct tct gtt ggt gct cgc gga ttg      1968
   Gly Tyr Phe Asn Glu Ser Ile Glu Ala Ser Val Gly Ala Arg Gly Leu
                   645                 650                 655
   gtt gaa gcc gtt cag gtt att ggc cat aac gcg acg ggg act gtc gtt      2016
   Val Glu Ala Val Gln Val Ile Gly His Asn Ala Thr Gly Thr Val Val
               660                 665                 670
   gaa atc agt ttg acg acc ggt aag aag tac gta ttt gcg att tct aat      2064
   Glu Ile Ser Leu Thr Thr Gly Lys Lys Tyr Val Phe Ala Ile Ser Asn
           675                 680                 685
   aag ccg aat cca gaa ggt gag cac aaa gtt gaa ttc aac ggc gta caa      2112
   Lys Pro Asn Pro Glu Gly Glu His Lys Val Glu Phe Asn Gly Val Gln
       690                 695                 700
   tac caa tgg acg gga gca acg gct cag tta taa                          2145
   Tyr Gln Trp Thr Gly Ala Thr Ala Gln Leu
   705                 710
<210> 13
   <211> 714
   <212> PRT
   <213> Vibrio sp.
<400> 13
   Met Asn Tyr Gln Pro Leu Leu Met Asn Phe Glu Glu Ala Ala Glu Leu
   1               5                   10                  15
   Ala Lys Glu Leu Gly Lys Asp Thr Leu Leu Gly Asn Ala Leu Ala Arg
               20                  25                  30
   Asp Ile Ala Gln Thr Asp Ala Tyr Met Ala Asn Val Gly Ile Glu Val
           35                  40                  45
   Pro Gly His Gly Glu Gly Gly Gly Tyr Glu His Asn Arg His Lys Gln
       50                  55                  60
   Asn Tyr Ile His Met Asp Leu Ala Gly Arg Leu Phe Leu Ile Thr Gly
   65                  70                  75                  80
   Glu Gln Lys Tyr Arg Asp Tyr Ile Val Glu Leu Leu Thr Ala Tyr Ala
                   85                  90                  95
   Thr Val Tyr Pro Thr Leu Glu Ser Asn Val Ser Arg Asp Ser Asn Pro
               100                 105                 110
   Pro Gly Lys Leu Phe His Gln Thr Leu Asn Glu Asn Met Trp Met Leu
           115                 120                 125
   Tyr Ser Ser Cys Ala Tyr Ser Cys Val Tyr His Thr Leu Glu Glu Ser
       130                 135                 140
   Gln Lys Arg Gln Ile Glu Asp Asp Leu Phe Lys His Met Ile His Met
   145                 150                 155                 160
   Phe Val Val Thr Tyr Gly His Asp Phe Asp Ile Val His Asn His Gly
                   165                 170                 175
   Leu Trp Ala Val Ala Ala Val Gly Ile Cys Gly Tyr Ala Ile Asn Asp
               180                 185                 190
   Gln Glu Ser Val Asp Lys Ala Leu Tyr Gly Leu Lys Leu Asp Lys Val
           195                 200                 205
   Ser Gly Gly Phe Leu Ala Gln Leu Asp Gln Leu Phe Ser Pro Asp Gly
       210                 215                 220
   Tyr Tyr Met Glu Gly Pro Tyr Tyr His Arg Phe Ser Leu Arg Pro Ile
   225                 230                 235                 240
   Tyr Leu Phe Ala Glu Ala Ile Glu Arg Arg Gln Pro Glu Leu Gly Ile
                   245                 250                 255
   Tyr Glu Phe Asn Asp Ser Val Ile Lys Thr Thr Ser Tyr Ala Val Phe
               260                 265                 270
   Lys Thr Ala Phe Pro Asp Gly Thr Leu Pro Ala Leu Asn Asp Ser Ser
           275                 280                 285
   Lys Thr Ile Ser Ile Asn Asp Glu Gly Val Ile Met Ala Thr Ser Val
       290                 295                 300
   Cys Tyr His Arg Tyr Glu Gln Thr Glu Val Leu Leu Gly Met Ala Ser
   305                 310                 315                 320
   His Gln Gln Asn Val Trp Val His Lys Ala Gly Leu Thr Leu Ser Gln
                   325                 330                 335
   Ala Val Glu Ala Ala Asp Asp Ile Lys Pro Phe Asn Trp Gly Ser Leu
               340                 345                 350
   Phe Met Thr Asp Gly Pro Gln Gly Glu Lys Gly Gly Val Ser Ile Leu
           355                 360                 365
   Arg His Arg Asp Val Lys Asp Asp Asp Thr Met Ala Leu Ile Trp Phe
       370                 375                 380
   Gly Gln His Gly Ser Asp His Gln Tyr His Ser Ala Leu Asp His Gly
   385                 390                 395                 400
   His Tyr Asp Gly Leu His Leu Ser Val Phe Asn Arg Gly Asn Glu Val
                   405                 410                 415
   Leu His Asp Tyr Gly Phe Gly Arg Trp Val Asn Val Glu Pro Lys Phe
               420                 425                 430
   Gly Gly Arg Tyr Ile Pro Glu Asn Lys Ser Tyr Cys Lys Gln Thr Val
           435                 440                 445
   Ala His Asn Thr Val Val Val Asp Gln Lys Thr Gln Asn Asn Phe Asn
       450                 455                 460
   Thr Ala Leu Ala Glu Ser Lys Phe Gly Gln Lys His Phe Phe Leu Thr
   465                 470                 475                 480
   Glu Asp Gln Lys Phe Gln Ala Met Ser Gly Thr Ile Ser Glu Tyr Tyr
                   485                 490                 495
   Glu Gly Val Asp Met Gln Arg Ser Val Leu Leu Pro Glu Leu Glu Glu
               500                 505                 510
   Phe Glu Lys Pro Leu Val Ile Asp Val Tyr Arg Ile Thr Ala Glu Thr
           515                 520                 525
   Glu His Glu Tyr Asp Leu Pro Tyr His His Lys Gly Gln Ile Ile Arg
       530                 535                 540
   Thr Asp Phe Asp Tyr Gln Thr Glu Ser Lys Leu Arg Pro Leu Gly Asp
   545                 550                 555                 560
   Ser Asp Gly Tyr Gln His Leu Trp Asn Val Ala Ser Gly Asn Val Glu
                   565                 570                 575
   Gly Ser Ser Leu Val Ser Trp Leu His Asp Ser Ser Tyr Tyr Ser Leu
               580                 585                 590
   Val Ser Ala Ala Pro Thr Gly Ser Gln Val Ile Phe Ala Arg Thr Gly
           595                 600                 605
   Ala Asn Asp Pro Asp Phe Asn Leu Arg Ser Glu Pro Thr Leu Ile Leu
       610                 615                 620
   Arg Gln Lys Ala Lys Asp His Val Phe Ala Ser Val Leu Glu Thr His
   625                 630                 635                 640
   Gly Tyr Phe Asn Glu Ser Ile Glu Ala Ser Val Gly Ala Arg Gly Leu
                   645                 650                 655
   Val Glu Ala Val Gln Val Ile Gly His Asn Ala Thr Gly Thr Val Val
               660                 665                 670
   Glu Ile Ser Leu Thr Thr Gly Lys Lys Tyr Val Phe Ala Ile Ser Asn
   675 680 685
   Lys Pro Asn Pro Glu Gly Glu His Lys Val Glu Phe Asn Gly Val Gln
   690 695 700
   Tyr Gln Trp Thr Gly Ala Thr Ala Gln Leu
   705 710
<210> 14
   <211> 720
   <212> DNA
   <213> Vibrio sp.
<220>
   <221> CDS
   <222> (1)..(720)
<400> 14
   atg ttc aaa tta aat aaa gtt tta ctt gcc act act gcc gct cta ctt          48
   Met Phe Lys Leu Asn Lys Val Leu Leu Ala Thr Thr Ala Ala Leu Leu
   1               5                   10                  15
   gct acg aca gca gtt aat gta aat gct gct tct gtt gat att cgc cat          96
   Ala Thr Thr Ala Val Asn Val Asn Ala Ala Ser Val Asp Ile Arg His
               20                  25                  30
   gaa tac aaa cat gac act gaa tcg aat aac aac cgt atc aaa att ggt         144
   Glu Tyr Lys His Asp Thr Glu Ser Asn Asn Asn Arg Ile Lys Ile Gly
           35                  40                  45
   ggt agt act aag act gac cta ggt act cac tac tat agt gtt gaa atg         192
   Gly Ser Thr Lys Thr Asp Leu Gly Thr His Tyr Tyr Ser Val Glu Met
       50                  55                  60
   aaa ttc aat ggc cgt gat ggt act tat ggt ttt gat gat tta gag cgt         240
   Lys Phe Asn Gly Arg Asp Gly Thr Tyr Gly Phe Asp Asp Leu Glu Arg
   65                  70                  75                  80
   ggc gat tca gaa gtt gtt tat gaa ttt aaa cac tat gta aca gac aag         288
   Gly Asp Ser Glu Val Val Tyr Glu Phe Lys His Tyr Val Thr Asp Lys
                   85                  90                  95
   ttc tac gta caa cca ggt atg ccg atc act atg ggt gaa gag cgt gtc         336
   Phe Tyr Val Gln Pro Gly Met Pro Ile Thr Met Gly Glu Glu Arg Val
               100                 105                 110
   acc tat aaa cca cag cta cgt att ggc tat gat ttt gat tct ggt atc         384
   Thr Tyr Lys Pro Gln Leu Arg Ile Gly Tyr Asp Phe Asp Ser Gly Ile
           115                 120                 125
   aca act aaa tta cgc tac cgc cat gag ttc cgc gac ttt act tca tca         432
   Thr Thr Lys Leu Arg Tyr Arg His Glu Phe Arg Asp Phe Thr Ser Ser
       130                 135                 140
   aaa gag aat ggt gaa aca tgg cag aaa agt aaa gtg act gca aac cta         480
   Lys Glu Asn Gly Glu Thr Trp Gln Lys Ser Lys Val Thr Ala Asn Leu
   145                 150                 155                 160
   gat tac aca tgg aac aat atg atc caa ttt ggt ttt gaa gct aac tat         528
   Asp Tyr Thr Trp Asn Asn Met Ile Gln Phe Gly Phe Glu Ala Asn Tyr
                   165                 170                 175
   gaa aaa gcg cat gaa ggc gat aac tgg att ttg tac gat aac aaa act         576
   Glu Lys Ala His Glu Gly Asp Asn Trp Ile Leu Tyr Asp Asn Lys Thr
               180                 185                 190
   tgg aac tac gat tac aac cta aaa atc ggt tac aaa aac gaa agc aac         624
   Trp Asn Tyr Asp Tyr Asn Leu Lys Ile Gly Tyr Lys Asn Glu Ser Asn
           195                 200                 205
   tgg cgt ccg tat gtt gag ttc ggt aac gta agt gaa agt agt aaa aca         672
   Trp Arg Pro Tyr Val Glu Phe Gly Asn Val Ser Glu Ser Ser Lys Thr
       210                 215                 220
   gat gct cgt cag cta cgt agc cgt gta ggt ctt act tat agc ttc taa         720
   Asp Ala Arg Gln Leu Arg Ser Arg Val Gly Leu Thr Tyr Ser Phe
   225                 230                 235
<210> 15
   <211> 239
   <212> PRT
   <213> Vibrio sp.
<400> 15
   Met Phe Lys Leu Asn Lys Val Leu Leu Ala Thr Thr Ala Ala Leu Leu
   1               5                   10                  15
   Ala Thr Thr Ala Val Asn Val Asn Ala Ala Ser Val Asp Ile Arg His
               20                  25                  30
   Glu Tyr Lys His Asp Thr Glu Ser Asn Asn Asn Arg Ile Lys Ile Gly
           35                  40                  45
   Gly Ser Thr Lys Thr Asp Leu Gly Thr His Tyr Tyr Ser Val Glu Met
       50                  55                  60
   Lys Phe Asn Gly Arg Asp Gly Thr Tyr Gly Phe Asp Asp Leu Glu Arg
   65                  70                  75                  80
   Gly Asp Ser Glu Val Val Tyr Glu Phe Lys His Tyr Val Thr Asp Lys
                   85                  90                  95
   Phe Tyr Val Gln Pro Gly Met Pro Ile Thr Met Gly Glu Glu Arg Val
               100                 105                 110
   Thr Tyr Lys Pro Gln Leu Arg Ile Gly Tyr Asp Phe Asp Ser Gly Ile
           115                 120                 125
   Thr Thr Lys Leu Arg Tyr Arg His Glu Phe Arg Asp Phe Thr Ser Ser
       130                 135                 140
   Lys Glu Asn Gly Glu Thr Trp Gln Lys Ser Lys Val Thr Ala Asn Leu
   145 150 155 160
   Asp Tyr Thr Trp Asn Asn Met Ile Gln Phe Gly Phe Glu Ala Asn Tyr
   165 170 175
   Glu Lys Ala His Glu Gly Asp Asn Trp Ile Leu Tyr Asp Asn Lys Thr
   180 185 190
   Trp Asn Tyr Asp Tyr Asn Leu Lys Ile Gly Tyr Lys Asn Glu Ser Asn
   195 200 205
   Trp Arg Pro Tyr Val Glu Phe Gly Asn Val Ser Glu Ser Ser Lys Thr
   210 215 220
   Asp Ala Arg Gln Leu Arg Ser Arg Val Gly Leu Thr Tyr Ser Phe
   225 230 235
<210> 16
   <211> 1062
   <212> DNA
   <213> Vibrio sp.
<220>
   <221> CDS
   <222> (1)..(1062)
<400> 16
   atg cct tta aaa acc gat gag cta aga acc caa gcg tta ggg cct atg         48
   Met Pro Leu Lys Thr Asp Glu Leu Arg Thr Gln Ala Leu Gly Pro Met
   1               5                   10                  15
   cct acg cca atg gaa tta atg caa gct cac cca ctg cct gat gat gtg         96
   Pro Thr Pro Met Glu Leu Met Gln Ala His Pro Leu Pro Asp Asp Val
               20                  25                  30
   gca gaa cat att gaa aac tcg cgt cgc caa ata gaa aac atc ctg tca        144
   Ala Glu His Ile Glu Asn Ser Arg Arg Gln Ile Glu Asn Ile Leu Ser
           35                  40                  45
   ggt aaa gac tct cgc cta ctg gtt att gta ggg ccg tgt tct gta cat        192
   Gly Lys Asp Ser Arg Leu Leu Val Ile Val Gly Pro Cys Ser Val His
       50                  55                  60
   gat acc gac gcg gct cta gac tat gcg caa cgc ctt aat gca cta caa        240
   Asp Thr Asp Ala Ala Leu Asp Tyr Ala Gln Arg Leu Asn Ala Leu Gln
   65                  70                  75                  80
   gag caa cac aaa gat cag ctg ttt att gtg atg cgt act tac ttt gag        288
   Glu Gln His Lys Asp Gln Leu Phe Ile Val Met Arg Thr Tyr Phe Glu
                   85                  90                  95
   aaa cct aga acc gta att ggt tgg aaa gga tta att acc gat cct aat        336
   Lys Pro Arg Thr Val Ile Gly Trp Lys Gly Leu Ile Thr Asp Pro Asn
               100                 105                 110
   ctt gac ggt agc tac gct tta gaa gac ggt tta aac aaa gct cgt aaa        384
   Leu Asp Gly Ser Tyr Ala Leu Glu Asp Gly Leu Asn Lys Ala Arg Lys
           115                 120                 125
   tta ctg ctc gac atc aat aaa ctg ggt tta gcc act gcc act gaa ttt        432
   Leu Leu Leu Asp Ile Asn Lys Leu Gly Leu Ala Thr Ala Thr Glu Phe
       130                 135                 140
   tta gac atg gtg aca ggt caa tat att gct gac ctt att tca tgg ggg        480
   Leu Asp Met Val Thr Gly Gln Tyr Ile Ala Asp Leu Ile Ser Trp Gly
   145                 150                 155                 160
   gca att ggc gca cgt acc act gag tcc caa att cac cgt gaa atg gct        528
   Ala Ile Gly Ala Arg Thr Thr Glu Ser Gln Ile His Arg Glu Met Ala
                   165                 170                 175
   tcg gcg ctt tct tgc cct gtt ggg ttt aag aat ggc acc aat ggt aat        576
   Ser Ala Leu Ser Cys Pro Val Gly Phe Lys Asn Gly Thr Asn Gly Asn
               180                 185                 190
   gtg aag att tca acc gat gcc att cgt gcc gcg caa gcc tcc cat tac        624
   Val Lys Ile Ser Thr Asp Ala Ile Arg Ala Ala Gln Ala Ser His Tyr
           195                 200                 205
   ttc tgt tct cca gat aag aat ggt cga atg acg gtt tat cgc aca tcc        672
   Phe Cys Ser Pro Asp Lys Asn Gly Arg Met Thr Val Tyr Arg Thr Ser
       210                 215                 220
   ggt aac cca tac ggt cat acg att tta cgt ggc ggt gat aca ggc cct        720
   Gly Asn Pro Tyr Gly His Thr Ile Leu Arg Gly Gly Asp Thr Gly Pro
   225                 230                 235                 240
   aac ttt gat gca gaa tca att caa tct gct tgt gaa caa tta agt gaa        768
   Asn Phe Asp Ala Glu Ser Ile Gln Ser Ala Cys Glu Gln Leu Ser Glu
                   245                 250                 255
   gta ggc tta act ccc cgt tta gtc gtg gat ttc agt cac gct aac tgc        816
   Val Gly Leu Thr Pro Arg Leu Val Val Asp Phe Ser His Ala Asn Cys
               260                 265                 270
   caa aaa gag cat cgt aag caa tta gat gta gcg caa gat att tgc aac        864
   Gln Lys Glu His Arg Lys Gln Leu Asp Val Ala Gln Asp Ile Cys Asn
           275                 280                 285
   caa atc aac aat ggt tct caa tca att gca ggt att atg gcg gaa agc        912
   Gln Ile Asn Asn Gly Ser Gln Ser Ile Ala Gly Ile Met Ala Glu Ser
       290                 295                 300
   ttt atc gag gaa ggt aat caa cca atg acg gac tta aat gcc ctc acc        960
   Phe Ile Glu Glu Gly Asn Gln Pro Met Thr Asp Leu Asn Ala Leu Thr
   305                 310                 315                 320
   tat ggt aaa tcg att act gat cca tgc cta agt tgg aaa gat acg gta       1008
   Tyr Gly Lys Ser Ile Thr Asp Pro Cys Leu Ser Trp Lys Asp Thr Val
                   325                 330                 335
   acc atg ctc gac atg ctt gcc aat acc att aag aat caa tcg gag aag       1056
   Thr Met Leu Asp Met Leu Ala Asn Thr Ile Lys Asn Gln Ser Glu Lys
               340                 345                 350
   aac tga                                                               1062
   Asn
<210> 17
   <211> 353
   <212> PRT
   <213> Vibrio sp.
<400> 17
   Met Pro Leu Lys Thr Asp Glu Leu Arg Thr Gln Ala Leu Gly Pro Met
   1               5                   10                  15
   Pro Thr Pro Met Glu Leu Met Gln Ala His Pro Leu Pro Asp Asp Val
               20                  25                  30
   Ala Glu His Ile Glu Asn Ser Arg Arg Gln Ile Glu Asn Ile Leu Ser
           35                  40                  45
   Gly Lys Asp Ser Arg Leu Leu Val Ile Val Gly Pro Cys Ser Val His
       50                  55                  60
   Asp Thr Asp Ala Ala Leu Asp Tyr Ala Gln Arg Leu Asn Ala Leu Gln
   65                  70                  75                  80
   Glu Gln His Lys Asp Gln Leu Phe Ile Val Met Arg Thr Tyr Phe Glu
                   85                  90                  95
   Lys Pro Arg Thr Val Ile Gly Trp Lys Gly Leu Ile Thr Asp Pro Asn
               100                 105                 110
   Leu Asp Gly Ser Tyr Ala Leu Glu Asp Gly Leu Asn Lys Ala Arg Lys
           115                 120                 125
   Leu Leu Leu Asp Ile Asn Lys Leu Gly Leu Ala Thr Ala Thr Glu Phe
       130                 135                 140
   Leu Asp Met Val Thr Gly Gln Tyr Ile Ala Asp Leu Ile Ser Trp Gly
   145                 150                 155                 160
   Ala Ile Gly Ala Arg Thr Thr Glu Ser Gln Ile His Arg Glu Met Ala
                   165                 170                 175
   Ser Ala Leu Ser Cys Pro Val Gly Phe Lys Asn Gly Thr Asn Gly Asn
               180                 185                 190
   Val Lys Ile Ser Thr Asp Ala Ile Arg Ala Ala Gln Ala Ser His Tyr
           195                 200                 205
   Phe Cys Ser Pro Asp Lys Asn Gly Arg Met Thr Val Tyr Arg Thr Ser
       210                 215                 220
   Gly Asn Pro Tyr Gly His Thr Ile Leu Arg Gly Gly Asp Thr Gly Pro
   225                 230                 235                 240
   Asn Phe Asp Ala Glu Ser Ile Gln Ser Ala Cys Glu Gln Leu Ser Glu
                   245                 250                 255
   Val Gly Leu Thr Pro Arg Leu Val Val Asp Phe Ser His Ala Asn Cys
               260                 265                 270
   Gln Lys Glu His Arg Lys Gln Leu Asp Val Ala Gln Asp Ile Cys Asn
           275                 280                 285
   Gln Ile Asn Asn Gly Ser Gln Ser Ile Ala Gly Ile Met Ala Glu Ser
       290                 295                 300
   Phe Ile Glu Glu Gly Asn Gln Pro Met Thr Asp Leu Asn Ala Leu Thr
   305                 310                 315                 320
   Tyr Gly Lys Ser Ile Thr Asp Pro Cys Leu Ser Trp Lys Asp Thr Val
                   325                 330                 335
   Thr Met Leu Asp Met Leu Ala Asn Thr Ile Lys Asn Gln Ser Glu Lys
               340                 345                 350
   Asn
<210> 18
   <211> 483
   <212> DNA
   <213> Vibrio sp.
<220>
   <221> CDS
   <222> (1)..(483)
<400> 18
   atg cct tca ttt gac ata att tct gaa gtg gac agc gtt gaa gta aga      48
   Met Pro Ser Phe Asp Ile Ile Ser Glu Val Asp Ser Val Glu Val Arg
   1               5                   10                  15
   aat gcc gca gaa aac gcc aac cgt gaa cta gat aca cgt ttc gat ttt      96
   Asn Ala Ala Glu Asn Ala Asn Arg Glu Leu Asp Thr Arg Phe Asp Phe
               20                  25                  30
   cgt aac gta aac gct aat ttt gag ttt aaa aac gat att gta aaa ctg      144
   Arg Asn Val Asn Ala Asn Phe Glu Phe Lys Asn Asp Ile Val Lys Leu
           35                  40                  45
   aca gca gaa aat gaa tac cag ctt gga caa atg atg acg atg ctt cgt      192
   Thr Ala Glu Asn Glu Tyr Gln Leu Gly Gln Met Met Thr Met Leu Arg
       50                  55                  60
   ggt caa ctt gct aaa cgt ggt gtt gat gcc aat gcg gta gat tta caa      240
   Gly Gln Leu Ala Lys Arg Gly Val Asp Ala Asn Ala Val Asp Leu Gln
   65                  70                  75                  80
   aaa gcc gat caa act gga aaa aca cat act caa gag gcc agt ttc aaa      288
   Lys Ala Asp Gln Thr Gly Lys Thr His Thr Gln Glu Ala Ser Phe Lys
                   85                  90                  95
   caa ggt att gat gtt gcc att gcc aaa aaa gtg gta aaa cta atc aaa      336
   Gln Gly Ile Asp Val Ala Ile Ala Lys Lys Val Val Lys Leu Ile Lys
               100                 105                 110
   gac tca aag atc aaa gtt caa gct tcg att cag ggt gat aaa gtt cgt      384
   Asp Ser Lys Ile Lys Val Gln Ala Ser Ile Gln Gly Asp Lys Val Arg
           115                 120                 125
   gta acg ggt aaa aag cgt gat gat ttg cag caa gtt atg gca tta gtt      432
   Val Thr Gly Lys Lys Arg Asp Asp Leu Gln Gln Val Met Ala Leu Val
       130                 135                 140
   aaa ggt gcg gaa atg gat caa ccc ttc caa ttc aac aac ttt aaa gac      480
   Lys Gly Ala Glu Met Asp Gln Pro Phe Gln Phe Asn Asn Phe Lys Asp
   145                 150                 155                 160
   taa                                                                  483
<210> 19
   <211> 160
   <212> PRT
   <213> Vibrio sp.
<400> 19
   Met Pro Ser Phe Asp Ile Ile Ser Glu Val Asp Ser Val Glu Val Arg
   1 5 10 15
   Asn Ala Ala Glu Asn Ala Asn Arg Glu Leu Asp Thr Arg Phe Asp Phe
   20 25 30
   Arg Asn Val Asn Ala Asn Phe Glu Phe Lys Asn Asp Ile Val Lys Leu
   35 40 45
   Thr Ala Glu Asn Glu Tyr Gln Leu Gly Gln Met Met Thr Met Leu Arg
   50 55 60
   Gly Gln Leu Ala Lys Arg Gly Val Asp Ala Asn Ala Val Asp Leu Gln
   65 70 75 80
   Lys Ala Asp Gln Thr Gly Lys Thr His Thr Gln Glu Ala Ser Phe Lys
   85 90 95
   Gln Gly Ile Asp Val Ala Ile Ala Lys Lys Val Val Lys Leu Ile Lys
   100 105 110
   Asp Ser Lys Ile Lys Val Gln Ala Ser Ile Gln Gly Asp Lys Val Arg
   115 120 125
   Val Thr Gly Lys Lys Arg Asp Asp Leu Gln Gln Val Met Ala Leu Val
   130 135 140
   Lys Gly Ala Glu Met Asp Gln Pro Phe Gln Phe Asn Asn Phe Lys Asp
   145 150 155 160
<210> 20
   <211> 1581
   <212> DNA
   <213> Vibrio sp.
<220>
   <221> CDS
   <222> (1)..(1581)
<400> 20
   atg aaa cat att ttt cta aaa agc ttg cta gct tca tct gtt gta tta        48
   Met Lys His Ile Phe Leu Lys Ser Leu Leu Ala Ser Ser Val Val Leu
   1               5                   10                  15
   gca atg ggc tgt tca tct aat ggc gcc gat gct caa aaa gcc gcg gaa        96
   Ala Met Gly Cys Ser Ser Asn Gly Ala Asp Ala Gln Lys Ala Ala Glu
               20                  25                  30
   atg gca gca aaa caa gaa ggc ccg cgt gtt gca att ttg acg cct act       144
   Met Ala Ala Lys Gln Glu Gly Pro Arg Val Ala Ile Leu Thr Pro Thr
           35                  40                  45
   gcc att gaa gca agt agc cat gat ggt aat ggt cct gat cgt ctt ttt       192
   Ala Ile Glu Ala Ser Ser His Asp Gly Asn Gly Pro Asp Arg Leu Phe
       50                  55                  60
   gac caa gat att acg act cgt tgg tct gca aat ggt gac ggt gag tgg       240
   Asp Gln Asp Ile Thr Thr Arg Trp Ser Ala Asn Gly Asp Gly Glu Trp
   65                  70                  75                  80
   gcg atg ttt gat tac ggc tcg gta cat gaa ttt gat gct gtg cgc gcg       288
   Ala Met Phe Asp Tyr Gly Ser Val His Glu Phe Asp Ala Val Arg Ala
                   85                  90                  95
   gct ttc agt aaa ggt aac gag cgt gta acc aaa ttt gat gtt ctt gtc       336
   Ala Phe Ser Lys Gly Asn Glu Arg Val Thr Lys Phe Asp Val Leu Val
               100                 105                 110
   agt gtt gat gga aaa act tgg tca cca gtt ttg gaa ggt caa gaa agt       384
   Ser Val Asp Gly Lys Thr Trp Ser Pro Val Leu Glu Gly Gln Glu Ser
           115                 120                 125
   tca ggc cgt gta gtt ggt tta gaa cgt ttt gat ttt gaa cct gta aaa       432
   Ser Gly Arg Val Val Gly Leu Glu Arg Phe Asp Phe Glu Pro Val Lys
       130                 135                 140
   gct cgc tat gtt aaa tat gtt ggt cac ggt aac agt agc aat gac tgg       480
   Ala Arg Tyr Val Lys Tyr Val Gly His Gly Asn Ser Ser Asn Asp Trp
   145                 150                 155                 160
   aac agt gta aca gaa ttt gca gca gtc aac tgt tca ttg aac tct tgc      528
   Asn Ser Val Thr Glu Phe Ala Ala Val Asn Cys Ser Leu Asn Ser Cys
                   165                 170                 175
   cct acg tca gaa gta att aca gat gat gta gtt aaa gca gaa caa gca      576
   Pro Thr Ser Glu Val Ile Thr Asp Asp Val Val Lys Ala Glu Gln Ala
               180                 185                 190
   atg atc gct caa atg gcg gca gat gca gcg gca cag aaa gaa gca cgt      624
   Met Ile Ala Gln Met Ala Ala Asp Ala Ala Ala Gln Lys Glu Ala Arg
           195                 200                 205
   aaa gat tta cgt aaa gga aac ttc ggt gcg ccg gcg gtt tac cca tgt      672
   Lys Asp Leu Arg Lys Gly Asn Phe Gly Ala Pro Ala Val Tyr Pro Cys
       210                 215                 220
   gac act acg gtt aaa tgt gac aca aga agt gca cta tct gcg cct aca      720
   Asp Thr Thr Val Lys Cys Asp Thr Arg Ser Ala Leu Ser Ala Pro Thr
   225                 230                 235                 240
   ggt ttg cca gca acg cca aaa gcg ggc aat aag cca agc caa aac ttt      768
   Gly Leu Pro Ala Thr Pro Lys Ala Gly Asn Lys Pro Ser Gln Asn Phe
                   245                 250                 255
   gac tta acc gaa tgg tac cta tct caa cca ttt gac cat gat ggc aac      816
   Asp Leu Thr Glu Trp Tyr Leu Ser Gln Pro Phe Asp His Asp Gly Asn
               260                 265                 270
   ggt cgc cct gat gat gtt tca gag tgg aac tta gcc aac ggt tat gaa      864
   Gly Arg Pro Asp Asp Val Ser Glu Trp Asn Leu Ala Asn Gly Tyr Glu
           275                 280                 285
   cac cca gat gta ttc tac aca gcg gat gat ggt ggt tta gta ttt aaa      912
   His Pro Asp Val Phe Tyr Thr Ala Asp Asp Gly Gly Leu Val Phe Lys
       290                 295                 300
   acc ttc att aaa ggt gta cgt act tcg aag aat act aag tac gca cgt      960
   Thr Phe Ile Lys Gly Val Arg Thr Ser Lys Asn Thr Lys Tyr Ala Arg
   305                 310                 315                 320
   act gaa atg cgc gaa atg cta cgt aaa ggt gat caa tca att tca act     1008
   Thr Glu Met Arg Glu Met Leu Arg Lys Gly Asp Gln Ser Ile Ser Thr
                   325                 330                 335
   aaa ggt gta aac aaa aat aac tgg gta ttt agc tca gca ccg gtt gaa     1056
   Lys Gly Val Asn Lys Asn Asn Trp Val Phe Ser Ser Ala Pro Val Glu
               340                 345                 350
   gat caa aaa gca gcc ggc ggc gtt gat ggt gta ctt gaa gca aca tta     1104
   Asp Gln Lys Ala Ala Gly Gly Val Asp Gly Val Leu Glu Ala Thr Leu
           355                 360                 365
   aaa gtc gat cac aca acc act aca ggt gaa gca ggc caa gta ggg cgc     1152
   Lys Val Asp His Thr Thr Thr Thr Gly Glu Ala Gly Gln Val Gly Arg
       370                 375                 380
   ttt atc att ggt cag atc cat gac caa gat gat gag cca att cgt ttg     1200
   Phe Ile Ile Gly Gln Ile His Asp Gln Asp Asp Glu Pro Ile Arg Leu
   385                 390                 395                 400
   tac tac cgt aaa cta cca aac cac aac aca ggt aca gtt tac ttt gct     1248
   Tyr Tyr Arg Lys Leu Pro Asn His Asn Thr Gly Thr Val Tyr Phe Ala
                   405                 410                 415
   cat gaa aat aca tta aaa ggt act gat gcg ttc tat gac ctt gta ggt      1296
   His Glu Asn Thr Leu Lys Gly Thr Asp Ala Phe Tyr Asp Leu Val Gly
               420                 425                 430
   ggc atg acg ggc gaa att ggt gat gat ggt att gct tta ggt gaa aaa      1344
   Gly Met Thr Gly Glu Ile Gly Asp Asp Gly Ile Ala Leu Gly Glu Lys
           435                 440                 445
   ttc act tac cgc att gaa gtt aaa ggt aat act cta acg gtt act gtt      1392
   Phe Thr Tyr Arg Ile Glu Val Lys Gly Asn Thr Leu Thr Val Thr Val
       450                 455                 460
   aag cgt gac ggt aaa gaa gac gta caa caa gtc gtt gac atg agt gaa      1440
   Lys Arg Asp Gly Lys Glu Asp Val Gln Gln Val Val Asp Met Ser Glu
   465                 470                 475                 480
   agt ggt tat gat gtg ggt ggt aag tac atg tac ttt aaa gct ggc gta      1488
   Ser Gly Tyr Asp Val Gly Gly Lys Tyr Met Tyr Phe Lys Ala Gly Val
                   485                 490                 495
   tat aat caa aac atc tct ggc gat tta gat gat tat gtt caa gca aca      1536
   Tyr Asn Gln Asn Ile Ser Gly Asp Leu Asp Asp Tyr Val Gln Ala Thr
               500                 505                 510
   ttc tat aaa cta gaa aaa tcg cac ggt aca tac aca ggt aaa taa          1581
   Phe Tyr Lys Leu Glu Lys Ser His Gly Thr Tyr Thr Gly Lys
           515                 520                 525
<210> 21
   <211> 526
   <212> PRT
   <213> Vibrio sp.
<400> 21
   Met Lys His Ile Phe Leu Lys Ser Leu Leu Ala Ser Ser Val Val Leu
   1               5                   10                  15
   Ala Met Gly Cys Ser Ser Asn Gly Ala Asp Ala Gln Lys Ala Ala Glu
               20                  25                  30
   Met Ala Ala Lys Gln Glu Gly Pro Arg Val Ala Ile Leu Thr Pro Thr
           35                  40                  45
   Ala Ile Glu Ala Ser Ser His Asp Gly Asn Gly Pro Asp Arg Leu Phe
       50                  55                  60
   Asp Gln Asp Ile Thr Thr Arg Trp Ser Ala Asn Gly Asp Gly Glu Trp
   65                  70                  75                  80
   Ala Met Phe Asp Tyr Gly Ser Val His Glu Phe Asp Ala Val Arg Ala
                   85                  90                  95
   Ala Phe Ser Lys Gly Asn Glu Arg Val Thr Lys Phe Asp Val Leu Val
               100                 105                 110
   Ser Val Asp Gly Lys Thr Trp Ser Pro Val Leu Glu Gly Gln Glu Ser
           115                 120                 125
   Ser Gly Arg Val Val Gly Leu Glu Arg Phe Asp Phe Glu Pro Val Lys
       130                 135                 140
   Ala Arg Tyr Val Lys Tyr Val Gly His Gly Asn Ser Ser Asn Asp Trp
   145                 150                 155                 160
   Asn Ser Val Thr Glu Phe Ala Ala Val Asn Cys Ser Leu Asn Ser Cys
                   165                 170                 175
   Pro Thr Ser Glu Val Ile Thr Asp Asp Val Val Lys Ala Glu Gln Ala
               180                 185                 190
   Met Ile Ala Gln Met Ala Ala Asp Ala Ala Ala Gln Lys Glu Ala Arg
           195                 200                 205
   Lys Asp Leu Arg Lys Gly Asn Phe Gly Ala Pro Ala Val Tyr Pro Cys
       210                 215                 220
   Asp Thr Thr Val Lys Cys Asp Thr Arg Ser Ala Leu Ser Ala Pro Thr
   225                 230                 235                 240
   Gly Leu Pro Ala Thr Pro Lys Ala Gly Asn Lys Pro Ser Gln Asn Phe
                   245                 250                 255
   Asp Leu Thr Glu Trp Tyr Leu Ser Gln Pro Phe Asp His Asp Gly Asn
               260                 265                 270
   Gly Arg Pro Asp Asp Val Ser Glu Trp Asn Leu Ala Asn Gly Tyr Glu
           275                 280                 285
   His Pro Asp Val Phe Tyr Thr Ala Asp Asp Gly Gly Leu Val Phe Lys
       290                 295                 300
   Thr Phe Ile Lys Gly Val Arg Thr Ser Lys Asn Thr Lys Tyr Ala Arg
   305                 310                 315                 320
   Thr Glu Met Arg Glu Met Leu Arg Lys Gly Asp Gln Ser Ile Ser Thr
                   325                 330                 335
   Lys Gly Val Asn Lys Asn Asn Trp Val Phe Ser Ser Ala Pro Val Glu
               340                 345                 350
   Asp Gln Lys Ala Ala Gly Gly Val Asp Gly Val Leu Glu Ala Thr Leu
           355                 360                 365
   Lys Val Asp His Thr Thr Thr Thr Gly Glu Ala Gly Gln Val Gly Arg
       370                 375                 380
   Phe Ile Ile Gly Gln Ile His Asp Gln Asp Asp Glu Pro Ile Arg Leu
   385                 390                 395                 400
   Tyr Tyr Arg Lys Leu Pro Asn His Asn Thr Gly Thr Val Tyr Phe Ala
                   405                 410                 415
   His Glu Asn Thr Leu Lys Gly Thr Asp Ala Phe Tyr Asp Leu Val Gly
               420                 425                 430
   Gly Met Thr Gly Glu Ile Gly Asp Asp Gly Ile Ala Leu Gly Glu Lys
           435                 440                 445
   Phe Thr Tyr Arg Ile Glu Val Lys Gly Asn Thr Leu Thr Val Thr Val
       450                 455                 460
   Lys Arg Asp Gly Lys Glu Asp Val Gln Gln Val Val Asp Met Ser Glu
   465                 470                 475                 480
   Ser Gly Tyr Asp Val Gly Gly Lys Tyr Met Tyr Phe Lys Ala Gly Val
                   485                 490                 495
   Tyr Asn Gln Asn Ile Ser Gly Asp Leu Asp Asp Tyr Val Gln Ala Thr
               500                 505                 510
   Phe Tyr Lys Leu Glu Lys Ser His Gly Thr Tyr Thr Gly Lys
           515                 520                 525
<210> 22
   <211> 885
   <212> DNA
   <213> Vibrio sp.
<220>
   <221> CDS
   <222> (1)..(885)
<400> 22
   atg atg act aaa cct gtt att ggt ttt atc ggc cta ggc ctt atg ggc        48
   Met Met Thr Lys Pro Val Ile Gly Phe Ile Gly Leu Gly Leu Met Gly
   1               5                   10                  15
   ggc aac atg gta gag aac cta caa act cgt ggc tac caa gta aat gta        96
   Gly Asn Met Val Glu Asn Leu Gln Thr Arg Gly Tyr Gln Val Asn Val
               20                  25                  30
   atg gat ctt aat aaa gac gct gtt gca gct gtt cta gct cgt ggt aac        144
   Met Asp Leu Asn Lys Asp Ala Val Ala Ala Val Leu Ala Arg Gly Asn
           35                  40                  45
   gcg gca gaa gca tca tct ggt aaa gaa ctt gct gaa aaa agc gac atc        192
   Ala Ala Glu Ala Ser Ser Gly Lys Glu Leu Ala Glu Lys Ser Asp Ile
       50                  55                  60
   gta atg ctt tgc cta aca acg tct gaa gtt gta gaa aaa gta gta tac        240
   Val Met Leu Cys Leu Thr Thr Ser Glu Val Val Glu Lys Val Val Tyr
   65                  70                  75                  80
   ggt gaa aca ggt atc cta gcg ggt atg tct gaa ggc aaa acg cta atc        288
   Gly Glu Thr Gly Ile Leu Ala Gly Met Ser Glu Gly Lys Thr Leu Ile
                   85                  90                  95
   gac ttt ggt act tct att cct gct tct act aag aag att ggt gct gat        336
   Asp Phe Gly Thr Ser Ile Pro Ala Ser Thr Lys Lys Ile Gly Ala Asp
               100                 105                 110
   ctt gcg gct aaa ggc gca ggt atg atc gat gct cct ctt ggt cgt act        384
   Leu Ala Ala Lys Gly Ala Gly Met Ile Asp Ala Pro Leu Gly Arg Thr
           115                 120                 125
   cca gct cac gct aaa gat ggt cta cta aac atc atg gca gct ggc gat        432
   Pro Ala His Ala Lys Asp Gly Leu Leu Asn Ile Met Ala Ala Gly Asp
       130                 135                 140
   atg gaa aca ttc aac aaa gtg aaa cct gtt ctt gat gat caa ggt gaa        480
   Met Glu Thr Phe Asn Lys Val Lys Pro Val Leu Asp Asp Gln Gly Glu
   145                 150                 155                 160
   aac gta ttc cac tta ggt gct cta ggt tct ggc cac gtt act aag ctt        528
   Asn Val Phe His Leu Gly Ala Leu Gly Ser Gly His Val Thr Lys Leu
                   165                 170                 175
   gtg aac aac ttc atg ggt atg act act gtt gca act atg tct caa gca        576
   Val Asn Asn Phe Met Gly Met Thr Thr Val Ala Thr Met Ser Gln Ala
               180                 185                 190
   ttc gca gtg gca aaa caa gcc ggt gtt gac ggt cag caa tta ttc gac        624
   Phe Ala Val Ala Lys Gln Ala Gly Val Asp Gly Gln Gln Leu Phe Asp
           195                 200                 205
   atc atg tca gct ggc cca tct aac tct cca ttc atg caa ttc tgt aag        672
   Ile Met Ser Ala Gly Pro Ser Asn Ser Pro Phe Met Gln Phe Cys Lys
       210                 215                 220
   ttc tac gca gta gac ggc gaa gaa aaa cta ggt ttc tct gtt gca aac        720
   Phe Tyr Ala Val Asp Gly Glu Glu Lys Leu Gly Phe Ser Val Ala Asn
   225                 230                 235                 240
   gca aac aaa gac ctt ggt tac ttc ctt gca cta tgt gaa gag tta ggt        768
   Ala Asn Lys Asp Leu Gly Tyr Phe Leu Ala Leu Cys Glu Glu Leu Gly
                   245                 250                 255
   act gag tct cta atc gct caa ggt act tct aca agc cta caa gca gct        816
   Thr Glu Ser Leu Ile Ala Gln Gly Thr Ser Thr Ser Leu Gln Ala Ala
               260                 265                 270
   gtt gat gct ggt ctt ggc caa aat gat gtg cca gtt atc ttt gat tac        864
   Val Asp Ala Gly Leu Gly Gln Asn Asp Val Pro Val Ile Phe Asp Tyr
   275 280 285
   ttc atg ggt ttg aaa aag taa                                     885
   Phe Met Gly Leu Lys Lys
       290
<210> 23
   <211> 294
   <212> PRT
   <213> Vibrio sp.
<400> 23
   Met Met Thr Lys Pro Val Ile Gly Phe Ile Gly Leu Gly Leu Met Gly
   1               5                   10                  15
   Gly Asn Met Val Glu Asn Leu Gln Thr Arg Gly Tyr Gln Val Asn Val
               20                  25                  30
   Met Asp Leu Asn Lys Asp Ala Val Ala Ala Val Leu Ala Arg Gly Asn
           35                  40                  45
   Ala Ala Glu Ala Ser Ser Gly Lys Glu Leu Ala Glu Lys Ser Asp Ile
       50                  55                  60
   Val Met Leu Cys Leu Thr Thr Ser Glu Val Val Glu Lys Val Val Tyr
   65                  70                  75                  80
   Gly Glu Thr Gly Ile Leu Ala Gly Met Ser Glu Gly Lys Thr Leu Ile
                   85                  90                  95
   Asp Phe Gly Thr Ser Ile Pro Ala Ser Thr Lys Lys Ile Gly Ala Asp
               100                 105                 110
   Leu Ala Ala Lys Gly Ala Gly Met Ile Asp Ala Pro Leu Gly Arg Thr
           115                 120                 125
   Pro Ala His Ala Lys Asp Gly Leu Leu Asn Ile Met Ala Ala Gly Asp
       130                 135                 140
   Met Glu Thr Phe Asn Lys Val Lys Pro Val Leu Asp Asp Gln Gly Glu
   145                 150                 155                 160
   Asn Val Phe His Leu Gly Ala Leu Gly Ser Gly His Val Thr Lys Leu
                   165                 170                 175
   Val Asn Asn Phe Met Gly Met Thr Thr Val Ala Thr Met Ser Gln Ala
               180                 185                 190
   Phe Ala Val Ala Lys Gln Ala Gly Val Asp Gly Gln Gln Leu Phe Asp
               195                 200                 205
   Ile Met Ser Ala Gly Pro Ser Asn Ser Pro Phe Met Gln Phe Cys Lys
       210                 215                 220
   Phe Tyr Ala Val Asp Gly Glu Glu Lys Leu Gly Phe Ser Val Ala Asn
   225                 230                 235                 240
   Ala Asn Lys Asp Leu Gly Tyr Phe Leu Ala Leu Cys Glu Glu Leu Gly
                   245                 250                 255
   Thr Glu Ser Leu Ile Ala Gln Gly Thr Ser Thr Ser Leu Gln Ala Ala
               260                 265                 270
   Val Asp Ala Gly Leu Gly Gln Asn Asp Val Pro Val Ile Phe Asp Tyr
           275                 280                 285
   Phe Met Gly Leu Lys Lys
       290
<210> 24
   <211> 9323
   <212> DNA
   <213> Artificial Sequence
<220>
   <223> Plasmid pSA2S2
<400> 24
   cgtatggcaa tgaaagacgg tgagctggtg atatgggata gtgttcaccc ttgttacacc      60
   gttttccatg agcaaactga aacgttttca tcgctctgga gtgaatacca cgacgatttc     120
   cggcagtttc tacacatata ttcgcaagat gtggcgtgtt acggtgaaaa cctggcctat     180
   ttccctaaag ggtttattga gaatatgttt ttcgtctcag ccaatccctg ggtgagtttc     240
   accagttttg atttaaacgt ggccaatatg gacaacttct tcgcccccgt tttcaccatg     300
   ggcaaatatt atacgcaagg cgacaaggtg ctgatgccgc tggcgattca ggttcatcat     360
   gccgtctgtg atggcttcca tgtcggcaga atgcttaatg aattacaaca gtactgcgat     420
   gagtggcagg gcggggcgta atttttttaa ggcagttatt ggtgccctta aacgcctggt     480
   gctacgcctg aataagtgat aataagcgga tgaatggcag aaattcgaaa gcaaattcga     540
   cccggtcgtc ggttcagggc agggtcgtta aatagccgct tatgtctatt gctggtttac     600
   cggtttattg actaccggaa gcagtgtgac cgtgtgcttc tcaaatgcct gaggccagtt     660
   tgctcaggct ctccccgtgg aggtaataat tgacgatatg atcatttatt ctgcctccca     720
   gagcctgata aaaacggtta gcgcttcgtt aatacagatg taggtgttcc acagggtagc     780
   cagcagcatc ctgcgatgca gatccggaac ataatggtgc agggcgcttg tttcggcgtg     840
   ggtatggtgg caggccccgt ggccggggga ctgttgggcg ctgccggcac ctgtcctacg 900
   agttgcatga taaagaagac agtcataagt gcggcgacga tagtcatgcc ccgcgcccac 960
   cggaaggagc taccggacag cggtgcggac tgttgtaact cagaataaga aatgaggccg 1020
   ctcatggcgt tccaatacgc aaaccgcctc tccccgcgcg ttggccgatt cattaatgca 1080
   gctggcacga caggtttccc gactggaaag cgggcagtga gcgcaacgca attaatgtga 1140
   gttagctcac tcattaggca ccccaggctt tacactttat gcttccggct cgtatgttgt 1200
   gtggaattgt gagcggataa caatttcaca caggaaacag ctatgaccat gattacgcca 1260
   agcttaggag gttaaatgtt caaattaaat aaagttttac ttgccactac tgccgctcta 1320
   cttgctacga cagcagttaa tgtaaatgct gcttctgttg atattcgcca tgaatacaaa 1380
   catgacactg aatcgaataa caaccgtatc aaaattggtg gtagtactaa gactgaccta 1440
   ggtactcact actatagtgt tgaaatgaaa ttcaatggcc gtgatggtac ttatggtttt 1500
   gatgatttag agcgtggcga ttcagaagtt gtttatgaat ttaaacacta tgtaacagac 1560
   aagttctacg tacaaccagg tatgccgatc actatgggtg aagagcgtgt cacctataaa 1620
   ccacagctac gtattggcta tgattttgat tctggtatca caactaaatt acgctaccgc 1680
   catgagttcc gcgactttac ttcatcaaaa gagaatggtg aaacatggca gaaaagtaaa 1740
   gtgactgcaa acctagatta cacatggaac aatatgatcc aatttggttt tgaagctaac 1800
   tatgaaaaag cgcatgaagg cgataactgg attttgtacg ataacaaaac ttggaactac 1860
   gattacaacc taaaaatcgg ttacaaaaac gaaagcaact ggcgtccgta tgttgagttc 1920
   ggtaacgtaa gtgaaagtag taaaacagat gctcgtcagc tacgtagccg tgtaggtctt 1980
   acttatagct tctaaaggag gttaaatgaa acatattttt ctaaaaagct tgctagcttc 2040
   atctgttgta ttagcaatgg gctgttcatc taatggcgcc gatgctcaaa aagccgcgga 2100
   aatggcagca aaacaagaag gcccgcgtgt tgcaattttg acgcctactg ccattgaagc 2160
   aagtagccat gatggtaatg gtcctgatcg tctttttgac caagatatta cgactcgttg 2220
   gtctgcaaat ggtgacggtg agtgggcgat gtttgattac ggctcggtac atgaatttga 2280
   tgctgtgcgc gcggctttca gtaaaggtaa cgagcgtgta accaaatttg atgttcttgt 2340
   cagtgttgat ggaaaaactt ggtcaccagt tttggaaggt caagaaagtt caggccgtgt 2400
   agttggttta gaacgttttg attttgaacc tgtaaaagct cgctatgtta aatatgttgg 2460
   tcacggtaac agtagcaatg actggaacag tgtaacagaa tttgcagcag tcaactgttc 2520
   attgaactct tgccctacgt cagaagtaat tacagatgat gtagttaaag cagaacaagc 2580
   aatgatcgct caaatggcgg cagatgcagc ggcacagaaa gaagcacgta aagatttacg 2640
   taaaggaaac ttcggtgcgc cggcggttta cccatgtgac actacggtta aatgtgacac 2700
   aagaagtgca ctatctgcgc ctacaggttt gccagcaacg ccaaaagcgg gcaataagcc 2760
   aagccaaaac tttgacttaa ccgaatggta cctatctcaa ccatttgacc atgatggcaa 2820
   cggtcgccct gatgatgttt cagagtggaa cttagccaac ggttatgaac acccagatgt 2880
   attctacaca gcggatgatg gtggtttagt atttaaaacc ttcattaaag gtgtacgtac 2940
   ttcgaagaat actaagtacg cacgtactga aatgcgcgaa atgctacgta aaggtgatca 3000
   atcaatttca actaaaggtg taaacaaaaa taactgggta tttagctcag caccggttga 3060
   agatcaaaaa gcagccggcg gcgttgatgg tgtacttgaa gcaacattaa aagtcgatca 3120
   cacaaccact acaggtgaag caggccaagt agggcgcttt atcattggtc agatccatga 3180
   ccaagatgat gagccaattc gtttgtacta ccgtaaacta ccaaaccaca acacaggtac 3240
   agtttacttt gctcatgaaa atacattaaa aggtactgat gcgttctatg accttgtagg 3300
   tggcatgacg ggcgaaattg gtgatgatgg tattgcttta ggtgaaaaat tcacttaccg 3360
   cattgaagtt aaaggtaata ctctaacggt tactgttaag cgtgacggta aagaagacgt 3420
   acaacaagtc gttgacatga gtgaaagtgg ttatgatgtg ggtggtaagt acatgtactt 3480
   taaagctggc gtatataatc aaaacatctc tggcgattta gatgattatg ttcaagcaac 3540
   attctataaa ctagaaaaat cgcacggtac atacacaggt aaataaagga ggttaaatga 3600
   ctgtcgatac tattgttgtt gctgcctatt ttctctttct aattgcgatt ggttggatgt 3660
   tccgtacatt tactacctct actagtgatt actttagggg gggaggtaag atgttatggt 3720
   ggatggtagg tgctactgcc ttcatgactc aattctcagc ctggaccttc actggagcag 3780
   cagggcgcgc ttatagcgat ggctttgttg tcgtaatctt attcttagca aacgcatttg 3840
   gttactttgt taactatatg tacttcgcgc ctaaattccg tcagcttcgt gtagtgactg 3900
   caattgaagc tattcgtcaa cgttttggta aaacttcaga acagttcttt acctggggcg 3960
   gtatgccaga tagcttaatt tctgcgggta tttggctaaa tggcctggct atctttgtgg 4020
   cggcagtttt tgatatccca atggaagcaa cgattatcgt cacaggctta gttcttatgc 4080
   ttatgtctgt aaccggtggc tcttgggcag ttgttgcttc tgactttatg caaatgctgg 4140
   tgatcatggc ggtaaccatt acttgtgccg ttgctgctta cttccaaggt ggtggtatta 4200
   ctaacattgt aaataacttc caaggcgatt tctttgttgg taacaacttg aattacatga 4260
   gcatctttgt tctttgggta gtgttcatct tcatcaagca gtttggtgta atgaataatt 4320
   cgattaacgc ttatcgttat ttatgtgcaa aagacagtga aaatgctcgt aaagcggctg 4380
   gccttgcttg tgtattgatg attattggcc ctgtaatttg gttcttacct gcttggtatg 4440
   ttggtgcatt tatgcctaac tttgccactg agtatggtgc tgtgggcaat gatgcagctt 4500
   accttgcttt cgtacaacac atgatgccag cgggtatggt tggcttgctt atgtcagcaa 4560
   tgtttgccgc gacaatgtct tcaatggatt ctggcttaaa ccgtaatgca ggcatctttg 4620
   tgatgaactt ctatagcccg attcttcgtc caaatgcttc acaaaaagaa ttggtggtag 4680
   tgagtaaaac gaccactatc gtgatgggct ttattatcat tgcgattggc ttgtttatta 4740
   actcattacg tcacttaagc ttatttgata tcgtgttaaa tgtgggtgcg ttgattggct 4800
   tcccaatgtt gattccagtg ttattaggca tgtggattcg taaaacacca gattggtcag 4860
   cttggacaac tttgattgtc ggcggtatcg tttcttgggt tttcggtatt tcattacaag 4920
   cacacgatgt agaacacttc ttcggtttag aacaagcact gaccggacgt gaatggtctg 4980
   acttgaaagt agcattaagc cttgcagcac acgttgtatt tactggcggt ttcttcttgc 5040
   taacgatgct tttctacaga ggacgcactc cagagcgtga agctgaagta gaaagactat 5100
   tcgaaaactg gaacactccg gtggtttcaa gtggtgaagc gcagcatgat ttagatacta 5160
   aacaacgctc tatgctaggt cgattaatct gtactgctgg atttggtgtt ttagcaatgg 5220
   cactactacc aaatgaaatg acaggacgta tgttgttcct attatgtggt tcaattgttt 5280
   tggttgttgg tgtgttgttg gtgaatgcct caaaagtaca acatcaaacg gtttcagaat 5340
   cgtaaaggct ttacacttta tgcttccggc tcgtatgttg tgtggaattg tgagcggata 5400
   acaatttcac acaggaaaca gctatgacca tgattacgcc aagcttagga ggttaaatga 5460
   attaccaacc attattaatg aattttgaag aagcggctga actggcaaaa gagttaggta 5520
   aagatactct gctaggtaat gcactggctc gcgatattgc tcaaacagat gcatatatgg 5580
   caaatgtggg tattgaagtg ccaggacatg gtgaaggtgg tggttatgag cataatcgtc 5640
   acaagcaaaa ctatattcat atggacttag caggccgctt atttctgatt actggtgagc 5700
   aaaaataccg tgactacatt gttgaactac taacagctta cgctactgtg tatccaacac 5760
   ttgaaagcaa tgtaagccgc gacagtaacc ctccaggtaa actgttccac caaacattga 5820
   atgaaaatat gtggatgctg tactcatcat gtgcatatag ctgtgtttac cacacgcttg 5880
   aagagtcgca aaaacgtcag attgaagatg acctgttcaa acacatgatc cacatgtttg 5940
   tagtgactta cggtcatgac tttgacattg tgcacaacca cggtctatgg gccgttgcgg 6000
   cagtaggtat ttgtggttac gccatcaatg accaagaatc tgttgataaa gcactatacg 6060
   gtttgaagct tgataaagtg agcggtggct tcttggctca actggatcag ttgttctcgc 6120
   cagatggcta ctatatggaa ggtccgtact atcaccgctt ctcattgcgt ccaatttact 6180
   tgtttgccga agcaattgag cgtcgtcagc cagagttggg catttatgaa tttaacgatt 6240
   cagtgatcaa aacgacgtca tacgcagtat ttaaaaccgc ttttccagat ggcactctac 6300
   ctgcgcttaa tgattcatca aaaaccatca gtattaatga tgaaggtgtg atcatggcaa 6360
   cgtctgtgtg ttaccaccgc tatgagcaaa ctgaagtatt gttgggtatg gccagtcacc 6420
   aacaaaatgt ctgggtacat aaagcaggtc ttactctatc tcaagccgtt gaagcagcag 6480
   atgacatcaa acctttcaac tggggcagct tgtttatgac agatggccca caaggtgaga 6540
   aaggcggtgt gagtattctt cgtcatcgtg atgttaaaga tgatgacacc atggcactaa 6600
   tttggtttgg tcaacatggt tccgatcacc aatatcattc agcattagat catggtcatt 6660
   acgatggctt acatctaagt gtgtttaacc gtggtaatga agtgctgcat gattatgggt 6720
   ttggtcgttg ggtaaacgtt gagccaaaat ttggtggtcg ttatattcca gaaaataagt 6780
   cttactgtaa gcaaactgtg gcacataaca cagttgtcgt tgaccaaaaa acgcaaaata 6840
   acttcaatac ggctttagcg gaatctaaat tcggtcagaa gcactttttc ttaaccgaag 6900
   atcaaaaatt ccaagcgatg agcggtacta tttctgagta ctacgaaggt gttgatatgc 6960
   aacgtagcgt attactgcct gagctagaag aatttgaaaa accacttgtg attgacgtat 7020
   accgtattac tgcagagacg gaacatgaat acgatctgcc ttaccaccat aaaggacaaa 7080
   tcattcgaac cgattttgac taccaaacgg aaagcaagtt gcgtccatta ggtgattcag 7140
   atggttatca gcacctgtgg aacgtggctt caggtaatgt ggaaggtagc tcattagtta 7200
   gttggttaca tgattctagc tactacagcc tagtctctgc agcaccaaca ggcagtcaag 7260
   tgatctttgc acgcactggt gctaatgacc ctgatttcaa cttacgctca gagcctactt 7320
   tgatcttgcg tcaaaaagcc aaagatcatg tgtttgcttc agtactcgaa acgcatggtt 7380
   actttaatga atccatcgaa gcttctgttg gtgctcgcgg attggttgaa gccgttcagg 7440
   ttattggcca taacgcgacg gggactgtcg ttgaaatcag tttgacgacc ggtaagaagt 7500
   acgtatttgc gatttctaat aagccgaatc cagaaggtga gcacaaagtt gaattcaacg 7560
   gcgtacaata ccaatggacg ggagcaacgg ctcagttata agtcgactct agaggatccc 7620
   cgggtaccga gctcgaattc actggccgtc gttttacaac gtcgtgactg ggaaaaccct 7680
   ggcgttaccc aacttaatcg ccttgcagca catccccctt tcgccagctg gcgtaatagc 7740
   gaagaggccc gcaccgatcg cccttcccaa cagttgcgca gcctgaatgg cgaatgagct 7800
   tatcgatgat aagctgtcaa acatgagaat tacaacttat atcgtatggg gctgacttca 7860
   ggtgctacat ttgaagagat aaattgcact gaaatctaga aatattttat ctgattaata 7920
   agatgatctt cttgagatcg ttttggtctg cgcgtaatct cttgctctga aaacgaaaaa 7980
   accgccttgc agggcggttt ttcgaaggtt ctctgagcta ccaactcttt gaaccgaggt 8040
   aactggcttg gaggagcgca gtcaccaaaa cttgtccttt cagtttagcc ttaaccggcg 8100
   catgacttca agactaactc ctctaaatca attaccagtg gctgctgcca gtggtgcttt 8160
   tgcatgtctt tccgggttgg actcaagacg atagttaccg gataaggcgc agcggtcgga 8220
   ctgaacgggg ggttcgtgca tacagtccag cttggagcga actgcctacc cggaactgag 8280
   tgtcaggcgt ggaatgagac aaacgcggcc ataacagcgg aatgacaccg gtaaaccgaa 8340
   aggcaggaac aggagagcgc acgagggagc cgccagggga aacgcctggt atctttatag 8400
   tcctgtcggg tttcgccacc actgatttga gcgtcagatt tcgtgatgct tgtcaggggg 8460
   gcggagccta tggaaaaacg gctttgccgc ggccctctca cttccctgtt aagtatcttc 8520
   ctggcatctt ccaggaaatc tccgccccgt tcgtaagcca tttccgctcg ccgcagtcga 8580
   acgaccgagc gtagcgagtc agtgagcgag gaagcggaat atatcctgta tcacatattc 8640
   tgctgacgca ccggtgcagc cttttttctc ctgccacatg aagcacttca ctgacaccct 8700
   catcagtgcc aacatagtaa gccagtatac actccgctag cgctgatgtc cggcggtgct 8760
   tttgccgtta cgcaccaccc cgtcagtagc tgaacaggag ggacagctga tagaaacaga 8820
   agccactgga gcacctcaaa aacaccatca tacactaaat cagtaagttg gcagcatcac 8880
   ccgacgcact ttgcgccgaa taaatacctg tgacggaaga tcacttcgca gaataaataa 8940
   atcctggtgt ccctgttgat accgggaagc cctgggccaa cttttggcga aaatgagacg 9000
   ttgatcggca cgtaagaggt tccaactttc accataatga aataagatca ctaccgggcg 9060
   tattttttga gttatcgaga ttttcaggag ctaaggaagc taaaatggag aaaaaaatca 9120
   ctggatatac caccgttgat atatcccaat ggcatcgtaa agaacatttt gaggcatttc 9180
   agtcagttgc tcaatgtacc tataaccaga ccgttcagct ggatattacg gcctttttaa 9240
   agaccgtaaa gaaaaataag cacaagtttt atccggcctt tattcacatt cttgcccgcc 9300
   tgatgaatgc tcatccggaa ttt 9323
<210> 25
   <211> 11292
   <212> DNA
   <213> Artificial Sequence
<220>
   <223> Plasmid pSA2M4
<400> 25
   aattcttgaa gacgaaaggg cctcgtgata cgcctatttt tataggttaa tgtcatgata      60
   ataatggttt cttagacgtc aggtggcact tttcggggaa atgtgcgcgg aacccctatt     120
   tgtttatttt tctaaataca ttcaaatatg tatccgctca tgagacaata accctgataa     180
   atgcttcaat aatattgaaa aaggaagagt atgagtattc aacatttccg tgtcgccctt     240
   attccctttt ttgcggcatt ttgccttcct gtttttgctc acccagaaac gctggtgaaa     300
   gtaaaagatg ctgaagatca gttgggtgca cgagtgggtt acatcgaact ggatctcaac     360
   agcggtaaga tccttgagag ttttcgcccc gaagaacgct catgtttgac agcttatcat     420
   cgatatgctt taatgcggta gtgatcaaga gacaggatga ggatcgtttc gcatgattga     480
   acaagatgga ttgcacgcag gttctccggc cgcttgggtg gagaggctat tcggctatga     540
   ctgggcacaa cagacaatcg gctgctctga tgccgccgtg ttccggctgt cagcgcaggg     600
   gcgcccggtt ctttttgtca agaccgacct gtccggtgcc ctgaatgaac tgcaggacga 660
   ggcagcgcgg ctatcgtggc tggccacgac gggcgttcct tgcgcagctg tgctcgacgt 720
   tggcactgaa gcgggaaggg actggctgct attgggcgaa gtgccggggc aggatctcct 780
   gtcatctcac cttgctcctg ccgagaaagt atccatcatg gctgatgcaa tgcggcggct 840
   gcatacgctt gatccggcta cctgcccatt cgaccaccaa gcgaaacatc gcatcgagcg 900
   agcacgtact cggatggaag ccggtcttgt cgatcaggat gatctggacg aagagcatca 960
   ggggctcgcg ccagccgaac tgttcgccag gctcaaggcg cgcatgcccg acggcgagga 1020
   tctcgtcgtg acccatggcg atgcctgctt gccgaatatc atggtggaaa atggccgctt 1080
   ttctggattc atcgactgtg gccggctggg tgtggcggac cgctatcagg acatagcgtt 1140
   ggctacccgt gatattgctg aagagcttgg cggcgaatgg gctgaccgct tcctcgtgct 1200
   ttacggtatc gccgctcccg attcgcagcg catcgccttc tatcgccttc ttgacgagtt 1260
   cttctgagcg ggactctggg gttcggcaca cagcccagct tggagcgaac gacctacacc 1320
   gaactgagat acctacagcg tgagctatga gaaagcgccc aatacgcaaa ccgcctctcc 1380
   ccgcgcgttg gccgattcat taatgcagct ggcacgacag gtttcccgac tggaaagcgg 1440
   gcagtgagcg caacgcaatt aatgtgagtt agctcactca ttaggcaccc caggctttac 1500
   actttatgct tccggctcgt atgttgtgtg gaattgtgag cggataacaa tttcacacag 1560
   gaaacagcta tgaccatgat tacgccaagc ttaggaggtt aaatgatgac taaacctgtt 1620
   attggtttta tcggcctagg ccttatgggc ggcaacatgg tagagaacct acaaactcgt 1680
   ggctaccaag taaatgtaat ggatcttaat aaagacgctg ttgcagctgt tctagctcgt 1740
   ggtaacgcgg cagaagcatc atctggtaaa gaacttgctg aaaaaagcga catcgtaatg 1800
   ctttgcctaa caacgtctga agttgtagaa aaagtagtat acggtgaaac aggtatccta 1860
   gcgggtatgt ctgaaggcaa aacgctaatc gactttggta cttctattcc tgcttctact 1920
   aagaagattg gtgctgatct tgcggctaaa ggcgcaggta tgatcgatgc tcctcttggt 1980
   cgtactccag ctcacgctaa agatggtcta ctaaacatca tggcagctgg cgatatggaa 2040
   acattcaaca aagtgaaacc tgttcttgat gatcaaggtg aaaacgtatt ccacttaggt 2100
   gctctaggtt ctggccacgt tactaagctt gtgaacaact tcatgggtat gactactgtt 2160
   gcaactatgt ctcaagcatt cgcagtggca aaacaagccg gtgttgacgg tcagcaatta 2220
   ttcgacatca tgtcagctgg cccatctaac tctccattca tgcaattctg taagttctac 2280
   gcagtagacg gcgaagaaaa actaggtttc tctgttgcaa acgcaaacaa agaccttggt 2340
   tacttccttg cactatgtga agagttaggt actgagtctc taatcgctca aggtacttct 2400
   acaagcctac aagcagctgt tgatgctggt cttggccaaa atgatgtgcc agttatcttt 2460
   gattacttca tgggtttgaa aaagtaaagg aggttaaatg aaaaatgaag tatcagctgt 2520
   attgcttaac actgcagaag tgaaagcgtt acagaaagaa gtcggccgtg acagtttgat 2580
   gggtaacact attgcgacca ttaaaaaaga tgttgatgcc tttatgacac tgggctacga 2640
   cgtacctggg catggtgaag cgggtggtta tgaacacaat cgccataagc aaaactataa 2700
   ctttatgaac cttgctggtc gtctttatct agtaactaaa gaagaaaaat acgctcgttt 2760
   tgtagctgaa ctattaacta tctatgctga caaatacttg caattcgatt tccatattca 2820
   aaaaaacacc aacccaacgg gtcgtttatt ccaccagatc ttaaatgaac acatgtggct 2880
   aatgtattcg agcttggcgt attcttgtgt gaaaggattt atgtcgctta ctgagcgtaa 2940
   tcatgtagaa agcaatttat tcctacctat gcttgatatg tttaccgtga aatatggcca 3000
   tgattttgac cgtattcata accacggtat gtgggccgtt gccgcggtag gtatttgtgg 3060
   tttagctatt gataaacgtg agtatctaga aatggccgta tttggtctag ataactttaa 3120
   cgctggcaaa aaagaaatgg gtgagtgtgg tttcttagcg caaatctcta aattatttgc 3180
   cccttctggt tactacatgg aaggtcctta ctaccaccgc tttgcgattc gtcctctatg 3240
   tgtgtttgca gaagttttac accgtcacat gcctgagcta gatatttata actttaaaga 3300
   caaagtgatt ggtaatacgg ttcaaacact tttagctacc gcttatccaa atggtgagtt 3360
   tccagcgtta aatgattcat ctcatacaat gggcattacc gatatgggcg tacaagtcgc 3420
   ggtgagctta tacagcaagc actacggttt agatgataac ttgcttggta tggcgaaaat 3480
   tcaaaaccaa gtatggatgc atgcgtgtgg tctagagtta tctcgtgcgt ttgaaaaagc 3540
   acaaaatgag cgtcctgatg atattggttt accatttttc ccaagtgtgg agttaaatga 3600
   aggcccagcg ggtcataacg gtgcacaagg ttttatccgt atgcaagatg aaaccaaaga 3660
   tgtcttccaa ttagtgatga actatggcca acatggtatg gatcatggtc actttgatac 3720
   tttaggtatt agttatttta accgtggtca agaagtgtta cgtgaatatg gtttctgccg 3780
   ttgggttaat gttgaaccta agttcggtgg ccgctacctt gatgagaata aatcttatgc 3840
   ccgtcaaacc attgcacata acgctgtgac gattgatgag aaatgccaaa ataacttctc 3900
   aattgatgaa gcagatacgg ttcatggctt gccgcatttc tttaaagtag aagatgcaaa 3960
   agtgaatggt atgagcgctt ttgccaatga tcattaccaa gatttcgaca tgcagcgtag 4020
   tgtgttcatg ctaaaactgg atgaactaga agcaccattg ctgctcgatt tataccgctt 4080
   aacggcagaa gataacaaaa cacatcagtt tgactactca caccaatatc aaggtcaaat 4140
   tgttcgtact aattttgatt atcaagccca ccaagagctt gacacactag gtgacaactt 4200
   cggttatcaa catctatgga atgtggcaag tggagaagtt aaaggcagtg ctttagtcag 4260
   ttggctacaa aataatactt actacacttg gttaggtacg agttcgaaca atgatggtga 4320
   agtcattttc acacgtactg gcgctaatga tccaagtttt aacttacgta gtgagcctgc 4380
   ctttatctta cgtgctaaag gccaatcaag cttatttgca tcagtcttag aaacgcatgg 4440
   ttactttaac gaagagtttg aacaatcagt gaatgctcgt ggtcaagttc aagacgtacg 4500
   tgtgattgcc cataatgagg ttggatctgt gattcaaatt gaaacgcaac aatcggtggt 4560
   tacttttatg gtaagcaatc aattagccgc gacagatacc actgtgcatc aaatttcagt 4620
   cgatgacaaa acttatcaat ggaatggctt ctattcatta gaagtacaag tgaaataaag 4680
   gaggttaaat gcctttaaaa accgatgagc taagaaccca agcgttaggg cctatgccta 4740
   cgccaatgga attaatgcaa gctcacccac tgcctgatga tgtggcagaa catattgaaa 4800
   actcgcgtcg ccaaatagaa aacatcctgt caggtaaaga ctctcgccta ctggttattg 4860
   tagggccgtg ttctgtacat gataccgacg cggctctaga ctatgcgcaa cgccttaatg 4920
   cactacaaga gcaacacaaa gatcagctgt ttattgtgat gcgtacttac tttgagaaac 4980
   ctagaaccgt aattggttgg aaaggattaa ttaccgatcc taatcttgac ggtagctacg 5040
   ctttagaaga cggtttaaac aaagctcgta aattactgct cgacatcaat aaactgggtt 5100
   tagccactgc cactgaattt ttagacatgg tgacaggtca atatattgct gaccttattt 5160
   catggggggc aattggcgca cgtaccactg agtcccaaat tcaccgtgaa atggcttcgg 5220
   cgctttcttg ccctgttggg tttaagaatg gcaccaatgg taatgtgaag atttcaaccg 5280
   atgccattcg tgccgcgcaa gcctcccatt acttctgttc tccagataag aatggtcgaa 5340
   tgacggttta tcgcacatcc ggtaacccat acggtcatac gattttacgt ggcggtgata 5400
   caggccctaa ctttgatgca gaatcaattc aatctgcttg tgaacaatta agtgaagtag 5460
   gcttaactcc ccgtttagtc gtggatttca gtcacgctaa ctgccaaaaa gagcatcgta 5520
   agcaattaga tgtagcgcaa gatatttgca accaaatcaa caatggttct caatcaattg 5580
   caggtattat ggcggaaagc tttatcgagg aaggtaatca accaatgacg gacttaaatg 5640
   ccctcaccta tggtaaatcg attactgatc catgcctaag ttggaaagat acggtaacca 5700
   tgctcgacat gcttgccaat accattaaga atcaatcgga gaagaactga tcatgccttc 5760
   atttgacata atttctgaag tggacagcgt tgaagtaaga aatgccgcag aaaacgccaa 5820
   ccgtgaacta gatacacgtt tcgattttcg taacgtaaac gctaattttg agtttaaaaa 5880
   cgatattgta aaactgacag cagaaaatga ataccagctt ggacaaatga tgacgatgct 5940
   tcgtggtcaa cttgctaaac gtggtgttga tgccaatgcg gtagatttac aaaaagccga 6000
   tcaaactgga aaaacacata ctcaagaggc cagtttcaaa caaggtattg atgttgccat 6060
   tgccaaaaaa gtggtaaaac taatcaaaga ctcaaagatc aaagttcaag cttcgattca 6120
   gggtgataaa gttcgtgtaa cgggtaaaaa gcgtgatgat ttgcagcaag ttatggcatt 6180
   agttaaaggt gcggaaatgg atcaaccctt ccaattcaac aactttaaag actaaaggct 6240
   ttacacttta tgcttccggc tcgtatgttg tgtggaattg tgagcggata acaatttcac 6300
   acaggaaaca gctatgacca tgattacgcc aagcttagga ggttaaatgt taaaaaaatt 6360
   cctctgcatg tcggttatcc taggctgctc agcaaacgca atggcgcaag ccgctccagt 6420
   accagctgac aagtttgaca tgatgaactg gaaaattacc gttccagaag atagagataa 6480
   aaacggtaag attgatgaaa ttgaaggtgt agcgatgttc agctactcaa acccggacta 6540
   tttcttttta gatgacgatg gcaacatggt tttccaagtt caaaataaag ctatcacaac 6600
   atctggctca tcaaacgctc gttcagaact acgccaaatg ctacgtggca ccgatcttac 6660
   tatcgatgtc aaagctccag caaataactt cgctttagca tctcatccca aagcaaaaga 6720
   atatggtgct gttggtggta aattagaagc aaccttaaaa gtaaaccatg ttgctactca 6780
   tgctaatcaa ccaaataaac ttcctgccta ttcagtggtt gttggacaaa ttcatgccaa 6840
   caaagatcct aaactaatgg aagcaaacac tggctttggc tatggtaatg aacctttaaa 6900
   aatcttctac aaaaaatggc cagatcatga taaaggttct gtattcttta actacgagcg 6960
   caacttagca aaagatgatc caaaccgtat cgatattgcc tacccagttt ggggcaatac 7020
   ttgggaaaac aaagatgatc ctaaagatgc tggtgtagca cttggggaag agtttagtta 7080
   tgtagtggat gttgaaggta acatcatgca cttaactttc acttcgaaaa accacaagac 7140
   cgttgaatat aaaatcgacc tatctaaaaa cctgaagcct gacggcacag ttgatgagaa 7200
   agataatcca cgtggttacg cagaagattc tttctacttt aaagccggtg cttatggcca 7260
   atgtagtgtg aaagatgatg aaggtttctg gtctccaggt tgtgcaggta caggtgactt 7320
   cgcaactgac gtgaagaatg gcgactacaa cagtgtgaca ttctctaagt tagaattaac 7380
   atcgcataaa aaatagagga ggttaaatgt ctaaattaga tcaacaactt gctgacttaa 7440
   aagtcattcc agtgattgca ttgaacaaag ttgaacatgc ccttcctctg gcgaaagcat 7500
   taatggaaaa cggcatgcct tgcgcagaaa ttacttttcg tactgaagcc gcagtcggcg 7560
   caattaaagc catgcgtgaa gcctaccctg aaatgctaat tggtgcaggt acgattttaa 7620
   cacctgaaca agttgaccaa gcaatagatg ctggtgttga tttcattgta agcccaggct 7680
   taaacccaac aacggttaaa tactgtcaac aaaagaacat gccaatcgtt ccgggtgtta 7740
   acaacccaag cctagttgag caagcaatgg aactgggtct taaaaccgtt aagttcttcc 7800
   cagctgagcc atctggtggc gttggtatgt tgaaagcatt aactgcggtt tacccagtga 7860
   aatttatgcc aactggcggt gttagtttat caaacattga tgactaccta tctattccag 7920
   ctgtactggc atgtggcggt acttggatgg ttcctacatc tcttattgat aacgagcaat 7980
   gggatgaact aggtgcatta gttaaagatg ctgtagccaa agttaactag aggaggttaa 8040
   atgacaacat tcaatattgc agtcattggc gaatgtatga ttgaactgca aaaatcaggc 8100
   gaattcctaa cgcaaaaatt tggtggtgat acgcttaata ctgcgttgta tcttgctcgc 8160
   ctaaccaaac acactgcgat taaaacaagc tacattaccg cgcttggtcg tgaaggcttt 8220
   agccaaaaca tgattgatag ctggtcagaa gaagcgatta atacgcaaca cattcttcgt 8280
   tttgaaaaca agcaacccgg tatgtactac atcgaaaccg atgacactgg tgagcgtagt 8340
   ttctattact ggcgtaatga tgccgcggca aaatacatgt ttgaccaagc ggagagcgct 8400
   caactgatcg aaacattatc aaccttcgat gcagtctatt taagtgggat cacactcgct 8460
   attttgactg agcaaggtcg tgagcagtta ttcacattac ttagcaaatt gaaagctaat 8520
   ggcagcaaag ttatttttga taataacttc cgtccaaaat tgtgggcatc ggttgaagaa 8580
   gctcaacaag cgtatcttaa aacactatcg ttctgtgata ttgccttatt aacattcgat 8640
   gatgaacaag atctctttgg tgacgacaat atcgaacaat gtattgctcg tactcaaggt 8700
   caaggcgtta aagagattgc gattaaacgt ggcagcaaag attgtgttgt tgtagaagaa 8760
   gatagcattc aatacgttgc gacaaaaccg gttgataacg tggtggacac gactgccgca 8820
   ggtgactcat tcagtgctgg ctatttagct aagcgtttaa atggttacag cgcaaaagac 8880
   tctgccctat caggtcataa agtagcaggc accgttatcc aacatcaagg cgcgattatt 8940
   ccaagtgagg caatgcctga tatcggctta taagtcgact ctagaggatc cccgggtacc 9000
   gagctcgaat tcactggccg tcgttttaca acgtcgtgac tgggaaaacc ctggcgttac 9060
   ccaacttaat cgccttgcag cacatccccc tttcgccagc tggcgtaata gcgaagaggc 9120
   ccgcaccgat cgcccttccc aacagttgcg cagcctgaat ggcgaatggc gctatttctt 9180
   ccagaattgc catgattttt tccccacggg aggcgtcact ggctcccgtg ttgtcggcag 9240
   ctttgattcg ataagcagca tcgcctgttt caggctgtct atgtgtgact gttgagctgt 9300
   aacaagttgt ctcaggtgtt caatttcatg ttctagttgc tttgttttac tggtttcacc 9360
   tgttctatta ggtgttacat gctgttcatc tgttacattg tcgatctgtt catggtgaac 9420
   agctttgaat gcaccaaaaa ctcgtaaaag ctctgatgta tctatctttt ttacaccgtt 9480
   ttcatctgtg catatggaca gttttccctt tgatatgtaa cggtgaacag ttgttctact 9540
   tttgtttgtt agtcttgatg cttcactgat agatacaaga gccataagaa cctcagatcc 9600
   ttccgtattt agccagtatg ttctctagtg tggttcgttg tttttgcgtg agccatgaga 9660
   acgaaccatt gagatcatgc ttactttgca tgtcactcaa aaattttgcc tcaaaactgg 9720
   tgagctgaat ttttgcagtt aaagcatcgt gtagtgtttt tcttagtccg ttacgtaggt 9780
   aggaatctga tgtaatggtt gttggtattt tgtcaccatt catttttatc tggttgttct 9840
   caagttcggt tacgagatcc atttgtctat ctagttcaac ttggaaaatc aacgtatcag 9900
   tcgggcggcc tcgcttatca accaccaatt tcatattgct gtaagtgttt aaatctttac 9960
   ttattggttt caaaacccat tggttaagcc ttttaaactc atggtagtta ttttcaagca 10020
   ttaacatgaa cttaaattca tcaaggctaa tctctatatt tgccttgtga gttttctttt 10080
   gtgttagttc ttttaataac cactcataaa tcctcataga gtatttgttt tcaaaagact   10140
   taacatgttc cagattatat tttatgaatt tttttaactg gaaaagataa ggcaatatct   10200
   cttcactaaa aactaattct aatttttcgc ttgagaactt ggcatagttt gtccactgga   10260
   aaatctcaaa gcctttaacc aaaggattcc tgatttccac agttctcgtc atcagctctc   10320
   tggttgcttt agctaataca ccataagcat tttccctact gatgttcatc atctgagcgt   10380
   attggttata agtgaacgat accgtccgtt ctttccttgt agggttttca atcgtggggt   10440
   tgagtagtgc cacacagcat aaaattagct tggtttcatg ctccgttaag tcatagcgac   10500
   taatcgctag ttcatttgct ttgaaaacaa ctaattcaga catacatctc aattggtcta   10560
   ggtgatttta atcactatac caattgagat gggctagtca atgataatta ctagtccttt   10620
   tcctttgagt tgtgggtatc tgtaaattct gctagacctt tgctggaaaa cttgtaaatt   10680
   ctgctagacc ctctgtaaat tccgctagac ctttgtgtgt ttttttgttt tatattcaag   10740
   tggttataat ttatagaata aagaaagaat aaaaaaagat aaaaagaata gatcccagcc   10800
   ctgtgtataa ctcactactt tagtcagttc cgcagtatta caaaaggatg tcgcaaacgc   10860
   tgtttgctcc tctacaaaac agaccttaaa accctaaagg cttaagtagc accctcgcaa   10920
   gctcgggcaa atcgctgaat attccttttg tctccgacca tcaggcacct gagtcgctgt   10980
   ctttttcgtg acattcagtt cgctgcgctc acggctctgg cagtgaatgg gggtaaatgg   11040
   cactacaggc gccttttatg gattcatgca aggaaactac ccataataca agaaaagccc   11100
   gtcacgggct tctcagggcg ttttatggcg ggtctgctat gtggtgctat ctgacttttt   11160
   gctgttctgc agttcctgcc ctctgatttt ccagtctgac cacttcggat tatcccgtga   11220
   caggtcattc agactggcta atgcacccag taaggcagcg gtatcatcaa caggcttacc   11280
   cgtcttactg tc                                                        11292
<210> 26
   <211> 65
   <212> DNA
   <213> Artificial Sequence
<220>
   <223> primer 1 for kdgN
<400> 26
   tgattacgcc aagcttagga ggttaaatgt tcaaattaaa taaagtttta cttgccacta      60
   ctgcc                                                                  65
<210> 27
   <211> 38
   <212> DNA
   <213> Artificial Sequence
<220>
   <223> primer 2 for kdgN
<400> 27
   ttagaagcta taagtaagac ctacacggct acgtagct 38
<210> 28
   <211> 65
   <212> DNA
   <213> Artificial Sequence
<220>
   <223> primer 1 for alyB
<400> 28
   tacttatagc ttctaaagga ggttaaatga aacatatttt tctaaaaagc ttgctagctt 60
   catct 65
<210> 29
   <211> 39
   <212> DNA
   <213> Artificial Sequence
<220>
   <223> primer 2 for alyB
<400> 29
   ttatttacct gtgtatgtac cgtgcgattt ttctagttt 39
<210> 30
   <211> 65
   <212> DNA
   <213> Artificial Sequence
<220>
   <223> primer 1 for toaA
<400> 30
   atacacaggt aaataaagga ggttaaatga ctgtcgatac tattgttgtt gctgcctatt 60
   ttctc 65
<210> 31
   <211> 54
   <212> DNA
   <213> Artificial Sequence
<220>
   <223> primer 2 for toaA
<400> 31
   atcctctaga gtcgacttac gattctgaaa ccgtttgatg ttgtactttt gagg 54
<210> 32
   <211> 65
   <212> DNA
   <213> Artificial Sequence
<220>
   <223> primer 1 for oalC
<400> 32
   tgattacgcc aagcttagga ggttaaatga attaccaacc attattaatg aattttgaag 60
   aagcg 65
<210> 33
   <211> 54
   <212> DNA
   <213> Artificial Sequence
<220>
   <223> primer 2 for oalC
<400> 33
   atcctctaga gtcgacttat aactgagccg ttgctcccgt ccattggtat tgta 54
<210> 34
   <211> 37
   <212> DNA
   <213> Artificial Sequence
<220>
   <223> primer 1 for pSA2S2 construction
<400> 34
   gtcgactcta gaggatcccc gggtaccgag ctcgaat 37
<210> 35
   <211> 36
   <212> DNA
   <213> Artificial Sequence
<220>
   <223> primer 2 for pSA2S2 construction
<400> 35
   ttacgattct gaaaccgttt gatgttgtac ttttga 36
<210> 36
   <211> 54
   <212> DNA
   <213> Artificial Sequence
<220>
   <223> primer 3 for pSA2S2 construction
<400> 36
   ggtttcagaa tcgtaaaggc tttacacttt atgcttccgg ctcgtatgtt gtgt 54
<210> 37
   <211> 54
   <212> DNA
   <213> Artificial Sequence
<220>
   <223> primer 4 fpr pSA2S2 construction
<400> 37
   atcctctaga gtcgacttat aactgagccg ttgctcccgt ccattggtat tgta 54
<210> 38
   <211> 65
   <212> DNA
   <213> Artificial Sequence
<220>
   <223> primer 1 for dehR
<400> 38
   tgattacgcc aagcttagga ggttaaatga tgactaaacc tgttattggt tttatcggcc 60
   taggc 65
<210> 39
   <211> 39
   <212> DNA
   <213> Artificial Sequence
<220>
   <223> primer 2 for dehR
<400> 39
   ttactttttc aaacccatga agtaatcaaa gataactgg 39
<210> 40
   <211> 65
   <212> DNA
   <213> Artificial Sequence
<220>
   <223> primer 1 for oalB
<400> 40
   gggtttgaaa aagtaaagga ggttaaatga aaaatgaagt atcagctgta ttgcttaaca 60
   ctgca 65
<210> 41
   <211> 39
   <212> DNA
   <213> Artificial Sequence
<220>
   <223> primer 2 for oalB
<400> 41
   ttatttcact tgtacttcta atgaatagaa gccattcca 39
<210> 42
   <211> 65
   <212> DNA
   <213> Artificial Sequence
<220>
   <223> primer 1 for toaB toaC
<400> 42
   agtacaagtg aaataaagga ggttaaatgc ctttaaaaac cgatgagcta agaacccaag 60
   cgtta 65
<210> 43
   <211> 54
   <212> DNA
   <213> Artificial Sequence
<220>
   <223> primer 2for toaB toaC
<400> 43
   atcctctaga gtcgacttag tctttaaagt tgttgaattg gaagggttga tcca 54
<210> 44
   <211> 65
   <212> DNA
   <213> Artificial Sequence
<220>
   <223> primer1 for alyD
<400> 44
   tgattacgcc aagcttagga ggttaaatgt taaaaaaatt cctctgcatg tcggttatcc 60
   taggc 65
<210> 45
   <211> 39
   <212> DNA
   <213> Artificial Sequence
<220>
   <223> primer 2 for alyD
<400> 45
   ctatttttta tgcgatgtta attctaactt agagaatgt 39
<210> 46
   <211> 65
   <212> DNA
   <213> Artificial Sequence
<220>
   <223> primer 1 for eda
<400> 46
   atcgcataaa aaatagagga ggttaaatgt ctaaattaga tcaacaactt gctgacttaa 60
   aagtc 65
<210> 47
   <211> 39
   <212> DNA
   <213> Artificial Sequence
<220>
   <223> primer 2 for eda
<400> 47
   ctagttaact ttggctacag catctttaac taatgcacc 39
<210> 48
   <211> 65
   <212> DNA
   <213> Artificial Sequence
<220>
   <223> primer 1 for kdgK
<400> 48
   agccaaagtt aactagagga ggttaaatga caacattcaa tattgcagtc attggcgaat 60
   gtatg 65
<210> 49
   <211> 54
   <212> DNA
   <213> Artificial Sequence
<220>
   <223> primer 2 for kdgK
<400> 49
   atcctctaga gtcgacttat aagccgatat caggcattgc ctcacttgga ataa 54
<210> 50
   <211> 35
   <212> DNA
   <213> Artificial Sequence
<220>
   <223> primer1 for pSA2M4
<400> 50
   gtcgactcta gaggatcccc gggtaccgag ctcga 35
<210> 51
   <211> 36
   <212> DNA
   <213> Artificial Sequence
<220>
   <223> primer 2 pSA2M4
<400> 51
   ttagtcttta aagttgttga attggaaggg ttgatc 36
<210> 52
   <211> 54
   <212> DNA
   <213> Artificial Sequence
<220>
   <223> primer 3 for pSA2M4
<400> 52
   caactttaaa gactaaaggc tttacacttt atgcttccgg ctcgtatgtt gtgt 54
<210> 53
   <211> 54
   <212> DNA
   <213> Artificial Sequence
<220>
   <223> primer 4 for pSA2M4
<400> 53
   atcctctaga gtcgacttat aagccgatat caggcattgc ctcacttgga ataa 54
<210> 54
   <211> 1185
   <212> DNA
   <213> Escherichia coli
<220>
   <221> CDS
   <222> (1)..(1185)
<400> 54
   atg gaa cag acc tgg cgc tgg tac ggc cca aac gat ccg gtt tct tta        48
   Met Glu Gln Thr Trp Arg Trp Tyr Gly Pro Asn Asp Pro Val Ser Leu
   1               5                   10                  15
   gct gat gtc cgt cag gcg ggc gca act ggc gtg gtt acc gcg ctg cac        96
   Ala Asp Val Arg Gln Ala Gly Ala Thr Gly Val Val Thr Ala Leu His
               20                  25                  30
   cat atc ccg aac ggc gaa gta tgg tcc gtt gaa gag atc ctc aaa cgc       144
   His Ile Pro Asn Gly Glu Val Trp Ser Val Glu Glu Ile Leu Lys Arg
           35                  40                  45
   aag gcg atc att gaa gac gca ggc ctg gtg tgg tct gtc gta gaa agc       192
   Lys Ala Ile Ile Glu Asp Ala Gly Leu Val Trp Ser Val Val Glu Ser
       50                  55                  60
   gtg cca att cac gaa gat atc aaa acc cac act ggc aac tat gag cag       240
   Val Pro Ile His Glu Asp Ile Lys Thr His Thr Gly Asn Tyr Glu Gln
   65                  70                  75                  80
   tgg atc gct aac tat cag cag act ctg cgc aac ctg gcg cag tgt ggc       288
   Trp Ile Ala Asn Tyr Gln Gln Thr Leu Arg Asn Leu Ala Gln Cys Gly
                   85                  90                  95
   att cgc acc gtg tgc tac aac ttc atg ccg gtg ctc gac tgg acc cgt       336
   Ile Arg Thr Val Cys Tyr Asn Phe Met Pro Val Leu Asp Trp Thr Arg
               100                 105                 110
   act gac ctc gaa tac gtg ctg cca gac ggc tcc aaa gct ctg cgc ttc       384
   Thr Asp Leu Glu Tyr Val Leu Pro Asp Gly Ser Lys Ala Leu Arg Phe
           115                 120                 125
   gac cag atc gaa ttc gct gca ttc gaa atg cat atc ctg aaa cgc cca        432
   Asp Gln Ile Glu Phe Ala Ala Phe Glu Met His Ile Leu Lys Arg Pro
       130                 135                 140
   ggc gcg gaa gcg gat tac acc gaa gaa gaa att gct cag gca gct gaa        480
   Gly Ala Glu Ala Asp Tyr Thr Glu Glu Glu Ile Ala Gln Ala Ala Glu
   145                 150                 155                 160
   cgc ttc gcc act atg agc gat gaa gac aaa gcg cgt ctg acc cgt aac        528
   Arg Phe Ala Thr Met Ser Asp Glu Asp Lys Ala Arg Leu Thr Arg Asn
                   165                 170                 175
   atc att gct ggt ctt ccg ggc gcg gaa gaa ggc tac acc ctc gac cag        576
   Ile Ile Ala Gly Leu Pro Gly Ala Glu Glu Gly Tyr Thr Leu Asp Gln
               180                 185                 190
   ttc cgt aaa cac ctg gag ctg tac aaa gat atc gac aaa gcg aag ctg        624
   Phe Arg Lys His Leu Glu Leu Tyr Lys Asp Ile Asp Lys Ala Lys Leu
           195                 200                 205
   cgc gaa aac ttt gcc gtc ttc ctg aaa gcg att att cca gtt gct gaa        672
   Arg Glu Asn Phe Ala Val Phe Leu Lys Ala Ile Ile Pro Val Ala Glu
       210                 215                 220
   gaa gtc ggc gtg cgt atg gct gtt cac ccg gac gat ccg ccg cgc ccg        720
   Glu Val Gly Val Arg Met Ala Val His Pro Asp Asp Pro Pro Arg Pro
   225                 230                 235                 240
   atc ctc ggc ctg ccg cgc att gtt tcc acc att gaa gat atg cag tgg        768
   Ile Leu Gly Leu Pro Arg Ile Val Ser Thr Ile Glu Asp Met Gln Trp
                   245                 250                 255
   atg gtt gat acc gta aac agc atg gca aac ggt ttt acc atg tgc acc        816
   Met Val Asp Thr Val Asn Ser Met Ala Asn Gly Phe Thr Met Cys Thr
               260                 265                 270
   ggt tcc tac ggc gtg cgt gct gac aac gat ctg gtt gat atg atc aag        864
   Gly Ser Tyr Gly Val Arg Ala Asp Asn Asp Leu Val Asp Met Ile Lys
           275                 280                 285
   cag ttc ggt ccg cgt att tac ttc acc cat ctg cgc tcc acc atg cgt        912
   Gln Phe Gly Pro Arg Ile Tyr Phe Thr His Leu Arg Ser Thr Met Arg
       290                 295                 300
   gaa gat aac ccg aaa acc ttc cac gaa gcg gcg cac ctg aac ggt gac        960
   Glu Asp Asn Pro Lys Thr Phe His Glu Ala Ala His Leu Asn Gly Asp
   305                 310                 315                 320
   gtt gat atg tac gaa gtg gtg aaa gcg att gtt gaa gaa gaa cac cgt       1008
   Val Asp Met Tyr Glu Val Val Lys Ala Ile Val Glu Glu Glu His Arg
                   325                 330                 335
   cgt aaa gcg gaa ggc aaa gaa gac ctg atc ccg atg cgt ccg gac cac       1056
   Arg Lys Ala Glu Gly Lys Glu Asp Leu Ile Pro Met Arg Pro Asp His
               340                 345                 350
   ggt cat cag atg ctg gac gac ctg aag aag aaa acc aac cca ggt tac       1104
   Gly His Gln Met Leu Asp Asp Leu Lys Lys Lys Thr Asn Pro Gly Tyr
           355                 360                 365
   tcc gca att ggt cgt ctg aaa ggc ctg gcc gaa gtt cgc ggt gtc gaa       1152
   Ser Ala Ile Gly Arg Leu Lys Gly Leu Ala Glu Val Arg Gly Val Glu
       370                 375                 380
   ctg gcg atc cag cgc gct ttc ttt agc cgt taa                          1185
   Leu Ala Ile Gln Arg Ala Phe Phe Ser Arg
   385                 390
<210> 55
   <211> 394
   <212> PRT
   <213> Escherichia coli
<400> 55
   Met Glu Gln Thr Trp Arg Trp Tyr Gly Pro Asn Asp Pro Val Ser Leu
   1               5                   10                  15
   Ala Asp Val Arg Gln Ala Gly Ala Thr Gly Val Val Thr Ala Leu His
               20                  25                  30
   His Ile Pro Asn Gly Glu Val Trp Ser Val Glu Glu Ile Leu Lys Arg
           35                  40                  45
   Lys Ala Ile Ile Glu Asp Ala Gly Leu Val Trp Ser Val Val Glu Ser
       50                  55                  60
   Val Pro Ile His Glu Asp Ile Lys Thr His Thr Gly Asn Tyr Glu Gln
   65                  70                  75                  80
   Trp Ile Ala Asn Tyr Gln Gln Thr Leu Arg Asn Leu Ala Gln Cys Gly
                   85                  90                  95
   Ile Arg Thr Val Cys Tyr Asn Phe Met Pro Val Leu Asp Trp Thr Arg
               100                 105                 110
   Thr Asp Leu Glu Tyr Val Leu Pro Asp Gly Ser Lys Ala Leu Arg Phe
           115                 120                 125
   Asp Gln Ile Glu Phe Ala Ala Phe Glu Met His Ile Leu Lys Arg Pro
       130                 135                 140
   Gly Ala Glu Ala Asp Tyr Thr Glu Glu Glu Ile Ala Gln Ala Ala Glu
   145                 150                 155                 160
   Arg Phe Ala Thr Met Ser Asp Glu Asp Lys Ala Arg Leu Thr Arg Asn
                   165                 170                 175
   Ile Ile Ala Gly Leu Pro Gly Ala Glu Glu Gly Tyr Thr Leu Asp Gln
               180                 185                 190
   Phe Arg Lys His Leu Glu Leu Tyr Lys Asp Ile Asp Lys Ala Lys Leu
           195                 200                 205
   Arg Glu Asn Phe Ala Val Phe Leu Lys Ala Ile Ile Pro Val Ala Glu
       210                 215                 220
   Glu Val Gly Val Arg Met Ala Val His Pro Asp Asp Pro Pro Arg Pro
   225                 230                 235                 240
   Ile Leu Gly Leu Pro Arg Ile Val Ser Thr Ile Glu Asp Met Gln Trp
                   245                 250                 255
   Met Val Asp Thr Val Asn Ser Met Ala Asn Gly Phe Thr Met Cys Thr
               260                 265                 270
   Gly Ser Tyr Gly Val Arg Ala Asp Asn Asp Leu Val Asp Met Ile Lys
           275                 280                 285
   Gln Phe Gly Pro Arg Ile Tyr Phe Thr His Leu Arg Ser Thr Met Arg
       290                 295                 300
   Glu Asp Asn Pro Lys Thr Phe His Glu Ala Ala His Leu Asn Gly Asp
   305                 310                 315                 320
   Val Asp Met Tyr Glu Val Val Lys Ala Ile Val Glu Glu Glu His Arg
                   325                 330                 335
   Arg Lys Ala Glu Gly Lys Glu Asp Leu Ile Pro Met Arg Pro Asp His
               340                 345                 350
   Gly His Gln Met Leu Asp Asp Leu Lys Lys Lys Thr Asn Pro Gly Tyr
           355                 360                 365
   Ser Ala Ile Gly Arg Leu Lys Gly Leu Ala Glu Val Arg Gly Val Glu
       370                 375                 380
   Leu Ala Ile Gln Arg Ala Phe Phe Ser Arg
   385                 390
<210> 56
   <211> 930
   <212> DNA
   <213> Escherichia coli
<220>
   <221> CDS
   <222> (1)..(930)
<400> 56
   atg tcc aaa aag att gcc gtg att ggc gaa tgc atg att gag ctt tcc        48
   Met Ser Lys Lys Ile Ala Val Ile Gly Glu Cys Met Ile Glu Leu Ser
   1               5                   10                  15
   gag aaa ggc gcg gac gtt aag cgc ggt ttc ggc ggc gat acc ctg aac        96
   Glu Lys Gly Ala Asp Val Lys Arg Gly Phe Gly Gly Asp Thr Leu Asn
               20                  25                  30
   act tcc gtc tat atc gcc cgt cag gtc gat cct gcg gca tta acc gtt       144
   Thr Ser Val Tyr Ile Ala Arg Gln Val Asp Pro Ala Ala Leu Thr Val
           35                  40                  45
   cat tac gta acg gcg ctg gga acg gac agt ttt agc cag cag atg ctg       192
   His Tyr Val Thr Ala Leu Gly Thr Asp Ser Phe Ser Gln Gln Met Leu
       50                  55                  60
   gac gcc tgg cac ggc gag aac gtt gat act tcc ctg acc caa cgg atg       240
   Asp Ala Trp His Gly Glu Asn Val Asp Thr Ser Leu Thr Gln Arg Met
   65                  70                  75                  80
   gaa aac cgt ctg ccg ggc ctt tac tac att gaa acc gac agc acc ggc       288
   Glu Asn Arg Leu Pro Gly Leu Tyr Tyr Ile Glu Thr Asp Ser Thr Gly
                   85                  90                  95
   gag cgt acg ttc tac tac tgg cgg aac gaa gcc gcc gcc aaa ttc tgg       336
   Glu Arg Thr Phe Tyr Tyr Trp Arg Asn Glu Ala Ala Ala Lys Phe Trp
               100                 105                 110
   ctg gag agt gag cag tct gcg gcg att tgc gaa gag ctg gcg aat ttc       384
   Leu Glu Ser Glu Gln Ser Ala Ala Ile Cys Glu Glu Leu Ala Asn Phe
           115                 120                 125
   gat tat ctc tac ctg agc ggg att agc ctg gcg atc tta agc ccg acc       432
   Asp Tyr Leu Tyr Leu Ser Gly Ile Ser Leu Ala Ile Leu Ser Pro Thr
       130                 135                 140
   agc cgc gaa aag ctg ctt tcc ctg ctg cgc gaa tgc cgc gcc aac ggc       480
   Ser Arg Glu Lys Leu Leu Ser Leu Leu Arg Glu Cys Arg Ala Asn Gly
   145                 150                 155                 160
   gga aaa gtg att ttc gac aat aac tat cgt ccg cgc ctg tgg gcc agc       528
   Gly Lys Val Ile Phe Asp Asn Asn Tyr Arg Pro Arg Leu Trp Ala Ser
                   165                 170                 175
   aaa gaa gag aca cag cag gtg tac caa caa atg ctg gaa tgc acg gat       576
   Lys Glu Glu Thr Gln Gln Val Tyr Gln Gln Met Leu Glu Cys Thr Asp
               180                 185                 190
   atc gcc ttc ctg acg ctg gac gac gaa gac gcg ctg tgg ggt caa cag       624
   Ile Ala Phe Leu Thr Leu Asp Asp Glu Asp Ala Leu Trp Gly Gln Gln
           195                 200                 205
   ccg gtg gaa gac gtc att gcg cgc acc cat aac gcg ggc gtg aaa gaa       672
   Pro Val Glu Asp Val Ile Ala Arg Thr His Asn Ala Gly Val Lys Glu
       210                 215                 220
   gtg gtg gtg aaa cgc ggg gcg gat tct tgc ctg gtg tcc att gct ggc       720
   Val Val Val Lys Arg Gly Ala Asp Ser Cys Leu Val Ser Ile Ala Gly
   225                 230                 235                 240
   gaa ggg tta gtg gat gtt ccg gcg gtg aaa ctg ccg aaa gaa aaa gtg       768
   Glu Gly Leu Val Asp Val Pro Ala Val Lys Leu Pro Lys Glu Lys Val
                   245                 250                 255
   atc gat acc acc gca gct ggc gac tct ttc agt gcc ggt tat ctg gcg        816
   Ile Asp Thr Thr Ala Ala Gly Asp Ser Phe Ser Ala Gly Tyr Leu Ala
               260                 265                 270
   gta cgt ctg aca ggc ggc agc gcg gaa gac gcg gcg aaa cgt ggg cac        864
   Val Arg Leu Thr Gly Gly Ser Ala Glu Asp Ala Ala Lys Arg Gly His
           275                 280                 285
   ctg acc gca agt acc gtt att cag tat cgc ggc gcg att atc ccg cgt        912
   Leu Thr Ala Ser Thr Val Ile Gln Tyr Arg Gly Ala Ile Ile Pro Arg
       290                 295                 300
   gag gcg atg cca gcg taa                                                930
   Glu Ala Met Pro Ala
   305
<210> 57
   <211> 309
   <212> PRT
   <213> Escherichia coli
<400> 57
   Met Ser Lys Lys Ile Ala Val Ile Gly Glu Cys Met Ile Glu Leu Ser
   1               5                   10                  15
   Glu Lys Gly Ala Asp Val Lys Arg Gly Phe Gly Gly Asp Thr Leu Asn
               20                  25                  30
   Thr Ser Val Tyr Ile Ala Arg Gln Val Asp Pro Ala Ala Leu Thr Val
           35                  40                  45
   His Tyr Val Thr Ala Leu Gly Thr Asp Ser Phe Ser Gln Gln Met Leu
       50                  55                  60
   Asp Ala Trp His Gly Glu Asn Val Asp Thr Ser Leu Thr Gln Arg Met
   65                  70                  75                  80
   Glu Asn Arg Leu Pro Gly Leu Tyr Tyr Ile Glu Thr Asp Ser Thr Gly
                   85                  90                  95
   Glu Arg Thr Phe Tyr Tyr Trp Arg Asn Glu Ala Ala Ala Lys Phe Trp
               100                 105                 110
   Leu Glu Ser Glu Gln Ser Ala Ala Ile Cys Glu Glu Leu Ala Asn Phe
           115                 120                 125
   Asp Tyr Leu Tyr Leu Ser Gly Ile Ser Leu Ala Ile Leu Ser Pro Thr
       130                 135                 140
   Ser Arg Glu Lys Leu Leu Ser Leu Leu Arg Glu Cys Arg Ala Asn Gly
   145                 150                 155                 160
   Gly Lys Val Ile Phe Asp Asn Asn Tyr Arg Pro Arg Leu Trp Ala Ser
                   165                 170                 175
   Lys Glu Glu Thr Gln Gln Val Tyr Gln Gln Met Leu Glu Cys Thr Asp
               180                 185                 190
   Ile Ala Phe Leu Thr Leu Asp Asp Glu Asp Ala Leu Trp Gly Gln Gln
           195                 200                 205
   Pro Val Glu Asp Val Ile Ala Arg Thr His Asn Ala Gly Val Lys Glu
       210                 215                 220
   Val Val Val Lys Arg Gly Ala Asp Ser Cys Leu Val Ser Ile Ala Gly
   225                 230                 235                 240
   Glu Gly Leu Val Asp Val Pro Ala Val Lys Leu Pro Lys Glu Lys Val
                   245                 250                 255
   Ile Asp Thr Thr Ala Ala Gly Asp Ser Phe Ser Ala Gly Tyr Leu Ala
               260                 265                 270
   Val Arg Leu Thr Gly Gly Ser Ala Glu Asp Ala Ala Lys Arg Gly His
           275                 280                 285
   Leu Thr Ala Ser Thr Val Ile Gln Tyr Arg Gly Ala Ile Ile Pro Arg
       290                 295                 300
   Glu Ala Met Pro Ala
   305
<210> 58
   <211> 642
   <212> DNA
   <213> Escherichia coli
<220>
   <221> CDS
   <222> (1)..(642)
<400> 58
   atg aaa aac tgg aaa aca agt gca gaa tca atc ctg acc acc ggc ccg      48
   Met Lys Asn Trp Lys Thr Ser Ala Glu Ser Ile Leu Thr Thr Gly Pro
   1               5                   10                  15
   gtt gta ccg gtt atc gtg gta aaa aaa ctg gaa cac gcg gtg ccg atg      96
   Val Val Pro Val Ile Val Val Lys Lys Leu Glu His Ala Val Pro Met
               20                  25                  30
   gca aaa gcg ttg gtt gct ggt ggg gtg cgc gtt ctg gaa gtg act ctg     144
   Ala Lys Ala Leu Val Ala Gly Gly Val Arg Val Leu Glu Val Thr Leu
           35                  40                  45
   cgt acc gag tgt gca gtt gac gct atc cgt gct atc gcc aaa gaa gtg        192
   Arg Thr Glu Cys Ala Val Asp Ala Ile Arg Ala Ile Ala Lys Glu Val
       50                  55                  60
   cct gaa gcg att gtg ggt gcc ggt acg gtg ctg aat cca cag cag ctg        240
   Pro Glu Ala Ile Val Gly Ala Gly Thr Val Leu Asn Pro Gln Gln Leu
   65                  70                  75                  80
   gca gaa gtc act gaa gcg ggt gca cag ttc gca att agc ccg ggt ctg        288
   Ala Glu Val Thr Glu Ala Gly Ala Gln Phe Ala Ile Ser Pro Gly Leu
                   85                  90                  95
   acc gag ccg ctg ctg aaa gct gct acc gaa ggg act att cct ctg att        336
   Thr Glu Pro Leu Leu Lys Ala Ala Thr Glu Gly Thr Ile Pro Leu Ile
               100                 105                 110
   ccg ggg atc agc act gtt tcc gaa ctg atg ctg ggt atg gac tac ggt        384
   Pro Gly Ile Ser Thr Val Ser Glu Leu Met Leu Gly Met Asp Tyr Gly
           115                 120                 125
   ttg aaa gag ttc aaa ttc ttc ccg gct gaa gct aac ggc ggc gtg aaa        432
   Leu Lys Glu Phe Lys Phe Phe Pro Ala Glu Ala Asn Gly Gly Val Lys
       130                 135                 140
   gcc ctg cag gcg atc gcg ggt ccg ttc tcc cag gtc cgt ttc tgc ccg        480
   Ala Leu Gln Ala Ile Ala Gly Pro Phe Ser Gln Val Arg Phe Cys Pro
   145                 150                 155                 160
   acg ggt ggt att tct ccg gct aac tac cgt gac tac ctg gcg ctg aaa        528
   Thr Gly Gly Ile Ser Pro Ala Asn Tyr Arg Asp Tyr Leu Ala Leu Lys
                   165                 170                 175
   agc gtg ctg tgc atc ggt ggt tcc tgg ctg gtt ccg gca gat gcg ctg        576
   Ser Val Leu Cys Ile Gly Gly Ser Trp Leu Val Pro Ala Asp Ala Leu
               180                 185                 190
   gaa gcg ggc gat tac gac cgc att act aag ctg gcg cgt gaa gct gta        624
   Glu Ala Gly Asp Tyr Asp Arg Ile Thr Lys Leu Ala Arg Glu Ala Val
           195                 200                 205
   gaa ggc gct aag ctg taa                                                642
   Glu Gly Ala Lys Leu
       210
<210> 59
   <211> 213
   <212> PRT
   <213> Escherichia coli
<400> 59
   Met Lys Asn Trp Lys Thr Ser Ala Glu Ser Ile Leu Thr Thr Gly Pro
   1               5                   10                  15
   Val Val Pro Val Ile Val Val Lys Lys Leu Glu His Ala Val Pro Met
               20                  25                  30
   Ala Lys Ala Leu Val Ala Gly Gly Val Arg Val Leu Glu Val Thr Leu
           35                  40                  45
   Arg Thr Glu Cys Ala Val Asp Ala Ile Arg Ala Ile Ala Lys Glu Val
       50                  55                  60
   Pro Glu Ala Ile Val Gly Ala Gly Thr Val Leu Asn Pro Gln Gln Leu
   65                  70                  75                  80
   Ala Glu Val Thr Glu Ala Gly Ala Gln Phe Ala Ile Ser Pro Gly Leu
                   85                  90                  95
   Thr Glu Pro Leu Leu Lys Ala Ala Thr Glu Gly Thr Ile Pro Leu Ile
               100                 105                 110
   Pro Gly Ile Ser Thr Val Ser Glu Leu Met Leu Gly Met Asp Tyr Gly
           115                 120                 125
   Leu Lys Glu Phe Lys Phe Phe Pro Ala Glu Ala Asn Gly Gly Val Lys
       130                 135                 140
   Ala Leu Gln Ala Ile Ala Gly Pro Phe Ser Gln Val Arg Phe Cys Pro
   145                 150                 155                 160
   Thr Gly Gly Ile Ser Pro Ala Asn Tyr Arg Asp Tyr Leu Ala Leu Lys
                   165                 170                 175
   Ser Val Leu Cys Ile Gly Gly Ser Trp Leu Val Pro Ala Asp Ala Leu
               180                 185                 190
   Glu Ala Gly Asp Tyr Asp Arg Ile Thr Lys Leu Ala Arg Glu Ala Val
           195                 200                 205
   Glu Gly Ala Lys Leu
       210
<210> 60
   <211> 27
   <212> DNA
   <213> Artificial Sequence
<220>
   <223> forward primer for EPI
<400> 60
   ggatgtgctg caaggcgatt aagttgg 27
<210> 61
   <211> 26
   <212> DNA
   <213> Artificial Sequence
<220>
   <223> reverse primer for EPI
<400> 61
   ctcgtatgtt gtgtggaatt gtgagc 26
<210> 62
   <211> 51
   <212> DNA
   <213> Artificial Sequence
<220>
   <223> forward primer for pMW119-attR-cat-attL-P14-alyB construction
<400> 62
   taagggtttt atatctatga aacatatttt tctaaaaagc ttgctagctt c 51
<210> 63
   <211> 51
   <212> DNA
   <213> Artificial Sequence
<220>
   <223> reverse primer for pMW119-attR-cat-attL-P14-alyB construction
<400> 63
   atcctctaga gtcgacttat ttacctgtgt atgtaccgtg cgatttttct a 51
<210> 64
   <211> 31
   <212> DNA
   <213> Artificial Sequence
<220>
   <223> forward primer for pMW119-attR-cat-attL-P14 construction
<400> 64
   agatataaaa cccttatata ttaatacgat t 31
<210> 65
   <211> 31
   <212> DNA
   <213> Artificial Sequence
<220>
   <223> reverse primer for pMW119-attR-cat-attL-P14 construction
<400> 65
   gtcgactcta gaggatcccc gggtaccgag c 31
<210> 66
   <211> 51
   <212> DNA
   <213> Artificial Sequence
<220>
   <223> forward primer for pMW119-attR-cat-attL-P14-alyD construction
<400> 66
   taagggtttt atatctatgt taaaaaaatt cctctgcatg tcggttatcc t 51
<210> 67
   <211> 51
   <212> DNA
   <213> Artificial Sequence
<220>
   <223> reverse primer for pMW119-attR-cat-attL-P14-alyD construction
<400> 67
   atcctctaga gtcgacctat tttttatgcg atgttaattc taacttagag a 51
<210> 68
   <211> 45
   <212> DNA
   <213> Artificial Sequence
<220>
   <223> forward primer for alyB
<400> 68
   taagggtttt atatctatga aacaaattac tctaaaaact ttact 45
<210> 69
   <211> 45
   <212> DNA
   <213> reverse primer for alyB
<400> Artificial Sequence
<220>
   <223> 69
   atcctctaga gtcgacttac tttttgtatt gatcgtgcga tacat 45
<210> 70
   <211> 51
   <212> DNA
   <213> Artificial Sequence
<220>
   <223> forward primer for pMW119-attR-cat-attL-P14-dehR construction
<400> 70
   taagggtttt atatctatga tgactaaacc tgttattggt tttatcggcc t 51
<210> 71
   <211> 51
   <212> DNA
   <213> Artificial Sequence
<220>
   <223> reverse primer for pMW119-attR-cat-attL-P14-dehR construction
<400> 71
   atcctctaga gtcgacttac tttttcaaac ccatgaagta atcaaagata a 51
<210> 72
   <211> 80
   <212> DNA
   <213> Artificial Sequence
<220>
   <223> forward primer for dehR
<400> 72
   atagccgggg cggtcttcct gattggtagc tggctgcgtt atgactacgg tctagacgct 60
   caagttagta taaaaaagct 80
<210> 73
   <211> 80
   <212> DNA
   <213> Artificial Sequence
<220>
   <223> reverse primer dehR
<400> 73
   cggtacgctc cagatgtgta tcagacgcga gaacgggaac agcaggaata ttactttttc 60
   aaacccatga agtaatcaaa 80
<210> 74
   <211> 80
   <212> DNA
   <213> Artificial Sequence
<220>
   <223> forward primer for alyD
<400> 74
   ttaccggtgt cgctattttg aacatccagc tctggtattc cgcaaaagca tctagacgct 60
   caagttagta taaaaaagct 80
<210> 75
   <211> 80
   <212> DNA
   <213> Artificial Sequence
<220>
   <223> reverse primer for alyD
<400> 75
   gggtctgcgt ttacgcggcc aacgaaactg tgatcaattt tgatataatc ctatttttta      60
   tgcgatgtta attctaactt                                                  80
<210> 76
   <211> 80
   <212> DNA
   <213> Artificial Sequence
<220>
   <223> forward primer for alyB
<400> 76
   gatggtgaca gaattacagg agataccgcc gatccatcag gaaacctcta tctagacgct      60
   caagttagta taaaaaagct                                                  80
<210> 77
   <211> 80
   <212> DNA
   <213> Artificial Sequence
<220>
   <223> reverse primer for alyB
<400> 77
   ccgttattcc agccattacc tttgaaactg tacttctccc ttatttacct gtgtatgtac      60
   cgtgcgattt ttctagttta                                                  80
<210> 78
   <211> 119
   <212> DNA
   <213> Artificial Sequence
<220>
   <223> promoter P14
<400> 78
   cctgggtcat ttttttcttg acaaccgtca cattcttgat ggtatagtcg aaaactgcaa     60
   aagcacatga cataaacaac ataagcacaa tcgtattaat atataagggt tttatatct     119
<210> 79
   <211> 64
   <212> DNA
   <213> Artificial Sequence
<220>
   <223> primer
<400> 79
   gcgggtaccg cggccgccct gggtcatttt tttcttgaca accgtcacat tcttgatggt    60
   atag                                                                 64
<210> 80
   <211> 47
   <212> DNA
   <213> Artificial Sequence
<220>
   <223> primer
<400> 80
   gcgcgcgcgc gagctcgcgg ccgctcataa acgcctgaaa ttttgcc 47
<210> 81
   <211> 56
   <212> DNA
   <213> Artificial Sequence
<220>
   <223> primer
<400> 81
   caggcttcaa gatctcctgg gtcatttttt tcttgacaac cgtcacattc ttgatg 56
<210> 82
   <211> 67
   <212> DNA
   <213> Artificial Sequence
<220>
   <223> primer
<400> 82
   atcctctaga gtcgacgcgg ccgctacggc aaaaacgcat cacttcacct tcgctttttc    60
   ctttcgg                                                              67
<210> 83
   <211> 67
   <212> DNA
   <213> Artificial Sequence
<220>
   <223> primer
<400> 83
   tgattacgcc aagcttagga ggttaatcta gacgctcaag ttagtataaa aaagctgaac 60
   gagaaac 67
<210> 84
   <211> 51
   <212> DNA
   <213> Artificial Sequence
<220>
   <223> primer
<400> 84
   agatcttgaa gcctgctttt ttatactaag ttggcattat aaaaaagcat t 51
<210> 85
   <211> 1686
   <212> DNA
   <213> Escherichia coli
<220>
   <221> CDS
   <222> (1)..(1686)
<400> 85
   gtg aat ata aac gtc gcc gaa ttg tta aat ggg aat tac att ctg tta 48
   Val Asn Ile Asn Val Ala Glu Leu Leu Asn Gly Asn Tyr Ile Leu Leu
   1 5 10 15
   tta ttt gtg gtc ctc gcg ctt ggg cta tgt ctc gga aag tta cga ctt 96
   Leu Phe Val Val Leu Ala Leu Gly Leu Cys Leu Gly Lys Leu Arg Leu
               20                  25                  30
   ggt tcg atc caa ctg ggt aat tcc att ggc gtt tta gtc gta tcg ctg       144
   Gly Ser Ile Gln Leu Gly Asn Ser Ile Gly Val Leu Val Val Ser Leu
           35                  40                  45
   tta tta ggc caa caa cat ttc agc att aac acc gat gcg ctt aat ctt       192
   Leu Leu Gly Gln Gln His Phe Ser Ile Asn Thr Asp Ala Leu Asn Leu
       50                  55                  60
   ggc ttt atg ctg ttt att ttc tgc gtc ggg gtc gaa gcc gga ccg aac       240
   Gly Phe Met Leu Phe Ile Phe Cys Val Gly Val Glu Ala Gly Pro Asn
   65                  70                  75                  80
   ttt ttt tcc att ttt ttt cgc gat ggg aaa aat tac cta atg tta gca       288
   Phe Phe Ser Ile Phe Phe Arg Asp Gly Lys Asn Tyr Leu Met Leu Ala
                   85                  90                  95
   ctg gtg atg gtt ggc agt gcg ctg gtg atc gcc tta ggg tta ggt aag       336
   Leu Val Met Val Gly Ser Ala Leu Val Ile Ala Leu Gly Leu Gly Lys
               100                 105                 110
   ctg ttt ggc tgg gat att ggc ctg acg gcc ggt atg tta gca ggc tct       384
   Leu Phe Gly Trp Asp Ile Gly Leu Thr Ala Gly Met Leu Ala Gly Ser
           115                 120                 125
   atg acg tcg aca ccg gtt ctg gtc ggt gct ggc gat aca ctg cgt cat       432
   Met Thr Ser Thr Pro Val Leu Val Gly Ala Gly Asp Thr Leu Arg His
       130                 135                 140
   tcc ggc atg gaa agc agg cag ctc tca ctg gca ctg gat aat ctg agc       480
   Ser Gly Met Glu Ser Arg Gln Leu Ser Leu Ala Leu Asp Asn Leu Ser
   145                 150                 155                 160
   ctc ggg tat gcc tta acc tat tta atc ggt ctg gtg agt ttg att gtt       528
   Leu Gly Tyr Ala Leu Thr Tyr Leu Ile Gly Leu Val Ser Leu Ile Val
                   165                 170                 175
   ggt gcg cgt tac ttg ccg aaa ttg cag cat cag gac tta cag acc agc       576
   Gly Ala Arg Tyr Leu Pro Lys Leu Gln His Gln Asp Leu Gln Thr Ser
               180                 185                 190
   gcc cag caa atc gcc cgc gaa cgt ggc ctg gac act gat gcc aac cgt       624
   Ala Gln Gln Ile Ala Arg Glu Arg Gly Leu Asp Thr Asp Ala Asn Arg
           195                 200                 205
   aag gtt tat tta ccg gtg atc cgc gcc tat cgc gtc ggc ccg gaa ctg       672
   Lys Val Tyr Leu Pro Val Ile Arg Ala Tyr Arg Val Gly Pro Glu Leu
       210                 215                 220
   gtg gcc tgg acc gac ggc aaa aat ctg cgt gaa ctg ggt att tat cga       720
   Val Ala Trp Thr Asp Gly Lys Asn Leu Arg Glu Leu Gly Ile Tyr Arg
   225                 230                 235                 240
   caa acc ggc tgc tac att gaa cgt att cga cgt aac ggg att ctg gca       768
   Gln Thr Gly Cys Tyr Ile Glu Arg Ile Arg Arg Asn Gly Ile Leu Ala
                   245                 250                 255
   aat cca gac ggt gat gcc gtg cta caa atg ggc gat gaa ata gcg ttg       816
   Asn Pro Asp Gly Asp Ala Val Leu Gln Met Gly Asp Glu Ile Ala Leu
               260                 265                 270
   gta ggc tat ccc gac gcc cat gcc cga ctc gat ccc agc ttc cgt aac        864
   Val Gly Tyr Pro Asp Ala His Ala Arg Leu Asp Pro Ser Phe Arg Asn
           275                 280                 285
   ggt aaa gaa gtt ttc gat cgt gac ctt ctc gac atg cgt atc gtc act        912
   Gly Lys Glu Val Phe Asp Arg Asp Leu Leu Asp Met Arg Ile Val Thr
       290                 295                 300
   gaa gaa gtg gtc gtt aaa aac cat aac gct gta ggt aaa cgt ctc gca        960
   Glu Glu Val Val Val Lys Asn His Asn Ala Val Gly Lys Arg Leu Ala
   305                 310                 315                 320
   caa ctg aag ttg acc gat cac ggt tgc ttc ctt aac cgc gtc att cgt       1008
   Gln Leu Lys Leu Thr Asp His Gly Cys Phe Leu Asn Arg Val Ile Arg
                   325                 330                 335
   agc cag att gag atg ccg ata gat gac aac gtc gtg ctt aac aaa ggt       1056
   Ser Gln Ile Glu Met Pro Ile Asp Asp Asn Val Val Leu Asn Lys Gly
               340                 345                 350
   gac gtt tta caa gtc agc ggc gat gcc cgc cgc gta aaa acc atc gcc       1104
   Asp Val Leu Gln Val Ser Gly Asp Ala Arg Arg Val Lys Thr Ile Ala
           355                 360                 365
   gat cgc atc ggc ttt atc tcg att cac agc cag gtc act gac ctg ctg       1152
   Asp Arg Ile Gly Phe Ile Ser Ile His Ser Gln Val Thr Asp Leu Leu
       370                 375                 380
   gca ttc tgc gcc ttc ttt gtt att ggg ctg atg atc ggg atg atc acc       1200
   Ala Phe Cys Ala Phe Phe Val Ile Gly Leu Met Ile Gly Met Ile Thr
   385                 390                 395                 400
   ttc cag ttc agc aca ttc agt ttc ggc atg ggg aac gct gcc ggg ttg       1248
   Phe Gln Phe Ser Thr Phe Ser Phe Gly Met Gly Asn Ala Ala Gly Leu
                   405                 410                 415
   tta ttc gcc gga att atg ctg ggc ttt atg cgt gct aac cac ccg acc       1296
   Leu Phe Ala Gly Ile Met Leu Gly Phe Met Arg Ala Asn His Pro Thr
               420                 425                 430
   ttc ggt tac att ccg cag ggt gca tta agc atg gtg aaa gag ttc ggc       1344
   Phe Gly Tyr Ile Pro Gln Gly Ala Leu Ser Met Val Lys Glu Phe Gly
           435                 440                 445
   ttg atg gtg ttt atg gca ggc gtt ggt ctg agc gcc ggt agc ggt att       1392
   Leu Met Val Phe Met Ala Gly Val Gly Leu Ser Ala Gly Ser Gly Ile
       450                 455                 460
   aat aac ggc ctg ggc gcg att ggc ggt cag atg ttg att gcc gga ttg       1440
   Asn Asn Gly Leu Gly Ala Ile Gly Gly Gln Met Leu Ile Ala Gly Leu
   465                 470                 475                 480
   att gtc agt ctg gtg ccc gtg gtt atc tgt ttc ttg ttc ggt gct tat       1488
   Ile Val Ser Leu Val Pro Val Val Ile Cys Phe Leu Phe Gly Ala Tyr
                   485                 490                 495
   gta ttg cga atg aac cgc gcg ctg ttg ttc ggc gca atg atg ggc gca       1536
   Val Leu Arg Met Asn Arg Ala Leu Leu Phe Gly Ala Met Met Gly Ala
               500                 505                 510
   cgt acc tgc gcg ccg gca atg gag atc atc agt gat aca gct cgc agt       1584
   Arg Thr Cys Ala Pro Ala Met Glu Ile Ile Ser Asp Thr Ala Arg Ser
           515                 520                 525
   aac atc ccg gcg ctg ggc tat gcg ggc acc tat gca atc gcc aac gtc      1632
   Asn Ile Pro Ala Leu Gly Tyr Ala Gly Thr Tyr Ala Ile Ala Asn Val
       530                 535                 540
   ctg ctg acg ctg gca ggg aca atc atc gtc atg gta tgg cca gga tta      1680
   Leu Leu Thr Leu Ala Gly Thr Ile Ile Val Met Val Trp Pro Gly Leu
   545                 550                 555                 560
   gga taa                                                              1686
   Gly
<210> 86
   <211> 561
   <212> PRT
   <213> Escherichia coli
<400> 86
   Val Asn Ile Asn Val Ala Glu Leu Leu Asn Gly Asn Tyr Ile Leu Leu
   1               5                   10                  15
   Leu Phe Val Val Leu Ala Leu Gly Leu Cys Leu Gly Lys Leu Arg Leu
               20                  25                  30
   Gly Ser Ile Gln Leu Gly Asn Ser Ile Gly Val Leu Val Val Ser Leu
           35                  40                  45
   Leu Leu Gly Gln Gln His Phe Ser Ile Asn Thr Asp Ala Leu Asn Leu
       50                  55                  60
   Gly Phe Met Leu Phe Ile Phe Cys Val Gly Val Glu Ala Gly Pro Asn
   65                  70                  75                  80
   Phe Phe Ser Ile Phe Phe Arg Asp Gly Lys Asn Tyr Leu Met Leu Ala
                   85                  90                  95
   Leu Val Met Val Gly Ser Ala Leu Val Ile Ala Leu Gly Leu Gly Lys
               100                 105                 110
   Leu Phe Gly Trp Asp Ile Gly Leu Thr Ala Gly Met Leu Ala Gly Ser
           115                 120                 125
   Met Thr Ser Thr Pro Val Leu Val Gly Ala Gly Asp Thr Leu Arg His
       130                 135                 140
   Ser Gly Met Glu Ser Arg Gln Leu Ser Leu Ala Leu Asp Asn Leu Ser
   145                 150                 155                 160
   Leu Gly Tyr Ala Leu Thr Tyr Leu Ile Gly Leu Val Ser Leu Ile Val
                   165                 170                 175
   Gly Ala Arg Tyr Leu Pro Lys Leu Gln His Gln Asp Leu Gln Thr Ser
               180                 185                 190
   Ala Gln Gln Ile Ala Arg Glu Arg Gly Leu Asp Thr Asp Ala Asn Arg
           195                 200                 205
   Lys Val Tyr Leu Pro Val Ile Arg Ala Tyr Arg Val Gly Pro Glu Leu
       210                 215                 220
   Val Ala Trp Thr Asp Gly Lys Asn Leu Arg Glu Leu Gly Ile Tyr Arg
   225                 230                 235                 240
   Gln Thr Gly Cys Tyr Ile Glu Arg Ile Arg Arg Asn Gly Ile Leu Ala
                   245                 250                 255
   Asn Pro Asp Gly Asp Ala Val Leu Gln Met Gly Asp Glu Ile Ala Leu
               260                 265                 270
   Val Gly Tyr Pro Asp Ala His Ala Arg Leu Asp Pro Ser Phe Arg Asn
           275                 280                 285
   Gly Lys Glu Val Phe Asp Arg Asp Leu Leu Asp Met Arg Ile Val Thr
       290                 295                 300
   Glu Glu Val Val Val Lys Asn His Asn Ala Val Gly Lys Arg Leu Ala
   305                 310                 315                 320
   Gln Leu Lys Leu Thr Asp His Gly Cys Phe Leu Asn Arg Val Ile Arg
                   325                 330                 335
   Ser Gln Ile Glu Met Pro Ile Asp Asp Asn Val Val Leu Asn Lys Gly
               340                 345                 350
   Asp Val Leu Gln Val Ser Gly Asp Ala Arg Arg Val Lys Thr Ile Ala
           355                 360                 365
   Asp Arg Ile Gly Phe Ile Ser Ile His Ser Gln Val Thr Asp Leu Leu
       370                 375                 380
   Ala Phe Cys Ala Phe Phe Val Ile Gly Leu Met Ile Gly Met Ile Thr
   385                 390                 395                 400
   Phe Gln Phe Ser Thr Phe Ser Phe Gly Met Gly Asn Ala Ala Gly Leu
                   405                 410                 415
   Leu Phe Ala Gly Ile Met Leu Gly Phe Met Arg Ala Asn His Pro Thr
               420                 425                 430
   Phe Gly Tyr Ile Pro Gln Gly Ala Leu Ser Met Val Lys Glu Phe Gly
           435                 440                 445
   Leu Met Val Phe Met Ala Gly Val Gly Leu Ser Ala Gly Ser Gly Ile
       450                 455                 460
   Asn Asn Gly Leu Gly Ala Ile Gly Gly Gln Met Leu Ile Ala Gly Leu
   465                 470                 475                 480
   Ile Val Ser Leu Val Pro Val Val Ile Cys Phe Leu Phe Gly Ala Tyr
                   485                 490                 495
   Val Leu Arg Met Asn Arg Ala Leu Leu Phe Gly Ala Met Met Gly Ala
               500                 505                 510
   Arg Thr Cys Ala Pro Ala Met Glu Ile Ile Ser Asp Thr Ala Arg Ser
           515                 520                 525
   Asn Ile Pro Ala Leu Gly Tyr Ala Gly Thr Tyr Ala Ile Ala Asn Val
       530                 535                 540
   Leu Leu Thr Leu Ala Gly Thr Ile Ile Val Met Val Trp Pro Gly Leu
   545                 550                 555                 560
   Gly
<210> 87
   <211> 56
   <212> DNA
   <213> Artificial Sequence
<220>
   <223> primer for ybjL
<400> 87
   tgattacgcc aagcttagga ggttaaatga atataaacgt cgccgaattg ttaaat 56
<210> 88
   <211> 46
   <212> DNA
   <213> Artificial Sequence
<220>
   <223> primer for ybjL
<400> 88
   atcctctaga gtcgacttat cctaatcctg gccataccat gacgat 46
<210> 89
   <211> 1542
   <212> DNA
   <213> Vibrio sp.
<400> 89
   atgcaactta attccacaga aattagcgag ctgattaaac agcgtatcga gaaattcgat      60
   gttgtgagtg aagcacgcaa tgaaggtact atcgtctctg tgagcgatgg tattatccgc     120
   atccacggtt tagccgatgt gatgcagggt gaaatgattg aactaccggg cggtctttac     180
   gcgcttgcac ttaaccttga gcgtgactcg gttggtgcgg ttgtaatggg cccgtatgct     240
   aaccttcagg aaggcatgaa agtaacgggt actggtcgta tccttgaagt acctgtaggt     300
   cctgaattgc ttggtcgtgt ggtaaacaca ctaggtcaac caattgatgg taaaggccct     360
   atcgatgcaa aactcacttc tccagtagaa atgattgcac caggtgttat cgatcgtaaa     420
   tcagtagacc aaccagtaca aactggttat aagtctgttg actcaatgat tccaatcggc     480
   cgtggtcagc gtgagcttat catcggtgac cgtcagactg gtaaaacagc gatggcgatt     540
   gatgcaatca ttaaccagaa agattctgga atcttctcta tctatgttgc gattggccaa     600
   aaagcctcta ctatcgccaa cgtagtacgt aaacttgaag agcacggtgc actagcaaac     660
   actatcgtgg ttgtagcatc ggcttctgag tctgcggcat tgcaatacct ggctccatat     720
   tcaggttgtg caatgggtga atacttccgt gatcgcggtg aagatgcact gattgtttat     780
   gatgatttat ctaaacaagc ggttgcttac cgtcagattt ctctacttct aaaacgtcca     840
   ccaggtcgtg aagcattccc aggtgatgtt ttctatcttc actctcgtct actagagcgt     900
   gctgctcgtg taaatgaaga atacgtagaa agattcacta acggtgaagt gaaaggtaag     960
   acaggttctt taacggctct acctatcatt gaaacacaag ctggtgacgt atctgcattc    1020
   gtaccgacta acgtaatctc gattaccgat ggtcagatct tcctacaaac tgagctattt    1080
   aatgctggtg ttcgccctgc ggttgaccca ggtatttcgg tatctcgtgt aggtggttct    1140
   gcgcaaacta agatcattaa gaaactgtct ggtggtattc gtactgcatt ggcacagtat    1200
   cgtgaactag ccgcgtttgc tcagttctca tctgatcttg atgctgcaac taagaagcag    1260
   ttggaccatg gtcaaaaagt aactgagtta atgaagcaga aacaatacgc tccattctca    1320
   gtatttgacc aagcattggt tatctttgct gctgagcgcg gttatttaga cacaatcgag    1380
   ctaaacaaat tagctgattt tgaagccgct cttctatcgt acgctcacgg tcaatttgct    1440
   gatttcgttg ctgaaatcaa caagacgggt gcttataacg atgaaatcga agccacactt    1500
   aagaagttgg ttgatggttt cgtagcaacc cagacctggt aa                       1542
<210> 90
   <211> 735
   <212> DNA
   <213> Vibrio sp.
<400> 90
   atgacaacga atcctaagcc tgcatatcag cgtattttgt taaaacttag tggcgaagct      60
   ctacagggtg aagaaggttt tggtatcgat ccggctattt tggatcgcat ggctcaagaa     120
   attaaagaac tcgttgaact tggcgtacaa gttggcgttg ttattggtgg tggtaaccta     180
   ttccgtggtg ctggtctagc ggaagctggc atgaatcgtg tagttggtga tcacatgggg     240
   atgctagcga ctgtgatgaa tggcctagca atgcgtgatg cgcttcatcg tgcttatgtg     300
   aatgcgcgag taatgtctgc gattcaatta aaaggtgtat gtgatgatta caactgggcg     360
   gatgcgatca gtcaacttcg tcaaggccgt gtagtgatct tttctgctgg tacgggtaac     420
   ccattcttta caactgattc agcagcatgt ctacgcggga tcgaaattga agcagatatt     480
   gtactaaaag caaccaaagt ggatggcgtt tatactgctg acccagttgc taacccagac     540
   gctgttttgt gtgataaact gagttacaac agcgtgctgg aaaaagaatt aaaagtgatg     600
   gacttagctg ccttcacttt agctcgtgat cataaaatgc caattcgcgt attcaacatg     660
   aacaagccag gtgctttgcg tcgtgtcgta atgggtgagc aagaaggcac actaatcggc     720
   gagctgccag aataa                                                     735
<210> 91
   <211> 1047
   <212> DNA
   <213> Vibrio sp.
<400> 91
   atggacgaga acaaacaaaa ggctctagca gcggcacttg gccaaatcga aaaacaattt      60
   ggtaaaggct caattatgaa attgggtgac aaccgcacta tggacgtaga aactatttct     120
   acaggttctc ttgctttgga tatcgcattg ggtgcgggtg gtttaccaat gggacgtatt     180
   gtagaagtat acggtcctga aagttctggt aaaacaacat taaccttaga attaattgcg     240
   gctgcgcaac gctcgggcaa aacctgtgca ttcgttgatg cggagcatgc actagaccca     300
   atttacgcga agaaattagg cgtaaatatc gatgagttat tagtttctca gccagatact     360
   ggtgagcaag cgcttgaaat ctgtgatgcg ttagcacgct ctggtgcgat tgacgttttg     420
   gttgtcgatt ctgtagcagc attaactccg aaagcagaaa ttgaaggtga aatgggcgat     480
   agccacatgg ggcttcaagc gcgtatgctt tctcaagcaa tgcgtaaact tacaggtaat     540
   ctaaaacaat ctaactgtat gtgtatcttc attaaccaaa ttcgtatgaa gattggtgtg     600
   atgtttggtt cacctgaaac cacaactggt ggtaatgcat taaaattcta cgcctctgtt     660
   cgtcttgata tccgccgtac tggtgcgata aaagatggtg acgaagtggt tggtaacgaa     720
   acacgtatta aggttgttaa gaacaagatt gcggcaccgt ttaaacaagc tgaaacacaa     780
   attctttatg gtaaaggctt taaccgtgaa ggtgagttaa tcgacctagg tgtgaaaaac     840
   aagttagtag agaaagcagg cgcatggtac agctaccaag gtgataaaat tggccaaggt     900
   aaagcaaact cttgcaacta cttacgtgaa aatccagcga ttgcacaaga aattgatact     960
   aaacttcgtg aaatgctatt agcacctgtg gttgcagaag aagaactaca agaagctgct    1020
   gaagttgaat ctggtgatga gttttaa                                        1047
<210> 92
   <211> 993
   <212> DNA
   <213> Vibrio sp.
<400> 92
   atgcagggtt ctgtaacaga atttcttaag ccacgtcttg ttgacatcga acagatcagc      60
   acgacacatg caaaagtaac tcttgagcct ttagagcgtg gttttggcca cactttaggt     120
   aatgcacttc gccgtattct tctatcttct atgccaggtt gtgctgtaac agaagttgag     180
   attgaaggcg ttctacacga gtacagcacc aaagaaggcg ttcaagaaga tatccttgag     240
   atccttctta acttgaaagg tcttgctgtg agcgttgccg aaggcaaaga tgaagtgttt     300
   attacactaa aaaaatcagg ctcgggccct gttgttgcag gtgacatcat ccatgatggt     360
   gatgtagaga tcgctaaccc agaacacgtt atttgtcacc taacggatga caaagctgaa     420
   atcgctatgc gtattaaagt tcaacgtggt cgtggttatg ttccgtcttc agctcgtatc     480
   catactgaag aagatgagcg tccaatcggt cgtctacttg ttgatgctac atacagcccg     540
   gttgataaaa tcgcctacgc tgtagaagca gcacgtgtag aacaacgtac tgacttagac     600
   aagcttgtta tcgatatgga aacaaatggc actttagaac ctgaggaagc tatccgtcgt     660
   gcagctacaa ttttagctga gcaattggat gcattcgtag atcttcgtga tgtacgtgtt     720
   ccagaagaga aagaagagaa gccggagttc gatcctattc tactgcgtcc tgtagacgat     780
   cttgaactaa cagttcgctc tgctaactgc ttgaaagcag aagcgattca ctacatcggt     840
   gatcttgtac agcgtaccga ggttgagctt cttaaaacgc caaaccttgg taagaagtct     900
   ttaacagaga ttaaagatgt tctagcgtcg cgcggtctgt ctctaggtat gcgcctagaa     960
   aactggccac cagcgtcgat tgctgaagat taa                                  993
<210> 93
   <211> 1842
   <212> DNA
   <213> Vibrio sp.
<400> 93
   atggatcaaa atccgcagtc acagctaaag ttacttgtcg caaaaggtaa ggaacaaggc      60
   tatctgacct acgccgaagt aaatgaccac ctacctgaag aaatcgtaga ttcagaacag     120
   gtagaagaca tcattcaaat gattaacgac atgggtatcc aagtcgttga aaccgcacct     180
   gatgcggacg atcttatgtt gaatgaggca gtagcagatg aagacgctgc cgaagccgca     240
   gcagccgcac tttccagtgt agaaagtgaa attggccgaa ctactgaccc agttcgcatg     300
   tacatgcgtg aaatgggtac ggttgaacta ttaactcgtg aaggtgagat cgacattgct     360
   aaacgcattg aagatggcat caaccaagtt caatgttcag tcgctgaata ccctggcact     420
   atttcttata ttcttgagca atttgataaa gttcaagctg aagaacttcg tttaactgac     480
   attatttcgg gtttcgttga tcctgatgat gatggcaatg ccgcaccaac agcaacacac     540
   attggttcag agcttgctgt tactgatgat gatctggatg acgatgatga agatgcagat     600
   tcaacagagg acgaagaaga ggaagataca ggcatcgatc ctgaacttgc tctagaacgc     660
   ttcacagagc tgcgtaatac ttatcagaac ttacagcttg cgatcaatga acatggtcgt     720
   gaaagcaaag caattgctga tagcaaacaa gaattccttg atgtttttcg tcaattccgt     780
   cttattccaa aacagtttga ttatttagtt aataatctta aaaactcaat ggatcgtgta     840
   cgtacacaag aacgtttaat tattcgtact tgtgttgaat acggaaaaat gccgaagaaa     900
   gcattcgtgc aagcctttac gggtaatgaa tcatcagatg catggctaga tgatgtttta     960
   gcatcaggta agccatatgc agataaaatt aaacaaagcg aagaagatct gcgccgtagt    1020
   atcttcaagc taaaatcaat cgaagaagaa acgtctttga atgttcaaac catcaaagat    1080
   atcagccgta gaatgtcaat cggtgaagca aaagctcgtc gtgcgaaaaa agaaatggtt    1140
   gaagccaact tacgtttggt tatttcgatt gcgaaaaaat acaccaaccg tggcctacaa    1200
   ttcttggatc ttattcaaga aggtaacatt ggcctcatga aagcggttga taagttcgaa    1260
   tatcgtcgtg gttataagtt ctcaacttat gcaacatggt ggattcgtca ggcgatcact    1320
   cgttcaattg ctgaccaagc acgtacgatc cgtattccgg tacacatgat cgaaaccatt    1380
   aacaaattga accgtatctc tcgtcagatg cttcaagaaa tgggtcgtga gccgttacca    1440
   gaagaattgg ctgaacgcat gcaaatgcct gaagacaaga ttcgcaaagt actaaaaatt    1500
   gctaaagagc caatttcaat ggaaacgcct atcggtgatg atgaagattc gcacctaggt    1560
   gattttattg aagatactac gcttgaatta cctttagatt cagccacttc gactagcttg    1620
   aagttcgcca ctaaagatgt tttagctggc ctaacgcctc gtgaagcaaa agtccttcgc    1680
   atgcgtttcg gtatcgatat gaatactgac cacactttgg aagaagtggg taagcagttc    1740
   gacgtaaccc gtgaacgtat tcgtcagatt gaagccaaag cattgcgcaa acttcgtcat    1800
   ccaagccgct cagaaacact gcgcagcttc ttggacgaat aa                        1842
<210> 94
   <211> 890
   <212> DNA
   <213> Vibrio casei
<220>
   <221> misc_feature
   <222> (30)..(30)
   <223> n is a, c, g, or t
<220>
   <221> misc_feature
   <222> (39)..(39)
   <223> n is a, c, g, or t
<220>
   <221> misc_feature
   <222> (42)..(42)
   <223> n is a, c, g, or t
<220>
   <221> misc_feature
   <222> (861)..(861)
   <223> n is a, c, g, or t
<220>
   <221> misc_feature
   <222> (867)..(867)
   <223> n is a, c, g, or t
<400> 94
   acgactgacc cggtacgcat gtayatgmgn garatgggna cngttgaact attgacccgt      60
   gaaggtgaaa tcgacattgc aaaacgcatt gaagatggca tcaatcaagt tcaatgttca     120
   gttgccgaat acccaggtac tatttcatac atccttgagc aatttgataa agttcaagct     180
   gaagagttac gtcttactga tatcatttca ggctttgttg atcctgatga tgatggtaat     240
   gccgcaccaa cagcaactca cgttggttca gagcttgccg tcagtgatga tgagctggac     300
   gatgatgatg attcagacga tgattcagac gatgacgaag aagaagatgt tggcatcgat     360
   ccagagcttg ctttagagcg cttcactgaa ttacgtaata cgtatcagaa ccttcaacta     420
   gccatcaatg aacatggccg tgaaagtaaa gcgattgccg cgagcaaaca agaatttctt     480
   gatgttttcc gtcaattccg cctgatccca aaacagtttg attacttagt caatggtttg     540
   aaaaactcaa tggatcgtgt tcgtactcaa gaacgcttga ttattcgcac cgtggttgag     600
   tacggaaaaa tgcctaagaa agctttcgtt aatgccttta ctggcaacga atcttctgac     660
   gcatggttag ataatattct agcttcaggc aaaccatacg cagaaaagat taagcaaagt     720
   gaagaagatc ttcgtcgctc aatctataaa cttaagagca tcgaagaaga aacgtcttta     780
   aatgtacaaa ccattaaaga catcagccgc cgcatgtcga ttggtgaagc gaaagctcgt     840
   cgtgcgaara argaratggt ngargcnaac ttacgtctgg ttatttctat                890
<210> 95
   <211> 887
   <212> DNA
   <213> Vibrio litoralis
<220>
   <221> misc_feature
   <222> (30)..(30)
   <223> n is a, c, g, or t
<220>
   <221> misc_feature
   <222> (39)..(39)
   <223> n is a, c, g, or t
<220>
   <221> misc_feature
   <222> (42)..(42)
   <223> n is a, c, g, or t
<220>
   <221> misc_feature
   <222> (858)..(858)
   <223> n is a, c, g, or t
<220>
   <221> misc_feature
   <222> (864)..(864)
   <223> n is a, c, g, or t
<400> 95
   acgactgacc cggtacgcat gtayatgmgn garatgggna cngttgagct attaactcgt      60
   gaaggtgaga tcgacattgc taaacgcatt gaagatggca tcaaccaagt acaatgttca     120
   gtcgctgaat acccaggcac tatttcttac attcttgagc aatttgataa agttcaagct     180
   gaagaacttc gtttaaccga cattatctca ggcttcgttg atcctgatga tgatggcaat     240
   gccgcaccaa ctgcaacaca cgttggttca gagcttgctg ttacggatga tgatctggat     300
   gacgatgatg atgatgattc agatgaggac gaagaagagg aagatacagg tatcgatcct     360
   gagcttgctc tagaacgttt tactgaactt cgcaatacct atcagaacct gcaacttgcg     420
   atcaatgaac acggtcgtga aagcaaagca attgctgata gaaagcaaga gttccttgat     480
   gttttccgtc aattccgtct gattccaaaa cagtttgatt atttagtcaa taatcttaaa     540
   aattcaatgg atcgcgtacg tacccaagag cgtttgatta ttcgtacttg tgttgaatac     600
   ggaaaaatgc cgaaaaaagc attcgtacaa gcctttactg gtaatgaatc atcagaagca     660
   tggctagacg aagttctggc atcaggcaag ccatacgcag aaaagatcaa acaaagcgaa     720
   gaagaactgc gtcgtagtat cttcaagcta aaatcaatcg aagaagaaac atctttgaat     780
   gttcaaacta tcaaagatat cagccgtcgc atgtcaatcg gtgaagcaaa agctcgtcgt     840
   gcgaaraarg aratggtnga rgcnaactta cgtctggtta tttctat                   887
<210> 96
   <211> 893
   <212> DNA
   <213> Vibrio rumoiensis
<220>
   <221> misc_feature
   <222> (30)..(30)
   <223> n is a, c, g, or t
<220>
   <221> misc_feature
   <222> (39)..(39)
   <223> n is a, c, g, or t
<220>
   <221> misc_feature
   <222> (42)..(42)
   <223> n is a, c, g, or t
<220>
   <221> misc_feature
   <222> (864)..(864)
   <223> n is a, c, g, or t
<220>
   <221> misc_feature
   <222> (870)..(870)
   <223> n is a, c, g, or t
<400> 96
   acgactgacc cggtacgcat gtayatgmgn garatgggna cngttgaact attaactcgt 60
   gaaggtgaaa tcgacattgc taaacgcatt gaagatggca tcaaccaagt acaatgttca 120
   gtcgctgaat acccagggac tatttcttat attcttgagc aatttgataa agttcaagct 180
   gaagaacttc gtctaacaga tattatctct ggttttgttg acccagatga tgacggtaat 240
   gcagctccaa cagccacaca cgttggttca gagcttgccg ttgatgatga cgaccttgat 300
   gatgacgatg acgaagattc ggattcagat gaggacgaag aagaggaaga tacaggcatc 360
   gatcctgagc ttgctcttga acgcttcact gaattacgca atacgtatca gaatttacaa 420
   ttagccatca atgagcatgg ccgtgacagt aaagcgattg ccgcgagcaa acaagacttt 480
   cttgatgtat tccgtcaatt ccgtcttgtt ccaaaacagt ttgattattt agttaataat 540
   cttaaaaact caatggaccg tgtgcgcact caggaacgtt taatcattcg tacttgtgtt 600
   gaatacggaa aaatgccgaa aaaagcattt gtgcaagcct ttacgggtaa tgaatcttct 660
   gacgcatggc tagatgatgt tttagcatca ggtaaaccat acgcagaaaa aatcaaacaa 720
   agcgaagaag atctacgtcg tagtatcttt aagctaaaaa ccattgaaga agaaacgtcg 780
   ttgaacgttc aaacgatcaa agacatcagt cgtcgcatgt caatcggtga agcgaaagtt 840
   cgtcgtgcga araargarat ggtngargcn aacttacgtc tggttatttc tat 893
<210> 97
   <211> 553
   <212> DNA
   <213> Vibrio rumoiensis
<400> 97
   taaacctgca tatcagcgta ttttgttaaa acttagtggc gaagctctac agggtgaaga 60
   aggttttggt attgatccag ccgttttgga tcgcatggct caagaaatta aagaactcgt 120
   tgaacttggt gtacaagttg gcgttgtgat tggtggtggt aacttattcc gtggcgctgg 180
   tttagccgaa gcggggatga accgtgtggt tggtgatcat atgggaatgt tagcaaccgt 240
   catgaatgga ctcgcaatgc gtgatgcgct tcatcgtgct tatgtgaatg cgcgtgtgat 300
   gtcagctatt caattaaaag gtgtgtgtga cgattacaat tgggctgatg cgattagcca 360
   acttcgtcaa ggccgagtgg ttatcttctc tgcaggtact ggtaacccat tctttacgac 420
   tgattcagca gcatgcttgc gtggtatcga aattgaagca gatatcgttc taaaagcgac 480
   caaagtggat ggtgtttata cagctgatcc ggttgctaac ccagatgcag ttttgtgtga 540
   taaactcagc tac 553
<210> 98
   <211> 1494
   <212> DNA
   <213> Vibrio rumoiensis
<220>
   <221> 16S rRNA gene
   <222> (1)..(1494)
<400> 98
   ttgaacgctg gcggcaggcc taacacatgc aagtcgagcg gaaacgagaa gtagcttgct      60
   acttcggcgt cgagcggcgg acgggtgagt aatgcatagg aagttgccca gtagaggggg     120
   ataaccattg gaaacgatgg ctaataccgc ataacctctt cggagcaaag cgggggacct     180
   tcgggcctcg cgctactgga tacgcctatg tgggattagc tagttggtga ggtaatggct     240
   caccaaggcg acgatcccta gctggtctga gaggatgatc agccacactg ggactgagac     300
   acggcccaga ctcctacggg aggcagcagt ggggaatatt gcacaatggg cgaaagcctg     360
   atgcagccat gccgcgtgta tgaagaaggc cttcgggttg taaagtactt tcagttgtga     420
   ggaaggtttc gtagttaata actgcgttgc ttgacgttag caacagaaga agcaccggct     480
   aactccgtgc cagcagccgc ggtaatacgg agggtgcgag cgttaatcgg aattactggg     540
   cgtaaagcgc atgcaggtgg tttgttaagt cagatgtgaa agcccggggc tcaacctcgg     600
   aaggtcattt gaaactggca aactagagta ctgtagaggg gggtagaatt tcaggtgtag     660
   cggtgaaatg cgtagagatc tgaaggaata ccagtggcga aggcggcccc ctggacagat     720
   actgacactc agatgcgaaa gcgtggggag caaacaggat tagataccct ggtagtccac     780
   gccgtaaacg atgtctactt ggaggttgtg gccttgagcc gtggctttcg gagctaacgc     840
   gttaagtaga ccgcctgggg agtacggtcg caagattaaa actcaaatga attgacgggg     900
   gcccgcacaa gcggtggagc atgtggttta attcgatgca acgcgaagaa ccttacctac     960
   tcttgacatc cagagaactt tccagagatg gattggtgcc ttcgggaact ctgagacagg    1020
   tgctgcatgg ctgtcgtcag ctcgtgttgt gaaatgttgg gttaagtccc gcaacgagcg    1080
   caacccttat ccttgtttgc cagcgagtaa tgtcgggaac tccagggaga ctgccggtga    1140
   taaaccggag gaaggtgggg acgacgtcaa gtcatcatgg cccttacgag tagggctaca    1200
   cacgtgctac aatggcgcat acagagggca gcaagctagc gatagtgagc gaatcccaaa    1260
   aagtgcgtcg tagtccggat tggagtctgc aactcgactc catgaagtcg gaatcgctag    1320
   taatcgtgga tcagaatgcc acggtgaata cgttcccggg ccttgtacac accgcccgtc    1380
   acaccatggg agtgggctgc aaaagaagta ggtagtttaa ccttcgggag gacgcttacc    1440
   actttgtggt tcatgactgg ggtgaagtcg taacaaggta gccctagggg aacc          1494